(19) Europäisches Patentamt
European Patent Office
Office européen des brevets

(11) EP 1 237 874 B1

(12) EUROPEAN PATENT SPECIFICATION

(45) Date of publication and mention of the grant of the patent:
**22.02.2006 Bulletin 2006/08**

(21) Application number: **00984340.0**

(22) Date of filing: **14.12.2000**

(51) Int Cl.:
***C07D 233/00*** *(2006.01)*

(86) International application number:
**PCT/US2000/033831**

(87) International publication number:
**WO 2001/044200 (21.06.2001 Gazette 2001/25)**

# (54) SELECTIVE NEUROKININ ANTAGONISTS

SELEKTIVE NEUROKININ-ANTAGONISTEN

ANTAGONISTES SELECTIFS DE LA NEUROKINE

(84) Designated Contracting States:
**AT BE CH CY DE DK ES FI FR GB GR IE IT LI LU MC NL PT SE**
Designated Extension States:
**RO SI**

(30) Priority: **17.12.1999 US 172489 P**

(43) Date of publication of application:
**11.09.2002 Bulletin 2002/37**

(73) Proprietor: **SCHERING CORPORATION**
**Kenilworth,**
**New Jersey 07033-0530 (US)**

(72) Inventors:
- **SHIH, Neng-Yang**
**North Caldwell, NJ 07006 (US)**
- **SHUE, Ho-Jane**
**Pine Brook, NJ 07058 (US)**
- **REICHARD, Gregory, A.**
**Morris Plains, NJ 07950 (US)**
- **PALIWAL, Sunil**
**Scotch Plains, NJ 07076 (US)**
- **BLYTHIN, David, J.**
**North Caldwell, NJ 07006 (US)**
- **PIWINSKI, John, J.**
**Clinton Township, NJ 08833 (US)**
- **XIAO, Dong**
**Scotch Plains, NJ 07076 (US)**
- **CHEN, Xiao**
**Edison, NJ 08820 (US)**

(74) Representative: **Adams, Harvey Vaughan John et al**
**Mathys & Squire,**
**120 Holborn**
**London EC1N 2SQ (GB)**

(56) References cited:
**EP-A- 0 790 248** **WO-A-94/10165**

- **HARRISON, T. ET AL: "Gem-disubstituted amino-ether based substance P antagonists" BIOORG. MED. CHEM. LETT. (1994), 4(23), 2733-4 , XP000995889**
- **OH, CHANG HYUN ET AL: "Synthesis of new hydantoin-3-acetic acid derivatives" BULL. KOREAN CHEM. SOC. (1988), 9(4), 231-5 , XP000995888**
- **KNABE, JOACHIM ET AL: "Racemates and enantiomers of basic substituted 5-phenylhydantoins. Syntheses and antiarrhythmic activity" PHARMAZIE (1997), 52 (12), 912-919 , XP000995887**
- **SCHULTE, KARL E. ET AL: "Hydantoin derivatives as potential antiinflammatory substances" EUR. J. MED. CHEM. - CHIM. THER. (1978), 13(1), 25-31 , XP000995882**
- **WU, X. ET AL: "Generation of Cyclopenta[c] piperidines and Pyrrolo[3,4-c]piperidines-Potential Substance P Antagonists-from Adducts of Cyclic Dienophiles and 5-Chloro-6-methyl-3-phenyl-2H-1,4-oxazin-2 -one" TETRAHEDRON (2000), 56(34), 6279-6290 , XP004214987**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 237 874 B1

## Description

BACKGROUND OF THE INVENTION

The present invention relates to a genus of substituted cyclic ureas and derivatives thereof useful as antagonists of tachykinin receptors, in particular as antagonists of the neuropeptides neurokinin-1 receptor ($NK_1$).

Neurokinin receptors are found in the nervous system and the circulatory system and peripheral tissues of mammals, and therefore are involved in a variety of biological processes. Neurokinin receptor antagonists are consequently expected to be useful in the treatment or prevention of various mammalian disease states, for example respiratory diseases such as chronic lung disease, bronchitis, pneumonia, asthma, allergy, cough, bronchospasm; inflammatory diseases such as arthritis and psoriasis; skin disorders such as atopic dermatitis and contact dermatitis; ophthalmalogical disorders such as retinitis, ocular hypertension and cataracts; addictions such as alcohol dependence and psychoactive substance abuse; stress related disorders such as post tramautic stress disorder; obsessive/ compulsive disorders; eating disorders such as bulemia, anorexia nervosa and binge eating disorders; mania; premenstrual syndrome; central nervous system conditions such as anxiety, general anxiety disorder, panic disorder, phobias, bipolar disorders, migraine, epilepsy, nociception, emesis, depression, psychosis, schizophrenia, Alzheimer's disease, AIDs related dementia and Towne's disease; gastrointestinal disorders such as Crohn's disease and colitis, nausea; bladder disorders; atherosclerosis; fibrosing disorders; obesity; Type II diabetes; pain related disorders such as neuropathic pain, post-operative pain, headache and chronic pain syndromes; and genitourinary disorders such as interstitial cystitis and urinary incontinence.

In particular, $NK_1$ receptors have been reported to be involved in microvascular leakage and mucus secretion, making $NK_1$ receptor antagonists especially useful in the treatment and prevention of asthma, emesis, nausea, depression, anxiety, cough, pain and migraine.

SUMMARY OF THE INVENTION

Compounds of the present invention are represented by the formula I

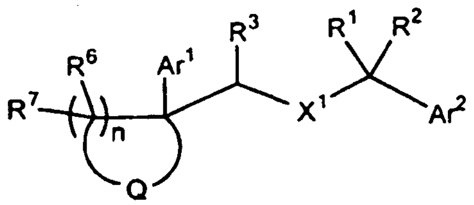

I

or a pharmaceutical acceptable salt thereof, wherein

$Ar^1$ and $Ar^2$ are independently selected from the group consisting of $R^{17}$. heteroaryl and

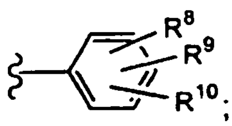

$X^1$ is -O-, -S-, -SO-, $-SO_2-$. $-NR^{12}-$, $-N(COR^{12})-$ or $-N(SO_2R^{15})-$;

$R^1$, $R^2$, $R^3$and $R^7$ are each independently selected from the group consisting of H, $C_1$-$C_6$ alkyl, hydroxy($C_1$-$C_3$)alkyl, $C_3$-$C_8$ cycloalkyl, $-CH_2F$, $-CHF_2$ and $-CF_3$; or $R^1$ and $R^2$, together with the carbon to which they are attached, form a $C_3$-$C_6$ alkylene ring; or, when $X^1$ is -O-, -S- or $-NR^{12}-$ , $R^1$ and $R^2$ together are =O;

each $R^6$ is independently selected from H, $C_1$-$C_6$ alkyl, $-OR^{13}$ or $-SR^{12}$;

n is 1-4, if n is greater than 1, then $R^6$ and $R^7$ can be the same or different on each carbon;

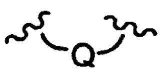

is selected from the group consisting of

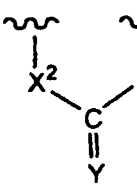 , 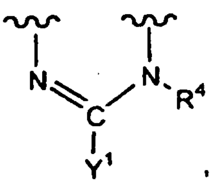 , 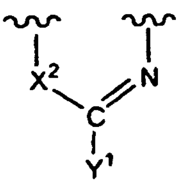 and 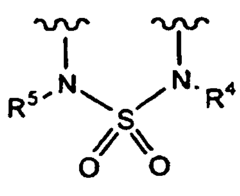 ;

$X^2$ is -O-. -S- or $-NR^5-$;
Y is =O, =S or $=NR^{11}$;
Y' is H, $C_1$-$C_6$ alkyl, $-NR^{17}R^{13}$, $-SCH_3$, $R^{19}$-aryl$(CH_2)_{n6}$-, $R^{19}$-heteroaryl-$(CH_2)_{n6}$-, $-(CH_2)_{n6}$-heterocycloalkyl, -($C_1$-$C_3$) alkyl-NH-C(O)O($C_1$-$C_6$)alkyl or $-NHC(O)R^{15}$;
$R^5$ is H or $-(CH_2)_{n1}$-G, wherein $n_1$ is 0-5, G is H, $-CF_3$, $-CHF_2$, $-CH_2F$, -OH, -O-($C_1$-$C_6$ alkyl), $-SO_2R^{13}$, -O-($C_3$-$C_8$ cycloalkyl), $-NR^{13}R^{14}$, $-SO_2NR^{13}R^{14}$, $-NR^{13}SO_2R^{15}$, - $NR^{13}COR^{12}$, $-NR^{12}(CONR^{13}R^{14})$, $-CONR^{13}R^{14}$, $-COOR^{12}$, $C_3$-$C_8$ cycloalkyl, $R^{19}$-aryl, $R^{19}$-heteroaryl,

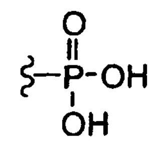 , 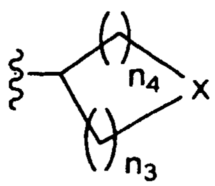 , 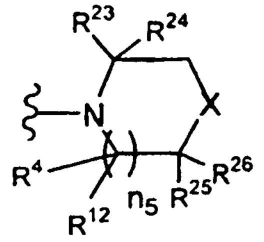 , 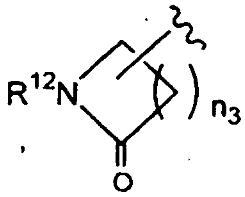 ,

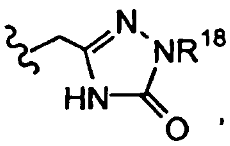 , 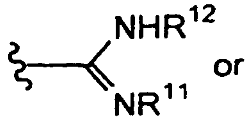 or 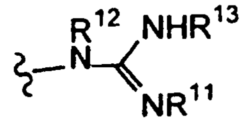 ,

or when $n_1$ is 0, $R^5$ can also be $-C(O)R^{13}$ or $-C(S)R^{13}$; provided that G is not H when n1=0;
X is $-NR^{20}$-, $-N(CONR^{13}R^{14})$-, $-N(CO_2R^{13})$-, $-N(SO_2R^{15})$-, $-N(COR^{12})$-, $N(SO_2NHR^{13})$-, -O-, -S-, -SO-, $-SO_{2-1}$ $-CF_2$-, $-CH_2$- or $-CR^{12}F$-;
$R^8$, $R^9$ and $R^{10}$ are independently selected from the group consisting of H, $C_1$-$C_6$ alkyl, $C_3$-$C_8$ cycloalkyl, $-OR^{12}$, halogen, -CN, $-NO_2$, $-CF_3$, $-CHF_2$, $-CH_2F$, $-OCF_3$, $-OCHF_2$, $-OCH_2F$, $-COOR^{12}$, $-CONR^{21}R^{22}$, $-NR^{21}COR^{12}$, $-NR^{21}CO_2R^{15}$, $-NR^{21}CONR^{21}R^{22}$, $-NR^{21}SO_2R^{15}$, $-NR^{21}R^{22}$, $-SO_2NR^{21}R^{22}$, $-S(O)_{n5}R^{15}$, $R^{16}$-aryl and $R^{19}$-heteroaryl;
$R^{11}$ is H, $C_1$-$C_6$ alkyl, $C_3$-$C_8$ cycloalkyl, $-NO_2$, -CN, OH, $-OR^{12}$, $-O(CH_2)_{n6}R^{12}$, -($C_1$-$C_3$)alkyl-$C(O)NHR^{12}$ , $R^{19}$-aryl $(CH_2)_{n6}$- or $R^{19}$-heteroaryl$(CH_2)_{n6}$-;
$R^4$ and $R^{12}$ are each independently selected from the group consisting of H, $C_1$-$C_6$ alkyl and $C_3$-$C_8$ cycloalkyl;
$R^{13}$ and $R^{14}$ are independently selected from the group consisting of H, $C_1$-$C_6$ alkyl, $C_3$-$C_8$ cycloalkyl, $R^{19}$-aryl$(CH_2)_{n6}$- or $R^{19}$-heteroaryl$(CH_2)_{n6}$-; or $R^{13}$ and $R^{14}$ together are $C_3$-$C_6$ alkylene and with the nitrogen to which they are attached form a 4-7 membered ring, or one of the carbon atoms in the alklyene chain formed by $R^{13}$ and $R^{14}$ is replaced by a heteroatom selected from the group consisting of -O-, -S- and - $NR^{12}$-;
$R^{15}$ is $C_1$-$C_6$ alkyl, $C_3$-$C_8$ cycloalkyl or $-CF_3$;
$R^{16}$ is 1 to 3 substituents independently selected from the group consisting of H, $C_1$-$C_6$ alkyl, $C_3$-$C_8$ cycloalkyl, $C_1$-$C_6$ alkoxy, halogen and $-CF_3$;
$R^{17}$ is H, $C_1$-$C_6$ alkyl, $C_3$-$C_8$ cycloalkyl, $-COOR^{12}$, $-CONR^{21}R^{22}$, $-NR^{21}R^{22}$, - $NR^{21}COR^{12}$, $-NR^{21}CO_2R^{12}$, $-NR^{21}CONR^{21}R^{22}$, $-NR^{21}SO_2R^{15}$ or $-S(O)_{n5}R^{15}$;
$R^{18}$ is H, $C_1$-$C_6$ alkyl or $-P(O)(OH)_2$;
$R^{19}$ is 1 to 3 substituents independently selected from the group consisting of H, $C_1$-$C_6$ alkyl, $C_3$-$C_8$ cycloalkyl, -OH, halogen, -CN, $-NO_2$, $-CF_3$, $-CHF_2$, $-CH_2F$, $-OCF_3$, $-OCHF_2$, $-OCH_2F$, -O-($C_1$-$C_6$ alkyl), -O-($C_3$-$C_8$ cycloalkyl), $-COOR^{12}$, $-CONR^{21}R^{22}$, $-NR^{21}R^{22}$, $-NR^{21}COR^{12}$, $-NR^{21}CO_2R^{12}$, $-NR^{21}CONR^{21}R^{22}$, $-NR^{21}SO_2R^{15}$ and $-S(O)_{n5}R^{15}$;
$R^{20}$ is H, $C_1$-$C_6$ alkyl, $C_3$-$C_8$ cycloalkyl or $-(CH_2)_{n6}$-heterocycloalkyl ;
$R^{21}$ and $R^{22}$ are independently selected from the group consisting of H, $C_1$-$C_6$ alkyl, $C_3$-$C_8$ cycloalkyl and benzyl; or $R^{21}$ and $R^{22}$ together are $C_3$-$C_6$ alkylene and with the nitrogen to which they are attached form a 4-7 membered ring, or one

of the carbon atoms in the alklyene chain formed by $R^{21}$ and $R^{22}$ is replaced by a heteroatom selected from the group consisting of -O-, -S- and $-NR^{12}-$;

$R^{23}$, $R^{24}$, $R^{25}$ and $R^{26}$ are H, $C_1$-$C_6$ alkyl and can be together as =O; when $n_5$ =0, and $R^{25}$ and $R^{26}$ =H, X is not O, N, S;

$n_3$ and $n_4$ are independently 1-5, provided that the sum of $n_3$ and $n_4$ is 2-6;

$n_5$ is independently 0-2; and

$n_6$ is independently 0-3.

Preferred are compounds of formula I wherein $R^4$ and $R^7$ are each H. Also preferred are compounds of formula I wherein $R^1$ and $R^3$ are each H. Also preferred are compounds of formula I wherein $R^1$, $R^3$, $R^4$and $R^7$ are each H. $R^6$ is preferably H or -OH. Preferably, $X^1$ is -O- or $-NR^{12}-$. $Ar^1$ and $Ar^2$ are each preferably $R^8$, $R^9$, $R^{10}$-phenyl, wherein $R^8$, $R^9$ and $R^{10}$ are independently selected. Y is preferably =O, and n is preferably 1 or 2. When Y is =O, $X^2$ is preferably $-NR^5-$. More preferred are compounds of formula I wherein Q is $-X^2-C(=Y)-NR^4-$ (i.e., the first structure shown in the definition of Q), $R^1$, $R^3$, $R^4$ and $R^7$ are each H; $R^6$ is H or -OH; $X^1$ is -O- or $-NR^{12}-$; $Ar^1$ and $Ar^2$ are each $R^8$,$R^9$,$R^{10}$-phenyl; Y is =O and $X^2$ is $-NR^5-$; n is 1 or 2; $R^5$ is H or $-(CH_2)_{n1}-$ G, G is not H when $n_1$=0; $R^{19}$-aryl or $R^{19}$-heteroaryl. Most preferred are compounds of formula I wherein $R^5$ is H.

This invention also relates to the use of a compound of formula I in the treatment of, for example, respiratory diseases such as chronic lung disease, bronchitis, pneumonia, asthma, allergy, cough, bronchospasm; inflammatory diseases such as arthritis and psoriasis; skin disorders such as atopic dermatitis and contact dermatitis; ophthalmalogical disorders such as retinitis, ocular hypertension and cataracts; addictions such as alcohol dependence and psychoactive substance abuse; stress related disorders such as post tramautic stress disorder, obsessive/compulsive disorders; eating disorders such as bulemia, anorexia nervosa and binge eating disorders; mania; premenstrual syndrome; central nervous system conditions such as anxiety, general anxiety disorder, panic disorder, phobias, bipolar disorders, migraine, epilepsy, nociception, emesis, depression, psychosis, schizophrenia, Alzheimer's disease, AIDs related dementia and Towne's disease; gastrointestinal disorders such as Crohn's disease and colitis; nausea; bladder disorders; atherosclerosis; fibrosing disorders; obesity; Type II diabetes; pain related disorders such as neuropathic pain, post-operative pain, headache and chronic pain syndromes; and genitourinary disorders such as interstitial cystitis and urinary incontinence. The treatment of mammals, both human and non-human, is contemplated.

Further, the invention relates to a method for antagonizing the effect of Substance P at its receptor site or for the blockade of neurokinin-1 receptors in a mammal, comprising administering an amount of a compound of formula I effective to antagonize the effect of Substance P at its receptor site in a mammal in need of such treatment.

In another aspect, the invention relates to a pharmaceutical composition comprising a compound of formula I in a pharmaceutically acceptable carrier. The invention also relates to the use of said pharmaceutical composition in the treatment of the mammalian disease states listed above.

The compounds of this invention can be combined with a selective serotonin reuptake inhibitor (SSRI) (i.e., the compounds of this invention can be combined with an SSRI in a pharmaceutical composition, or the compounds of this invention can be administered with an SSRI.

Numerous chemical substances are known to alter the synaptic availability of serotonin through their inhibition of presynaptic reaccumulation of neuronally released serotonin. Representative SSRIs include, without limitation; fluoxetine, fluvoxamine, paroxetine and sertaline, and pharmaceutically acceptable salts thereof. Other compounds can readily be evaluated to determine their ability to selectively inhibit serotonin reuptake.

In another aspect, the invention relates to a method of treating the above diseases and disorders comprising administering an effective amount of an NK1 antagonist of formula I in combination with an SSRI described above.

In another aspect, the invention relates to a method of treating the above diseases and disorders comprising administering an effective amount of an NK1 antagonist of formula I in combination with an SSRI selected from: fluoxetine, fluvoxamine, paroxetine and sertaline, and pharmaceutically acceptable salts thereof.

In another aspect, the invention relates to a method of treating emesis, depression, anxiety, and cough comprising administering an effective amount of an NK1 antagonist of formula I in combination with an SSRI described above.

In the methods of this invention wherein a combination of an NK1 antagonist of this invention (compound of formula I) is administered with an SSRI described above, the compound of formula I and SSRI can be administered simultaneously, consecutively (one after the other within a relatively short period of time), or sequentially (first one and then the other over a period of time). In general, when the antagonists are administered consecutively or sequentially, the NK1 antagonist of this invention (compound of formula I) is administered first.

Preferred are compounds of formula Ia and Ib

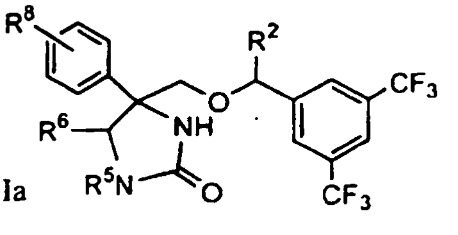

Ia

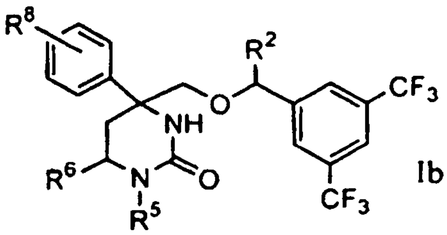

Ib

wherein $R^8$ is H or halogen; $R^2$ is H, $-CH_3$ or-$CH_2OH$; $R^6$ is H or -OH; and $R^5$ is selected from the group consisting of hydrogen and groups of the formula $-(CH_2)_{n1}$-G as follows,

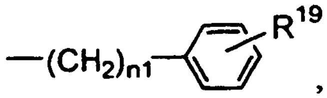

, $-(CH_2)_{n1}-R^{19}$-heteroaryl ,

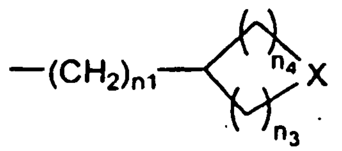

,

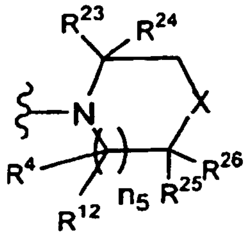

and

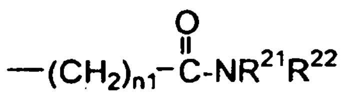

,

or $-(CH_2)_{n1}$-G', wherein $n_1$ is 2-4 and G' is H, -OH, $-OCH_3$-, -OEt, -O(i-Pr), -O-cyclopropyl, or $-CONR^{13}R^{14}$, wherein $R^{13}$ and $R^{14}$ are independently selected from the group consisting of H, $-CH_3$, Et, i-Pr, or cyclopropyl (Et is ethyl and i-Pr is isopropyl).

Also preferred are compounds of the formula Ic and Id

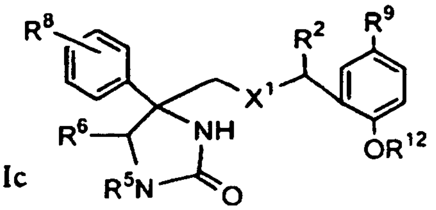

Ic

and

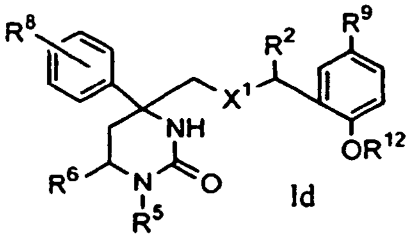

Id

wherein $X^1$ is -O-, -NH-, $-N(CH_3)$- or $-N(COCH_3)$-; $R^8$ is H or halogen; $R^2$ is H, $-CH_3$ or $-CH_2OH$; $R^9$ is $-OCF_3$ or 5-(trifluoromethyl)-1 H-tetrazol-1-yl; $R^6$ is H or -OH; $R^{12}$ is $-CH_3$ or cyclopropyl; and $R^5$ is selected from the group consisting of hydrogen and groups of the formula $-(CH_2)_{n1}$-G as shown above for structures Ia and Ib.

Preferred compounds of the invention are the compounds of examples 2, 61, 79a, 79b, 92, 93, 126, 127, 128, 129, 165a, 165b, 166a and 166b.

DETAILED DESCRIPTION

As used herein, the term "alkyl" means straight or branched alkyl chains. "Lower alkyl" refers to alkyl chains of 1-6 carbon atoms and, similarly, lower alkoxy refers to alkoxy chains of 1-6 carbon atoms.

"Cycloalkyl" means cyclic alkyl groups having 3 to 8 carbon atoms.

"Aryl" means phenyl, naphthyl, indenyl, tetrahydronaphthyl, indanyl, anthracenyl or fluorenyl. $R^{16}$-aryl and $R^{19}$-aryl refer to such groups wherein substitutable ring carbon atoms have an $R^{16}$ or an $R^{19}$ substituent.

"Halogen" refers to fluoro, chloro, bromo or iodo atoms.

"Heteroaryl" refers to 5- to 10-membered single or benzofused aromatic rings comprising 1 to 4 heteroatoms independently selected from the group consisting of - O-, -S-, -N= and -NH-, provided that the rings do not include adjacent oxygen and/or sulfur atoms. Examples of single-ring heteroaryl groups are furanyl, imidazolyl, isoxazolyl,

isothiazolyl, oxadiazolyl, oxazolyl, pyrazinyl, pyrazolyl, pyridyl, pyrimidyl, pyrrolyl, tetrazolyl, thiadiazolyl, thiazolyl, thienyl, triazolyl and triazinyl. Examples of benzofused heteroaryl groups are benzimidazolyl, benzofuranyl, benzo-thiophenyl, benzoxazolyl, indolyl and quinolyl. N-oxides of nitrogen-containing heteroaryl groups are also included. All positional isomers are contemplated, e.g., 2-pyridyl, 3-pyridyl and 4-pyridyl, and when $R^4$ or $R^5$ is heteroaryl, it can be joined to the nitrogen atom of the "-Q-" group either by a ring carbon or a ring nitrogen. $R^{19}$-heteroaryl refer to such groups wherein substitutable ring carbon atoms have an $R^{19}$ substituent. When $R^8$, $R^9$ or R'° is heteroaryl, it is preferably tetrazolyl substituted by H, $C_1$-$C_4$ alkyl, $C_3$-$C_8$ cycloalkyl, -$CF_3$ -$SO_2$-($C_1$-$C_6$ alkyl) or -$OCF_3$.

"Heterocycloalkyl" refers to a 4- to 7-membered saturated ring comprising 1 to 3 heteroatoms independently selected from the group consisting of -O-, -S- and - $NR^{21}$-, wherein $R^{21}$ is H or $C_1$-$C_6$ alkyl, and wherein the remaining ring members are carbon. Where a heterocyclic ring comprises more than one heteroatom, no rings are formed where there are adjacent oxygen atoms, adjacent sulfur atoms, or three consecutive heteroatoms. Examples of heterocyclic rings are tetrahydrofuranyl, pyrrolidinyl, tetrahydropyranyl, piperidinyl, morpholinyl, thiomorpholinyl and piperazinyl.

In the above definitions, wherein variables $R^1$ to $R^{26}$ are said to be independently selected from a group of substituents, we mean that $R^1$, $R^2$, $R^3$, etc., are independently selected, but also that where an $R^8$, for example, occurs more than once in a molecule, those occurrences are independently selected (e.g., if $X^1$ is -$NR^{12}$- wherein $R^{12}$ is hydrogen, G can be -$COOR^{12}$ wherein $R^{12}$ is methyl). Similarly, $R^8$, $R^9$ and $R^{10}$ can be independently selected from a group of substituents, and where more than one $R^8$, $R^9$ or $R^{10}$ is other than hydrogen, the substituents are independently selected; those skilled in the art will recognize that the size and nature of the substituent(s) will affect the number of substituents which can be present.

The "Q" groups are always joined to the rest of the molecule as shown, i.e., they are attached left-to-right, where -$X^2$- or -$NR^5$- is attached to the carbon to which $R^6$ and $R^7$ are attached, and -$NR^4$- is always attached to the carbon to which $Ar^1$ is attached.

Compounds of formula I can have at least one asymmetrical carbon atom and all isomers, including diastereomers, enantiomers and rotational isomers are contemplated as being part of this invention. The invention includes d and l isomers in both pure form and in admixture, including racemic mixtures. Isomers can be prepared using conventional techniques, either by reacting optically pure or optically enriched starting materials or by separating isomers of a compound of formula I.

Those skilled in the art will appreciate that for some compounds of formula I, one isomer will show greater pharmacological activity than other isomers.

Compounds of the invention which have an amino group can form pharmaceutically acceptable salts with organic and inorganic acids. Examples of suitable acids for salt formation are hydrochloric, sulfuric, phosphoric, acetic, citric, oxalic, malonic, salicylic, malic, fumaric, tartaric, succinic, ascorbic, maleic, methanesulfonic and other mineral and carboxylic acids well known to those in the art. The salt is prepared by contacting the free base form with a sufficient amount of the desired acid to produce a salt. The free base form may be regenerated by treating the salt with a suitable dilute aqueous base solution such as dilute aqueous sodium bicarbonate. The free base form differs from its respective salt form somewhat in certain physical properties, such as solubility in polar solvents, but the salt is otherwise equivalent to its respective free base forms for purposes of the invention.

Certain compounds of the invention which are acidic (e.g., those compounds which possess a carboxyl group) form pharmaceutically acceptable salts with inorganic and organic bases. Examples of such salts are the sodium, potassium, calcium, aluminum, gold and silver salts. Also included are salts formed with pharmaceutically acceptable amines such as ammonia, alkyl amines, hydroxyalkylamines, N-methylglucamine and the like.

Compounds of formula I can be prepared using methods known to those skilled in the art. Typical procedures are described below, although the skilled artisan will recognize that other procedures may be applicable, and that the procedure may be suitably modified to prepare other compounds within the scope of formula I.

Method 1:

Compounds of formula I wherein $X^1$ is -O- or -S- and n is 1 can be prepared by the following method.

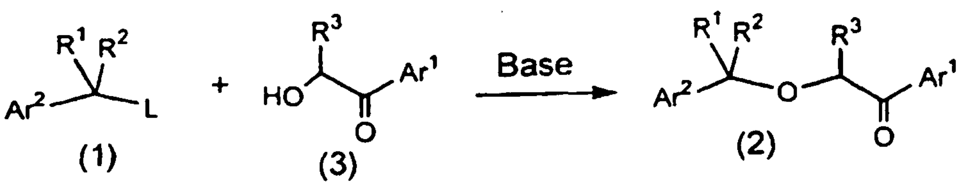


(1) (3) (2)

An alcohol (3), in which $Ar^1$ and $R^3$ are as defined above is converted to the ketone (2), by reaction with an

activated derivative (1) of the alcohol (4), in which $Ar^2$, $R^1$ and $R^2$ are as defined above.

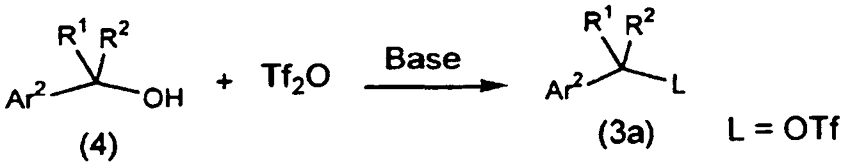

(4) (3a)

This reaction is most favorable when $R^1$, $R^2$ and $R^3$ are each H but, depending on the leaving group L, it may work effectively if either $R^1$, $R^2$ or $R^3$ is C1-C6 alkyl. A leaving group, L, of choice is $CF_3SO_2$- (triflate) but others also suffice, such as Br or I. The base used may vary but is preferably one of the hindered non-nucleophilic kind, of which an example is 2,6-di-tert-butyl-4-methyl pyridine.

The alkylating agent (3a) in which L is triflate may be prepared from the alcohol-type starting material (4) using triflic anhydride and the same hindered, non-nucleophilic base as is used for the alkylation.

The ketone (2) may be used to prepare compounds in which n is 1, $X^2$ is -$NR^5$- and Y is =O (5). Reaction of (2) with a metallic cyanide (e. g. KCN) and $(NH_4)_2CO_3$ results in the formation of the hydantoin, a process well known to those skilled in the art of organic synthesis:

(2)
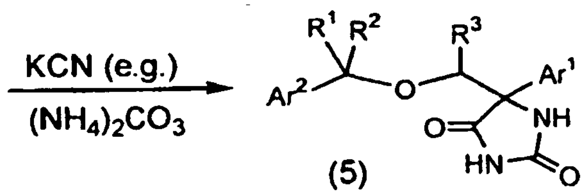

(5)

Selective reduction of the amide carbonyl and not the urea carbonyl may be accomplished by using a mixture of lithium aluminum hydride (LAH) and $AlCl_3$ as a preferred method although other methods are also available, such as the use of LAH in ether or THF at or above room temperature, up to the boiling point of the solution.

The reaction produces compounds of the invention in which $R^4$ and $R^5$ are both H. Introduction of a substituent, $R^5$, may be performed relatively selectively although in some cases a second substituent $R^4$ (where $R^4$ = $R^5$ in this case) may be introduced at the same time. Such substitution reaction at the nitrogen atoms of (5) may be accomplished using one of many sets of conditions used for such transformations, for example, use of an organic base, such as triethylamine or Hunig's Base (di-isopropyl ethylamine) and the appropriate alkylating agent, L-$R^5$.

Reaction of the hydantoin (5) with p-methoxybenzyl chloride in acetone in the presence of $K_2CO_3$ and a catalytic quantity of tetra-n-butylammonium iodide produces the derivative (5A) which can be readily reduced to a mixture of alcohols (5B) by use of mild reducing conditions. Suitable reagents are LAH in THF at 0-30°C for 1-6 hrs.

(5)
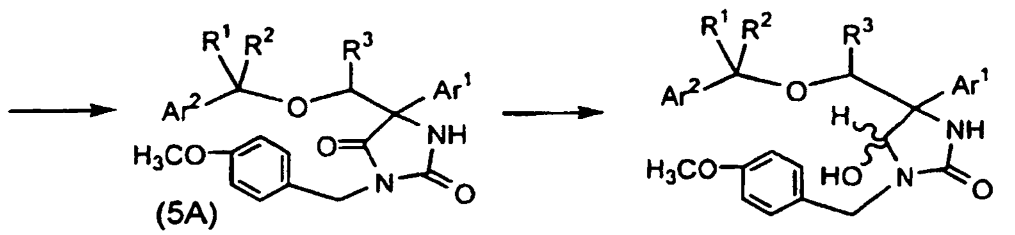
(5A) (5B)

Subsequent removal of the PMB protecting group may be carried out using $Ce(NH_4)_2(NO_3)_6$ (CAN) in a neutral solvent, preferably a $CH_3CN$/water mixture. The mixture of chiral alcohols can frequently be separated and purified by chiral HPLC, preferably on one of the carbohydrate-based columns, such as one of the Daicel Chiralcel® or Chiralpak® series of columns.

Method 2:

Compounds of formula I wherein $X^1$ is -O- or -S- and Y is =O or =S can be prepared by the following method.

The ketone (2) may also be made by the following sequence of reactions. The alcohol (1) may be converted to its alkoxide anion using a strong base, such as lithium bis(trimethyl silyl)amide or the like, followed by reaction, in an

inert solvent, such as THF, with the N,O-dimethyl amide of the iodo-acid (7) to produce (8) which is known as a "Weinreb amide".

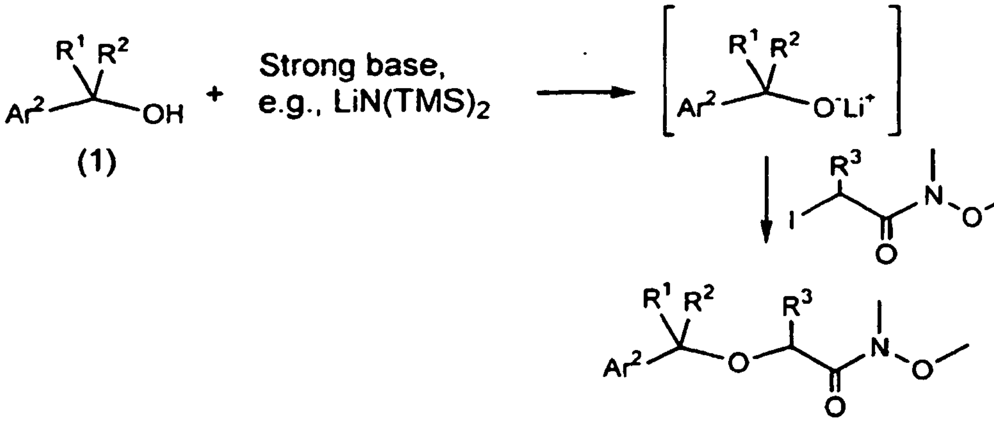


(1)

(8)

Addition of an organometallic derivative of $Ar^1$ (9) results in formation of the ketone (2). Suitable organometallic reagents include the Grignard (M is Mg) or lithium reagent. Suitable media for this reaction include neutral, non-reactive solvents such as ether or THF.

(8) + $Ar^1$-M (9) → (2)

The ketone (2) may next be reacted with trimethylsilylcyanide in the presence of a Lewis acid catalyst, such as $ZnI_2$, and subsequently treated with saturated $NH_3$-$CH_3OH$ at ambient temperature to yield an intermediate which may be reduced directly to diamine (10) using a powerful hydride reducing agent such as LAH in a neutral solvent such as THF.

(2)

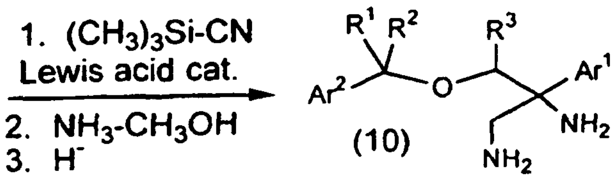


(10)

Reaction of the diamine (10) with a reagent known to introduce a carbonyl between two amines located in the correct position leads to the cyclic ureas (5) which are compounds of the invention. Examples of such reagents are $COCl_2$, carbonyl diimidazole and methyl or ethyl chloroformate. Subsequent modification by introduction of $R^4$ and $R^5$ groups may be performed as described in Method 1.

The reaction described above can also be used to prepare compounds of the invention in which $X^1$ is -S- by employing the thiol corresponding to the alcohol (4) shown above. In addition, reagents known to introduce the -C(=S)- function between two appropriately placed nitrogen atoms (such as thiocarbonyl diimidazole) may be used to prepare compounds in which Y is =S.

A further use of compounds such as 10A is to introduce the guanidine functions into compounds of the invention. Reacting (10A) with $CH_3$ I in a neutral solvent, such as $CH_3CN$, THF, or a mixture of the two, produces the S-methyl derivative (10B) which may then be reacted with an amine, $R^{11}$-$NH_2$, to produce guanidines (10C) or a tautomer.

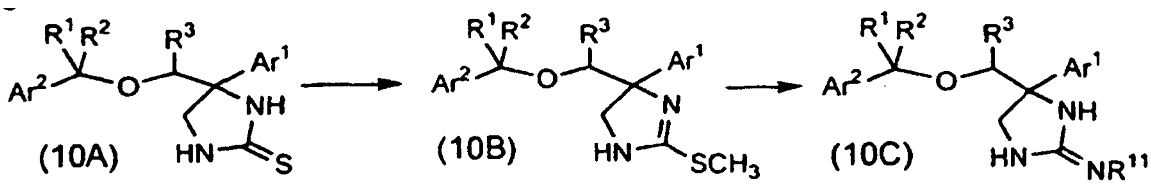

(10A) (10B) (10C)

The diamine (10) is also a useful intermediate for preparation of products of the invention in which $X^2$ is -$NR^5$-. The group $R^5$ is introduced by use of an aldehyde or ketone precursor of $R^5$ by a process of reductive amination (otherwise known as reductive alkylation of the amine (10)). A proviso of this method is that the $R^5$ group may not contain a quaternary carbon atom next to the nitrogen atom. It also cannot be H, nor certain other of the definitions of $R^5$, for example, when G is -OH, -$SO_2R^{13}$ or -$NR^{13}R^{14}$ etc., then $n_1$ cannot be 0. To describe the process, the starting material (10D) will be used. By reacting. (10) with (10D) in a neutral solvent, such as 1,2-dichloroethane, in the presence of a suitable reducing agent (sodium triacetoxyborohydride is particularly suitable for this reaction), and conventional work-up, a product (10E) is formed ($R^5$ is -$(CH_2)_{n1}$-G in which $n_1$ is 0 and G is

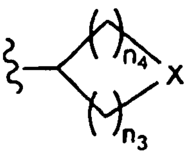

in which $n_3$ = $n_4$ = 2 and X = 0;

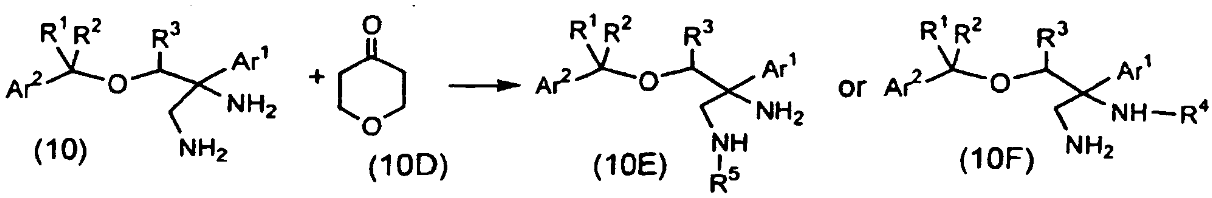

(10) + (10D) (10E) or (10F)

A variable amount of the isomeric structure (10F) (see above) may also form in this reaction, depending upon the reactants. It may be separated from (10E) by conventional chromatographic methods and reacted, as above, to yield compounds of the invention in which $R^4$ is the introduced substituent instead of $R^5$.

Reactions with other precursors of the $R^5$ group are also possible, as will be evident to one skilled in the arts of organic and medicinal, chemistry. Ring closing of (10E) may be carried out by direct cyclization to make many of the Q groups of the invention in which an $R^5$ group is present and where Y is =0 or =S, or compounds wherein Y is =N-$R^5$ can be made by the sequence of reactions described earlier for the synthesis of (10C).

A further use for the diamine (10) is in the preparation of compounds of the invention in which Y' is as defined above, except that it is not -$NH_2$, -$NHCH_3$ or - $SCH_3$. The diamine (10) may be heated with a carboxylic acid, $Y^1$-$CO_2H$, in a high boiling, neutral solvent, such as toluene to produce the amidines (10G) of the invention:

(10) + $Y^1$-$CO_2H$

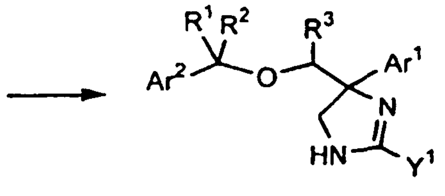

(10G) or tautomer

Method 3:

An alcohol (3) may be reacted with an O-substituted hydroxylamine derivative, preferably methoxylamine to yield the oxime derivative (11). Conversion of the oxime to the alkoxide may be performed using a strong base, such as NaH, in a non-hydroxylic solvent, such as THF. Reaction of this anion with the substituted alkyl halide (12), in which Hal is preferably I or Br, produces the oxime-ether (13).

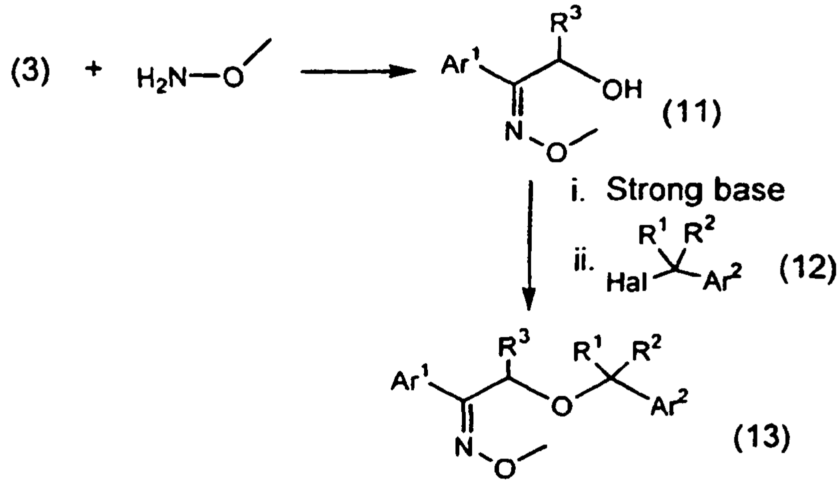

(3) (11) (12) (13)

Cleavage of the oxime-ether (13) under acidic conditions, for instance, using 6N HCl at elevated temperature for 5 to 50 hours results in isolation of the ketone (2).

Further processing of (2) may be performed as described above in Method 1.

Method 4:

Compounds of formula I wherein $X^1$ is -O- and n is 2-4 can be prepared by the following method (only $R^6$ is shown in the formulae, but both $R^6$ and $R^7$ can be present).

A diprotected aryl glycine ester, such as (14), in which "Prot" is a protecting group, preferably benzyl, and "E" is an ester group, preferably methyl or ethyl, may be converted to its anion using a strong base, such as lithium diisopropylamide, in an ether solvent, such as THF, at a temperature of about -78 °C. Reaction of the anion with a halo-nitrile (15), in which "Hal" is preferably I, at temperatures between -78 °C and 0°C results in the protected intermediate (16).

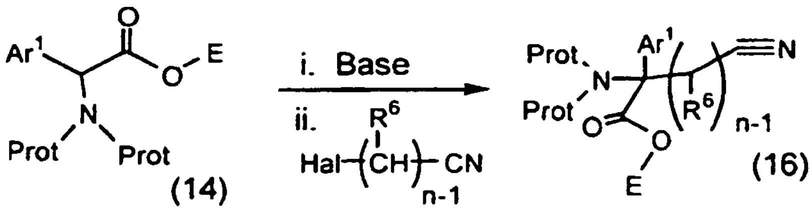

(14) (16)

Reduction of the ester and nitrile simultaneously using a powerful hydride reducing agent, such as LAH, in an inert solvent, such as THF, at a temperature between about -78 °C and 0 °C, produces aminoalcohol (17).

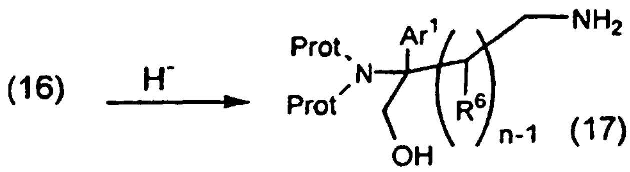

(16) (17)

Removal of the protecting groups under standard conditions (e.g. 20% $Pd(OH)_2$ on active carbon in methanol if "Prot" is benzyl) yields the di-amine alcohol (18) which may be cyclized using one of the reagents known to introduce a C=O, C=S or $-SO_2-$ group between two appropriately placed nitrogen atoms (*e.g.* $COCl_2$; carbonyl diimidazole; thiocarbonyl diimidazole, etc.) to yield (19).

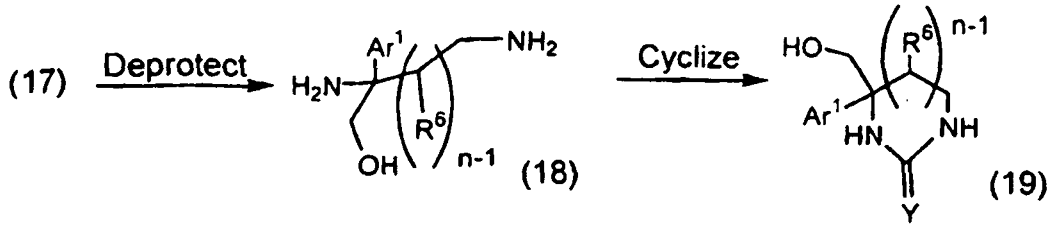

(17) (18) (19)

wherein =Y is =O or =S; the thio-urea can be prepared in a similar manner.

The alcohol (19) may be converted to a compound of the invention (20), by reaction of the mono-anion prepared by using a strong base, such as NaH, with a benzyl halide in the presence of $Ag_2O$. "Hal" is as defined above and the solvent is preferably a polar, non-hydroxylic solvent such as DMF.

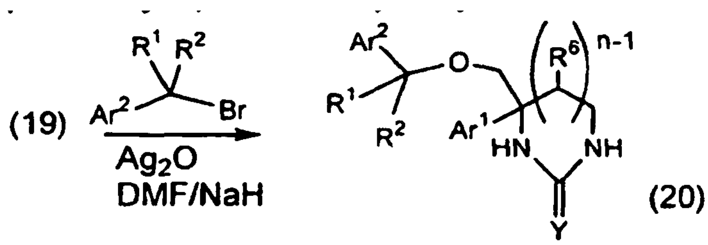

(19) (20)

Subsequent modification by introduction of $R^4$ and $R^5$ groups may be performed as described in Method 1.

Method 4a:

The intermediate (17), above, may also be converted to compounds of the invention via its di-BOC protected derivative (21).

Removal of the original protecting groups (if they are benzyl groups or similar) by hydrogenolysis in the presence of $(BOC)_2O$ yields the di-BOC derivative (21).

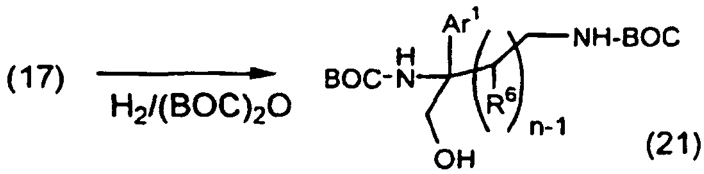

(17) (21)

Such intermediates may be converted to the ethers (22) by reaction with an aryl halide (23), preferably a bromide or iodide. Use of silver oxide ($Ag_2O$) as a catalyst and base is desirable.

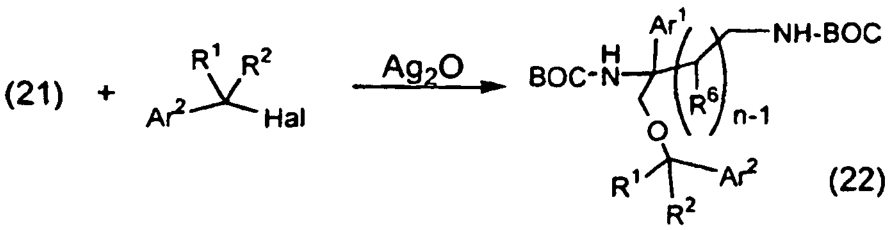

(21) + (22)

Removal of the BOC protecting groups under standard conditions (e.g. HCl/ether) produces the diamine which may be cyclized to compounds of the invention using the same reagents as described earlier in Method 2, *e.g.* $COCl_2$, carbonyl diimidazole, sulfonyl diimidazole, etc.

Method 5:

Compounds of the invention in which $X^1$ is one of the nitrogen-containing groups may be synthesized from the

amino ketone derivatives (10G), some of which may be commerically available while others can be synthesized by well-known literature techniques. Protection of the amino group in (10G), for instance as its BOC derivative, allows the hydantoin-forming reaction to occur to produce intermediates (10H) where "Prot" is the previously-introduced protecting group, e.g. BOC:

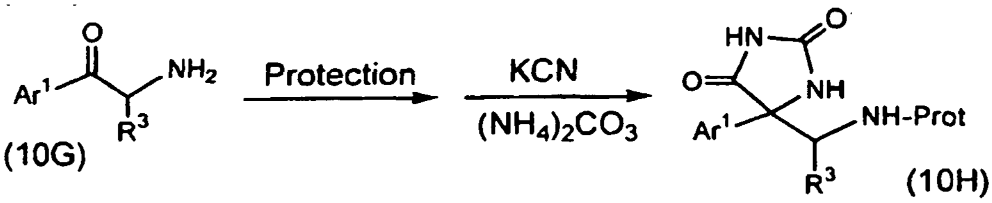


Reduction of one of the carbonyl groups preferentially, as described previously, using the $LiALH_4/AlCl_3$ mixed reagents, followed by removal of the protecting group by an appropriate method (e.g., $CF_3CO_2H$ or HCl if it is BOC) produces the amine (10I)

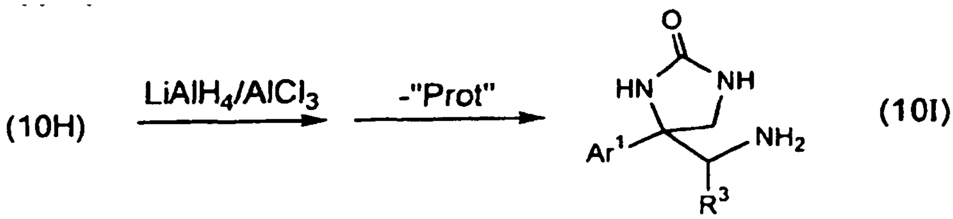


Reductive alkylation of the amine group with an aldehyde or ketone (10J) under conditions described for this transformation earlier (using sodium triacetoxyborohydride) or using one of the many published procedures known to carry out this reaction (e.g. sodium borohydride in an alcohol solvent) results in the amine (10K) which may be further modified by reactions well known in the art to produce other compounds of the invention, (10L) and (10M).

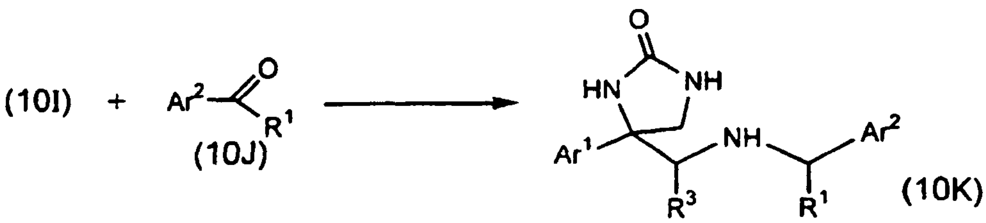


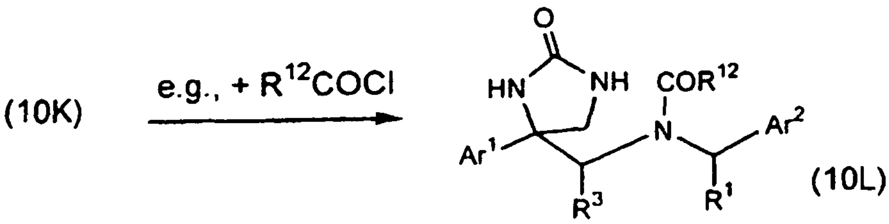


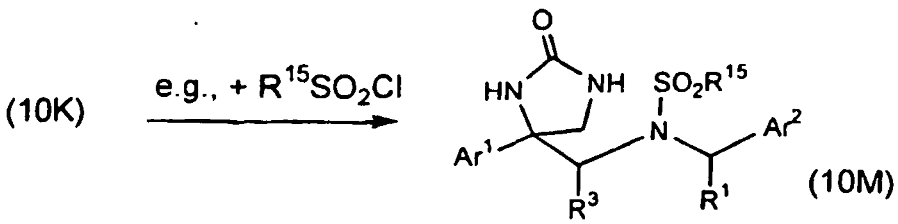

Reactive groups not involved in the above processes can be protected during the reactions with conventional protecting groups which can be removed by standard procedures after the reaction. The following Table 1 shows some typical protecting groups:

**Table 1**

| Group to be Protected | Group to be Protected and Protecting Group |
|---|---|
| $-COOH$ | $-COOalkyl$, $-COObenzyl$, $-COOphenyl$ |
| >NH | >NCOalkyl, >NCObenzyl, >NCOphenyl, >$NCH_2OCH_2CH_2Si(CH_3)_3$ >$NC(O)OC(CH_3)_3$, >N-benzyl, >$NSi(CH_3)_3$, 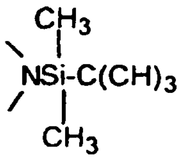 |
| $-NH_2$ | 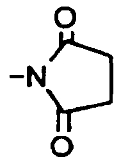 |
| $-OH$ | $-OCH_3$, $-OCH_2OCH_3$, $-OSi(CH_3)_3$, 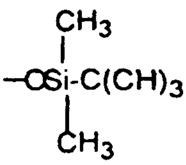 or $-OCH_2phenyl$ |

For preparing pharmaceutical compositions from the compounds described by this invention, inert, pharmaceutically acceptable carriers can be either solid or liquid. Solid form preparations include powders, tablets, dispersible granules, capsules, cachets and suppositories. The powders and tablets may be comprised of from about 5 to about 95 percent active ingredient. Suitable solid carriers are known in the art, e.g. magnesium carbonate, magnesium stearate, talc, sugar or lactose. Tablets, powders, cachets and capsules can be used as solid dosage forms suitable for oral administration. Examples of pharmaceutically acceptable carriers and methods of manufacture for various compositions may be found in A. Gennaro (ed.), Remington's Pharmaceutical Sciences, 18th Edition, (1990), Mack Publishing Co., Easton, Pennsylvania.

Liquid form preparations include solutions, suspensions and emulsions. As an example may be mentioned water or water-propylene glycol solutions for parenteral injection or addition of sweeteners and opacifiers for oral solutions, suspensions and emulsions. Liquid form preparations may also include solutions for intranasal administration.

Aerosol preparations suitable for inhalation may include solutions and solids in powder form, which may be in combination with a pharmaceutically acceptable carrier, such as an inert compressed gas, e.g. nitrogen.

Also included are solid form preparations which are intended to be converted, shortly before use, to liquid form preparations for either oral or parenteral administration. Such liquid forms include solutions, suspensions and emulsions.

The compounds of the invention may also be deliverable transdermally. The transdermal compositions can take the form of creams, lotions, aerosols and/or emulsions and can be included in a transdermal patch of the matrix or reservoir type as are conventional in the art for this purpose.

Preferably the compound is administered orally.

Preferably, the pharmaceutical preparation is in a unit dosage form. In such form, the preparation is subdivided into suitably sized unit doses containing appropriate quantities of the active component, e.g., an effective amount to achieve the desired purpose.

The quantity of active compound in a unit dose of preparation for treatment of respiratory diseases; inflammatory diseases; skin disorders; ophthalmalogical disorders; addictions; stress related disorders; obsessive/compulsive disorders; eating disorders; mania; premenstrual syndrome; central nervous system conditions; gastrointestinal disorders; bladder disorders; atherosclerosis; fibrosing disorders; obesity; Type II diabetes; pain related disorders; and genitourinary disorders; may be varied or adjusted from about 1 mg to about 1500 mg, preferably from about 50 mg to about 500 mg,

more preferably from about 20 mg to about 200 mg, according to the particular application.

The actual dosage employed may be varied depending upon the requirements of the patient and the severity of the condition being treated. Determination of the proper dosage regimen for a particular situation is within the skill of the art. For convenience, the total daily dosage may be divided and administered in portions during the day as required.

The amount and frequency of administration of the compounds of the invention and/or the pharmaceutically acceptable salts thereof will be regulated according to the judgment of the attending clinician considering such factors as age, condition and size of the patient as well as severity of the symptoms being treated. A typical recommended daily dosage regimen for oral administration can range from about 1 mg/day to about 1500 mg/day, in two to four divided doses.

Following are examples of preparing compounds of formula 1. As used herein, RT is room temperature, Me is methyl, Bu is butyl, Br is bromo, Ac is acetyl, Et is ethyl, Ph is phenyl, THF is tetrahydrofuran, EtOAc is ethyl acetate, $Et_2O$ is ether, LAH is lithium aluminum hydride, CDI is 1,1-carbonyl diimidazole; HOBT is hydroxybenzotriazole; DEC is 1,2-diethylaminoethyl chloride; TFA is trifluoroacetic acid; $Et_3N$ is triethylamine, MTBE is *t*-butyl methyl ether; DAST is diethylaminosulfur trifluoride.

Example 1

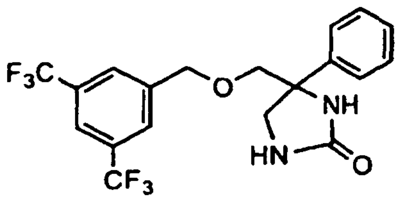

Method 1:

Step 1:

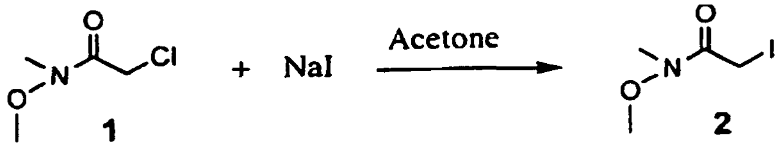


To a solution of 2-chloro-N-methylacetamide 98% (10.4 g, 74 mmol) in acetone (120 ml), Nal (12.2 g, 81.4 mmol) was added. The flask was filled with $N_2$ and covered with aluminum foil. After stirring at RT for 30 h, the reaction mixture was filtered. The filtrate was concentrated under vacuum to give a dark brown oil. The crude product was directly used without further purification in the next step.

Step 2:

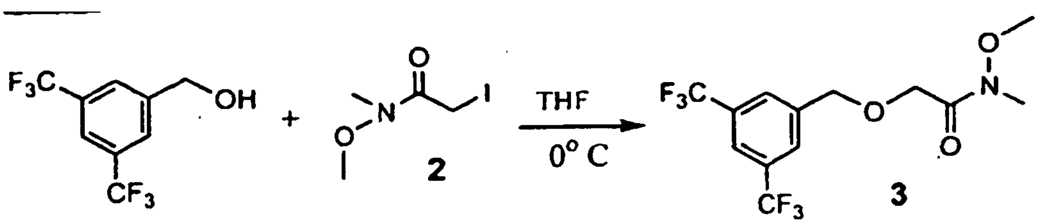


To a cooled solution of 3,5-bis(trifluoromethyl)benzyl alcohol (18.06 g, 74 mmol) in anhydrous THF (140 ml) at 0 °C, solid $KN(TMS)_2$ (16.24 g, 81.4 mmol) was added slowly. The reaction was kept at 0 °C for 1 h. A solution of 2 in anhydrous THF (60 ml) was then added dropwise into the cooled solution. The reaction was allowed to gradually

warm to RT over night under a $N_2$ atmosphere. After quenching with saturated $NH_4Cl$, the reaction was made slightly acidic with 1N HCl and extracted with EtOAc (200 ml × 4). The combined organic layer was washed with brine (200 ml x 2), dried over $MgSO_4$, filtered and concentrated. The crude material was purified by flash chromatography, eluting with 20% EtOAc in hexane to give 3 as an oil (12.3 g, 35.6 mmol, 48% yield).

Step 3:

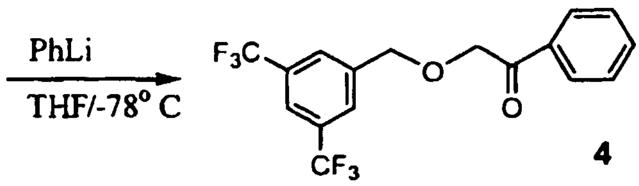


4

To a cooled solution of 3 (12.2 g, 35.34 mmol) in anhydrous THF (150 ml) at - 75°C, phenyl lithium (22.58 ml, 40.64 mmol) was added dropwise. The reaction mixture was kept at low temperature for 1.5 h, then the cold bath was removed and the reaction was allowed to warm up to RT under $N_2$ protection. The reaction was cooled in an ice-water bath and quenched with saturated $NH_4Cl$ solution (200 ml) using a dropping funnel followed by neutralizing the aqueous solution to pH -7 with 1N HCl. After stirring for 15 min, the mixture was extracted with EtOAc (200 ml x 4). The EtOAc extracts were combined, washed with brine (200 ml x 2), dried over $MgSO_4$, filtered and concentrated to give a brown oil. The crude material was purified by flash grade silica gel chromatography, eluting with 15% EtOAc in hexane to give compound 4 (10.7 g, 28.44 mmol) with -80% yield. FAB MS $[M+1]^+$ 263.1.

Step 4:

4

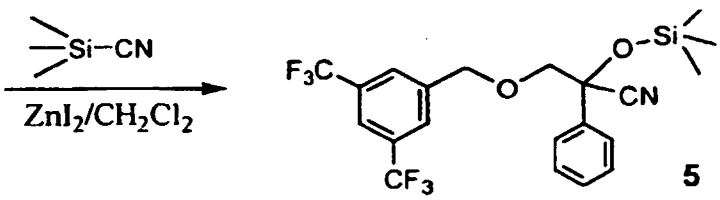


5

Trimethyl silyl cyanide (0.92 ml, 6.9 mmol) was added to a flask containing a solution of 4 (2.0 g, 5.52 mmol) in $CH_2Cl_2$ (11 ml), cooled with a water bath, followed by addition of $ZnI_2$ (88 mg, 0.276 mmol). The reaction was finished in 1 h. The insoluble $ZnI_2$ was filtered off and rinsed with $CH_2Cl_2$. Evaporation of the solvent yielded a light yellow oil as crude product which was used in the next step.

5

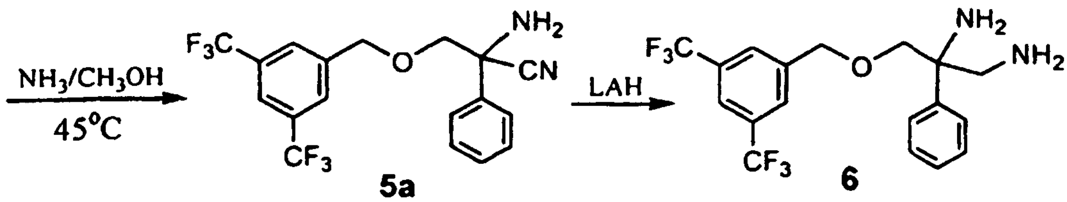


5a 6

The crude material **5** was treated with saturated $NH_3$-$CH_3OH$ (10 ml) and heated in an oil bath under $N_2$ at 45 °C. After 2 h of heating, solid was filtered off and the filtrate was concentrated to give **5a**.

To a suspension of LAH (0.84 g, 22 mmol) in anhydrous $Et_2O$ (40 ml) at -78 °C, a suspension of **5a** in anhydrous $Et_2O$ (40 ml) was added through an addition funnel under the protection of $N_2$. The reaction mixture was stirred at RT over night. After the reaction was complete, $Et_2O$ (250 ml) was added. The reaction was quenched with saturated $Na_2SO_4$ solution and stirred for 2 h. The solid was filtered off and the filtrate was dried over $MgSO_4$, filtered again and treated with 4M HCl in dioxane (3 ml). Evaporation of all the volatile solvents gave the crude product, which was further purified

on Biotage cartiledge, eluting with 7.5% $NH_4OH$-$CH_3OH$ (1:9) in 92.5% in $CH_2Cl_2$ to give 6 as an oil. FAB MS $[M+1]^+$ 393.1.

Step 5:

To a solution of 6 (0.155 g, 0.395 mmol) in anhydrous THF (12 ml) was added 3 Å molecular sieves (200 mg) and 1,1'-carbonyl diimidazole (76.6 mg, 0.474 mmol). The mixture was stirred at RT overnight under $N_2$. Diluted with 200 ml EtOAc. the reaction mixture was washed with brine (100 ml × 2), dried over $MgSO_4$. filtered and concentrated. The crude material was purified by flash chromatography, eluting with 5% $NH_4OH$-$CH_3OH$(1:9) in 95% $CH_2Cl_2$, to give the title compound as a solid with a 97% yield. FAB MS $[M+1]^+$ 419.1.

Method 2:

Step 1:

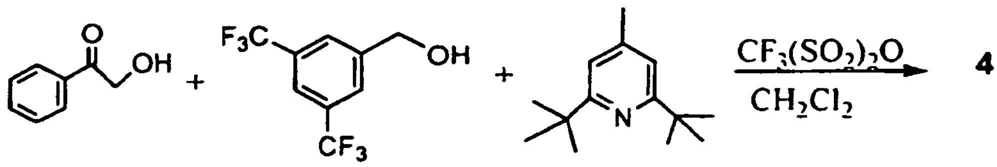


A mixture of 3,5-bis(trifluoromethyl)benzyl alcohol (39.9 g, 0.163 mol) and 2,6-di-t-butyl-4-methyl-pyridine (68.56g, 0.33 mol) was placed in a 2 liter, 3-neck flask and vacuum dried overnight. To this green mixture was added dry $CH_2Cl_2$ (600 ml) (cooled with a water bath), followed by slow addition of trifluoromethylmethane sulfonic anhydride (50 g, 0.177 mol) through a dropping funnel under a $N_2$ atmosphere. After stirring at RT for 4 h, a solution of 2-hydroxy-acetophone (20.36 g, 0.149 mol) in dry $CH_2Cl_2$ (120 ml) was added slowing through a dropping funnel under $N_2$. The reaction mixture was stirred at RT for 5 days, then solid was filtered off. The filtrate was washed with brine (200 ml, 3x), dried ($MgSO_4$), filtered and concentrated to give a dark brown oil which was purified with flash grade silica gel (1 Kg), eluting with 10 % EtOAc/hexane. Compound 4 was obtained as a solid (39.63 g, 0.11 mol, 74 % yield).

Step 2:

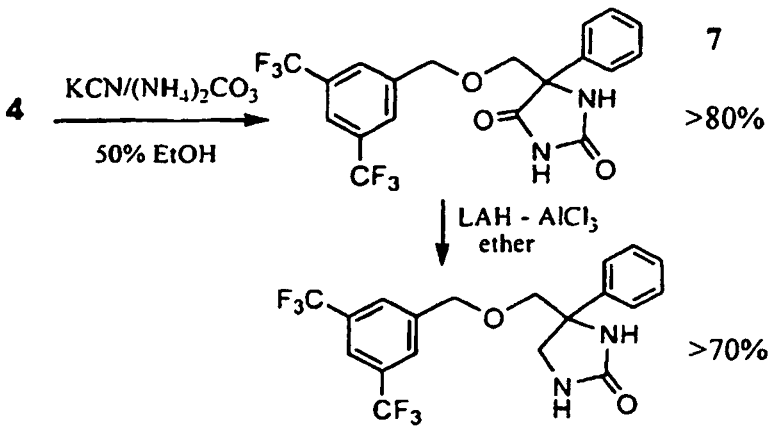


A mixture containing compound **4** (39.5 g, 109 mmol), KCN (10.64g, 163 mmol) and $(NH_4)_2CO_3$ (37.47 g, 380 mmol) in 50% EtOH / $H_2O$ was heated in an oil bath at 60 °C under the $N_2$ atmosphere for 22 h. After cooling, ice-water (780 ml) was added and the mixture was stirred for 1 h. The white solid was filtered and rinsed with water. The solid was crystallized from hot dichloroethane. Pure compound 7 was obtained as a white solid (39g, 90 mmol, 83%).

To a one-liter round bottom flask (cooled with an ice-water bath) containing $AlCl_3$ (24.6g, 186 mmol) was slowly added 1 M LAH solution in $Et_2O$ (140 ml, 140 mmol) under an $N_2$ atmosphere. After stirring at 0°C for 10 min, a solution of compound 7 (20g, 46.2 mmol) in dry THF (170 ml) was added slowly to the LAH-$AlCl_3$ mixture. It was gradually

warmed to RT and stirred at RT for 3 days. After completion, the reaction mixture was diluted with THF (200 ml) and excess LAH was decomposed with saturated $Na_2SO_4$ and 3 N NaOH. The mixture was stirred for 1 h at RT and solid was filtered off through a celite pad. The filtrate was dried over $MgSO_4$, filtered and concentrated to give the title compound as a foam which was purified on flash silica gel, eluting with 3.5 % (1:9) $NH_4OH/CH_3OH$ in 96.5 % $CH_2Cl_2$ to give pure title compound (16g, 40 mmol, 87% yield) as a solid.

The racemic title compound was separated on a chiral column by using either a ChiralPak AS (Hexane / IPA (80:20)) or a Chiralcel OD ($CH_3CN$) column to give enantiomer A and enantiomer B (mp 138-140 °C). Enantiomer B: HRMS calculated for [M+1]: $C_{19}H_{17}F_6N_2O_2$ 419.1194, Found: 419.1190; C, H, N analysis : calculated C, 54.57; H, 3.71; N, 6.55; F 27.25 Found: C 54.55; H, 3.89; N, 6.59;F, 27.22; rotation in $CH_3OH$ $[\alpha]^{20}_D$= -67.0 °

Example 2

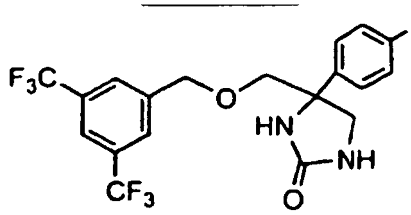

Part A:

Step 1:

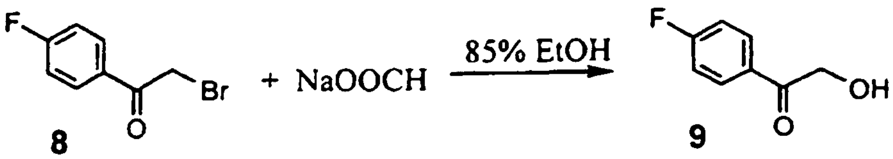


A suspension of 2-bromo-4'-fluoroacetophenone, 8, (30 g, 0.1368 mol) and HCOONa (59.2 g, 0.87 mol) in 85% EtOH (360 ml) was heated to 80 °C for 5h. After cooling, it was stirred at RT over night under $N_2$. The reaction was stopped by evaporating off the EtOH, and the residue was redissolved in brine (500 ml) and extracted with EtOAc (200 ml x 3). The combined organic layers were dried ($MgSO_4$), filtered and concentrated. The purified product was obtained by triturating the crude material with $CH_2Cl_2$, EtOAc and hexane to give 9 as a solid (18 g, 116 mmol, 85% yield).

Step 2:

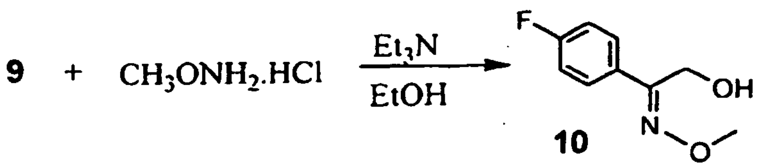


To a suspension of **9** (5.0 g, 32.44 mmol) in absolute EtOH (50 ml), 3Å molecular sieves (0.8 g), $CH_3NH_2$.HCl (4.146 g, 48.67 mmol) and $Et_3N$ (6.77 ml, 48.67 mmol) were added. The mixture was heated to 85 °C under $N_2$ for 2.5 h. The mixture was cooled to RT, the solvent was evaporated under reduced pressure, and the crude product was redissolved in EtOAc (350 ml). This organic layer was washed with brine (150 ml x 3), dried ($MgSO_4$), filtered and evaporated to dryness to give 10 as a solid (13.5 g, 7.36 mmol) in 97% yield.

Step 3:

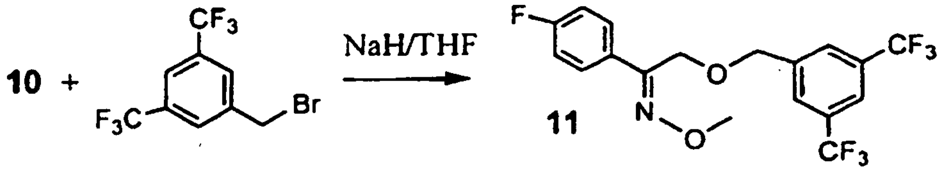


To a cooled solution of 10 (12.0 g, 65.5 mmol) in anhydrous THF (60 ml) (cooled in an ice-water bath) was added 60% NaH in mineral oil (3.14g, 78.61 mmol). After stirring at RT for 15 min, the yellow suspension was treated with a solution of 3,5-bis(trifluoromethyl)benzyl bromide (13.62 ml, 72.05 mmol) in THF (13.62 ml). TLC indicated that the reaction was complete after 1.5 h at RT. Solvent was evaporated and the residue was redissolved in EtOAc (200 ml). This solution was washed with brine (50 ml x 2), dried ($MgSO_4$), filtered and concentrated to give **11** as an oil (26.5g, 64.77 mmol, 98% yield) FAB MS $[M+1]^+$ 410.1.

Step 4:

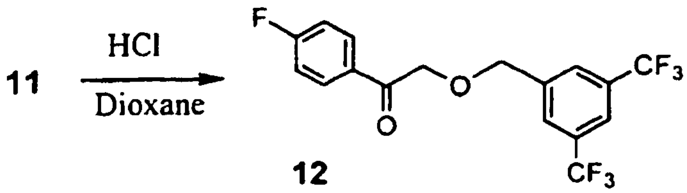


To a solution of 11 (26 g, 63.55 mmol) in dioxane (116 ml) was added 6N HCl (378 ml, 2.268 mol). The mixture was heated at 100 °C for 2 days. After cooling to RT, the reaction mixture was poured into ice-cold KOH solution (120 g, -2.142 mol in 240 ml $H_2O$). The products were extracted with $CH_2Cl_2$ (200 ml x 3) from aqueous solution, dried ($MgSO_4$) and filtered. Solvents were evaporated to give a crude product as a brown oil. The final product was further purified by flash chromatography, eluting with 10% EtOAc in hexane to give 12 as a solid (13.41 g, 35.27 mmol, 55.5% yield).

Part B:

**Step 1:**

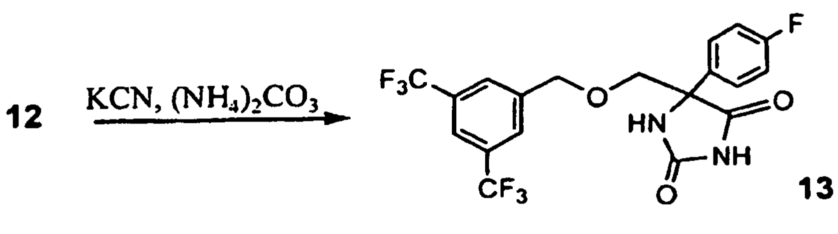


To the mixture of **12** (3.23 g, 8.5 mmol) in 1:1 EtOH/$H_2O$ (20 ml), KCN (0.83 g, 12.8 mmol) and $(NH_4)_2CO_3$ (2.86 g, 29.8 mmol) were added. The mixture was heated to 56 °C overnight, then cooled to RT. Iced water (60 ml) was added to the complete reaction. After 30 min, the solid was filtered off and rinsed with $CH_2Cl_2$ to give 13 as a white powdery product (3.12 g, 82%). FAB MS $[M+1]^+$ 451.1.

Step 2:

To a cooled 3-neck flask containing $AlCl_3$ (1.4 g, 10.7 mmol) at 0 °C, 1 M LAH in $Et_2O$ (8 ml, 8 mmol) was added dropwise via a syringe. A solution of 13 (1.2 g, 2.67 mmol) in anhydrous THF (10 ml) was added slowly and the reaction mixture was allowed to stir at RT overnight. After the reaction was complete, THF (100 ml) was added and excess LAH was decomposed with saturated $Na_2SO_4$ and 4N NaOH. After stirring for 1 h, the organic layer was separated, dried ($MgSO_4$), filtered and concentrated. The crude material was purified by flash silica gel eluting with 3.5% $NH_3$-$CH_3OH$ (1:9) / 96.5% $CH_2Cl_2$ to give the title compound as a white foam (0.95 g. 0.22 mmol, 82% yield). FAB MS $[M+1]^+$ 437.1.

The racemic compound was separated with a Chiralcel OD column, eluting with $CH_3CN$ to give enantiomers A and B. The enantiomers A and B were reduced separately with LAH-$AlCl_3$ to give chiral compounds A and B. Pure chiral B was obtained by crystallization from hot $CH_3CN$ and hexane. Enantiomer B: HRMS calculated for [M+1]: $C_{19}H_{16}F_7N_2O_2$ 437.1103, Found: 437.1100; C, H, N analysis : calculated C, 52.30; H, 3.46; N, 6.42; Found: C 52.38; H, 3.42; N, 6.31; rotation in $CH_3OH$ $[\alpha]^{20}_D$ = -55.7, mp: 130-132 °C.

Example 3

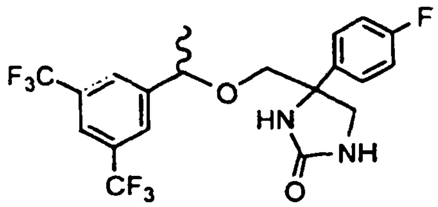

Step 1:

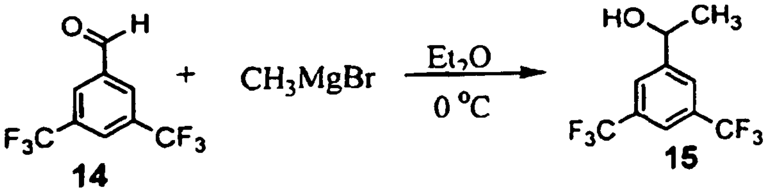


14 15

To the cooled solution of 3,5-bis(trifluoromethyl)benzaldehyde **14** (15 g, 62 mmol) in anhydrous $Et_2O$ (100 ml) at 0°C, 3 M $CH_3MgBr$ (25 ml, 75 mmol) was added dropwise. The reaction was kept at 0 °C for another 2 h, then stirred at RT for 2 h. The reaction was quenched by pouring into ice cold saturated $NH_4Cl$. Routine work-up gave 15 as an off-white solid product (15.4 g).

Step 2:

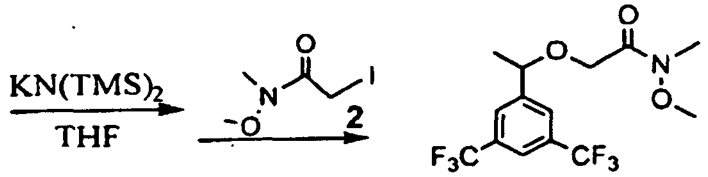


15 2 16

To the cooled solution of **15** (18.8 g, 72.9 mmol) in anhydrous THF (100 ml) at 0 °C was added solid $KN(TMS)_2$ (17.4 g, 87 mmol) portionwise. After stirring at 0 °C for 1h, the potassium salt solution of **15** was added dropwise to a solution of **2** (17.5 g, 76.4 mmol) in anhydrous THF (20 ml). The reaction was kept at 0 °C overnight and quenched with

saturated $NH_4Cl$ (300 ml). The aqueous layer was extracted with $CH_2Cl_2$ (300 ml x 3). The combined organic layer was washed with brine (300 ml), dried ($MgSO_4$), filtered and concentrated to a yellow oil (22 g). The crude product was further purified on silica gel (1:4 EtOAc/hexane) to give **16** as a pale yellow material (11 g, 31 mmol, 43% yield).

Step 3:

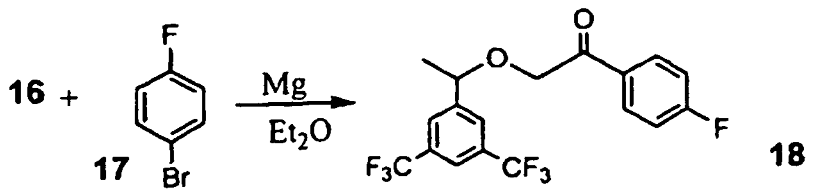

16 + 17 18

In a cooled flask (ice water bath) containing Mg turnings (1.12 g, 46.2 mmol) in anhydrous $Et_2O$ (100 ml) was slowly added 4-bromofluoro-benzene **17** (7.7 g, 44 mmol), followed by a catalytic amount of 1,2-dibromoethane. After completion of the addition, the reaction was heated at 80 °C overnight under a $N_2$ atmosphere. After cooling, a solution of **16** (7.9 g, 22 mmol) in anhydrous $Et_2O$ (40 ml) was added slowly to the Grignard solution at RT. After reaction was complete, a saturated $NH_4Cl$ solution was slowly added under a $N_2$ atmosphere. The product was extracted from aqueous solution with $CH_2Cl_2$, combined, dried ($Na_2SO_4$), filtered and evaporated to give **18** as an oil. The crude material was purified on flash grade silica gel (400 g) eluting with 15% EtOAc/85% hexane to give **18** (7 g, 1.78 mmol, 87% yield). FAB MS $(M+1)^+$ 395.0.

Step 4:

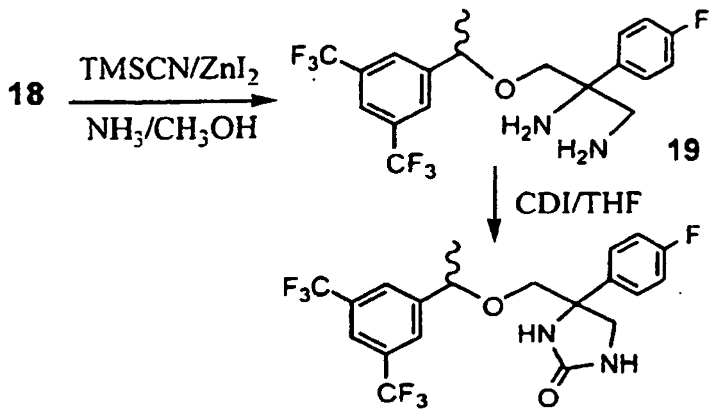

18 19

Compound **19** was prepared by an analogous method to that of Example 1 (step 4) using compound **18** in place of compound **4**.

The title compound was prepared by a method analogous to that described for Example 1 (step 5) using compound **19** in place of compound **6**. Two sets of diastereomers A and B were isolated by flash silica chromatography at compound **19** stage and these were cyclized separately to the title compounds with CDI reagent. Diastereomer A of the title compound, FAB MS $[M+1]^+$ 451.1265, calculated $[M+1]^+$ 451.1257; Diastereomer B of the title compound, FAB MS $[M+1]^+$ 451.1261, calculated $[M+1]^+$ 451.1257.

Example 4

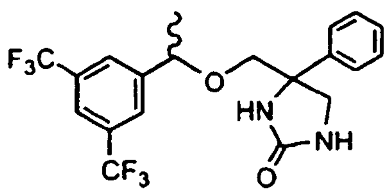

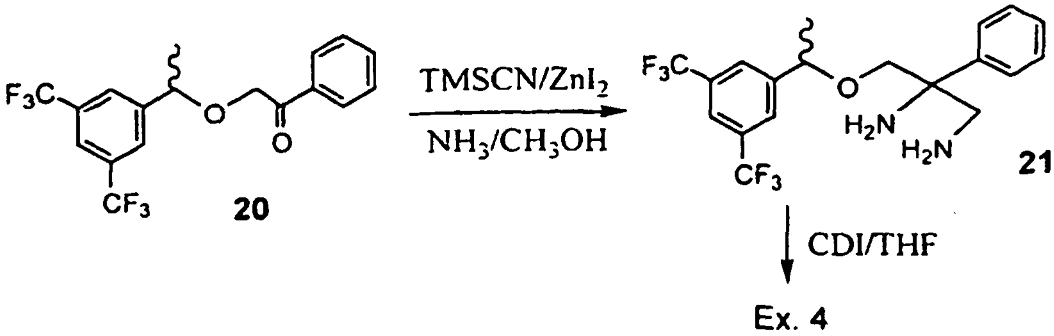


The title compound was prepared by an analogous method to that described for Examples 1 and 3 using α-methyl-3,5 -bis(trifluoromethyl)-benzyl alcohol in place of 3,5-bis(trifluoromethyl)benzyl alcohol. Two sets of diastereomers were isolated by flash silica chromatography at compound **21** stage and cyclized separately to the title compound with CDI reagent. Diastereomer A of the title compound, FAB MS $[M+1]^+$ 433.1358, calculated $[M+1]^+$ 433.1351; diastereomer B of the title compound, FAB MS $[M+1]^+$ 433.1358, calculated $[M+1]^+$ 433.1351.

Example 5

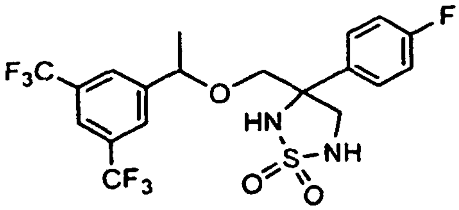

To a solution of compound **19** (0.22 g, 0.52 mmol) (prepared from compound **18** in Example 3) in pyridine (2 ml), sulfamide (50 mg, 0.52 mmol) was added. The mixture was refluxed for 24 h. After cooling, the reaction was diluted with EtOAc (200 ml), washed with 0.5 N HCl, brine, dried (MgSO4), filtered and concentrated to give a gum which was further purified on silica gel (1:4 EtOAc/hexane) to give the title compound as an off-white solid (40 mg, 16% yield). FAB MS $[M+1]^+$ 487.3.

Example 6

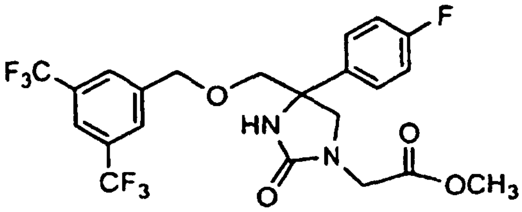

A mixture of the product of Example 2 (0.42 g, 0.96 mmol), toluene (5 ml), $CH_3ONa$ (0.13 g, 2.3 mmol) and

$Bu_4NHSO_4$ (3.3 mg, 0.01 mmol) was heated to 90 °C for 1h. Methyl bromoacetate (0.35 g, 2.3 mmol) was added later. After stirring at 80 °C for 48 h, the reaction was cooled to RT and product was extracted from the aqueous layer with $CH_2Cl_2$ (50 ml, 3x), washed with brine, and the organic layer was dried ($MgSO_4$), filtered and concentrated to give a cloudy gum (0.4 g) which was further chromatographed with 3% $NH_3$-$CH_3OH$ (1:9) / 97% $CH_2Cl_2$ to give pure title compound as a solid (35 mg, 0.07 mmol, 8% yield). Electrospray MS $[M+1]^+$ 509.1.

Example 7

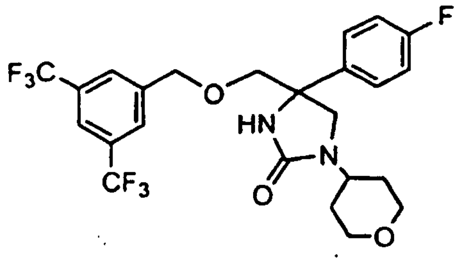

Step 1:

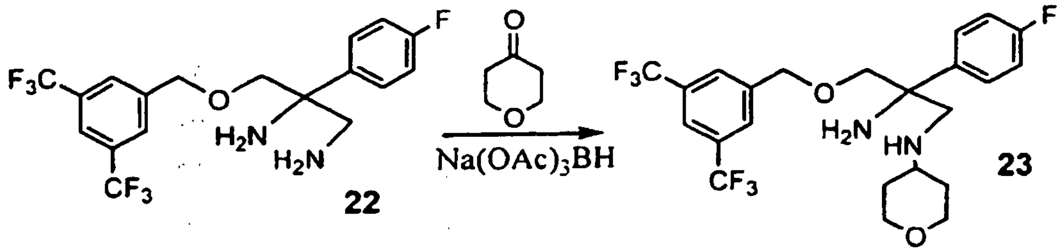


To the mixture of **22** (0.205 g, 0.5 mmol) (prepared analogously to the procedures described for **6** in Example 1) in dichloroethane (4 ml) were added tetrahydro-4H-pyranone (50 mg, 0.5 mmol) and sodium triacetoxy-borohydride (0.21 mg, 1 mmol). After stirring overnight at RT, the reaction mixture was worked-up. The crude product was further purified on silica gel, eluting with 3.5% [1:9] $NH_3$-$CH_3OH$ in 96.5% $CH_2Cl_2$. Compound **23** was obtained as an oil (0.21g, 0.43 mmol, 84% yield). FAB MS $[M+1]^+$ 495.45.

Step 2:

A mixture of **23** (0.15 g, 0.303 mmol) and CDI (120 mg, 0.74 mmol) in THF (5 ml) was stirred under a $N_2$ atmosphere at RT overnight. After work-up, a gummy material was obtained as a crude product which was then purified by chromatography, eluting with 2% $NH_3$-$CH_3OH$ (1:9) / 98% $CH_2Cl_2$ to give the title compound as a white foam (0.11 g, 0.22 mmol, 73%). FAB MS $[M+1]^+$ 521.2.

Example 8 (Preparative)

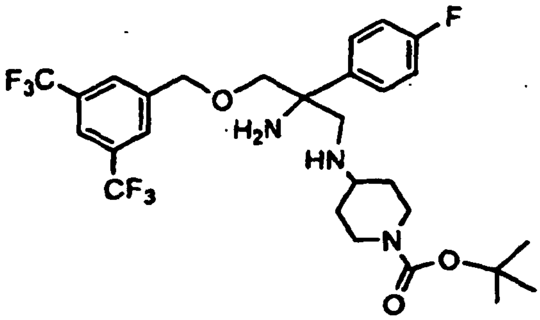

24

The title compound **24** was prepared by an analogous method to that described for compound **23** in Example 7 using 1-t-butoxylcarbonyl-4-piperidone in place of tetrahydro-4H-pyranone. FAB MS $[M+1]^+$ 594.1.

Example 9

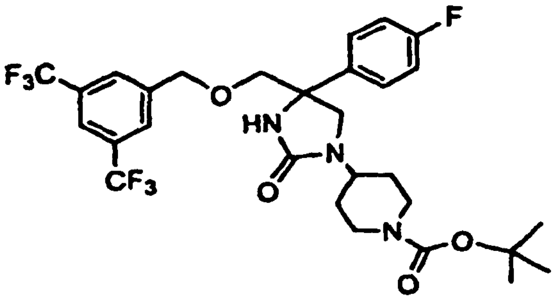

The title compound was prepared from compound **24** by an analogous method to that described for the title compound of Example 7. FAB MS $[M+1]^+$ 620.2.

Example 10

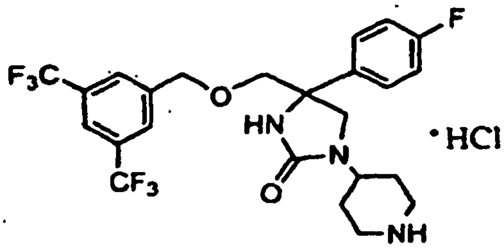

A solution of the product of Example 9 (95 mg. 0.153 mmol) in $CH_2Cl_2$ (1 ml) was treated with 4M HCl in dioxane (2 ml) for 2 h. The reaction was worked-up by evaporating solvents and excess HCl. The title compound was obtained as an off-white salt.
FAB MS $[M+1]^+$ 520.3.

Example 11

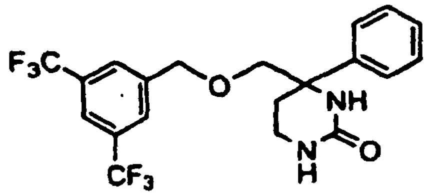

Step 1:

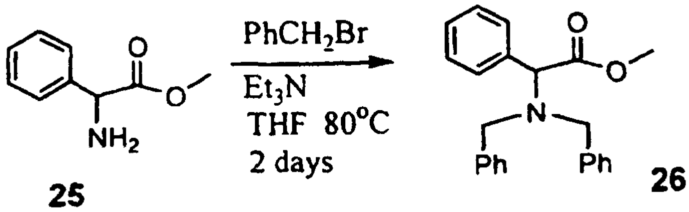


To a suspension of (R)-(-)-2-phenylglycine methyl ester hydrochloride salt **25** (10 g, 49.6 mmol) in anhydrous THF (30 ml) and anhydrous DMF (10 ml) at RT were added $Et_3N$ (22.13 ml, 158.9 mmol) and benzyl bromide (14.74 ml, 123.9 mmol). After 5 h of heating, the reaction mixture was treated with another equivalent of benzyl bromide (5.89 ml, 49.6 mmol) and $Et_3N$ (6.9 ml, 49.6 mmol). The solution was heated at 80 °C under a $N_2$ atmosphere overnight. Additional benzyl bromide (6.9 ml, 49.6 mmol) was added next day and heating continued at 80 °C overnight to complete the reaction. After completion, the reaction mixture was cooled to RT, and poured into a separatory funnel containing $NaHCO_3$ solution and EtOAc. The aqueous layer was extracted with EtOAc (200 ml x 3). The combined organic layer was washed with brine (300 ml), dried over $MgSO_4$, filtered and concentrated. Crude product was purified by chromatography (3% EtOAc in hexane) to give 26 as an oil (8.36 g, 22.62 mmol, 46 % of yield). Electrospray MS $[M+1]^+$ 346.1.

Step 2:

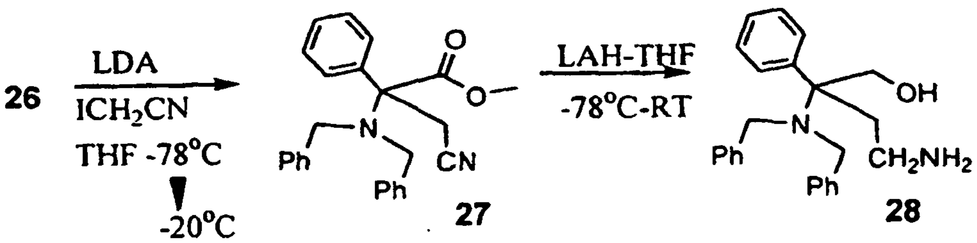


To a cooled solution of **26** (18 g, 52.11 mmol) in anhydrous THF (125 ml) at - 78 °C, a 2M LDA (32.6 ml, 65.2 mmol) solution in THF/n-heptane was added slowly. The reaction mixture was kept at this low temperature for another 2h under a $N_2$ atmosphere, then treated with 95% $ICH_2CN$ (4.97 ml, 65.2 mmol) dropwise through a syringe. The reaction was stirred at -78 °C for 4h and -20 °C overnight. A mixture of chilled saturated aqueous $NH_4Cl$ solution was added to quench the reaction. The separated aqueous layer was extracted with EtOAc (200 ml x 3). The combined organic layers were washed with brine (300 ml), dried over $MgSO_4$, filtered and concentrated. The residue was purified by flash chromatography (15% EtOAc in hexane) to afford **27** as an oil (10.24 g, 51.2%). Electrospray MS $[M+1]^+$ 385.1.

To a solution of **27** (6.0 g, 15.6 mmol) in anhydrous THF (100 ml) was added 1 M LAH in THF (100ml, 100 mmol) slowly at -78 °C under a $N_2$ atmosphere. The reaction mixture was let to stir from -78 °C to RT overnight. After completion, the reaction was diluted with THF (100 ml) and quenched (cooled in an ice-bath) with a $Na_2SO_4$ saturated aqueous solution slowly through a dropping funnel. The mixture was stirred at RT for 1h, then filtered and concentrated. The crude product was triturated with $CH_3OH$ and EtOAc and filtered. The solid (2.24 g) was the desired pure compound. The filtrate was further purified by flash grade silica gel, eluting with 6% $NH_4OH$-$CH_3OH$ (1:9) / 94 % $CH_2Cl_2$ to give compound **28** (1.3 g) as a solid with a total yield of 64 %.

Step 3:

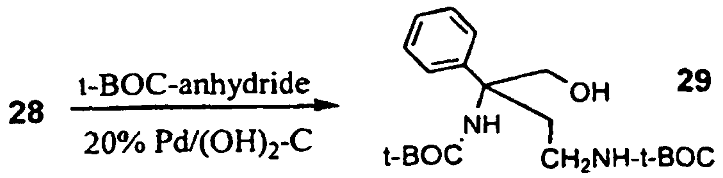


To a solution of 28 (1.27 g, 3.523 mmol) in $CH_3OH$ (150 ml) was added t-BOC anhydride (1.8 g, 8.24 mmol) and 20% $Pd(OH)_2$ on carbon (0.254 g). The mixture was hydrogenolyzed at 50 psi in a Parr Shaker overnight. After completion, excess catalyst was filtered and rinsed with $CH_3OH$. Solvent was concentrated and gave the crude product **29**, which was further purified on flash grade of silica gel, eluting with 5% $NH_3$-$CH_3OH$ (1:9) in 95% $CH_2Cl_2$ to give **29** as a white solid (1.14 g, 2.99 mmol) with a 85 % of yield. Electrospray MS $[M+1]^+$ 381.1.

Step 4:

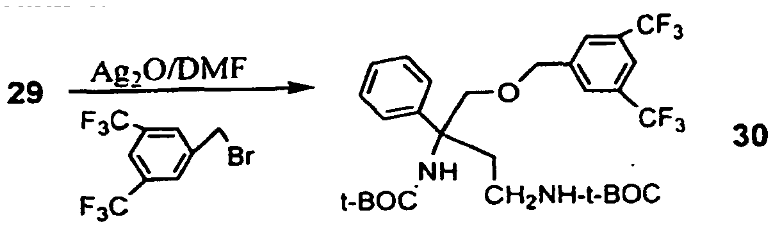


To a solution of **29** (2.4 g, 6.306 mmol) (from another batch) in anhydrous DMF (20 ml) was added 4 Å molecular sieves (1 g), 3,5-bis(trifluoromethyl)benzyl bromide (1.736 ml, 9.459 mmol) and $Ag_2O$ (2.998 g, 12.61 mmol) under a $N_2$ atmosphere. The reaction was stirred at RT in the dark overnight. EtOAc (300 ml) was added, and the mixture was washed with brine (100 ml x 2), dried over $MgSO_4$, filtered and evaporated to give **30** as a yellow oil. It was purified by chromatography, eluting with 0.75% $NH_3$-$CH_3OH$ (1:9) / 99.5 % $CH_2Cl_2$ to give 30 as a solid (2.4 g, 3.95 mmol, 63% yield). Electrospray MS $[M+1]^+$ 607.1.

Step 5:

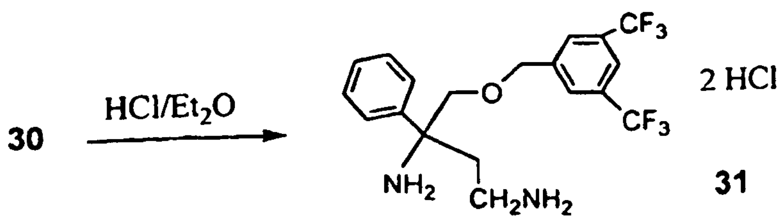


To a solution of **30** (1.38 g, 2.27 mmol) in $CH_2Cl_2$ (8.5 ml) was added 2N HCl /$Et_2O$ (8.5 ml, 17 mmol). The solution was stirred at RT overnight under a $N_2$ atmosphere. After the reaction was complete, all solvents were evaporated to give compound **31** as a solid. Crude yield: 1.08 g, 99.6%.

Step 6:

To a suspension of **31** (1.07 g, 2.233 mmol) in anhydrous THF (30 ml) was added 4 Å molecular sieves (1.0 g), $Et_3N$ (0.653 ml, 4.69 mmol) and CDI (0.434 g, 2.679 mmol) at 0 °C. The reaction mixture was stirred at ambient

temperature overnight under a $N_2$ atmosphere. After completion, the reaction mixture was evaporated and the residue was partitioned between EtOAc and water (300 ml). The organic layer was combined and washed with brine (50 ml x 3), dried over $MgSO_4$, filtered and concentrated to give a clear oil of the title compound. The crude product was further purified on flash grade silica gel, eluting with 5% $NH_4$ OH-MeOH (1:9) / 95 % $CH_2Cl_2$ to give the title compound as a solid (0.46 g, 1.06 mmol) in a 48% of yield. Electrospray MS $[M+1]^+$ 433.1.

Example 12

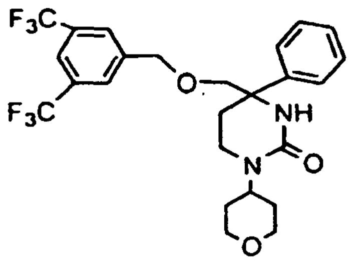

Step 1:

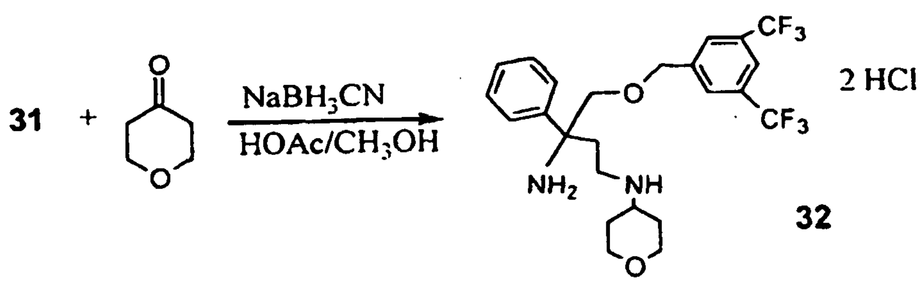


To a cooled solution of **31** (0.5 g, 1.044 mmol) in $CH_3OH$ (14.65 ml) was added $Et_3N$ (0.29 ml, 2.088 mmol), $Na_2SO_4$ (200 mg) and tetrahydro-4H-pyran-4-one (98 μl, 1.044 mmol) at 0 °C under $N_2$ atmosphere. After 1 h, $NaBH_3CN$ (103.5 mg, 1.566 mmol) and HOAc (125.6 μl, 2.088 mmol) were added. The reaction was stirred at 0 °C for 3 h and 1 N NaOH was added until solution reached pH=10. The volatile solvent was evaporated *in vacuo.* The residue was redissolved in $CH_2Cl_2$ (300 ml) and washed with aqueous mixture of saturated $NaHCO_3$-NaCl-$H_2O$. The combined organic layer was dried over $MgSO_4$, filtered and evaporated to give 32 as an oil. The crude product was passed through 50 g of flash grade silica gel, eluting with 5% $NH_3$-$CH_3OH$ (1:9) / 95 % $CH_2Cl_2$ to give **32** as an oil (0.28 g, 0.57 mmol, 55%). Part of this material (100 mg) was redisolved in $CH_2Cl_2$ and two equivalents of HCl-ether solution was added. After stirring for 10 min, solvents were evaporated to give HCl salt of compound **32** as a solid. Electrospray MS $[M+1]^+$ 491.1.

Step 2:

To a solution of **32** (180 mg, 0.367 mmol) in anhydrous THF (7.0 ml) was added 4Å molecular sieves (300 mg) and CDI (71.4 mg, 0.44 mmol). The reaction mixture was stirred at RT for over 50 h. A small amount of $NaHCO_3$ solution was added to quench the reaction. The cloudy mixture was diluted with EtOAc (200 ml). The organic layer was washed with brine (50 ml x 3), dried over $MgSO_4$, filtered and concentrated. The crude product was purified on 30 g of flash grade silica gel, eluting with 5% $NH_3$-$CH_3OH$ (1:9) / 95 % $CH_2Cl_2$ to give the title compound as a solid (0.118 g, 0.23 mmol, 63%). FAB MS $[M+1]^+$ 517.1.

Example 13

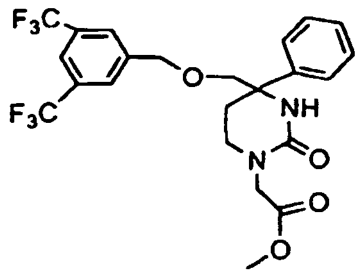

To a cooled solution of the product of Example 11 (0.36 g, 0.832 mmol) in anhydrous DMF (2 ml) at 0 °C was added 60% NaH (40 mg, 0.998 mmol) in mineral oil. After stirring 30 min, methylbromo acetate (88 μl, 0.915 mmol) was added. The reaction was stirred at 0 °C to RT overnight under a $N_2$ atmosphere. EtOAc (300 ml) was added and the mixture was washed with brine (100 ml × 3), dried over $MgSO_4$, filtered and concentrated to give a crude material. It was separated on flash grade silica gel (50 g), eluting with 5% $NH_3$-$CH_3OH$ (1:9) in 95% $CH_2Cl_2$ to give the title compound as a gummy oil (15 mg, 0.029 mmol, 3.5%). Electrospray MS $[M+1]^+$ 505.1.

Example 14

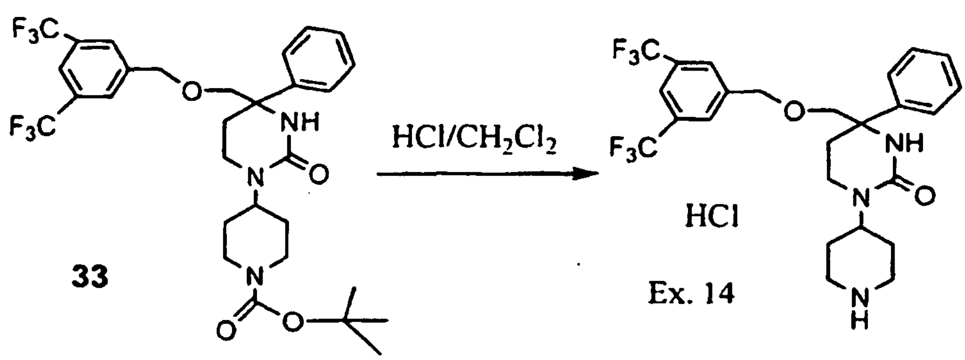


33

Ex. 14

Compound **33** was prepared by an analogous method to that described in Examples 8 and 9 using compound **31** as starting material in place of compound **22.** Electrospray MS $[M+1]^+$ 616.1.

To a solution of **33** (328 mg, 0.533 mmol) in $CH_2Cl_2$ (2.66 ml) was added 2N HCl-$Et_2O$ (2.66 ml, 5.33 mmol). The reaction mixture was stirred at RT for 18h under a $N_2$ atmosphere. Crude title compound was obtained by evaporating off the solvents and used directly in the following step. FAB MS $[M+1]^+$ 516.1.

Example 15

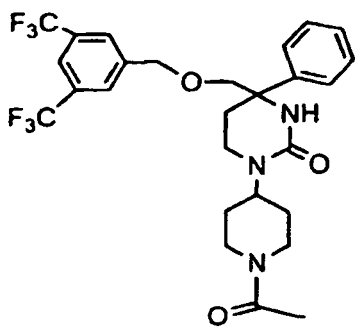

To a solution of the product of Example **14** (110 mg, 0.199 mmol) in anhydrous $CH_2Cl_2$ (2.2 ml) was added $Et_3N$ (61 μl, 0.438 mmol), followed by the addition of HOAc (12 μl, 0.2 mmol), HOBT (27 mg, 0.2 mmol) and DEC (46

mg, 0.24 mmol). The reaction mixture was stirred at RT for 3 h under a $N_2$ atmosphere. After completion, $CH_2Cl_2$ (200 ml) was added and the reaction mixture was washed with brine (50 ml x 2), dried over $MgSO_4$, filtered and evaporated to give the title compound as a foam. The crude material was purified on flash grade silica gel, eluting with 5 % $NH_4OH$ / $CH_3OH$ (1:9) / 95 % $CH_2Cl_2$ to afford the title compound as a white solid (90 mg, 0.16 mmol, 81%). Electrospray MS $[M+1]^+$ 558.1.

Example 16

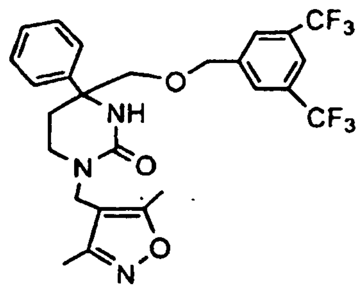

The title compound was prepared by an analogous method to that described in Example 13 using 4-chloromethyl-3, 5-dimethyl-isoxazole in place of methylbromo acetate and 60% NaH in mineral oil (1.5 equivalent). Electrospray MS $[M+1]^+$542.1.

Example 17

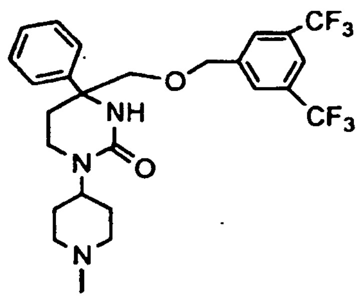

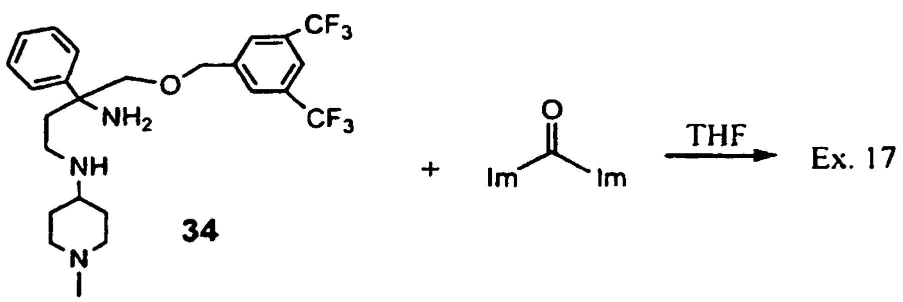


34

The title compound and compound 34 were prepared by a method analogous to that described in Examples 12 using 1-methyl-4-piperidone in place of tetrahydro-4H-pyran-4-one. Electrospray MS $[M+1]^+$ 530.1.

Example 18

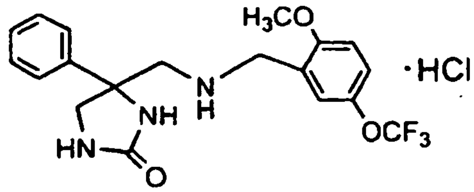

Step 1:

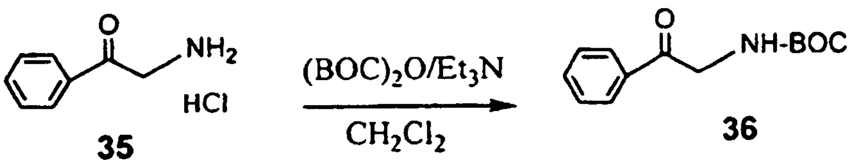

To a solution of **35** (14.8 g, 86.23 mmol) and $Et_3N$ (36 ml, 258.69 mmol) in $CH_2Cl_2$ (200 ml), $(BOC)_2O$ (20.70 g, 94.85 mmol) was added at RT. Stirred for 18 h, the reaction was stopped by diluting with $CH_2Cl_2$ (100 ml) and transferred into a separatory funnel. The organic mixture was washed with water (100 ml x 2), dried over $Na_2SO_4$, filtered, and concentrated to give a light yellow solid **36** (20 g, 85 mmol, 99%). This crude product was used without purification in the following step.

Step 2:

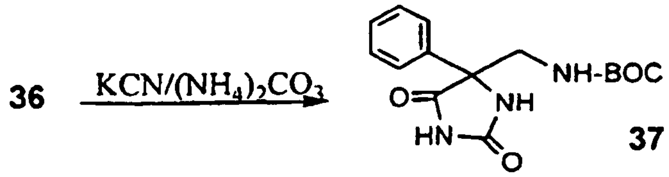

In a flask containing **36** (20.28 g, 86.195 mmol), $(NH_4)_2CO_3$ (28.99 g, 301.68 mmol) and KCN (8.42 g, 129.29 mmol) was added a (1:1) mixture of EtOH and water (180 ml). The mixture was heated to 56 °C under $N_2$ atmosphere for 48 h. The reaction was cooled with an ice bath and then the precipitate was filtered off. The residue was rinsed with water, then hexane, and air dried to give **37** as a white solid (20 g). The filtrate was diluted with EtOAc (500 ml) and the organic layer was separated, dried over $Na_2SO_4$, filtered and concentrated. The solid was triturated with $Et_2O$ then $CH_2Cl_2$, and dried in air to give additional **37** (4.8 g) as a solid (total 24.8 g, 81.2 mmol, 94%).

Step 3:

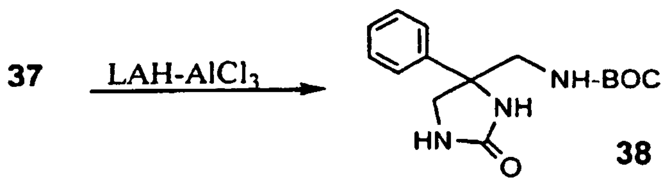

To a 3-neck flask equipped with a $N_2$ inlet was added $AlCl_3$ (10.48 g, 78.60 mmol). An ice-bath was placed under the flask. A solution of 1 M LAH in $Et_2O$ (59 ml, 59 mmol) was added dropwise to the cooled flask. After 10 min, a solution of hydantoin **37** (6 g, 19.65 mmol) in anhydrous THF (100 ml) was added to the reaction mixture slowly. The reaction was kept at 0 °C for additional 15 min then gradually warmed up to RT overnight. In a cooling bath, the reaction was carefully quenched with water (3 ml), followed by 15% NaOH (3 ml) and water (9 ml) under an atmosphere of $N_2$. Stirred for 15 min, the reaction mixture was filtered and rinsed with EtOAc, the THF. The filtrate was transferred to a separatory funnel containing water (50 ml). The organic layer was separated, dried over $Na_2SO_4$, filtered, and concentrated to give **38** as a white solid (4.5 g, 15.44 mmol, 79%). The crude product was used without further purification. FAB MS $[M+1]^+$ 292.1.

Step 4:

**38**
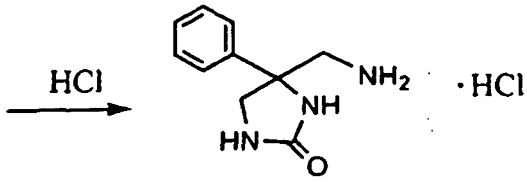

**39**

To a solution of **38** (0.9 g, 3.09 mmol) in $CH_3OH$ (20 ml), a 4 N HCl (8 ml, 31 mmol) solution in dioxane was added. After stirring at RT for 2 h, the reaction was stopped by evaporating off the volatile solvents. $CH_3OH$ was added twice with subsequent evaporation. A white foam (0.65 g, 2.85 mmol, 92 % yield) was obtained as crude product, which was used directly in the following step.

Step 5:

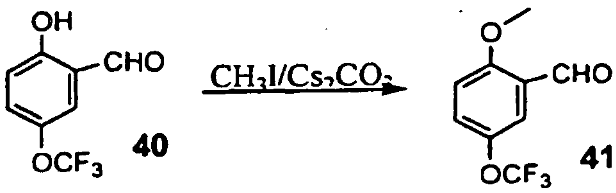

**40** **41**

To a solution of alcohol **40** (0.5 g, 2.43 mmol) in dioxane (2.5 ml), water (0.15 ml) was added, followed by $Cs_2CO_3$ (3.96 g, 12.15 mmol) and $CH_3I$ (0.45 ml, 7.29 mmol). The mixture was heated to 72 °C in a sealed tube for 3 h. The reaction mixture was then cooled to RT and concentrated. The residue was flashed through silica gel, eluting with 40 % EtOAc/ hexane. Compound **41** was obtained as an oil (0.52 g, 2.36 mmol, 97 % yield).

Step 6:

To a solution of **39** (0.28 g, 1.23 mmol) in (9:1) of trifluoroethanol / $Et_3N$ (10 ml), aldehyde **41** (0.27 g, 1.23 mmol) was added, followed by 3 Å molecular sieves (1.5 g) and sodium triacetoxyborohydride (0.91 g, 4.31 mmol). This turbid reaction was made clear by adding dichloroethane (15 ml). After 18 h, the reaction mixture was filtered and rinsed with EtOAc (200 ml) and $CH_3OH$ (200 ml). The combined filtrate was concentrated. The residue was redissolved in EtOAc (250 ml) and washed with saturated $NaHCO_3$ (100 ml). The organic layer was dried over $Na_2SO_4$, filtered and concentrated. The crude material was purified on flash silica gel, eluting with EtOAc /$Et_3N$ (9:1) to give the title compound as an oil (0.3 g, 0.76 mmol, 62 % yield), FAB MS $[M+1]^+$ 396.1. The compound was treated with one equivalent of 1 N HCl-$Et_2O$ solution and isolated as a HCl salt.

Example 19

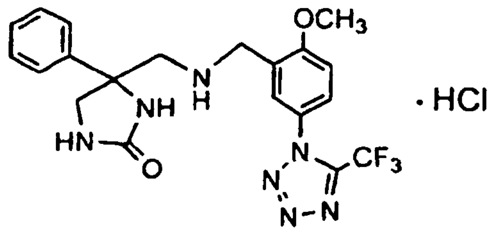

Step 1:

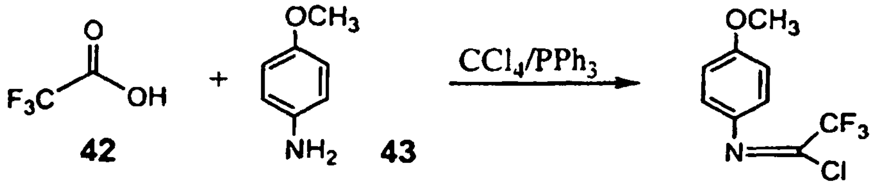


42 43 44

To a solution of triphenyl phosphine (99.5 g, 379.43 mmol) in $CCl_4$ (51 ml), $Et_3N$ (16.98 ml, 121.80 mmol) and trifluoro acetic acid, **42,** (7.8 ml, 101.08 mmol) were added. After stirring at 0 °C for 10 min, a solution of p- anisidine **43** (15 g, 121.80 mmol) in $CCl_4$ (51 ml) was added. The reaction mixture was refluxed for 3 h. Cooled down to RT, the reaction mixture was filtered and rinsed with hexane until there was no yellow color filtered off. The filtrate was concentrated and the brown residue was distilled at 110 °C to give the desired product **44** (17 g, 71.55 mmol, 59 % yield) as a yellow oil.

Step 2:

44

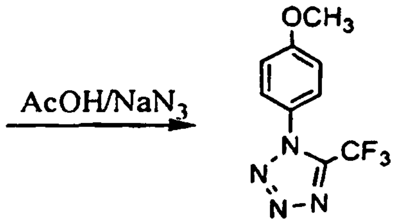


45

To a solution of compound **44** (7 g, 29.46 mmol) in AcOH (110 ml) was added $NaN_3$ (6.13 g, 94.27 mmol). The reaction mixture was heated at 70 °C overnight. After cooling to RT, the reaction mixture was filtered and the filtrate was diluted with $CH_2Cl_2$ (200 ml) and washed with saturated $NaHCO_3$ (100 ml) solution, water (100 ml) and dried over $Na_2SO_4$, filtered and concentrated. Compound **45** (7 g, 28.67 mmol, 98 % yield) was obtained and used directly without further purification.

Step 3:

45 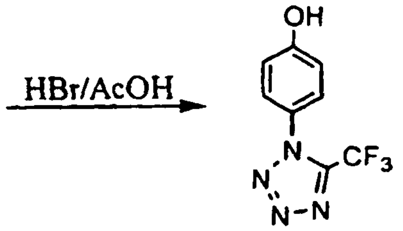

46

To the flask containing compound **45** (15 g, 61.43 mmol) and AcOH (100 ml) was added 48% aqueous HBr (100 ml). The reaction mixture was heated to 100 °C for 48 h. After cooling to RT, the reaction mixture was concentrated to half of its volume on the rotary evaporator and extracted with EtOAc (200 ml x 2). The combined organic layer was washed with water (150 ml x 3), dried over $Na_2SO_4$, filtered and concentrated to give **46** (8.0 g, 34.76 mmol, 57 % yield) which was used without further purification.

Step 4:

46 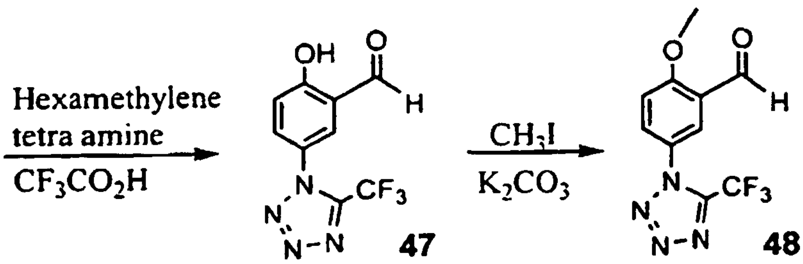

47 48

To the solution of phenol **46** (4 g, 17.39 mmol) in TFA (40 ml) was added hexamethylene tetramine (11.2 g, 79.89 mmol). The reaction mixture was heated at 70 °C under a $N_2$ atmosphere for 48 h. Cooled to RT, the reaction mixture was poured into 2 N $H_2SO_4$ (100 ml). This mixture was then extracted with EtOAc (200 ml, 2x), dried over $Na_2SO_4$, filtered and concentrated to give compound **47** (3.4 g, 13.17 mmol, 76 % yield) as a crude product which was used in the next step directly.

Compound **47** (3.4 g, 13.17 mmol) was dissolved in anhydrous DMF (20 ml). To this solution was added $K_2CO_3$ (3.6 g, 26.34 mmol) and $CH_3I$ (1.7 ml, 26.34 mmol). The reaction mixture was stirred at RT for 3 h and then poured into a separatory funnel containing water (125 ml) and EtOAc (250 ml). The organic layer was washed with water (100 ml x 2), brine (100 ml) and concentrated. The crude product was purified by flash silica gel, eluting with hexane/EtOAc (9:1) to give **48** as a white solid (3 g, 1.02 mmol, 84%)

Step 5:

To a solution of hydrochloride salt of **39** (0.25 g, 1.098 mmol), a (9:1) mixture of trifluoroethanol/$Et_3N$ (20 ml) and aldehyde **48** (0.3 g, 1.098 mmol) was added followed by 3Å molecular sieves (1.5 g). The reaction mixture was stirred under $N_2$ protection for 0.5 h before sodium triacetoxy borohydride (0.81 g, 3.83 mmol) was added. After 18 h of stirring, the reaction was filtered and rinsed with a (1:1) mixture of $CH_3OH$/EtOAc (200 ml). The filtrate was concentrated and purified with flash grade silica gel, eluting with EtOAc/$Et_3N$ (9:1) to give the title compound (0.3g, 0.67 mmol, 61% yield) FAB MS $[M+1]^+$ 448.1. The compound was treated with one equivalent of 1N HCl-$Et_2O$ solution and isolated as a HCl salt.

Example 20

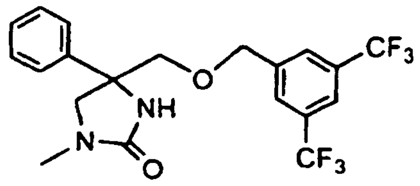

Step 1:

7

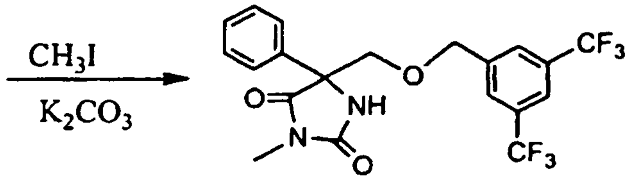


49

To the solution of hydantoin compound 7 (0.1 g, 0.23 mmol) (Example 1, method 2) in anhydrous DMF (1 ml), $K_2CO_3$ (45 mg, 0.322 mmol) was added. After stirring at RT for 25 min, $CH_3I$ (29 μl, 0.46 mmol) was added. After stirring at RT overnight, the reaction mixture was quenched by pouring into EtOAc (100ml). The organic layer was washed with a saturated $K_2CO_3$ (100 ml x 2) solution, dried over $Na_2SO_4$, filtered and concentrated. The crude product was purified by flash chromatography (EtOAc/hexane 1:4) to give compound **49** as a solid (67 mg, 0.17 mmol, 74 % yield). FAB MS $[M+1]^+$ 447.1.

Step 2:

To a 2-neck flask containing $AlCl_3$ (0.37 g, 2.77 mmol), 1M LAH(2.07 ml, 2.07 mmol) in $Et_2O$ was added slowly. Stirred for a few minutes, a solution of hydantoin **49** (0.31 g, 0.69 mmol) in anhydrous $Et_2O$ (5 ml) was added to the white suspension.
The reaction mixture was left at RT for 48 h and quenched by adding water (1 ml), dropwise, followed by 15% NaOH (1 ml). The white precipitate was filtered and rinsed with EtOAc. The filtrate was washed with brine (50 ml), dried over $Na_2SO_4$, filtered and concentrated. The crude product was purified by flash chromatography (2% $CH_3OH$ in $CH_2Cl_2$) and the title compound was isolated as a solid (0.22 g, 0.51 mmol, 74 % yield). FAB MS $[M+1]^+$433.1.

Example 21

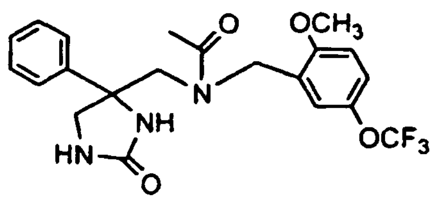

To the mixture of the compound of Example 18 (0.05 g, 0.126 mmol) in $CH_2Cl_2$ (2 ml), $Et_3N$ (35 μl, 0.252 mmol) and $CH_3COCl$ (18 μl, 0.252 mmol) were added. The reaction mixture was left at RT overnight and flashed through silica gel, eluting with EtOAc/$Et_3N$ (9:1), to give the title compound as a foam (35 mg, 0.075 mmol, 60 % yield).
FAB MS $[M+1]^+$438.1.

Example 22

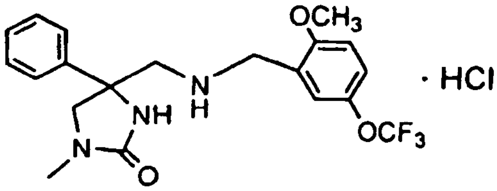


Step 1:

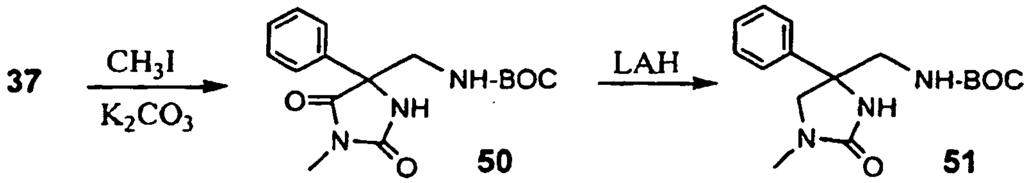


To the solution of **37** (2 g, 6.55 mmol) in anhydrous DMF (12 ml), $K_2CO_3$ (1.27 g, 9.19 mmol) was added. After stirring at RT for 0.5 h, $CH_3I$ (0.82 ml, 13.17 mmol) was added. After stirring at RT overnight, the reaction was diluted with EtOAc (150 ml) and washed with water (100 ml x 2), dried over $Na_2SO_4$, filtered, and concentrated to give a white solid as the crude **50**. The crude material was further purified on the flash grade silica gel, eluting with 20 % EtOAc in hexane to give pure **50** as a solid (1-88g, 5.89 mmol, 90 % yield).

To a cooled flask containing $AlCl_3$ (3.14 g, 23.55 mmol) at 0 °C, 1 M solution of LAH in anhydrous THF (18 ml 18 mmol) was added slowly. The resulting white slurry was treated with a solution of hydantoin 50 (1.88 g, 5.89 mmol) in anhydrous THF (40 ml). The reaction mixture was stirred at RT for 3 days. Water (2 ml) was added carefully to quench the remaining reactants, followed by 15% NaOH (2 ml) and water (6 ml). The mixture was then poured into a separatory funnel containing EtOAc (200 ml), and the organic layer was washed with water (100 ml x 2), dried over $Na_2SO_4$, filtered and concentrated. The crude product **51** (1.28 g, 4.19 mmol, 72 % yield) was used directly in the next step.

Step 2:

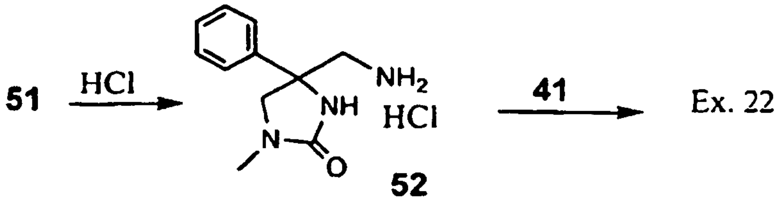


The mixture of BOC compound **51** (1.28 g, 4.19 mmol) in **4** M HCl solution of dioxane (25 ml, 100 mmol) was stirred at RT for 18 h. After completion, all solvents were evaporated off and the residue was dried by azotropical distillation with $CH_3OH$ twice. The foamy crude product **52** (0.1 g, 0.487 mmol) was dissolved in a solution of $CF_3CH_2OH/Et_3N$ 9:1 (10 ml). To this clear solution, aldehyde **41** (0.107g, 0.487 mmol) was added, followed by 3Å molecular sieves (0.7 g). After stirring at RT for 45 min, $NaBH(OAC)_3$ (0.36g, 1.7 mmol) was added. The reaction was stirred overnight at RT. After reaction was complete, the mixture was filtered and washed with $CH_3OH$ and EtOAc. Evaporation of all solvents gave the title compound as a crude product which was further purified by silica gel chromatography with EtOAc/hexane (1:1) as eluting solvents to give the title compound (80 mg, 0.195 mmol, 40 %) as an oil. FAB MS $[M+1]^+$410.1.

The compound was treated with one equivalent of 1 N HCl - $Et_2O$ solution and isolated as the HCl salt.

Example 23

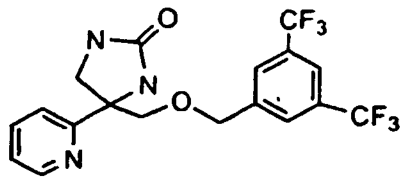

Step 1:

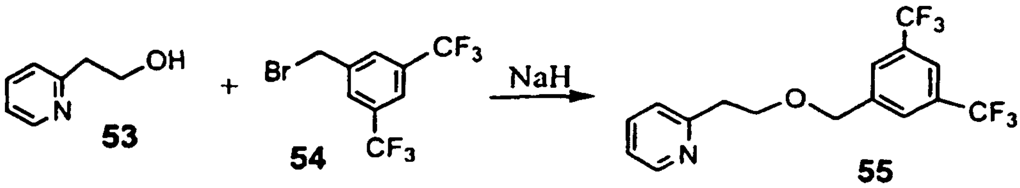


53 54 55

A solution of the alcohol **53** (10 g, 81.2 mmol) in anhydrous DMF (50 ml) was cooled to 0 °C and treated with 60% NaH (3.6 g, 89.3 mmol) in mineral oil portionwise. The cooling bath was removed after 30 min and the reaction was stirred at RT for 2 h. The reaction mixture was cooled again to 0 °C and then treated with bromide **54**. After stirring for 4 h, the reaction was quenched by pouring into EtOAc (250 ml). The organic mixture was washed with water (200 ml, x 4), brine (50 ml), dried over $Na_2SO_4$, filtered and concentrated. The crude product was purified on a Biotage 75S column, eluting with hexane/EtOAc (5:1) to give **55** (22.2 g, 63.6 mmol, 78 % yield). Electrospray MS $[M+1]^+$ 350.1.

Step 2:

55 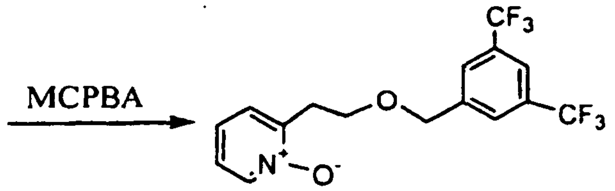

56

A solution of **55** (10 g, 28.6 mmol) in $CH_2Cl_2$ (50 ml) was treated with MCPBA (6.2 g, 28.6 mmol). After 2 h, additional 2 g of MCPBA was added abd the mixture was stirred for 6 h. The mixture was poured into EtOAc (250 ml) and washed with saturated aqueous $Na_2S_2O_3$ (200 ml), saturated aqueous $NaHCO_3$ (200 ml), and brine, dried over $Na_2SO_4$, filtered and concentrated to give the N-oxide **56** as a lightly colored solid (10.18 g, 27.9 mmol, 97.5% yield). Electrospray MS $[M+1]^+$ 366.1.

Step 3:

56 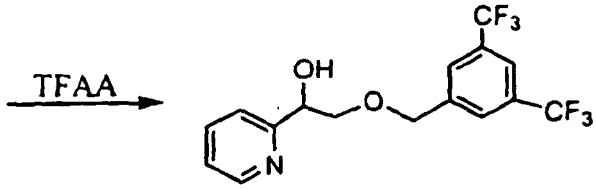

57

A mixture of N-oxide **56** (6.0 g, 16.43 mmol) and trifluoroacetic anhydride (17.3 g, 82.2 mmol) in $CH_2Cl_2$ (75 ml) was heated under reflux for 4 h and then stirred at RT overnight. The reaction mixture was concentrated *in vacuo.*

The residue was dissolved in THF (40 ml) and saturated aqueous $NaHCO_3$ was added until gas no longer evolved. EtOAc (200 ml) and brine (200 ml) were added and the organic phase was separated. The aqueous phase was extracted with EtOAc (150 ml, x 2). The combined organic layers were washed with brine (200 ml), dried over $Na_2SO_4$, filtered and concentrated to give a crude orange oil, which was further purified on the Biotage column, eluting with hexane/ EtOAc (4:1) to give **57** (4.11 g, 11.26 mmol, 68.5% yield). Electrospray MS $[M+1]^+$366.9.

Step 4:

57
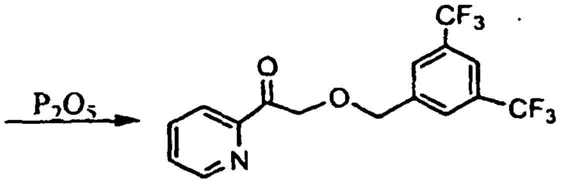

58

$CH_2Cl_2$ (100ml) was added to a solution of alcohol **57** (4.11g, 11.25 mmol) in warm DMSO (11 ml). The solution was cooled to 0°C and $P_2O_5$ (6.39 g, 22.5 mmol) was then added portionwise over 30 min, followed by addition of $Et_3N$ (6.3 ml, 45 mmol). The reaction mixture was stirred at RT for 18 h, then quenched with saturated aqueous $NaHCO_3$. The aqueous layer was extracted with EtOAc (200 ml, x 3). The combined organic phases were washed with brine (200 ml), dried over $Na_2SO_4$, filtered and concentrated. The crude product was triturated with hexane to give a light tan powder as desired product. The filtrate was further purified on the Biotage column, eluting with hexane/EtOAc (4:1) to give **58** as tan solid (2.46 g, 6.74 mmol, 60.25%). Electrospray MS $[M+1]^+$364.1.

Step 5:

58
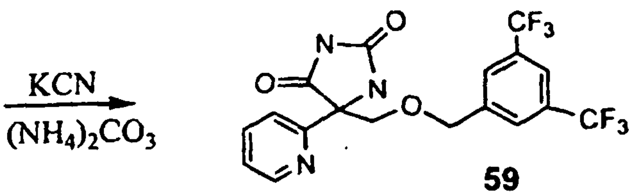

59

A suspension of ketone **58** (2.30 g, 6.30 mmol) in a mixture of (1:1) water/EtOAc (30 ml) was treated with $(NH_4)_2CO_3$ (2.12 g, 22.1 mmol) followed by KCN(0.62 g, 9.5 mmol). The solution was heated to 60 °C and stirred overnight. After cooling to RT, the reaction mixture was taken up in EtOAc (100 ml) and washed with water (100ml x 2). The separated organic layer was dried over $Na_2SO_4$, filtered and concentrated *in vacuo.* The crude product was purified on a Biotage column, eluting with hexane/EtOAc (1:1) to give the desired product **59** as a white solid (2.03 g, 4.69 mmol,. 74.5% yield). Electrospray MS $[M+1]^+$434.1.

Step 6:

To a cooled flask containing $AlCl_3$ (614 mg, 4.6 mmol) at 0 °C, a 1M solution of LAH in $Et_2O$ (3.5 ml, 3.5 mmol) was added dropwise. 10 min later, a solution of hydantoin **59** (500 mg, 1.15 mmol) in anhydrous THF (10 ml) was added slowly. After 25 min at 0 °C, the reaction was complete as determined by TLC. Water (3 ml) was added slowly to the reaction mixture, followed by 15% NaOH (3 ml) and water (9 ml). After stirring at RT for 15 min, the emulsion was filtered and rinsed with EtOAc and THF. The filtrate was washed with water (50 ml), dried over $Na_2SO_4$, filtered and concentrated. The crude product was purified on the Biotage column, eluting with EtOAc/$Et_3N$ (9:1) to give the title compound as a solid (0.229 g, 0.546 mmol, 47.5 % yield). Electrospray MS $[M+1]^+$420.1.

Example 24

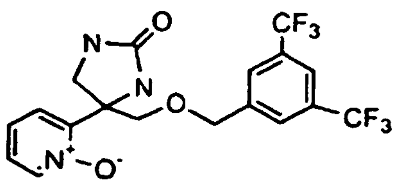

The title compound was prepared by a similar method to that described in Example 23, Step 2, using the cyclic urea, **56,** of Example 23 in place of ether **55.** Yield was 67.1%. Electrospray MS $[M+1]^+$ 436.1.

Example 25

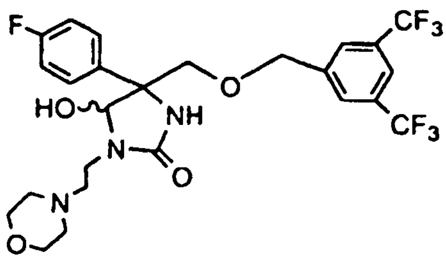

Step 1:

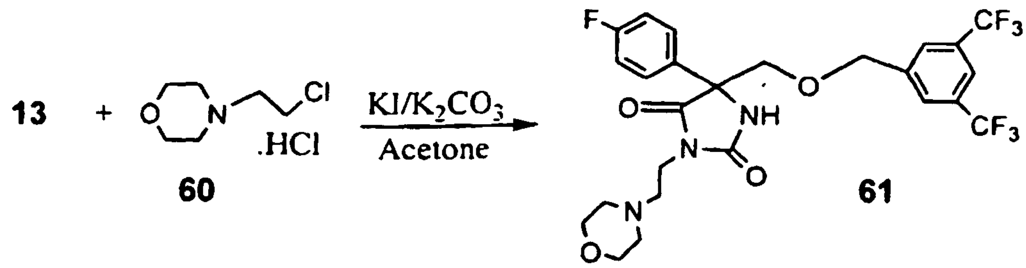


To the flask containing compound **13** (1.35 g, 3 mmol) in acetone (45.ml), 4-(2-chloroethyl)morpholine hydrochloride salt, **60**, (0.614 g, 3.3 mmol) was added, followed by KI (0.1 g, 0.6 mmol) and $K_2CO_3$ (1.24 g, 9 mmol). The reaction mixture was heated to reflux for 44 h. After cooling to RT, EtOAc (60 ml) was added and the organic mixture was washed with water (20 ml), dried over $MgSO_4$, filtered and concentrated to give 61 as an oil. The crude product was further purified on a silica gel column, eluting with 20% EtOAc/$CH_2Cl_2$to give **61** (1.3g, 2.3 mmol, 76% of yield).

Step 2:

To a cooled flask containing $AlCl_3$ (0.35 g, 2.63 mmol) at 0 °C under a $N_2$ atmosphere, 1 M solution of LAH in $Et_2O$ (2.0 ml, 2.0 mmol) was added dropwise. Stirred for 5 min, a solution of 61 (0.37 g, 0.66 mmol) in anhydrous THF (10 ml) was added slowly. The reaction mixture was allowed to stir from 0 °C to RT over the weekend and quenched with 20% NaOH (1 ml). The solids were filtered and rinsed well with $CH_2Cl_2$ after stirring for 15 min. The filtrate was concentrated and purified on flash grade silica gel, eluting with 5% $CH_3OH$ in $CH_2Cl_2$ to give the title compound as a white foam (70 mg, 0.13 mmol, 20% of yield). High Resolution MS: $[M+1]^+$ measured 566.1899, calculated 566.1890.

Example 26

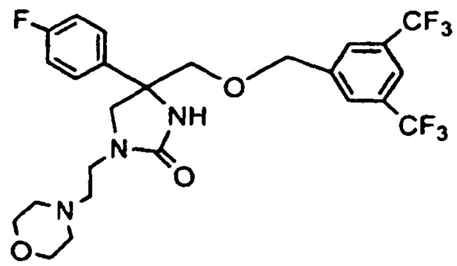

To a cooled flask containing $AlCl_3$ (1.23 g, 9.24 mmol) at 0 °C under $N_2$ atmosphere, 1 M solution of LAH in $Et_2O$ (7.0 ml, 7.0 mmol) was added dropwise. Stirred for 5 min, a solution of **61** (1.3 g, 2.3 mmol) in anhydrous THF (25 ml) was added slowly. The reaction mixture was allowed to stir at 0 °C for 15 min then heated at 70 °C for 64 h. The reaction was quenched with 20% NaOH (2 ml) and stirred at RT for 15 min. The solids were filtered and rinsed well with $CH_2Cl_2$. The filtrate was concentrated and purified on flash grade silica gel, eluting with 5% $CH_3OH$ in $CH_2Cl_2$ to give the title compound as a gummy solid (0.46g, 0.8 mmol, 36% yield). High Resolution MS: $[M+1]^+$ measured 550.1948, calculated 550.1941.

Example 27

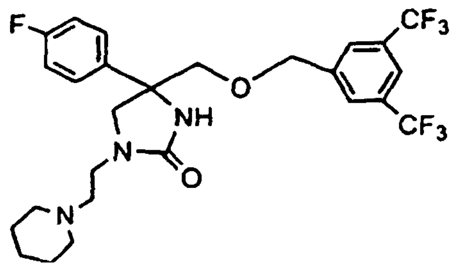

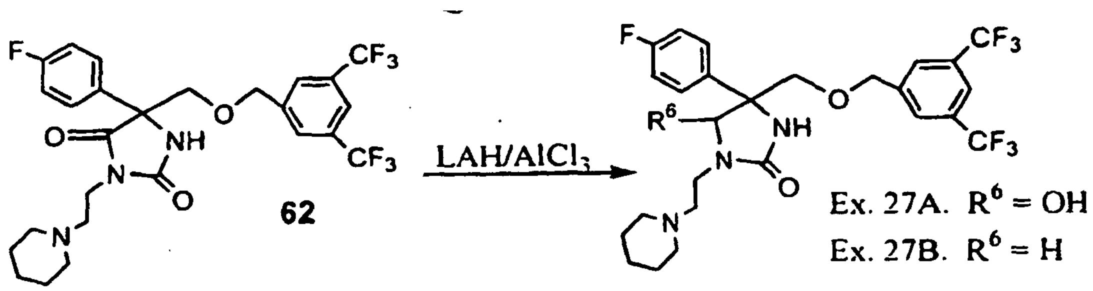


Compound **62** was prepared in a similar method as described in Example 25 using 2-chlorethyl-1-piperidine in place of 4-(2-chloroethyl)-morpholine. A mixture of Ex. 27A and Ex. 27B was separated by flash column chromatography, eluting with 2~5% $CH_3OH$ in $CH_2Cl_2$ to afford
Ex. 27A (33% yield) (FAB MS $[M+1]^+$ 564) and Ex. 27B (43% yield) FAB MS $[M+1]^+$ 549.

Example 28

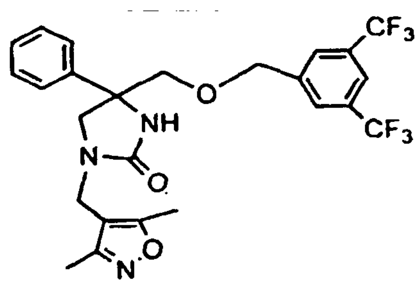

The title compound was prepared by an analogous method to that described in Example 16 using the compound of Example 1 in place of the compound of Example 11 to give the title compound in a 22 % yield.
FAB MS $[M+1]^+$ 546.3.

<u>Example 29</u>

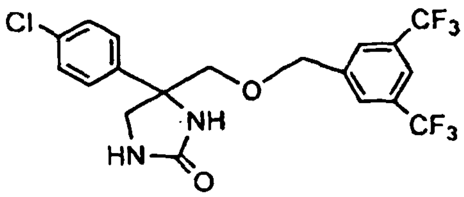

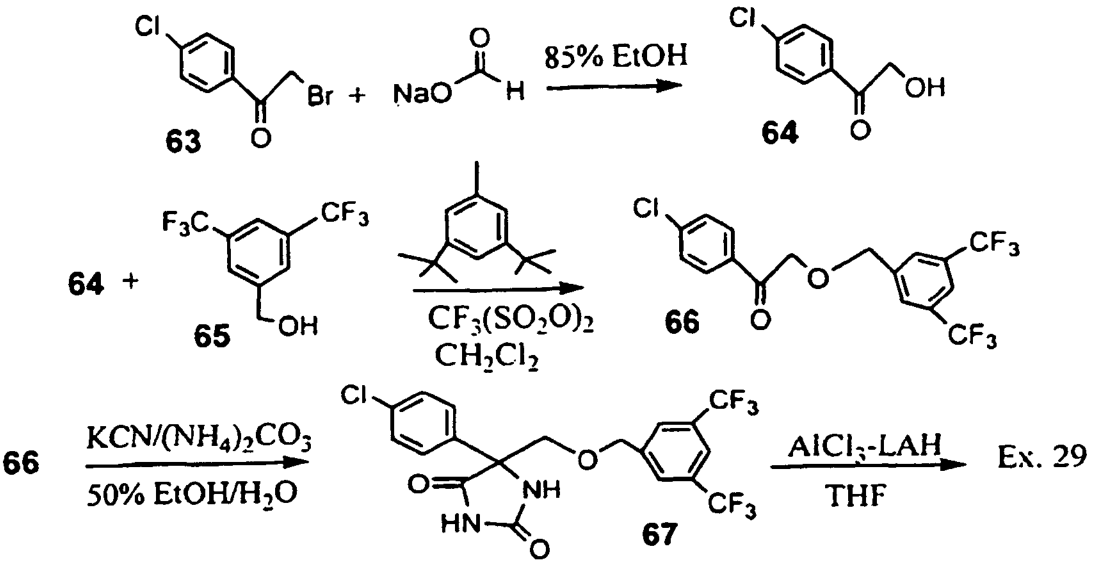


63 + → 64

64 + 65 → 66

66 → 67 → Ex. 29

The title compound was prepared by analogous methods as described for Examples 1 and 2, except for the preparation of ketone intermediate **66**. Compound **66** was prepared by treatment of compound **64** (24.1 g, 98.8 mmol) and 2, 6-di-t-butyl-4-methyl pyridine (42 g, 206 mmol) in dry $CH_2Cl_2$ (400 ml) at 0 °C with slow addition of trifluoromethyl-methane sulfonic anhydride (30 g, 107 mmol) under a $N_2$ atmosphere. After stirring at RT for 1 h, a solution of compound **64** (14 g, 82.3 mmol) in $CH_2Cl_2$ (200 ml) was added dropwise. After stirring at RT for 6 days, the solid was filtered and filtrate was washed with brine, dried ($MgSO_4$), filtered and concentrated to give a crude **66** as a brown oil. Compound **66** was obtained by purification through a flash grade silica gel, eluting with 8% EtOAc/hexane to give **66** as an oil (19.5 g, 50 mmol, 61 % of yield). Compound **67** was synthesized from compound **66** with a 60% of yield and title compound was prepared from **67** by selective reduction with $AlCl_3$-LAH in a 52 % yield. FAB MS $[M+1]^+$ 467.

Example 30

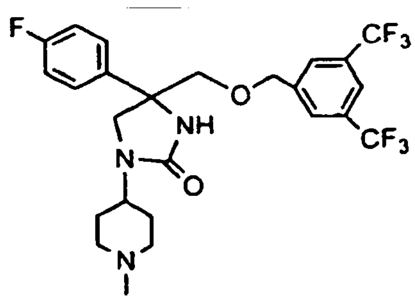

The title compound was prepared by a method analogous to that described in Example 7 by using compound 22 and replacing tetrahydro-4H-pyranone with 1-methyl-4-piperidone. HRMS $[M+1]^+$ calculated, 534.1992; found, 534.1987.

Example 31

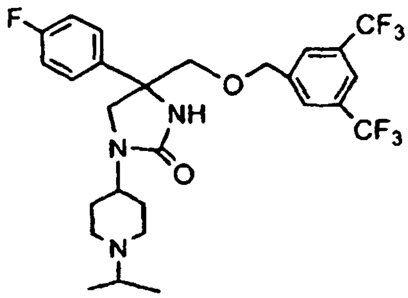

To a solution of the product of Example 10 (0.2g, 0.36 mmol) in dichloroethane (3 ml) and acetone (21 mg, 0.36 mmole) at RT was added $Na(OAc)_3BH$ (0.15g, 0.72 mmol). The mixture was stirred at RT for two days under a $N_2$ atmosphere. After completion, to the reaction was added $CH_2Cl_2$ (50 ml) and saturated $NaHCO_3$ solution, followed by routine work-up to give a crude product. Product was purified on flash grade silica gel, eluting with 3% $NH_4OH$-$CH_3OH$ (1:9) / 97 % $CH_2Cl_2$ to afford pure title compound as a solid (0.12g, 0.22 mmol) in a 62% of yield.
FAB MS $[M+1]^+$ 562.4.

Example 32

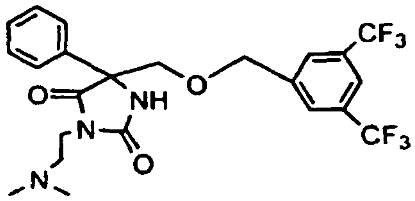

The title compound was prepared by a method analogous to that described in Example 25 for compound **61** in step 1, using compound **7** in place of compound **13** and N, N-dimethyaminoethyl chloride hydrochloride in place of 1-(2-chloro-ethyl)-morpholine hydrochloride. The title compound was obtained as a solid in a 96 % yield. Electrospray MS $[M+1]^+$ 504.1.

Example 33

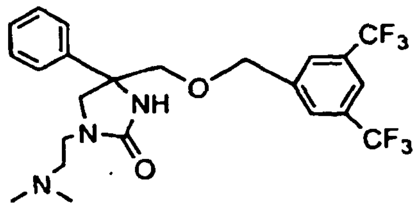

Reduction of the compound from Example 32 with LAH-$ALCl_3$ complex, analogous to the method described in Example 26, gave the title compound in a 32 % yield. Electrospray MS $[M+1]^+$ 490.1.

Example 34

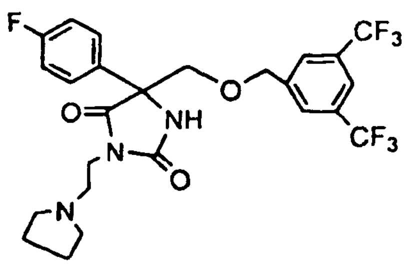

The title compound was prepared by an analogous method to that described in Example 25, Step 1, using 1-(2-chloroethyl) pyrrolidine HCl salt in place of 1-(2-chloroethyl)-morpholine hydrochloride salt. The title compound was obtained as a solid in a 77 % yield. Electrospray MS $[M+1]^+$ 548.1.

Example 35

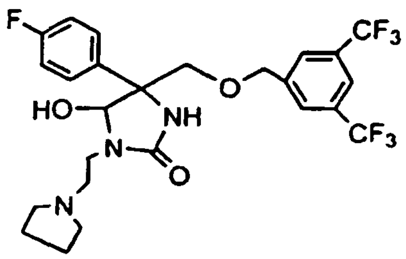

Reduction of compound the product of Example 34 with LAH-$ALCl_3$ complex as described in Example 26, heating at 70 °C for 1 day, gave the title compound in a 36% yield. Electrospray MS $[M+1]^+$ 550.1

Example 36

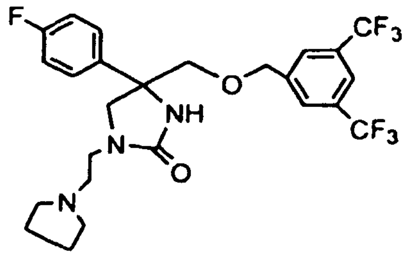

Reduction of the product of Example 34 with LAH-ALCl$_3$ complex at 70°°C for 1 day, analogous to the method as described in Example 26, gave the title compound in a 11% yield. Electrospray MS [M+1]$^+$ 534.1.

Example 37

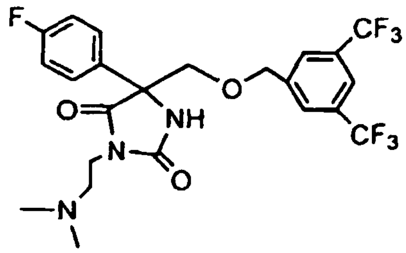

The title compound was prepared by an analogous method to that described in Example 25, Step 1, using 2-dimethylamine ethyl chloride hydrochloride salt in place of 1-(2-chloroethyl)-morpholine hydrochloride salt. The title compound was obtained as a solid in a 83 % of yield. Electrospray MS [M+1]$^+$ 522.1.

Example 38

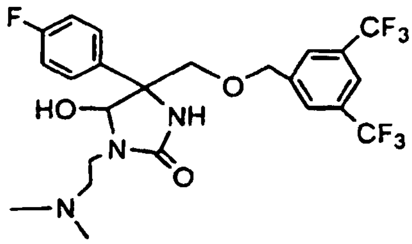

Reduction of the product of Example 37 with LAH-ALCl$_3$ complex at 70 °C for 1 day, analogous to the method described in Example 26, gave the title compound in a 26% yield. Electrospray MS [M+1]$^+$ 524.1

Example 39

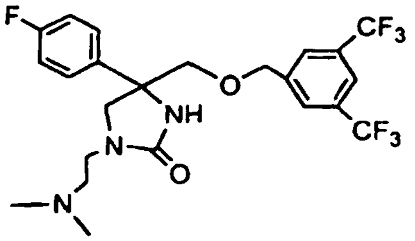

Reduction of the product of Example 38 with LAH-$ALCl_3$ complex at 70 °C for 1 day, analogous to the method described in Example 26, gave the title compound in a 15% yield. Electrospray MS $[M+1]^+$ 508.1.

Example 40

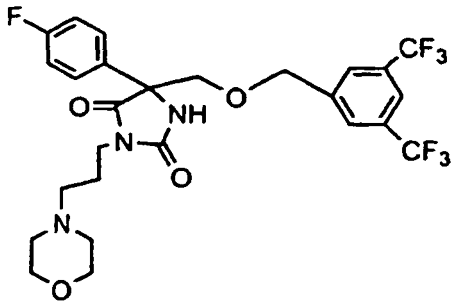

The title compound was prepared by an analogous method to that described in Example 25, Step 1, using 3-chloropropyl-morpholine in place of 1-(2-chloroethyl)-morpholine hydrochloride. The title compound was obtained as a solid in a 57 % yield. FAB MS $[M+1]^+$ 578.1.

Example 41

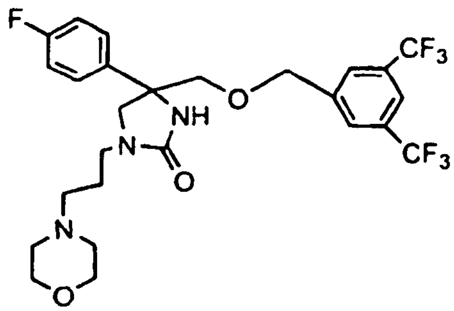

Reduction of the compound from Example 40 with LAH-$ALCl_3$ complex, analogous to the method as described in Example 26, gave the title compound in a 9 % yield. Electrospray MS $[M+1]^+$ 564.1.

Example 42

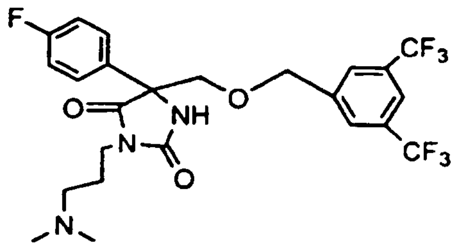

The title compound was prepared by an analogous method to that described in Example 25, Step 1, using N, N-dimethylaminopropyl chloride HCl in place of 1-(2-chloroethyl)-morpholine hydrochloride. The title compound was obtained as a solid in a 69 % yield. FAB MS $[M+1]^+$ 536.1.

Example 43

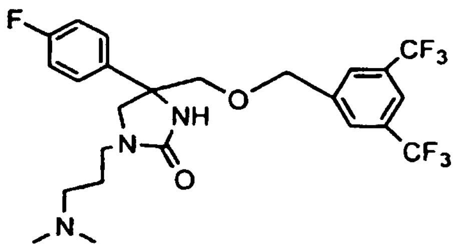

Reduction of the compound from Example 42 with LAH-$ALCl_3$ complex, analogous to the method as described in Example 26, gave the title compound in a 1.5 % yield. Electrospray MS $[M+1]^+$ 522.3.

Example 44

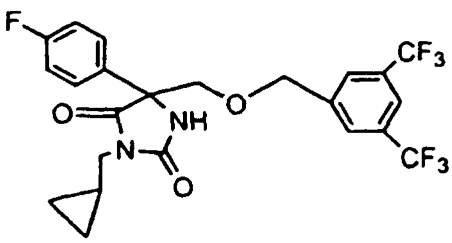

The title compound was prepared by an analogous method to that described in Example 25, Step 1, using bromomethyl cyclopropane replacing 1-(2-chloroethyl)-morpholine hydrochloride. The title compound was obtained as a solid in a 94 % of yield. FAB MS $[M+1]^+$ 505.1.

Example 45

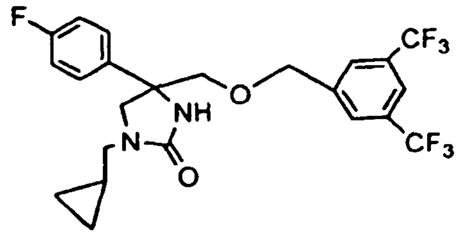

Reduction of the compound from Example 44 with LAH-ALCl$_3$ complex, analogous to the method as described in Example 26, gave the title compound in a 57 % yield. Electrospray MS $[M+1]^+$ 491.1.

Example 46

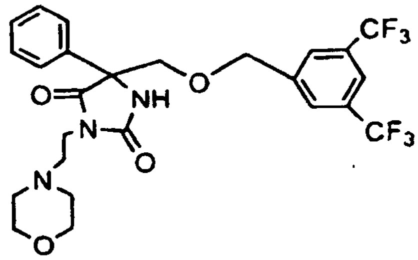

The title compound was prepared by an analogous method to that described in Example 25, Step 1, using compound **7** in place of compound **13**. The title compound was obtained as a solid in a 90 % yield. FAB MS $[M+1]^+$ 546.1.

Example 47

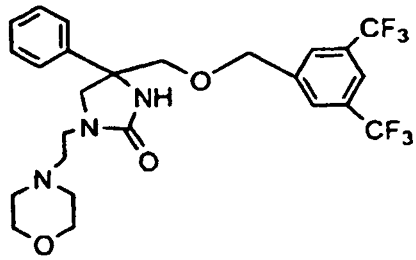

Reduction of the compound from Example 46 with LAH-ALCl$_3$ complex, analogous to the method as described in Example 26, gave the title compound in a 67 % yield. Electrospray MS $[M+1]^+$ 532.1.

Example 48

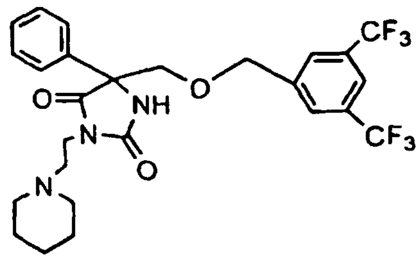

The title compound was prepared by an analogous method to that described in Example 25, Step 1, using compound **7** in place of compound **13** and 1-(2-chloroethyl)-piperidine hydrochloride in place of 1-(2-chloroethyl)-morpholine hydrochloride. The title compound was obtained as a solid in a 90 % yield. FAB MS $[M+1]^+$ 544.3.

Example 49

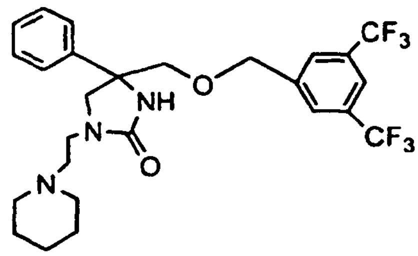

Reduction of the compound from Example 48 with LAH-$ALCl_3$ complex, analogous to the method as described for Example 26, gave the title compound in a 52 % yield. Electrospray MS $[M+1]^+$ 530.1.

Example 50

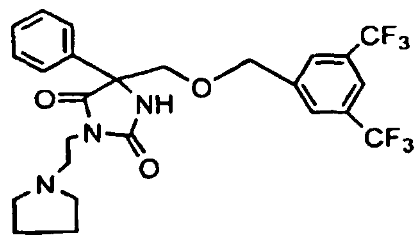

The title compound was prepared by an analogous method to that described in Example 25, Step **1**, using compound **7** in place of compound **13** and 1-(2-chloroethyl)-pyrrolidine hydrochloride in place of 1-(2-chloroethyl)-morpholine hydrochloride. The title compound was obtained as a solid in a 76 % yield. Electrospray MS $[M+1]^+$ 530.1.

Example 51

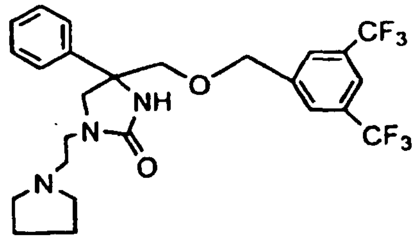

Reduction of the compound from Example 50 with LAH-ALCl$_3$ complex, analogous to the method as described in Example 26, gave the title compound in a 65 % yield. Electrospray MS $[M+1]^+$ 516.1.

Example 52

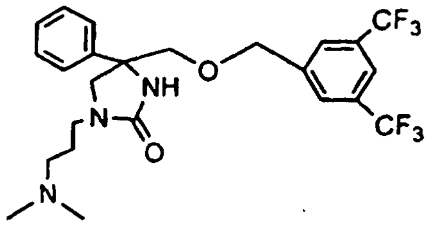

The title compound was prepared by a method analogous to Example 42, using **7** in place of **13**. Reduction of the resulting hydantoin with LAH/AlCl$_3$ complex, analogous to the method described in Example 26, gave the title compound in 8 % yield. FAB MS [M+1] 504.1.

Example 53

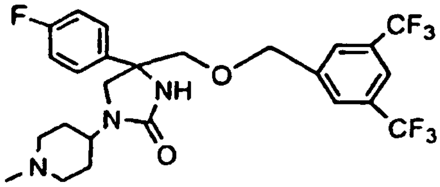

The title compound was prepared by a method analogous to that described in Example 7, using N-methylpiperidone in place of tetrahydro-4H-pyranone. The title compound was obtained as a solid in a 28 % yield. FAB MS [M+1] 534.

Example 54

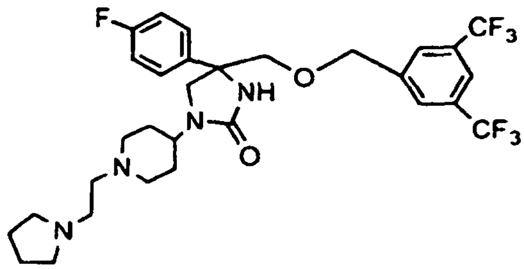

A solution of the product of Example 10 (0.3 g, 0.54 mmol) in $CH_2Cl_2$ (3 ml), N, N-diisopropylethylamine (0.24 g, 1.9 mmol), and 1-(2-chloroethyl)pyrrolidine hydrochloride (92 mg, 0.54 mmol) was stirred under an atmosphere of $N_2$ at RT for 14 days. After work-up, a cloudy gum was obtained as a crude product which was purified by chromatography, eluting with 4.5% $NH_3$-$CH_3OH$ (1:9) / 95.5 % $CH_2Cl_2$ to give the title compound as an off-white solid (20 mg, 7 %). FAB MS [M+1] 617.1.

Example 55

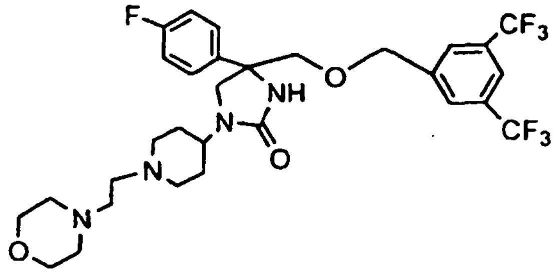

The title compound was prepared by a method analogous to Example 54, using 4-(2-chloroethyl)morpholine in place of 1-(2-chloroethyl)pyrrolidine. The title compound was obtained as a solid in a 35 % yield. FAB MS [M+1] 633.3.

Example 56

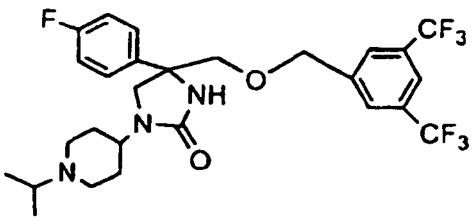

A solution of the product of Example 10 (0.2 g, 0.36 mmol) in 1, 2-dichloroethane (3 ml), acetone (21 mg, 0.36 mmol), and $Na(OAc)_3BH$ (0.15 g, 0.72 mmol) was stirred under $N_2$ atmosphere at RT for 2 days. After work-up, a solid was obtained as a crude product which was then purified by chromatography, eluting with 3 % $NH_3$-$CH_3OH$ (1:9) / 97 % $CH_2Cl_2$ to give the title compound as a white solid (120 mg, 60 %). FAB MS [M+1] 562.4.

Example 57

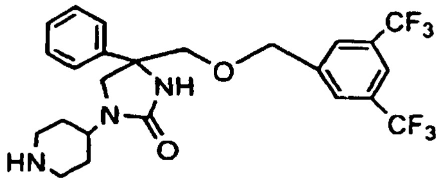

The title compound was prepared by a method analogous to Example 10, using **6** in place of **22.** The title compound was obtained as a HCl salt, FAB MS [M+1] 502.1.

Example 58

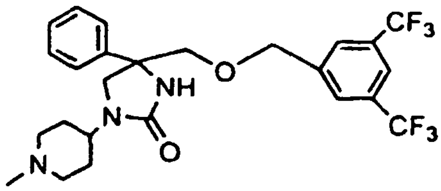

A solution of the product of Example 57 (0.3 g, 0.56 mmol) in $CH_2Cl_2$ (3 ml), N, N-diisopropylethylamine (0.14 g, 1.12 mmol), and $CH_3I$ (71 mg, 0.5 mmol) was stirred under $N_2$ atmosphere at 0°C for 2 h. After work-up, a solid was obtained as a crude product which was then purified by chromatography, eluting with 3.5% $NH_3$-$CH_3OH$ (1:9) / 96.5 % $CH_2Cl_2$ to give the title compound as a white solid (90 mg, 36 %). FAB MS [M+1] 516.1.

Example 59

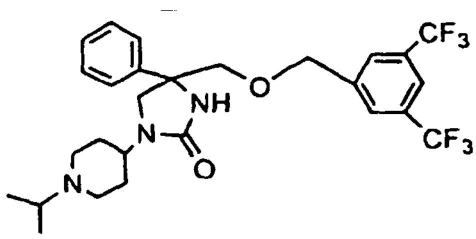

The title compound was prepared by a method analogous to Example 56, using the product of Example 57 in place of Example 10. The title compound was obtained as a solid in a 52 % yield. FAB MS [M+1] 544.2.

Example 60

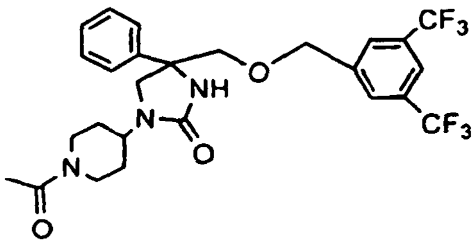

The title compound was prepared by a method analogous to Example 15, using Example 57 in place of Example 14. The title compound was obtained as a solid in a 80 % yield. FAB MS [M+1] 544.1.

Example 61

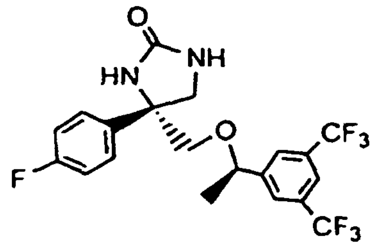

76

Step 1:

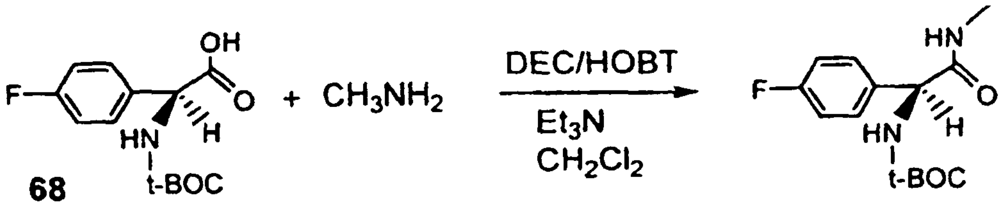


68 69

To a cooled solution of (S)-N-tBoc-4-fluoro-phenyl glycine in anhydrous $CH_2Cl_2$ at 0°C. HOBT (3.66 g, 22.4 mmol), $Et_3N$ (3.12 ml, 22.4 mmol), DEC (4.29 g, 22.4 mmol) and 2M $CH_3NH_2$ in THF (10.23 ml, 20.46 mmol) were added. After stirring at 0°C for 30 min, the reaction mixture was gradually warmed up to RT under $N_2$ protection over night. Diluted with $CH_2Cl_2$ (300 ml), the reaction mixture was washed with 10% citric acid (2X100 ml), saturated $NaHCO_3$-NaCl solution (2X100 ml), brine (1X100 ml). The combined organic layer was dried over $MgSO_4$, filtered and concentrated *in vacuo* to give crude **69** (4.88 g).

Step 2:

69

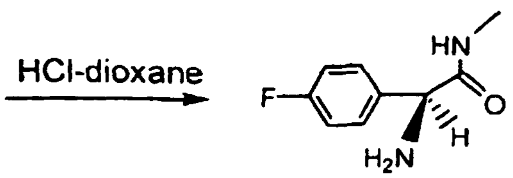


70

To a cooled solution of **69** (24.3 g, 86 mmol) in $CH_2Cl_2$ (58.8 ml) and $CH_3OH$ (30 ml) at 0 °C was added 4M HCl-dioxane solution (200 ml, 800 mmol). The clear solution was kept at 0 °C for 2.5 hours. Solvent was evaporated and the solid material was taken up with distilled water (200 ml), neutralized with NaOH (3.4 g) in water (100 ml). Final pH was adjusted to 9.5 with $K_2CO_3$. This aqueous solution was extracted with $CH_2Cl_2$ (4X250 ml). The combined organic layer was dried over $MgSO_4$, filtered and concentrated *in vacuo* to give a white solid which further turned into a pale yellow solid **70.** (14.9 g. 96%).

Step 3:

70 +
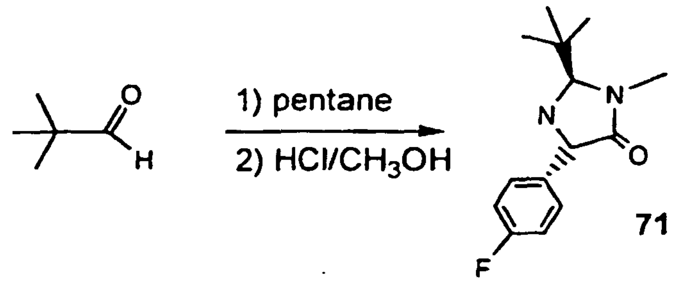

71

A mixture of compound 70 (14.84 g, 81.5 mmol), pentane (85 ml) and pivaldehyde (11.13 ml, 101.88 mmol) was heated to 65 °C with a Dean-Stark trap for 2.5 hours under $N_2$. The reaction mixture was then concentrated and the crude oil was taken up with $CH_3OH$ (22.5 ml), cooled down to 0 °C, and the crude product was treated with saturated HCl-$CH_3OH$ (52.5 ml) through a dropping funnel over 20 min. and stirred at ambient temperature over night. Volatile solvent was again evaporated and the residue was suspended in a mixture of brine (100 ml) and 2% $K_2CO_3$ solution (150 ml). This basic solution (pH=9.5) was extracted with $CH_2Cl_2$ (4X200 ml) and the combined organic layer was dried over $MgSO_4$, filtered and concentrated *in vacuo.* The crude product (20 g) was purified on a silica gel column, eluting with 25% EtOAc in hexane (4 l), 30% EtOAc in hexane (4 l) to give compound 71 (11.67 g, 58%) as an oil.

Step 4:

71 +
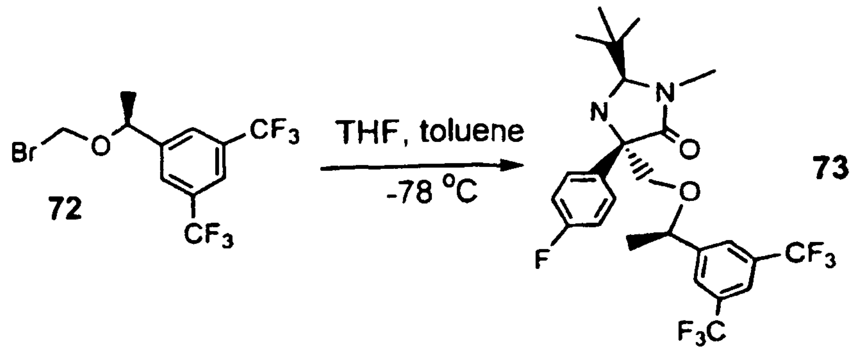

72
73

To a cooled solution of 71 (11.5 g, 46.08 mmol) in freshly distilled degassed THF (62 ml) and anhydrous degassed toluene (192 ml) at -78 °C, 0.5M KHMDS (97 ml, 48.5 mmol) in toluene was added slowly over 10 min through an addition funnel. The solution was stirred at -78 °C for 40 min before 72 (17 g, 49 mmol) was added via a syringe. Kept stirring at this low temperature for another 2.5 hours, the reaction was quenched with saturated $NH_4Cl$ solution (350 ml). The separated aqueous layer was extracted with $CH_2Cl_2$ (3X200 ml). The combined organic layer was dried over $MgSO_4$, filtered and concentrated. The crude product was further purified by flash chromatography, eluting with 15% EtOAc in hexane (6 l), 20% EtOAc in hexane (4 l). **73** was obtained as an oil (13.06 g, 56%).

Step 5:

73 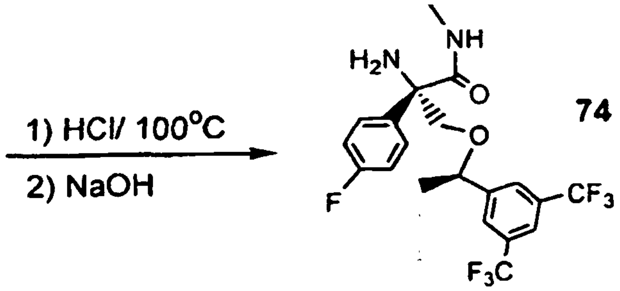

74

A mixture of **73** (12.55 g, 24.2 mmol) and 6N HCl (132 ml) was heated to 100°C under $N_2$ for 4 h. After cooling to RT, volatile solvent was evaporated. The gummy material was redissolved in 2.5% $K_2CO_3$ solution (300 ml) and brine (100 ml). The aqueous solution was extracted with $CH_2Cl_2$ (4X250 ml). The combined organic layer was dried over $MgSO_4$, filtered and concentrated *in vacuo* to give a gummy material (10.9 g) which was purified on a silica gel column, eluting with 20% EtOAc in hexane then 1.5% $NH_3$-$CH_3OH$ in $CH_2Cl_2$. **74** was obtained as an oil (8.45 g, 78%). Step 6:

74 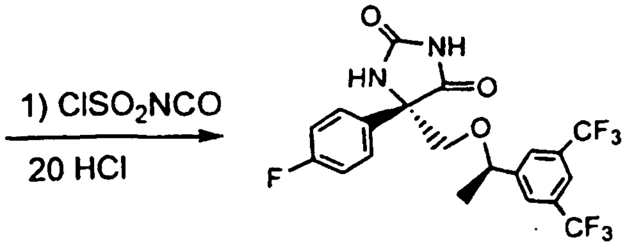

75

To a cooled solution of **74** (7.5 g, 16.6 mmol) in anhydrous $CH_2Cl_2$ (85 ml) at 0 °C was added $ClSO_2NCO$ (1.45 ml, 16.6 mmol) using a syringe. After stirring for 20 min, the reaction was finished and the solvent was evaporated. Taken up with 1,4-dioxane (70 ml) and 4N HCl (70 ml), the reaction mixture was then heated up to 95°C under $N_2$ for 4.5 hours. TLC showed completed reaction. Evaporation of volatile solvents yielded crude gummy material, which was taken up with 2.5% $K_2CO_3$ solution (300 ml) and $CH_2Cl_2$(200 ml). The aqueous solution was extracted with $CH_2Cl_2$ (4X200 ml). The combined organic layer was dried over $MgSO_4$, filtered and concentrated. The crude product was purified by flash chromatography, eluting with 3.5% (1:9) $NH_4OH$-$CH_3OH$ in $CH_2Cl_2$ to give 75 as a solid (6.88 g, 90%).

Step 7:

To a cooled flask containing $AlCl_3$ (4.3 g, 32.16 mmol) was added LAH (24.12 ml, 24.12 mmol) at 0°C under $N_2$. After 15 min, a solution of 75 (3.73 g, 8.04 mmol) in anhydrous THF (126 ml) was added through a syringe. The reaction mixture was stirred at 0 °C for 10 min before warming up to RT. After 4 h, the reaction was quenched with saturated $Na_2SO_4$ solution (10 ml) in an ice-bath. Diluted with THF (200 ml) and stirred for 1 h at RT, the reaction was dried over $MgSO_4$, filtered and concentrated to give crude 76 (3.5 g), which was purified by flash chromatography eluting with 1% $NH_4OH/CH_3OH$ (1:9) in $CH_2Cl_2$. mp 136-137 °C, FABMS $[M+1]^+$ 451.1; $[\alpha]_D$20 °C -49.4 °C.

<u>Example 62</u> (Preparative)

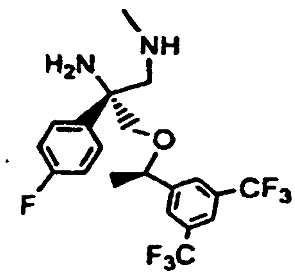

The title compound was prepared by a method analogous to step 7 of Example 61, using compound 74 in place of compound 75. FAB MS $[M+1]^+$ 439.1.

Example 63

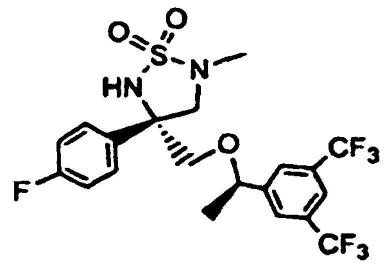

To a solution of Example **62** (180 mg, 0.41 mmol) in dioxane (4 ml), sulfamide (48.5 mg, 0.51 mmol) was added. After heating to 110°C for 5 h, the reaction was concentrated. The residue was purified by flash chromatography, eluting with 4% $NH_4OH$:$CH_3OH$(1:9) in $CH_2Cl_2$. The title compound was collected as off-white solid (35 mg, 17%). FAB MS $[M+1]^+$ 518.1.

Example 64

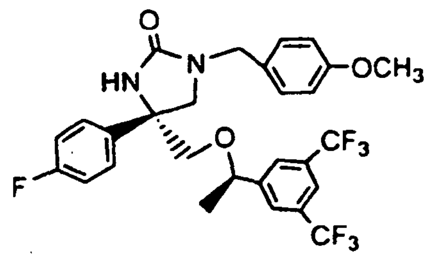

To a cooled solution of NaH (27 mg, 0.67 mmol) in anhydrous DMF (5 ml), was added compound **76** from Example 61 (0.25 g, 0.56 mmol) at 0 °C. After 15 min, the cold bath was removed. The reaction was warmed to RT for 45 min before p-methoxy benzyl-chloride (0.08 ml, 0.59 mmol) was added. The reaction was then stirred at RT for another 2 h. Water was used to quench the reaction and the aqueous solution was extracted with EtOAc. The combined organic layer was dried over $Na_2SO_4$, filtered and concentrated. Crude material was purified through a silica gel column, eluting with 2:1 hexane to EtOAc to give the title compound as a solid (86.8 mg, 27%). FAB MS $[M+1]^+$ 571.1.

Example 65

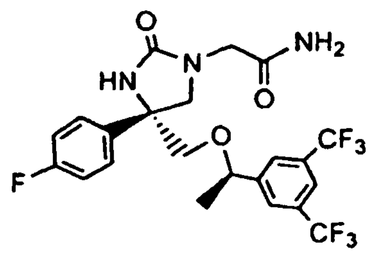

The title compound was prepared by a method analogous to Example 64, using bromo acetamide in place of

p-methoxy benzyl chloride, FAB MS $[M+1]^+$ 508.1.

Example 66

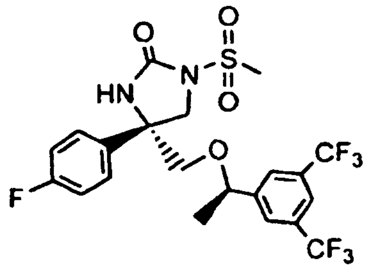

To a cooled solution of **76** (0.2 g, 0.44 mmol) in anhydrous $CH_2Cl_2$ (4.2 ml) at 0 °C, diethylisopropylamine (0.12 ml, 0.69 mmol) was added dropwise followed by $CH_3SO_2Cl$ (0.04 ml, 0.52 mmol). The reaction was warmed to RT and stirred overnight. The volatile solvent was evaporated and the crude product was purified on a silica gel column, eluting with 4% $CH_3OH$ (saturated with $NH_3$) in $CH_2Cl_2$. FAB MS $[M+1]^+$ 529.1.

Example 67

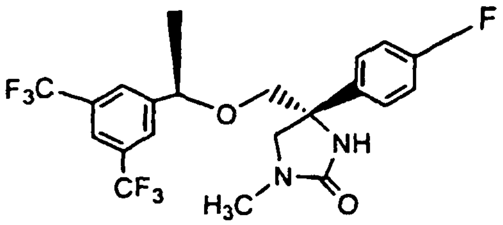

The title compound was prepared by methods analogous to Example 1, method 1, step 5, using the compound of Example 62 in place of 6. The title compound was obtained as a solid in a 51% yield. Electrospray MS $[M+1]^+$ 465.1.

Example 68

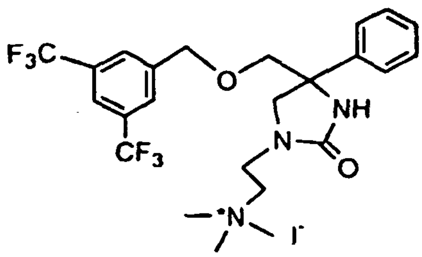

To a solution of compound of Example 67 (0.31g, 0.64 mmol) in anhydrous THF (12.8 ml) at RT, $CH_3I$ (0.09 ml, 1.28 mmol) was added. The reaction mixture was stirred under $N_2$ for 5 h. Evaporation of the solvent gave a yellow foam, which was further purified by flash chromatography eluting with 7.5% $CH_3OH$ (saturated with $NH_3$) in $CH_2Cl_2$. Electrospray MS $[M+1]^+$ 504.

Example 69

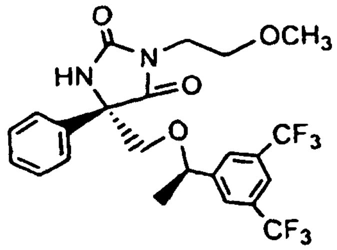

The title compound was prepared by a method analogous to Example 25, step 1, using **75** in place of **13** and 2-bromoethyl methyl ether in place of 4-(2- chloroethyl)-morpholine hydrochloride salt. The title compound was obtained in a 61% yield. Electrospray MS $[M+1]^+$ 505.1.

Example 70

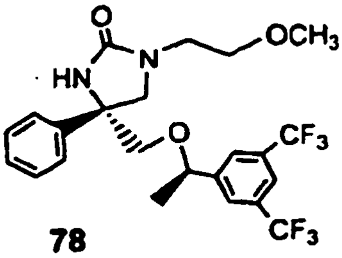

**78**

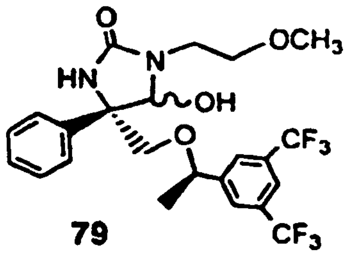

**79**

Reduction of the product of Example 69 with LAH-$AlCl_3$ complex at RT overnight, analogous to the method described in Example 26, gave the title compound **78** as a clear oil and compound **79** as a crystalline solid after separation of crude product using 3% $CH_3OH(NH_3)$ in $CH_2Cl_2$. Electrospray MS: compound **78,** MS $[M+1]^+$ 491.1 and compound **79,** MS $[M+1]^+$ 507.4.

Example 71 (Preparative)

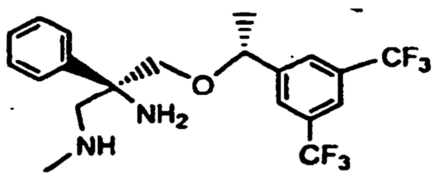

The title compound was prepared by analogous methods to that described in Examples 61, Step 7 and 62, using the phenyl analog in place of **74** in Example 62. The title compound was obtained as an oil. MS $[M+1]^+$ 407.1.

Example 72

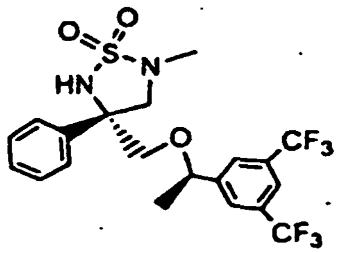

The title compound was prepared in a method analogous to Example 63, using Example 71 in place of the compound of Example 62. The title compound was obtained as an oil in 44% yield. NMR ($CDCl_3$) 1.45 (d, 3H), 2.38 (s, 3H), 3.87 (d, 1H), 3.27 (d, 1H), 3.45 (d, 1H), 3.55(d, 1H), 4.55 (q, 3H), and aromatic protons (7.3-.7.4, 7.58, 7.78).

Example 73

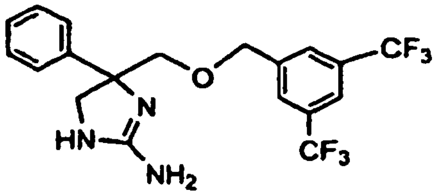

**82**

Step 1.

**6**

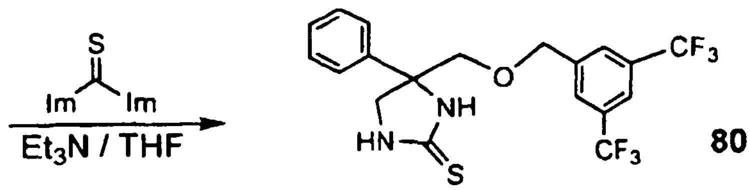


**80**

To a solution of compound 6 (1.85 g, 3.97 mmol) in dry THF (30 ml) was added $Et_3N$ (0.6 ml, 4.3 mmol), 4 Å molecular sieve (3 g) and thiocarbonyl diimidazole (1.179 g, 5.95 mmol). The mixture was stirred at 0 °C under $N_2$ for 20 h. After completion, molecular sieve was filtered off and THF evaporated. The residue was redissolved in EtOAc (300 ml), washed with 0.04 N HCl (50 ml, 2X), dried ($MgSO_4$), filtered and evaporated. Pure 80 was obtained as a solid (1.41 g, 3.25 mmol) in 82% yield by flash silica chromatography (50 g), eluting with 5 % (1:9) [$NH_4OH/CH_3OH$] /95 % $CH_2Cl_2$.

Step 2.

**80**

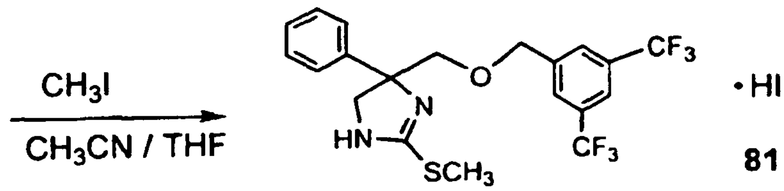


**81**

To a solution of **80** (0.75 g, 1.73 mmol) in $CH_3CN$ and THF (6 ml) was added $CH_3I$ (0.13 ml, 2.1 mmol). The

solution was stirred at RT overnight under an atmosphere of $N_2$. After completion, solvent was evaporated and the product used in the next step without further purification.

Step 3

**81** (150 mg, 0.334 mmol) was treated with 2 M $NH_3$-$CH_3OH$ solution (1 ml). The solution was stirred in a vial with a sealed cap for 5 days. After completion, solvent was evaporated and product was purified by flash silica gel (50 g), eluting with 5 % (1:9) $NH_4OH$- $CH_3OH$/95 % $CH_2Cl_2$. LCMS $[M+1]^+$ 418.1.

Example 74

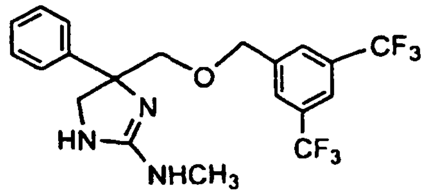

The title compound was prepared in a method analogous to Example 73, step 3, using 2M $NH_2CH_3$ in place of 2M $NH_3$. Product was purified on flash silica gel (50 g), eluting with 5 % (1:9) $NH_4OH$- $CH_3OH$/ 95 % $CH_2Cl_2$. LCMS $[M+1]^+$ 432.1.

Example 75

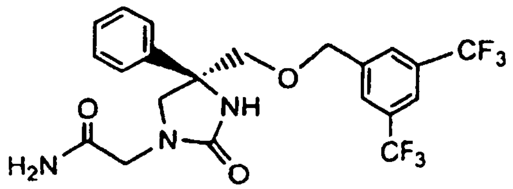

**84**

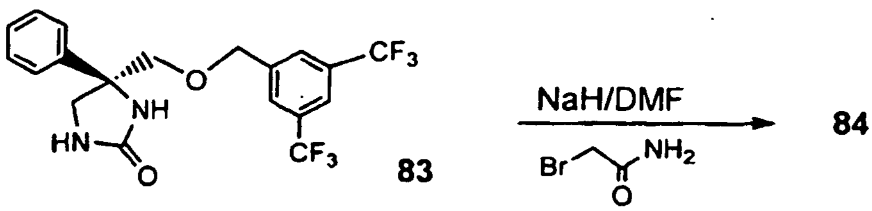


**83** **84**

To a solution of **83** (0.4 g, 0.96 mmol) in anhydrous DMF (2 ml) was added 60% NaH (46.4 mg, 1.16 mmol) under $N_2$. After 20 min, 2-bromoacetamide (163.3 mg, 1.16 mmol) was added. The reaction was stopped by evaporating off the DMF after 20 h. The residue was redissolved in EtOAc (200 ml) and washed with brine (2X 50 ml). The separated organic layer was dried over $MgSO_4$, filtered and concentrated *in vacuo.* Crude material was purified by flash silica chromatography, eluting with 4.5% (1:9) $NH_4OH$-$CH_3OH$ in 96.5% $CH_2Cl_2$ to give the title compound **84** in a 25% yield. Electrospray MS $[M+1]^+$ 476.1.

Example 76

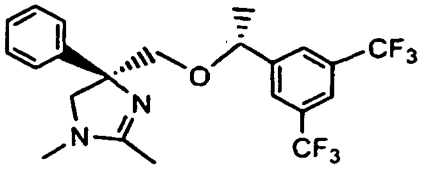

**85**

To a flask containing Example 71 (0.2 g, 0.48 mmol) and molecular sieves (0.18 g) in anhydrous toluene (4.5 ml), glacial acetic acid (0.3 ml, 5.24 mmol) was added. The reaction mixture was heated to 100 °C for 48 h. Cooled down to RT, the reaction was worked up by pouring into a separatory funnel containing EtOAc and saturated $NaHCO_3$. The aqueous layer was extracted with EtOAc (2X 50 ml). The combined organic layer was washed with brine (50 ml), dried over $Na_2SO_4$, filtered and concentrated. The title compound **85** was obtained by purifying through flash chromatography, eluting with 20% EtOAc in hexane. Yield: 43%. Electrospray MS $[M+1]^+$ 445.1.

Example 77

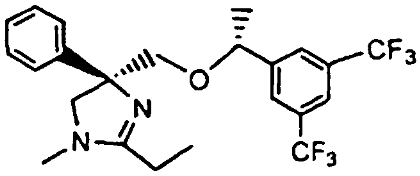

The title compound was prepared in a method analogous to Example 76, using propanoic acid in place of glacial acetic acid. The title compound was obtained in a 25% yield. Electrospray MS $[M+1]^+$ 459.1.

Example 78

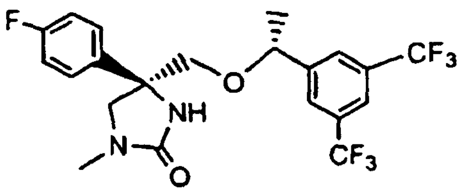

The title compound was prepared by a method analogous to Example 1, Method 1, Step 5, using Example 62 in place of **6**. Purification by flash silica chromatography, using 5% (1:9)$[NH_4OH/ CH_3OH]$/95 % $CH_2Cl_2$ gave the title compound as a solid with a yield of 51%. LCMS $[M+1]^+$ 465.1.

Example 79

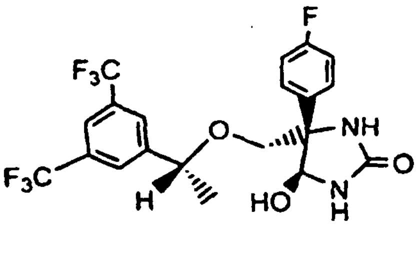

Ex. 79a

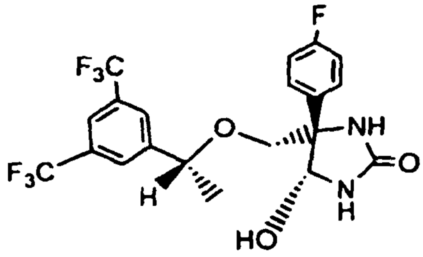

Ex. 79b

88

Step 1:

74

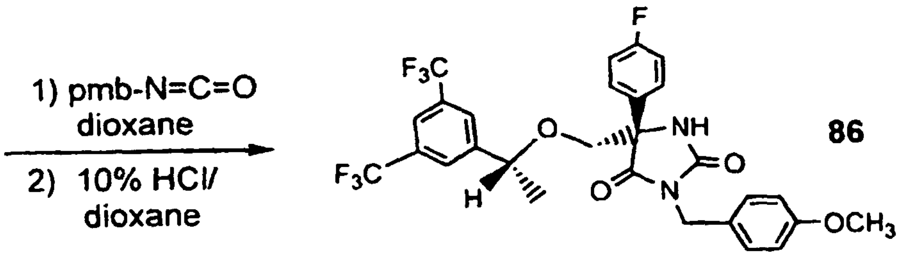


86

A solution of **74** (1.8 g, 4 mmol) in 1.4-dioxane (17 ml) and 4-methoxybenzylisocyanate (98 mg, 6 mmol) was stirred under $N_2$ at RT for 4 h. The mixture was mixed with 10% aqueous HCl in dioxane (17 ml), then heated to 90 °C for 5.5 h. After work-up, a solid was obtained as a crude product which was then purified by chromatography, eluting with 15% EtOAc/85% hexane to give **86** as a white solid (1.8g, 82 %). FAB MS [M+1] 584.

Step 2:

86

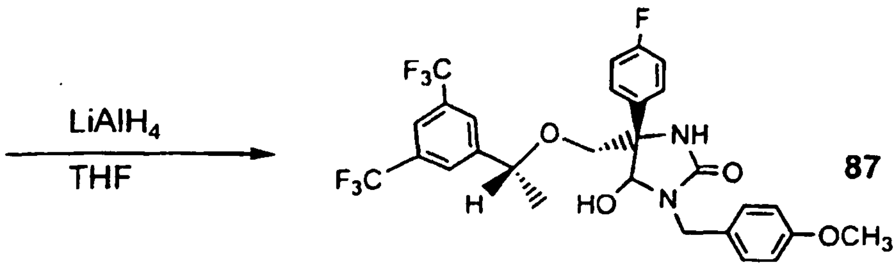


87

A mixture of **86** (1.7 g, 2.91 mmol) in THF (10 ml) and $LiAlH_4$ (1M in ether, 3.3 ml, 3.2 mmol) was stirred under $N_2$ at 0°C for 30 min., then RT for 3 h. After work-up, a solid was obtained as a crude product which was then purified by chromatography, eluting with 2 % $NH_3$-$CH_3OH$ (1:9) / 98 % $CH_2Cl_2$ to give **87** as a white solid (1.54g, 91 %). FAB MS [M+1] 587.4.

Step 3:

A mixture of **87** (1.48 g, 2.53 mmol) in $CH_3CN$ (33 ml)/ $H_2O$ (10 ml) and $Ce(NH_4)_2(NO_3)_6$ (5.54 g, 10.1 mmol) was stirred under $N_2$ at RT for 50 min. After work-up, an oil was obtained as a crude product which was then purified by chiral HPLC (Chiralpak® AD), to give the title compounds, examples 79a and 79b,as a white solid (0.19g, 16 %). FAB MS [M+1] 467.1.

Example 80

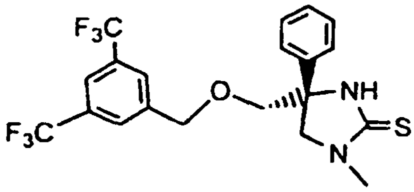

**90**

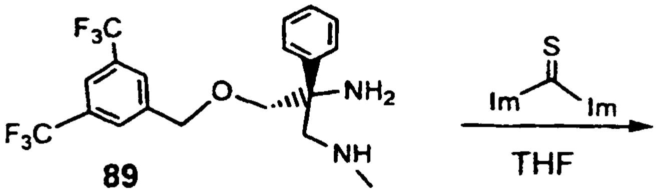


**89**

**90**

**89** was prepared by a method analogous to Examples 61 and 62, using s-(+)-2-phenylglycine methyl ester hydrochloride in place of (s)-N-BOC-4-fluorophenyl-glycine in Example 61. A mixture of **89** (2.03 g, 5 mmol) in THF (6 ml) and 1,1'-thiocarbonyldiimidazole(1.34 g, 7.5 mmol) was stirred under $N_2$ at RT for 18 h. After work-up, a yellow paste was obtained as a crude product which was then purified by chromatography, eluting with 25% EtOAc/75% hexanes, to give the title compound **90** as a solid (1.35 g, 62 %). FAB MS [M+1] 449.1.

Example 81

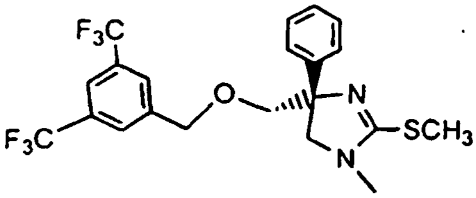

A solution of the product of Example 80 (1.2 g, 2.68 mmol) in THF (10 ml) and $CH_3I$ (0.45 g, 3.2 mmol) was stirred under $N_2$ at RT for 5 h. After work-up, a solid was obtained as a crude product which was then purified by trituation with ether to give the title compound as a pale yellow salt (1. 5 g, 94 %). FAB MS [M+1] 463.1.

Example 82

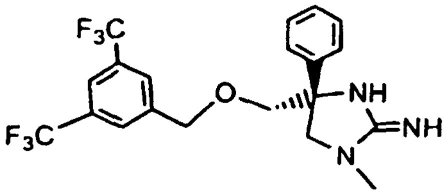

A solution of the product of Example 81 (0.3 g, 0.51 mmol) and 7 N $NH_3$ (2 ml, 14 mmol) in $CH_3OH$ was stirred under $N_2$ at RT for 4 days. After work-up, a solid was obtained as a crude product which was then purified by chromatography, eluting with 9 % $NH_3$-$CH_3OH$ (1:9) / 91 % $CH_2Cl_2$ to give the title compound as a solid (0.19 g, 86 %). FAB MS [M+1] 432.1.

Example 83

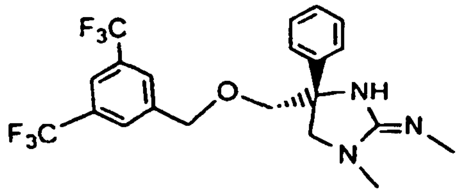

The title compound was prepared by a method analogous to Example 82, using 2 M $CH_3NH_2$ in $CH_3OH$ in place of 7 M $NH_3$ in $CH_3OH$. The title compound was obtained as a solid in a 95 % yield. FAB MS [M+1] 446.1.

Example 84

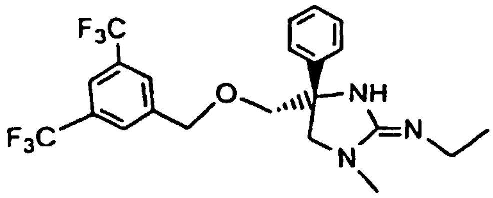

The title compound was prepared by a method analogous to Example 82, using 2 M ethylamine in $CH_3OH$ in place of 7 M $NH_3$ in $CH_3OH$. The title compound was obtained as a solid in a 17 % yield. FAB MS [M+1] 460.1.

Example 85

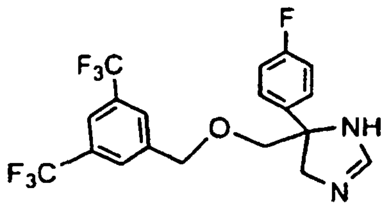

A mixture of **22** (0.3 g, 0.73 mmol) and N, N-dimethylformamide dimethyl acetal (87 mg, 0.73 mmol) was heated to 60 °C for 18 h. The reaction mixture was purified by chromatography, eluting with 3.5 % $NH_3$-$CH_3OH$ (1:9)/96.5 % $CH_2Cl_2$ to give the title compound as an off-white solid (180 mg, 60 %). FAB MS [M+1] 421.1.

Example 86

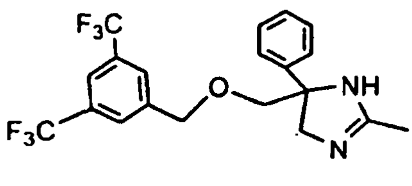

A mixture of **6** (0.4 g, 1 mmol) in toluene (3 ml) and acetic acid (0.24 g, 4 mmol) was heated to reflux for 6 days.

After work-up, an oil was obtained as a crude product which was purified by chromatography, eluting with 5 % $NH_3$-$CH_3OH$ (1:9) /95 % $CH_2Cl_2$ to give the title compound as a HCl salt (75 mg, 18 %) after treating the pure compound with 1 eq. of 2 N HCl-$Et_2O$ solution. FAB MS [M+1] 417.1.

Example 87

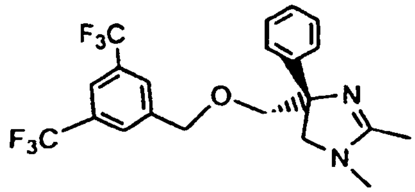

A mixture of **89** (0.4 g, 0.985 mmol) in toluene (3 ml) and acetic acid (0.59 g, 9.85 mmol) was heated to 100 °C for 40 h. After work-up, an oil was obtained as a crude product which was purified by chromatography, eluting with 3.5 % $NH_3$-$CH_3OH$ (1:9) / 96.5 % $CH_2Cl_2$ to give the title compound as a HCl salt (0.32 g, 76 %) after treating the pure compound with 1 eq. of 2 N HCl-$Et_2O$ solution. FAB MS [M+1] 431.1.

Example 88

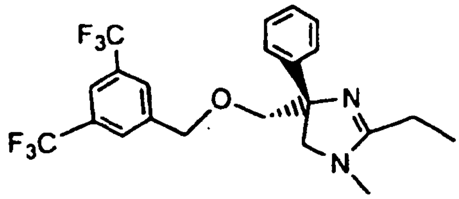

The title compound was prepared by a method analogous to Example 87, using propionic acid in place of acetic acid. The title compound was obtained as a HCl salt in a 59 % yield. FAB MS [M+1] 445.1.

Example 89

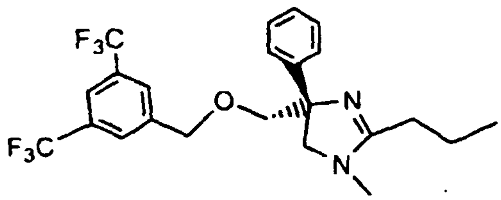

The title compound was prepared by a method analogous to Example 87, using n-butyric acid in place of acetic acid. The title compound was obtained as a HCl salt in a 88 % yield. FAB MS [M+1] 459.1.

Example 90

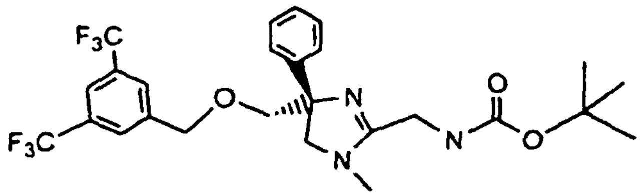

The title compound was prepared by a method analogous to Example 87, using N-t-BOC glycine in place of acetic acid. The title compound was obtained as a HCl salt in a 63 % yield. FAB MS [M+1] 546.1.

Example 91

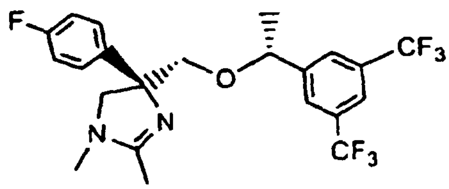

The title compound was prepared by a method analogous to Example 76 using Example 62 in place of Example 71. Purification the crude material by flash silica chromatography, using 5% (1:9)[$NH_4OH/CH_3OH$]/95 % $CH_2Cl_2$ gave the title compound as a solid with a yield of 75%. LCMS $[M+1]^+$ 463.1.

Example 92

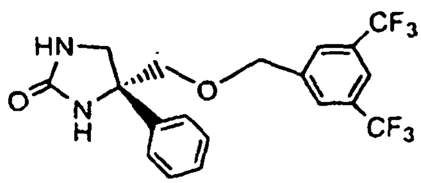

Step 1:

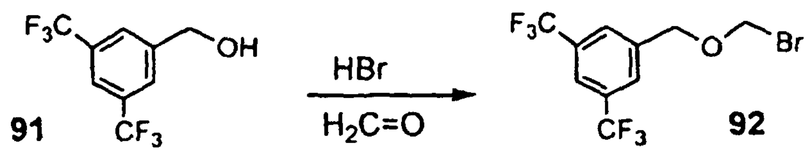


A flame-dried flask was charged with compound **91** (50 g, 204.8 mmol, 1 equiv.) and paraformaldehyde (6.76 g, 225.3 mmol, 1.1 equiv.). The solid mixture was dissolved under $N_2$ with a heat gun until the solution was homogeneous, approximately 20 min. The solution was allowed to cool to RT. HBr gas was bubbled into the solution at a fast rate at RT. The reaction was continued for 2.5 h. The layers were separated, the upper layer was diluted with hexane (150 ml) and any aqueous remnants were removed. The hexane layer was dried over $MgSO_4$ overnight, then filtered and concentrated. Short path distillation (high vac, 75°C) gave pure **92** (67.86 g, 98%) as a clear oil. $^1H$ NMR ($CDCl_3$) δ 4.85 (s, 2H), 5.79 (s, 2H), 7.84 (s, 2H), 7.87 (s, 1 H).

Step 2:

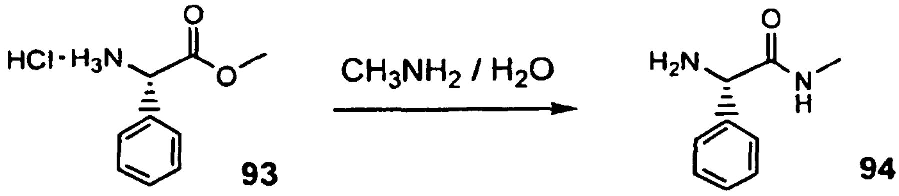


93 94

Compound **93** (100g, 496 mmol, 1 equiv.) was added to $CH_3NH_2$ (160 ml, 40% in $H_2O$, 4 equiv.) in a cool water bath at 10-16°C over a period of 15 min. After complete addition, the solution was warmed to RT and stirred for 1 h. The reaction was monitored by TLC in 98:2 EtOAc/$CH_3OH$. Upon completion, the reaction was quenched with brine (25% in $H_2O$, 500 ml). The solution was extracted with 1:1 THF/EtOAc (4 x 400 ml) and the combined organic phases were dried ($Na_2SO_4$) and concentrated. The residue was dried on high vacuum to give **94** (79.47 g, 98%). Electrospray MS $[M+1]^+$ 165.0.

Step 3:

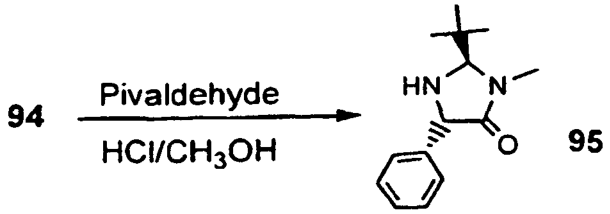


94 95

A mixture of **94** (79.44 g, 483.8 mmol, 1 equiv.), pentane (550 ml), and trimethylacetaldehyde (65.7 ml, 604.8 mmol, 1.25 equiv.) was heated to 65°C in a system equipped with a condenser, Dean-Stark trap and $N_2$ inlet. The mixture was heated for 3 h and the suspension dissolved. The solution was cooled to RT and stirred overnight (16 h). The solution was concentrated, redissolved in $CH_3OH$ (140-150 ml), cooled in an ice bath for 30 min, and then saturated HCl-$CH_3OH$ (300 ml) was slowly added via a dropping funnel over 30 min. The solution was stirred at 0°C, then warmed to RT under $N_2$ overnight and concentrated on high vac to yield a crude, yellow oil (109.1 g). The oil was redissolved in $CH_2Cl_2$ (800 ml) and washed with 25% $K_2CO_3$ (w/w, 400 ml). The aqueous portion was washed again with $CH_2Cl_2$ (2 x 400 ml). The combined organics were dried ($Na_2SO_4$) and concentrated to give a yellow solid (98.7 g). The crude solid was recrystallized out of hot MTBE (300 ml), then cooled to 0°C to give 95 as a white solid (54.88 g, 49%). Mother liquor still contained product which could be isolated by another recrystallization or by chromatography. Electrospray MS $[M+1]^+$ 451.1

Step 4:

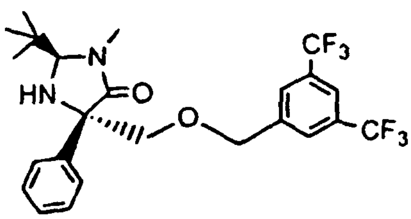

96

All reagents were deoxygenated under Ar prior to use. **95** (42.05 g, 181 mmol, 1 equiv.) was dissolved in dry THF from a still (550 ml) while agitating with a mechanical stirrer. The solution was cooled to approximately -70°C in dry

ice/acetone and a solution of 1.5 M LDA·THF in cyclohexane (124.3 ml, 186.5 mmol, 1.03 equiv.) was added over 20 min. The resultant dark orange/brown solution was allowed to stir at -78°C for 30 min. Bromide **92** (64.0 g, 190 mmol, 1.05 equiv.) was slowly added via syringe over 20 min. The solution was stirred at -78°C and monitored by TLC in 4:1 Hex/EtOAc. The reaction was complete after 2 h. The reaction was quenched with sat. aq. $NH_4Cl$ (300 ml) at -78°C and then warmed to RT while stirring vigorously. Phases were separated and the organic phase washed with $H_2O$ (2 x 150 ml). The aqueous layer was washed with EtOAc (300 ml). The combined organics were dried ($Na_2SO_4$) and concentrated to give a crude, light yellow solid (32.63 g), which was recrystallized out of hot pentane to give **96** (33.66 g). The mother liquor was again concentrated to an orange solid and recrystallized out of pentane (150 ml) to give an additional 10.50 g of product (Overall yield = 44.16 g, 50%). The mother liquor (46.7 g, approximately 50% pure by NMR) still contained starting product which could be isolated. Electrospray MS $[M+1]^+$ 489.1

Step 5:

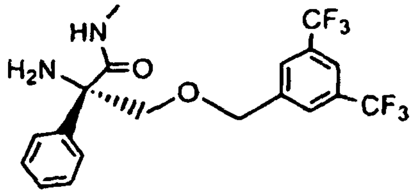

**97**

To a solution of **96** (33.58 g, 68.76 mmol, 1 equiv.) in $CH_3OH$ (300 ml), concentrated HCl (300 ml) was added dropwise with stirring. The solution was heated to 95°C and stirred vigorously, and was stirred and heated overnight. The reaction was monitored by TLC. Upon completion, solvent was evaporated and the residue was taken up in $CH_2Cl_2$ (750 ml). The solution was treated with 25% aq. $K_2CO_3$ (approximately 350 ml) until pH 12. The mixture was filtered and the layers separated. The organic layer was washed with 25% aq. $K_2CO_3$ (500 ml). Combined aqueous layers were extracted with $CH_2Cl_2$ (2 x 750 ml). Combined organic layers were dried ($Na_2SO_4$), and concentrated to a yellow oil. $^1H$ NMR shows pure **97** (29.20 g, 100%). $^1H$ NMR ($CDCl_3$) δ 2.04 (s, 2H), 2.80 (d, J = 4.8 Hz, 3H), 3.67 (d, J = 8.8 Hz, 1H), 4.53 (d, J = 8.8 Hz, 1H), 4.67 (d, J = 12.5 Hz, 1H), 4.77 (d, J = 12.5 Hz, 1 H), 7.26-7.36 (m, 3H), 7.51 (d, J = 8.1 Hz, 2H), 7.65 (d, J = 4.4 Hz, 1H), 7.75 (s, 2H), 7.79 (s, 1 H).

Step 6:

The title compound was prepared from **97** using procedures similar to Example 61, steps 6 and 7.

<u>Example 93</u>

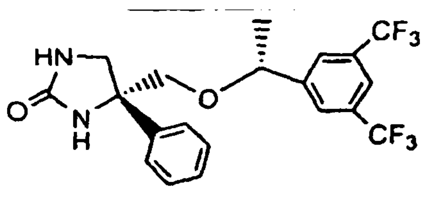

Step 1:

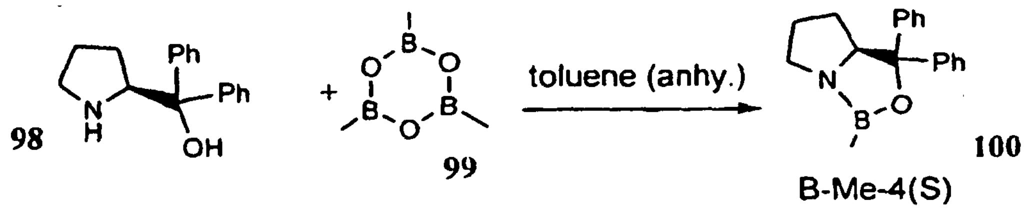


A flame-dried flask was charged with **98** (5.10 g, 20.1 mmol, 1.0 eq) and anhydrous toluene (56 ml). This cloudy solution was heated up to 140-150 °C. 36 ml of dry toluene was azeotropically distilled through a Dean-Stark trap with an air condenser. Another 36 ml of toluene was added. This azeotropical distillation was repeated three times to ensure **98** was totally dry. After the third azotropical distillation was done, another 36 ml of anhydrous toluene was added. The solution was allowed to cool down toRT. **99** (1.90 ml, 13.5 mmol, 0.67 eq) was syringed in within 5 min. White solid was formed at about 6 min after completion of the addition. The reaction mixture was stirred at RT for 30 min, then 36 ml of toluene was distilled off. Another 36 ml of dry toluene was added and distilled off again. The distillation was repeated one more time, and 20 ml of 1.0 M of **100** (CBS catalyst B-Me-4(S)solution in toluene) was prepared. The almost colorless solution can be used in CBS reduction directly.

Step 2:

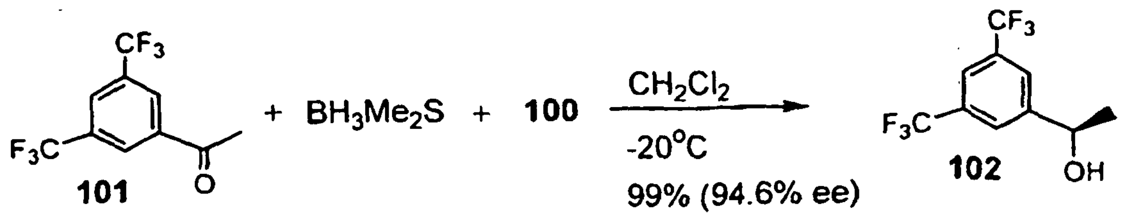


**101** (102.14 g, 0.4 mol, 1.0 eq) was dissolved in anhydrous $CH_2Cl_2$ (780 ml) and the resulting solution was transferred into a dry dropping funnel. An oven-dried 3L flask was cooled to -20°C, and 20 ml of **102** toluene solution was syringed in, followed by 40 ml of 10.0~10.3 M borane-methylsulfide complex. Then the **101** solution was added dropwise through the dropping funnel over 2 days. During the addition, the temperature was maintained at -20°C. Once the addition was finished, the reaction was monitored by TLC in 4:1 hexane/EtOAc. When **101** was completely consumed, 250 ml of $CH_3OH$ was added slowly, and hydrogen gas was emitted. The reaction solution was then concentrated to give white solid. The solid was dissolved in $Et_2O$ (500 ml), then 45 ml of 2.0 M of HCl in $Et_2O$ was added slowly at -20°C. White precipitate was formed. The reaction mixture was warmed to RT and stirred for 30-40 min. The mixture was filtered and the filtrate concentrated to give 101.5 g of white solid **102** (yield 98.7%). Chiral HPLC Chiral OD(Chiralcel) column (Hexane/IPA = 98/2) showed 94.6%ee.

Step 3:

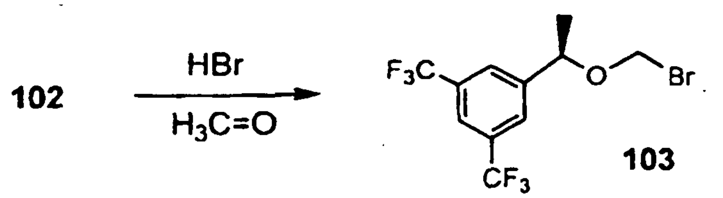


**103** was prepared in a method analogous to Example 92, Step 1 using **102** in place of **91**.

Step 4:

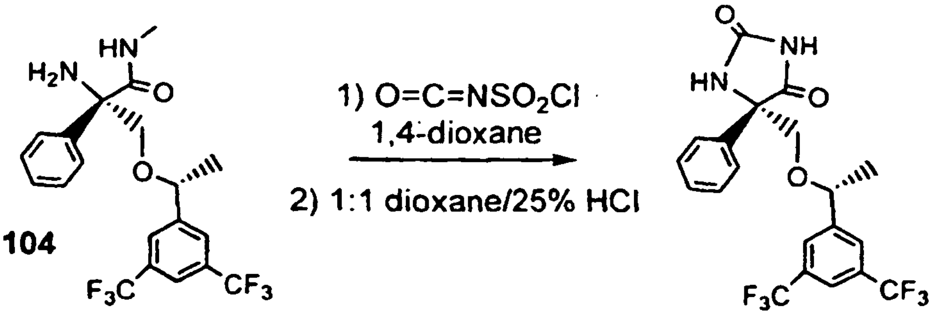


104

105

Amino amide **104** (14.14 g, 32.55 mmol, 1 equiv.) (prepared in a similar procedure to that described in Example 92, substituting **103** in place of **92** in step 4) was taken up in dry $CH_2Cl_2$ (120 ml). The solution was cooled to -78°C and chlorosulfonyl isocyanate (2.84 ml, 32.55 mmol, 1 equiv.) was added. The reaction was stirred at 0°C for 3 h and then concentrated to a white solid. The solid was dissolved in 1,4-dioxane (120 ml) and 3 N aqueous HCl (120 ml), then stirred at 90°C for 5 h, then at RT overnight. The solution was diluted with $H_2O$ (250 ml) and extracted with EtOAc (3 x 400 ml). The combined organics were dried ($Na_2SO_4$), and concentrated to a crude, white foam (15.80 g). the foam was purified by plug chromatography on a 600 ml fritted funnel, eluting with 2:1 Hex/EtOAc. The fractions 2-8 were collected and concentrated to give pure **105** (10.26 g, 71%). FAB MS $[M-+1]^+$ 447.1

Step 5:

$AlCl_3$ (12.2 g, 91.4 mmol, 4 equiv.) was added to a flask equipped with a $N_2$ inlet. The flask was cooled to 0°C and a solution of 1 M LAH in ether (68.6 ml, 68.6 mmol, 3 equiv.) was slowly added. The resultant white slurry was stirred at 0°C for 15 min. Next, a solution of hydantoin 105 (10.2 g, 22.85 mmol, 1 equiv.) in 150 ml dry THF was added via canula. The solution was warmed to RT and stirred for 41 h. The solution was again cooled to 0°C and $H_2O$ (20 ml) was added, then 15% aq. NaOH (w/w, 20 ml) followed by $H_2O$ (60 ml). The biphasic solution was stirred for 30 min. All emulsion formed was dissolved with 1 N HCl (approximately 300-400 ml) and the layers were separated. The aqueous layer was extracted with EtOAc (2 x 500 ml). The combined organics were washed with $H_2O$ (200 ml), dried ($Na_2SO_4$), and concentrated to give the crude (9.86 g). The crude material was initially purified by plug chromatography on a 2L fritted funnel, eluting with 1:1 Hex/EtOAc. followed by 98:2 EtOAc to give 8.0 g of material, which still contained 3% of a less polar impurity. The solid was recrystallized from hot MTBE (30 ml) to provide pure Example 93 (6.5 g, 79%). FAB MS $[M+1]^+$ 433.1

Example 94

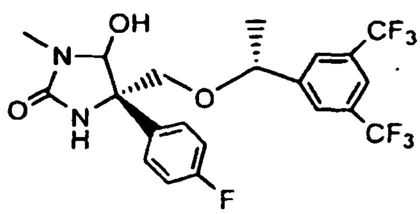

Step 1:

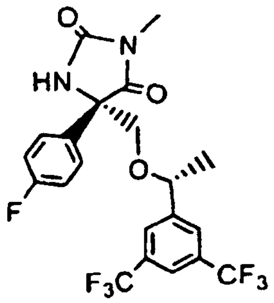

**106**

**104** (3.0 g, 6.63 mmol, 1 equiv.) (prepared analogously to the procedure described in a Example 92) was dissolved in $CH_2Cl_2$ (100 ml) and cooled to 0°C. $Et_3N$ (2.78 ml, 19.89 mmol, 3 equiv.) followed by triphosgene (787 mg, 2.65 mmol, 0.4 equiv.) was added and the solution was allowed to stir and warm to RT. Reaction was complete after 2 h as determined by TLC. The reaction was quenched with sat. aq. $NaHCO_3$ (100 ml) and the layers were separated. The organic layer was washed with brine (100 ml), dried ($Na_2SO_4$), and concentrated. The crude product was purified by Biotage chromatography, eluting with $CH_2Cl_2$ to give 2.024 g (64%) of **106.** FAB MS $[M+1]^+$ 479.1.

Step 2:

**106** (919 mg, 1.92 mmol, 1 equiv.) was dissolved in dry THF (10 ml) and cooled to 0°C. A 1 M solution of LAH in $Et_2O$ (1.92 ml, 1.92 mmol, 1 equiv.) was slowly added and the solution was stirred at 0°C for 10 min. The reaction was warmed to RT and completed after 2 h. The solution was cooled to 0°C and water (3 ml) was slowly added, followed by 15% aq. NaOH (3 ml), and then more water (10 ml). The emulsion was dissolved with 1 M HCl and the layers were separated. The aqueous layer was extracted with EtOAc (2 x 100 ml) and the organic layers were combined, washed with water (50 ml), dried ($Na_2SO_4$), and concentrated to give crude product (914 mg). Biotage chromatography, eluting with 98:2 $CH_2Cl_2/CH_3OH(NH_3)$ gave a 3:2 mixture of diastereomers (798 mg) (87%). Prep HPLC on 50 mg of product using a chiralcel OD column, eluting with 85:15 Hex/IPA gave the title compound ($t_R$ = 6.4 min), a white powder (12.7 mg, 51%, 98% de). HRMS $[M+1]^+$ 481.1362.

<u>Example 95</u>

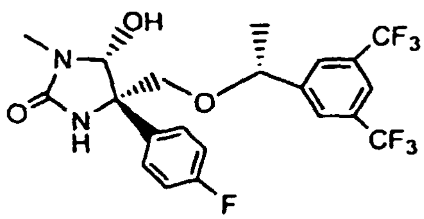

The title compound was prepared from **106** using the procedure of Example 94. Prep HPLC on 50 mg of material on chiralcel OD column, eluting with 85:15 Hex/IPA gave the title compound ($t_R$ = 8.2 min) as a clear oil (17.5 mg, 70%, 78% de). HRMS $[M+1]^+$ 481.1362

<u>Example 96</u>

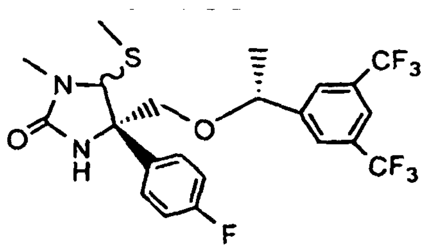

A 2:3 diastereomeric mixture of Examples 94 and 95 (100 mg, 0.208 mmol, 1 equiv.) was dissolved in $CH_2Cl_2$ (2 ml) and treated with $NaSCH_3$ (29.2 mg, 0.416 mmol, 2 equiv.). The suspension was treated with concentrated HCl (4-5 drops) and stirred at RT. The reaction was complete after 30 min as determined by TLC. The reaction mixture was diluted with $CH_2Cl_2$ (40 ml), washed with sat. aq. $NaHCO_3$ (30 ml) and brine (30 ml), dried ($Na_2SO_4$), and concentrated. The crude product was purified by Biotage chromatography, eluting with 1:1 Hex/EtOAc to give the title compound (92.1 mg, 87%) as a 1:1 mixture of diastereomers. Electrospray MS $[M+1]^+$ 511.1

Example 97

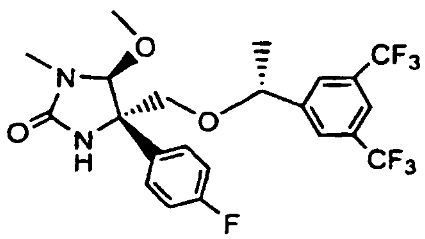

A 2:3 diastereomeric mixture of Examples 94 and 95 (100 mg, 0.208 mmol, 1 equiv.) was dissolved in $CH_3OH$ (3 ml), cooled to 0°C, and treated with concentrated HCl in $CH_3OH$ (14 drops). This was warmed to RT and stirred to completion (24h). The reaction mixture was diluted with $CH_2Cl_2$ (100 ml), washed with sat. aq. $NaHCO_3$ (30 ml) and then brine (30 ml), dried ($Na_2SO_4$), and concentrated. The crude mixture was purified by Biotage chromatography, eluting with 3:2 EtOAc/Hex (2% $NEt_3$) to give a product as a 3:2 mixture of diastereomers. Isolation by prep HPLC on an chiralcel OD column, eluting with 95:5 Hex/IPA gave the title compound ($t_R$ = 6.7 min) (29 mg, 28%). HRMS $[M+1]^+$ 495.1419

Example 98

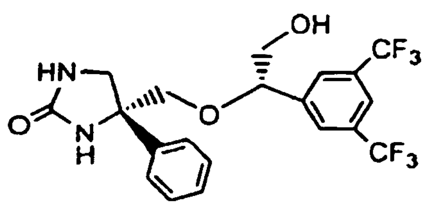

Step 1:

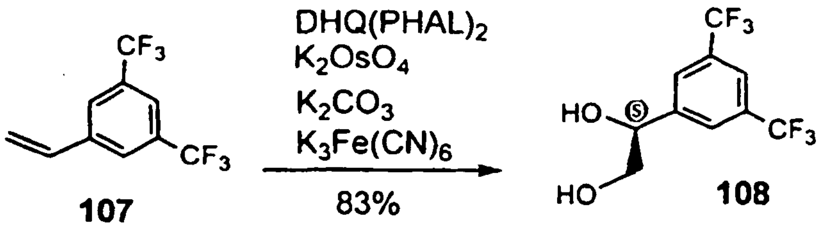


107 108

A solution of $K_2CO_3$ (31.0 g, 187.5 mmol, 3 equiv-), $K_3Fe(CN)_6$ (61.75 g, 187.5 mmol, 3 equiv.), $(DHQ)_2PHAL$ (2.44 g, 3.13 mmol, 0.05 equiv.), and $K_2OsO_4 \cdot 2H_2O$ (1.16 g, 3.13 mmol, 0.05 equiv.) in $^t$BuOH/$H_2O$ 1:1 (750 ml) was stirred and cooled to 0°C. The suspension was treated with **107** (11.25 ml, 62.5 mmol, 1 equiv.) and stirred at 0°C for 3.5 h. The reaction mixture was treated with $Na_2SO_3$ (95 g, 750 mmol, 12 equiv.) and warmed to RT. The biphasic solution was separated and the aqueous layer was extracted with EtOAc (500 ml). Combined organic fractions were washed with brine (200 ml), dried ($Na_2SO_4$), and concentrated to a yellow solid. The crude product was recrystallized from minimum 1,2-dichloroethane to give **108** (14.12 g, 83%) as a white solid. $^1$H NMR ($CD_3OD$) δ 3.67 (d, J = 5.6 Hz, 2H), 4.85 (t, J = 5.6 Hz, 1H), 7.85 (s, 1H), 7.99 (s, 2H).

Step 2:

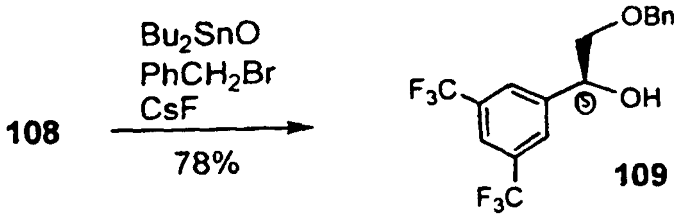


108 109

A mixture of **108** (4.0 g. 14.6 mmol, 1 equiv.) and dibutyltin oxide (3.64 g, 14.6 mmol, 1 equiv.) in toluene (60 ml) was refluxed for 2 h under a Dean Stark trap. The solution was concentrated to obtain a white solid. To this solid, CsF (4.30 g, 28.32 mmol, 1.94 equiv.) was added and the mixture was dried in vacuo overnight. Benzyl bromide (3.0 ml, 25.11 mmol, 1.72 equiv.) in dry DMF (60 ml) was added and the reaction mixture was stirred vigorously for 6 h. The solution was concentrated, taken up in EtOAc (200 ml) and the solid filtered off. The organics were extracted with water (4 x 100 ml), dried ($Na_2SO_4$), and concentrated. The crude product was purified by Biotage chromatography eluting with 9:1 Hex/EtOAc → 4:1 Hex/EtOAc to give 109 (4.15 g, 78%) as a pale yellow oil. FAB MS $[M+1]^+$ 363.0.

Step 3:

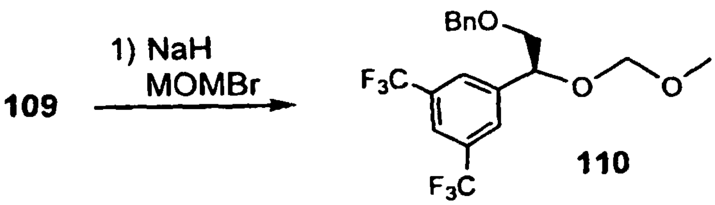


109 110

NaH (834 mg, 20.85 mmol, 1.1 equiv.) was added to a stirred solution of **109** (6.90 g, 18.95 mmol, 1 equiv.) in THF (20 ml) under $N_2$. The mixture was stirred for 1 h, then cooled to 0°C. MOMBr (2.63 g, 21.06 mmol, 1 equiv.) was added dropwise. The solution was warmed to RT and stirred for 1 h. The white mixture was filtered through a plug of celite and the filtrate was concentrated to give **110** (7.90 g, >95%) as a pure, yellow oil. $^1$H NMR ($CDCl_3$) δ 3.36 (s, 3H), 4.55 (s, 2H), 4.61 (d, 7.0 Hz, 1H), 4.75 (d, 6.6 Hz, 1H), 4.92 (t, J = 4.8 Hz, 1H), 7.21-7.31 (m, 5H), 7.82 (s, 1H), 7.83 (s, 2H).

Step 4:

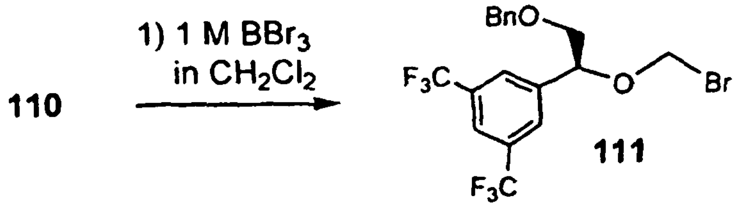


To an ice-cooled solution of **110** (5.85 g, 14.33 mmol, 3 equiv.) in $CH_2Cl_2$ (15 ml) was added a solution of 1 M $BBr_3$ in $CH_2Cl_2$ (1.64 ml, 1.64 mmol, 1 equiv.). The solution was warmed to RT and stirred for 4 h. The reaction mixture was concentrated to a crude brown oil **111** (6.1 g, 93%) which was used in the next step.

Step 5:

The title compound was prepared by a method analogous to Example 92 using **111** in place of **92** for step 4.

Example 99

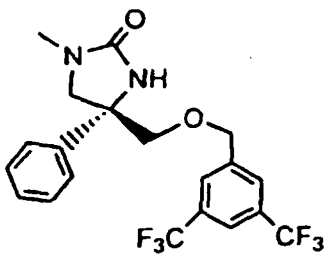

The product of Example 92 (0.25g, 0.59 mmol, 1.0 equiv.) was taken up in 4 ml dry DMF and cooled to 0 °C in a ice bath. NaH (60% dispersion in mineral oil) (0.0179g, 0.59 mmol, 1.0 equiv.) was added to the reaction mixture, the solution was warmed to RT and stirred for 45 min. $CH_3I$ (0.053 ml, 0.66 mmol, 1.1 equiv.) was added and reaction mixture was stirred at RT over night. The reaction was monitored by TLC in 4/1 EtOAc/Hexane. The reaction did not go to completion, hence was quenched with $H_2O$ (3x15ml). The mixture was extracted with EtOAc (2x15 ml) dried ($Na_2SO_4$), concentrated and dried on high vacuum. Purification using Biotage chromatography with 60/40 Hexane/EtOAc gave the title compound (0.127g, 50%).
MS $[M+1]^+$ 433.1.

Example 100

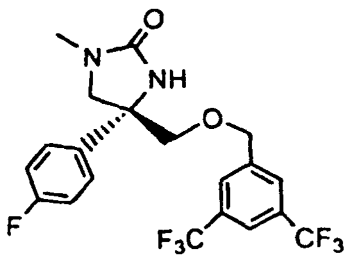

The title compound was prepared using a procedure similar to Example 99 using the product of Example 2B in place of Example 92. MS $[M+1]^+$ 451.1.

Example 101

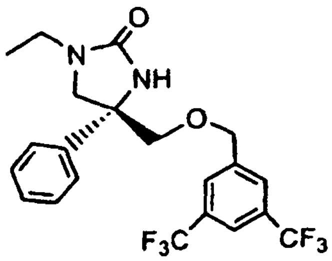

The title compound was prepared using a procedure similar to Example 99 using ethyl iodide in place of $CH_3I$ to give the title compound. MS $[M+1]^+$ of crude 447.1.

Example 102

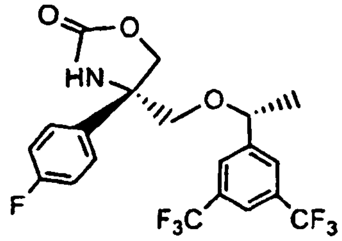

Step 1:

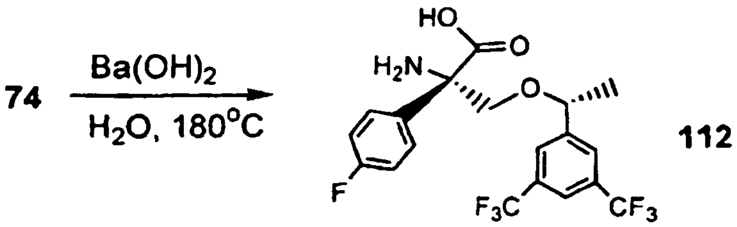


**74** (6.0 g, 13.7 mmol, 1.0 eqv), $Ba(OH)_2$ (20.8 g, 65.9 mmol, 4.8 equiv.) and $H_2O$ (50 ml) were combined in a high pressure bomb equipped with a stirring bar. It was heated at 165 °C for 64 h, and then the temperature was increased to 180 °C. The bomb was heated for another 3 days, then cooled, and the crude product was treated with $CH_3OH$ ($NH_3$) to transfer the hardened crude to solution. The mass gradually dissolved using 10 (40-50 ml) portions with sonication each time. The resulting turbid solution was filtered and concentrated. The crude was then dissolved in ether, filtered and concentrated to give pale yellow solid **112** (6.2 g, >95%).

**Step 2:**

112
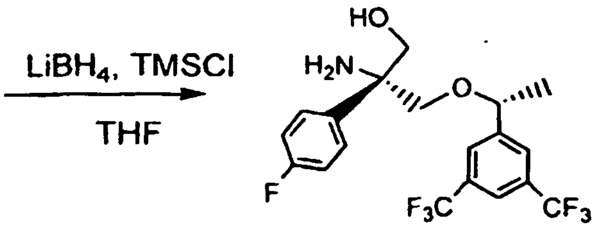

113

A solution of $LiBH_4$ (0.01 g, 0.46 mmol, 2.0 eqiv) was treated with TMSCl (0.115 ml, 0.θ1 mmol, 4.0 eqiv). After 5 min, **112** (0.1g, 0.227 mmol, 1.0 equiv.) was added as a powder and the mixture was stirred for 18 h. It was monitored by TLC 70:10:20 (EtOAc: $Et_3N$:$CH_3OH$). Upon completion, the reaction was quenched with 5ml $CH_3OH$ and stirred for another 1 h. Purification by Biotage in 97:3 $CH_2Cl_2$ /$CH_3OH$ ($NH_3$) gave the product **113** (0.65 g, 67 %).

<u>Step 3:</u>

A solution of **113** (0.63 g, 0.148 mmol, 1.0 equiv) in dry $CH_2Cl_2$ (4ml) was treated with diisopropyl ethyl amine (0.077 ml, 0.444 mmol, 3.0 equiv.) followed by triphosgene (0.0176 g, 0.059 mmol, 0.4 equiv.). The reaction mixture was stirred at RT for 10 min. Monitoring by TLC 2/1 Hexane/EtOAc showed reaction completion. The crude product was purified using Biotage chromatography, eluting with 98/2 $CH_2Cl_2$ / $CH_3OH$ to give the title compound as a white foam (0.58 g, 86 %). MS $[M+1]^+$ 452.

<u>Example 103</u>

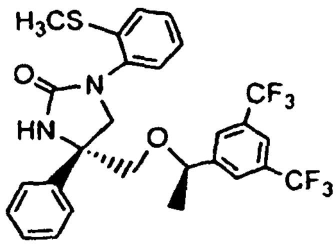

Step 1

104
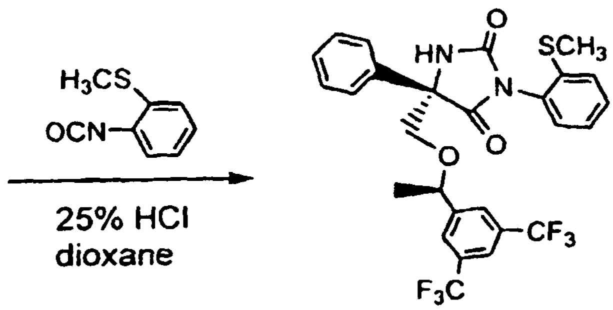

114

To a solution of **104** (0.225 g, 0.518 mmol) in $CH_2Cl_2$ (3 ml) was added 2-methylthiophenylisocyanate and the resulting solution was stirred for 12 h. The solvent was removed and the residue treated with dioxane/25% HCl (4 ml, 2:1., v/v) and heated at 90°C overnight. After cooling to RT, the solution was diluted with EtOAc and water and the layers separated. The aqueous layer was extracted with EtOAc three times. The combined organic layers were washed with half saturated $NaHCO_3$ solution and brine, dried over $MgSO_4$, filtered and concentrated. Flash chromatography afforded **114** (0.265 g, 95%). Electrospray MS $[M+1]^+$ 569.1

Step 2:

To solid $AlCl_3$ (346.7 mg, 2.6 mmol) at 0° C under $N_2$ was added $LiAlH_4$ (2.03 ml, 1M in $Et_2O$, 2.03 mmol). The resulting suspension was stirred for 10 min, then a solution of **114** (320 mg) in THF (19 ml) was cannulated into the hydride suspension dropwise. After 5 min, the solution was allowed to warm to 23 °C and stirred for 2 h. The reaction mixture was cooled to 0°C, quenched with 10 ml saturated sodium potassium tartrate, stirred at 23 °C for 2 h, then patitioned between water and EtOAc. The organic layer was washed with brine, dried over $MgSO_4$, filtered and concentrated.

Step 3

A solution of the crude hydroxyurea from Step 2 in $CH_2Cl_2$ (3 ml) was treated with triethylsilane (1.5 ml) followed by addition of TFA (0.178 ml), stirred overnight, further treated with TFA (0.098 ml) and stirred for 4 h. The mixture was concentrated, then re-dissolved in $CH_2Cl_2$ and stirred with solid $K_2CO_3$ for 2 h. The solution was filtered and concentrated. Flash chromatography afforded the title urea. Electrospray
MS $[M+1]^+$ 555.1.

Examples 104-111

The following examples were prepared in a similar fashion to Example 103 using the appropriate isocycanate:

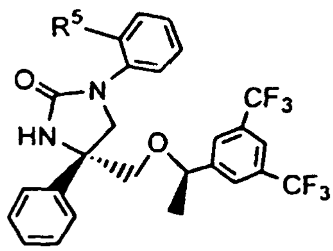

| Example | $R^5$ | MS $[M+1]^+$ |
|---|---|---|
| 104 | 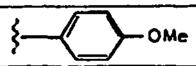 | 539.1 |
| 105 | 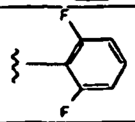 | 545.1 |
| 106 | 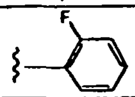 | 527.1 |
| 107 | 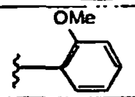 | 539.1 |
| 108 | 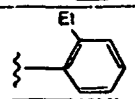 | 537.1 |
| 109 | 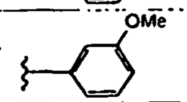 | 539.1 |
| 110 | 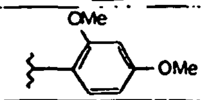 | 569.1 |
| 111 | 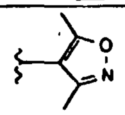 | 528.1 |

Examples 112-113

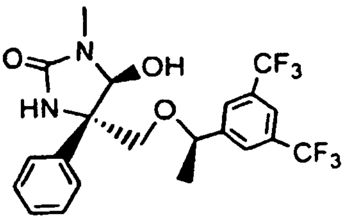

Ex. 112

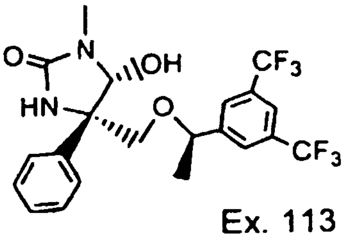

Ex. 113

Step 1:

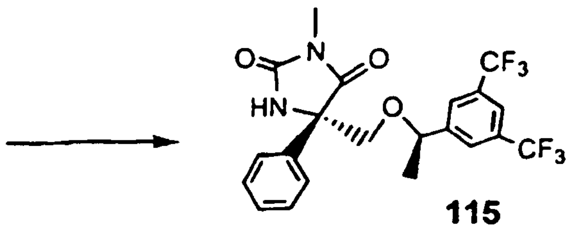

104

115

**104** (1.26 g, 2.9 mmol) was dissolved in $CH_2Cl_2$ (58 ml) at 0 °C, followed by addition of $Et_3N$ (1.2 ml, 8.7 mmol). Triphosgene (0.35g, 1.13 mmol) was added in one portion. The solution was allowed to warm to 23 °C and stirred for 2 h. The reaction was then diluted with EtOAc, washed with 5% HCl(aq.), half saturated $NaHCO_3$ solution and brine, then dried over $Na_2SO_4$, filtered, and concentrated under reduced pressure. The residue was purified by flash column chromatography to give **115** (1.31g, 98%).

Step 2:

Using a procedure similar to Example 94, step 2, a mixture of Examples 112 and 113 was prepared. Separation by silica gel chromatography using gradient of hexane to EtOAc/$NEt_3$ 9:1:provided the title compounds.

Examples 114-115

The following examples were prepared in a similar fashion to Example 103, steps 1-2, omitting step 3 and using the appropriate isocycanate:

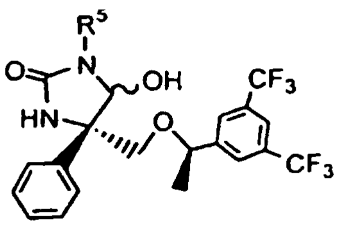

| Example | $R^5$ | MS $[M+1]^+$ |
|---|---|---|
| 114 | 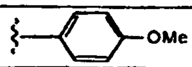 | 555.1 |
| 115 | 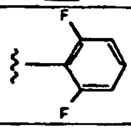 | 561.1 |

Examples 116-117

The following examples were prepared in a similar fashion to Example 96, using 116 to prepare Example 116 and Example 115 to prepare Example 117 in place of hydroxyureas from Examples 94 and 95:

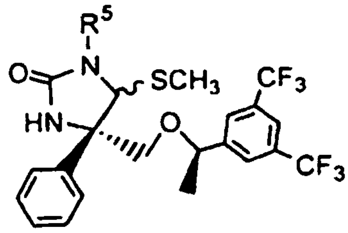

| Example | $R^5$ | MS $[M+1]^+$ |
|---|---|---|
| 116 | Me | 493.1 |
| 117 | 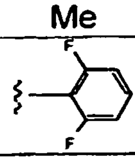 | 591.1 |

Examples 118-119

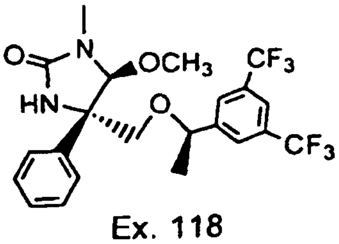

Ex. 118

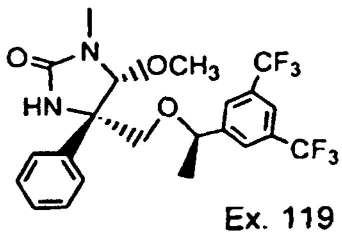

Ex. 119

To the alcohol mixture of Examples 112 and 113 was added saturated dry HCl in $CH_3OH$ (5 ml, precooled to -20 ° C). The solution was stirred at 23 °C for 2 h, then poured into 10% $Na_2CO_3$ (25 ml). The aqueous layer was extracted with EtOAc (3 X 30 ml). The combined organic layer was washed with brine, dried over $Na_2SO_4$, filtered and concentrated under reduced pressure. Flash chromatography afforded an inseparable mixture of two methyl ethers. The mixture was then separated on HPLC using Chiralpak OD eluted with (95:5) Hexane/iPA. Electrospray MS $[M+1]^+$ 477.1

Example 120

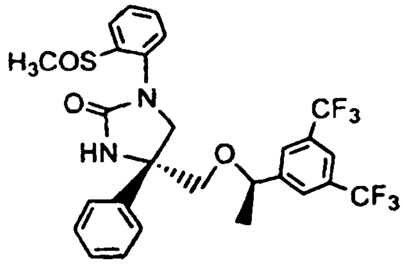

To a solution of Example 103 (170 mg, 0.306 mmol) in $CH_2Cl_2$ was added solid Oxone (1.14g, 17.4 mmol), the resulting suspension was stirred vigorously for 20 h, then quenched with saturated $NaHCO_3$ solution and extracted with EtOAc. The organic layers were washed with brine, dried over $Na_2SO_4$ and concentrated. Flash chromatography afforded the title compound. Electrospray MS $[M+1]^+$ 57 1.1

Example 121

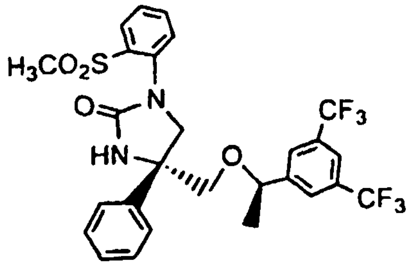

To a solution of Example 120 in $CH_2Cl_2$ was added m-CPBA and the resulting solution was stirred for 30 h, then quenched with $NaHCO_3$ solution and extracted with EtOAc. The organic layers were washed with brine, dried over $Na_2SO_4$ and concentrated. Flash chromatography afforded the title compound. Electrospray MS $[M+1]^+$ 587.1.

Example 122

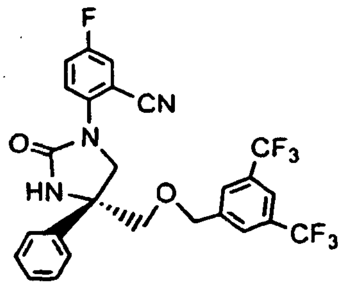

To Example 92 (0.150 g, 0.36 mmol), CsF (0.109 g, 0.72 mmol) in DMF (3.5 ml) at 0°C and NaH 0.018 g (60% in mineral oil) was added. After stirring for 10 min, 2,5-difluorobenzonitrile (0.0587 g, 0.0378 mmol) was added in small portions. The solution was allowed to warm to 23 °C and was stirred overnight. The reaction was quenched with saturated $NH_4Cl$ solution, extracted with EtOAc, and the combined organic layer was washed with water and brine, dried over $Na_2SO_4$ and concentrated. Flash chromatography afforded the title compound (0.051 g, 26%). Electrospray MS $[M+1]^+$ 538.1.

Example 123

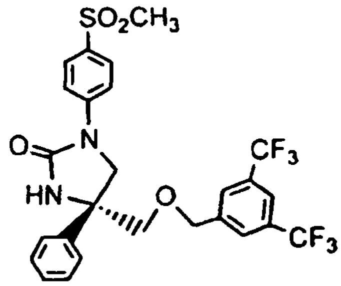

In a procedure similar to Example 122, 4-F-phenylmethylsulfone was used in place of 2,5-difluorobenzonitrile to obtain the title compound. Electrospray MS $[M+1]^+$ 573.1.

Example 124

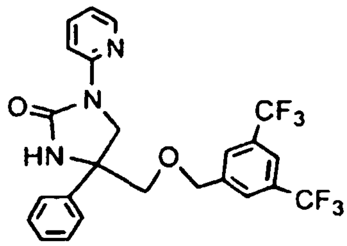

In a procedure similar to Example 122, 2-F-pyridine was used in place of 2,5-difluorobenzonitrile and Example 1 was used in place of Example 92 to obtain the title compound. Electrospray MS $[M+1]^+$ 496.1

Example 125

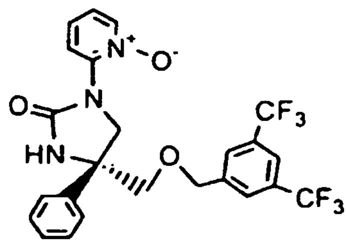

In a procedure similar to Example 122, 2-F-pyridine was used in place of 2,5-difluorobenzonitrile in step 1, followed by a procedure similar to that used in Example 121 for the oxidation to the N-oxide to obtain the title compound. Electrospray MS $[M+1]^+$ 512.1.

Examples 126-127

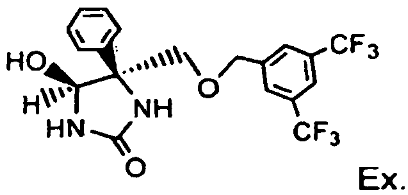

Ex. 126

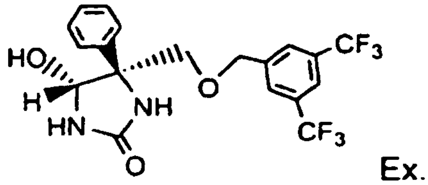

Ex. 127

Step 1:

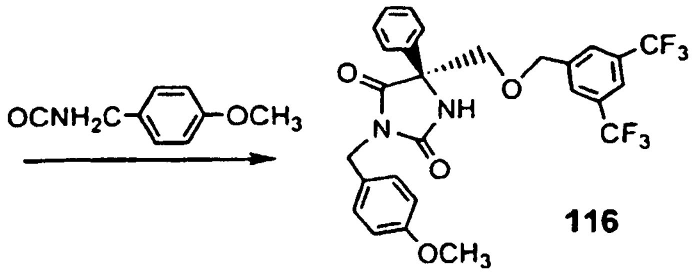

97

116

A mixture of hydantoin (prepared from **97** using procedures similar to Example 61, step 6) (5.0 g, 11.6 mmol, 1 equiv.), and p-methoxybenzylisocyanate (2.5 ml, 17.4 mmol, 1.5 equiv.) in dry dioxane (20 ml) was stirred at RT for 3 h, then 3N aqueous HCl (20 ml) was added and the mixture stirred at 90 °C for 14 h. The mixture was poured into 250 ml EtOAc and washed with $H_2O$ (2 x 125 ml). The organic layer was dried over anhydrous $Na_2SO_4$, filtered and concentrated to give 6.5 g of the crude racemic **116.**

Step 2

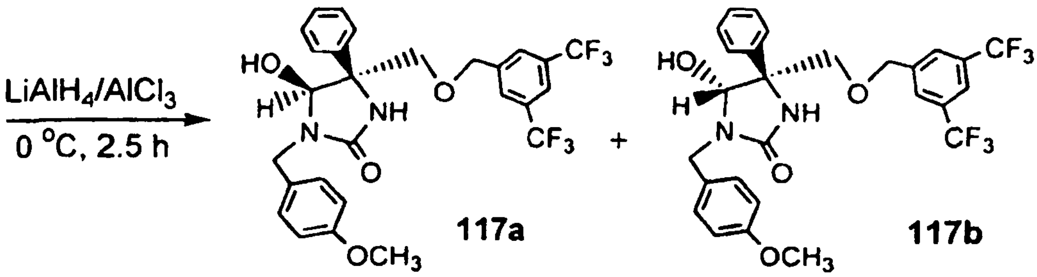


116

+

117a

117b

$LiAlH_4$ (35 ml of 1 M solution in ether, 35 mmol, 3 equiv.) was added slowly to $AlCl_3$ (6.3 g, 47.06 mmol, 4 equiv.) at 0°C. and stirred for 10 min, then a solution of **116** (6.5 g, 11.76 mmol, 1 equiv.) in dry THF (70 ml) was carefully added. After stirring at 23 °C for 2.5 h, the mixture was cooled to 0°C, quenched slowly with 30 ml saturated aqueous sodium potassium tartarate and stirred for 14 h at 23 °C. The mixture was diluted with water (100 ml), and extracted with EtOAc (2 x 200 ml). The organic layers were dried over anhydrous $Na_2SO_4$, filtered and concentrated. Purification using 400 ml silica and eluting with 2/1 hexane/EtOAc provided 4.62 g of the product as white solid.

Step 3:

To a suspension of **117a/b** (4.62 g, 8.33 mmol, 1 equiv.) in $CH_3CN$/water (150 ml, 2:1) at RT was added ceric ammonium nitrate (18.27 g, 33.33 mmol, 4 equiv.). After stirring at 23 °C for 1 h, the mixture was poured into 300 ml EtOAc/ 150 ml saturated aq. $NaHCO_3$ and filtered through a frit. The organic layer was isolated, the aqueous layer was washed with EtOAc (1 x 300 ml) and the combined organic layers were dried over anhydrous $Na_2SO_4$, filtered and

concentrated. Purification using the Biotage silica gel system, eluting with 1/1 hexane/EtOAc. 1 L, followed by 5% $CH_3OH$/EtOAc, 1 L, provided 2.0 g of the product as a mixture of two isomers. HPLC separation on chiralpak AD column, eluting with (90/10) hexane/IPA mixture gave Example 127 FAB, ($M^{+1}$) = 435.0, and Example 126, FAB, ($M^{+1}$) = 435.0.

Examples 128-129

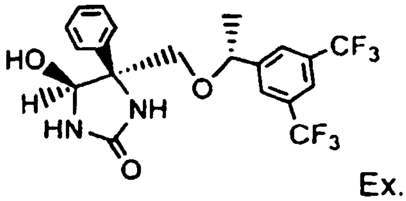

Ex. 128

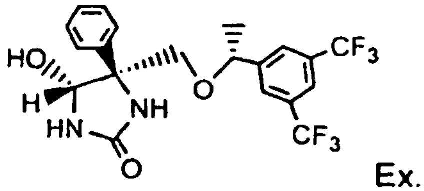

Ex. 129

Step 1:

A mixture of **105** (0.976 g, 2.19 mmol, 1 equiv.), $K_2CO_3$ (0.453 g, 3.28 mmol, 1.5 equiv.) in dry DMF (10 ml) was stirred at RT for 30 min. *p*-Methoxy-benzyl chloride (0.34 ml, 2.5 mmol, 1.15 equiv.) was added at once and the resulting mixture was stirred at 23°C for 14 h. The mixture was then poured into EtOAc (150 ml) and washed with $H_2O$ (3 x 100 ml) and saturated aq. NaCl (100 ml). The organic layer was dried over $Na_2SO_4$, filtered and concentrated to give 1.27 g of the crude product. It was used in the next step without further purification.

Step 2:

The product of step 1 was converted to Examples 128 and 129 using procedures similar to Examples 126 and 127, steps 2-3, using LAH in place of LAH/$AlCl_3$ in step 2 to provide 0.14 g of the products, obtained as mixture of two isomers. HPLC separation on chiralpak AD column using (85/15) hexane/IPA mixture gave 80 mg (29% yield) of Example 129, FAB, ($M^{+1}$) 449.2 and 30 mg (11% yield) of Example 128 FAB, ($M^{+1}$) 449.2.

Example 130

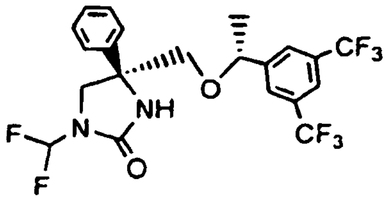

Step 1:

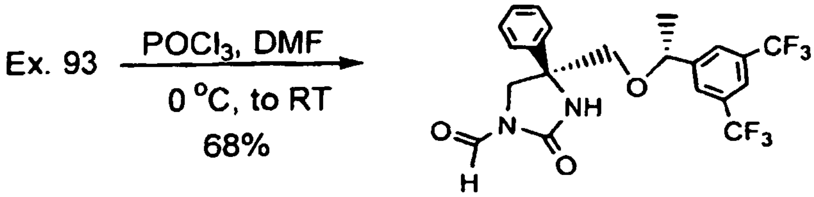


118

To a solution of Example 93 (0.1 g, 0.23 mmol, 1.0 equiv.) in dry DMF (0.5 ml) cooled to 0 °C, $POCl_3$ (0.024 ml, 0.254 mmol, 1.1 equiv.) was added slowly. The mixture was warmed to RT, stirred for 30 min, and poured into 5 g ice. The resultant mixture was poured into water (100 ml) and extracted with EtOAc (100 ml). The organic layer was separated, washed with saturated aq. NaCl (1 x 100 ml), dried over anhydrous $Na_2SO_4$ and concentrated. Flash chromatography over 200 ml silica using (1) 4/1 hex/EtOAc, and (2) 1/1 hex/EtOAc gave 0.070 g (66% yield) of 118 as solid. MS ($M^{+1}$) 461.1.

Step 2:

**118**

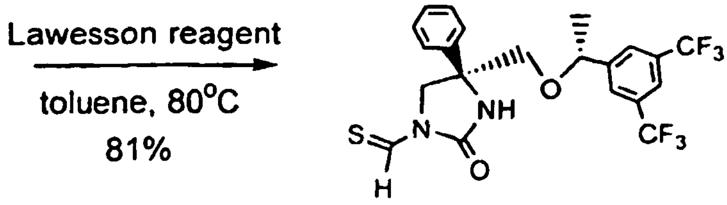


**119**

**118** (0.1 g, 0.22 mmol, 1.0 equiv.) and Lawesson's reagent (0.044 g, 0.108 mmol, 0.5 equiv.) in toluene (1 ml) were heated at 80 °C for 0.5 h. The solvent was evaporated and the residue purified by Biotage chromatography using 15% EtOAc/hexane to obtain **119** as a yellow foam, 0.085g (81% yield).

Step 3:

**119** (0.3 g, 0.626 mmol, 1.0 equiv.) was taken up in $CH_2Cl_2$ (3 ml) at RT. DAST (0.17 ml, 1.25 mmol, 2.0 equiv.) was added slowly and the mixture was stirred at RT overnight. The reaction was slowly quenched with saturated aq. $NaHCO_3$ (5 ml), the mixture was poured into saturated aq. $NaHCO_3$ (100 ml) and $CH_2Cl_2$ (100 ml) added. The organic layer was separated, dried over anhydrous $Na_2SO_4$, filtered and concentrated. The residue was purified by Biotage chromatography using 4/1 hex/EtOAc to obtain the title compound, 0.080 g (27% yield). MS ($M^{+1}$) = 483.1.1.

Example 131

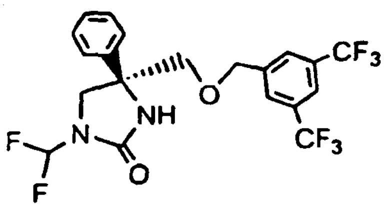

Using procedures similar to Example 130, substituting Example 92 for Example 93, the title compound was prepared. Electrospray MS $[M+1]^+$ 469.1

Example 132

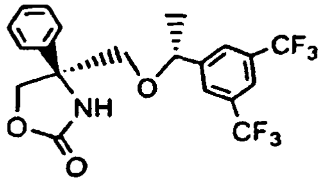

Using procedures similar to those in Example 102, and substituting 97 for 74 in step 1, the title compound was prepared. Electrospray MS $[M+1]^+$ 434.1.

Example 133

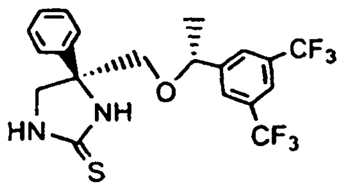

Example 93 (0.633 g, 1.465 mmol, 1.0 equiv.), Lawesson's reagent (0.81 g, 2.04 mmol, 1.37 equiv.) and toluene (12 ml) were heated at 85 °C for 1.5 h, then cooled to RT and concentrated. The residue was purified by silica gel chromatography using 15% EtOAc/hex, then 10% EtOAc/$CH_2Cl_2$ to give 0.61 g (93% Yield) of the title compound. MS $(M^{+1})$ = 449.1.

Example 134

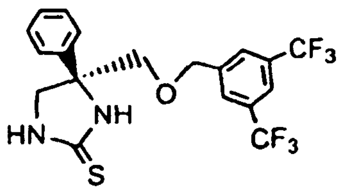

A procedure similar to that used in Example 133, using Example 92 in place of Example 93, provided the title compound. MS $(M^{+1})$ = 435.1

Examples 135-139

The compounds were prepared using procedures similar to those used in Example 99, using Example 92 and the appropriate alkyliodide . For Example 139, Example 76 was used as the starting cyclic urea.

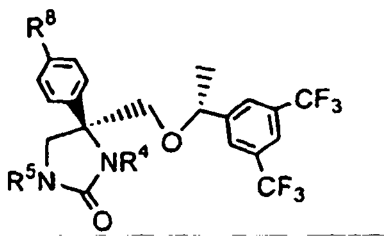

| Example | $R^5$ | $R^4$ | $R^8$ | MS $[M+1]^+$ |
|---|---|---|---|---|
| 135 | Me | H | H | 447.1. |
| 136 | Et | H | H | 461.1. |
| 137 | H | Et | H | 461.1. |
| 138 | *i*Pr | H | H | 475.1. |

Table continued

| Example | $R^5$ | $R^4$ | $R^8$ | MS $[M+1]^+$ |
|---|---|---|---|---|
| 139 | Et | H | F | 479.1. |

Examples 140-141

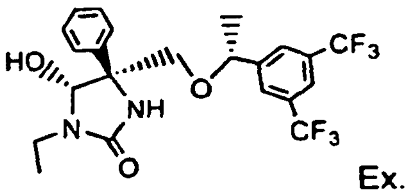

Ex. 140

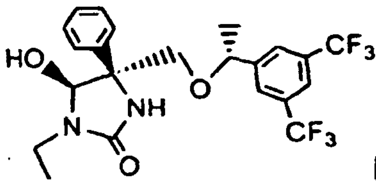

Ex. 141

The title compounds were prepared using procedures similar to those in Example 94, using ethyl isocyanate in place of methyl isocyanate. Purification via HPLC (Chiralpak AD column using 98/2 hex/isopropanol) provided Example 140 MS $[M+1]^+$ 477.1 and Example 141 MS $[M+1]^+$ 477.1.

Example 142

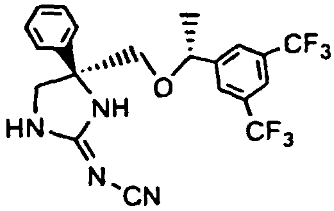

Example 133 (0.61 g, 1.36 mmol, 1.0 equiv.) in THF (20 ml) was treated with $CH_3I$ (0.10 ml, 1.63 mmol, 1.2 equiv.), stirred for 14 h, then concentrated. The crude was dissolved in $CH_3OH$ (20 ml), treated with $NH_2CN$ (0.37 g, 8.84 mmol, 6.7 equiv.) and heated to 60 °C for 14 h. The mixture was concentrated and purified by silica gel chromatography, using 1/1 EtOAc/hex to give the title compound, 0.1 g (17% yield) as a white solid. MS ($M^{+1}$) = 457.1.

Example 143

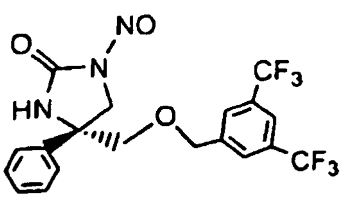

To Example 92 (2.0 g, 4.78 mmol) in THF (15 ml) at 0°C was added $NaNO_2$ (0.39 g, 5.7 mmol) in $H_2O$ (7.5 ml), then $H_2SO_4$ (conc.) (1 ml) was added slowly. The solution was allowed to warm to 23°C and stirred for 1 h. It was then diluted with water and extracted with EtOAc. The organic layers were washed with $NaHCO_3$ solution, brine, dried over $Na_2SO_4$ and concentrated to give the title compound. Electrospray MS $[M+1]^+$ 448.1.

Example 144

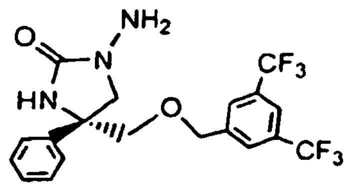

To Example 143 (0.95 g, 2.1 mmol) in $Et_2O$ (10 ml) at 0° C was added dropwise $LiAlH_4$ (4.2 ml, 1 M in $Et_2O$). The solution was allowed to warm to 23°C and stirred for 2 hr. It was quenched with saturated K,Na tartrate solution and then partitioned between water and EtOAc. The aqueous layer was extracted with EtOAc. The combined organic layers were washed with brine, dried over $Na_2SO_4$ and concentrated to give the title compound. Electrospray MS $[M+1]^+$434.1

Example 145

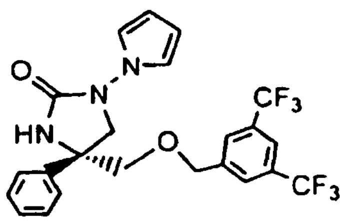

To Example 144 (0.3 g, 0.69 mmol) in acetic acid (3 ml) was added 2,5-dimethoxy-3-tetrohydrofuran (3 ml) and the mixture was heated at 70° C for 1.5 h. It was cooled to 23°C and diluted with EtOAc. The organic layers were washed with $NaHCO_3$, brine, dried over $Na_2SO_4$ and concentrated. A stream of $N_2$ was introduced into the residue to remove excess 2,5-dimethoxy-3-tetra-hydrofuran. The crude product was purified by silica gel chromatography to give the title compound. Electrospray MS $[M+1]^+$ 484.1.

Example 146

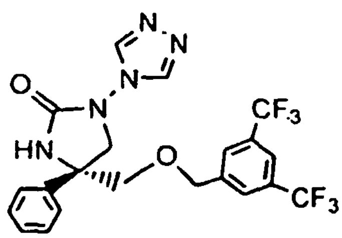

Example 144 (0.65 g, 1.5 mmol) in pyridine (15 ml) was concentrated under reduced pressure at 50° C using a water bath. The procedure was repeated twice. The residue was treated with 1,2-diformylhydrozine (0.34g, 3.6 mmol), followed by the addition of pyridine (7.5 ml), $Et_3N$ (1.5 ml) and TMSCl (3 ml). The thick paste was heated at 80 °C under $N_2$ for 65 h, then concentrated under reduced pressure to give a yellow residue. The residue was partitioned between water and EtOAc. The aqueous layer was extracted with EtOAc. The combined organic layers were washed with brine, dried over $Na_2SO_4$ and concentrated to give a crude product. Further purification using biotage followed by a prep TLC afforded the title compound. Electrospray MS $[M+1]^+$486.1.

Example 147

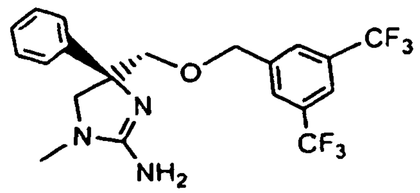

The title compound was prepared in a method analogous to Example 73, using 89 in place of 6. The title compound was obtained in 95% yield. Electrospray MS $[M+1]^+$ 432.1.

Example 148

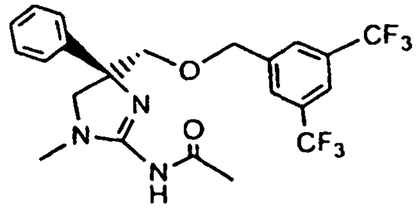

To a cooled solution of Example 147 (0.3 g, 0.70 mmol), DEC (0.14 g, 0.73 mmol), HOBT (0.1 g, 0.74 mmol) and $NaSO_4$ (0.59 g) in anhydrous $CH_2Cl_2$ (4.5 ml), glacial acetic acid (0.05 ml, 0.87 mmol) was added followed by $Et_3N$ (0.1 ml, 0.72 mmol). The reaction was allowed to react for 18 h at RT, then quenched with brine. The aqueous solution was extracted with $CH_2Cl_2$ (50 ml X 3). The combined organic layer was dried, filtered and concentrated. The pure title compound was obtained through flash chromatography, eluting with 2% (1:9) $NH_4OH$-$CH_3OH$ in $CH_2Cl_2$ to give the final product in 39% yield. Electrospray MS $[M+1]^+$ 474.1.

Example 149

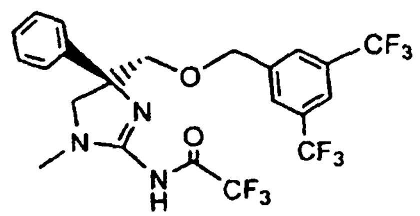

To a cooled solution of Example 147 (0.2 g, 0.46 mmol) in anhydrous $CH_2Cl_2$ (5 ml) at 0 °C, trifluoroacetic anhydride (0.09 ml, 0.64 mmol) neat was added followed by $Et_3N$ (0.08 ml, 0.57 mmol). The reaction was allowed to react for 18 h at RT. Volatile solvents were evaporated. The pure title compound was obtained through flash chromatography, eluting with 50% EtOAc in hexane to give the final product in 33% yield. Electrospray MS $[M+1]^+$ 528.1.

Example 150

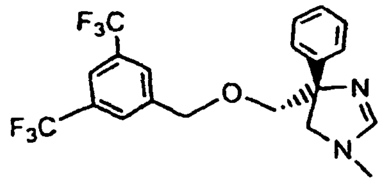

A mixture of **89** (0.38 g, 0.93 mmol) and N; N-dimethylformamide dimethyl acetal (0.11 g, 0.93 mmol) was heated to 60 °C for 18 h. The reaction mixture was purified by chromatography, eluting with 3.5 % $NH_3$-$CH_3OH$ (1:9)/ 96.5 % $CH_2Cl_2$ to give the title compound later as a HCl salt (0.2 g, 50 %). FAB MS [M+1] 417.

Example 151

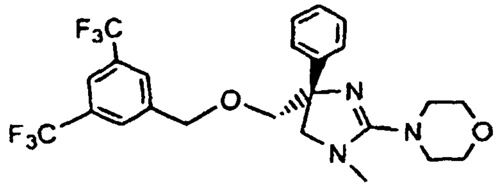

Using a method analogous to Example 82, using morpholine in place of 7 M $NH_3$ in $CH_3OH$, the title compound was obtained as a solid in a 36 % yield. FAB MS [M+1] 502.

Example 152

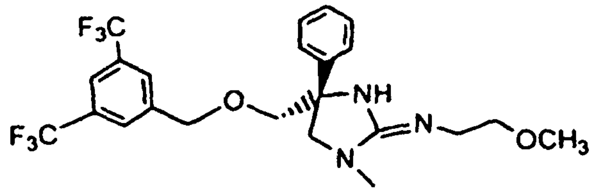

Using a method analogous to that Example 82, using 2-methoxyethyl-amine in place of 7 M $NH_3$ in $CH_3OH$, the title compound was obtained as a solid in a 80 % yield. FAB MS [M+1] 490.1.

Example 153

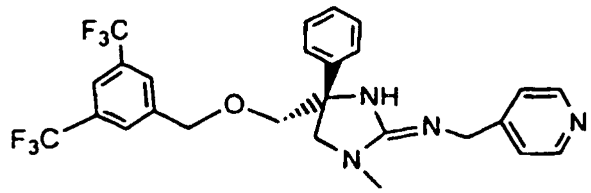

Using a method analogous to Example 82, using 4-(aminomethyl)pyridine in place of 7 M $NH_3$ in $CH_3OH$. the title compound was obtained as a solid in a 26 % yield. FAB MS [M+1] 523.4.

Example 154

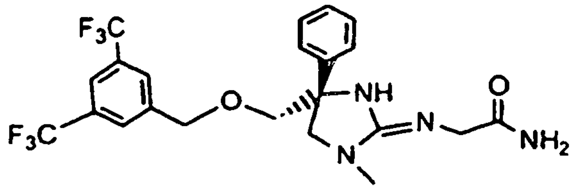

Using a method analogous to Example 82, using glycinamide in place of 7 M $NH_3$ in $CH_3OH$, the title compound was obtained as a solid in a 14 % yield. FAB MS [M+1] 489.2.

Example 155

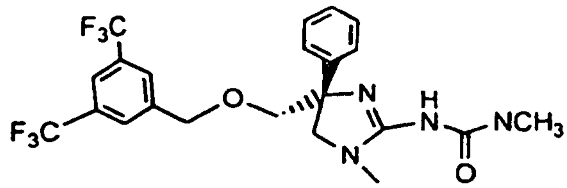

A solution of Example 82 (0.2 g, 0.464 mmol) in $CH_2Cl_2$ (2 ml) and isocyanate (53 mg, 0.93 mmol was stirred under $N_2$ at RT for 18 h. After work-up, a solid was obtained as a crude product which was then purified by chromatography, eluting with 2 % $NH_3$-$CH_3OH$ (1:9) / 98 % $CH_2Cl_2$ to give the title compound as a solid (55 mg, 24 %). FAB MS [M+1] 489.3.

Example 156

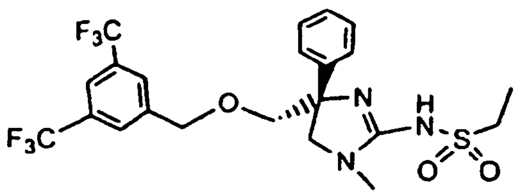

Using a method analogous to Example 155, using ethane sulfonyl chloride in place of isocyanate, the title compound was obtained later as a HCl salt in a 25 % yield. FAB MS [M+1] 524.3.

Example 157

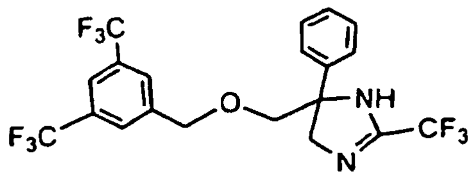

Using a method analogous to Example 87, using 6 in place of 89 and TFA in place of acetic acid, the title

compound was later obtained as a HCl salt in a 34 % yield. FAB MS [M+1] 471.

Example 158

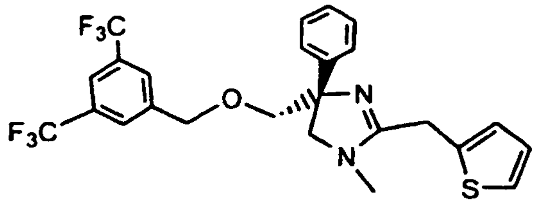

Using a method analogous to Example 89, using 2-thiophene acetic acid in place of acetic acid, the title compound was later obtained as a HCl salt in a 24 % yield. FAB MS [M+1] 512.9.

Example 159

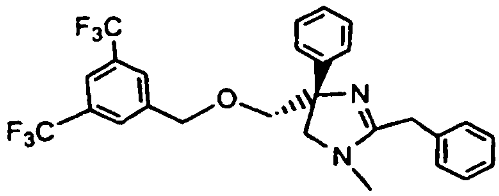

Using a method analogous to Example 89, using phenyl acetic acid in place of acetic acid, the title compound was later obtained as a HCl salt in a 15 % yield. FAB MS [M+1] 507.1.

Example 160

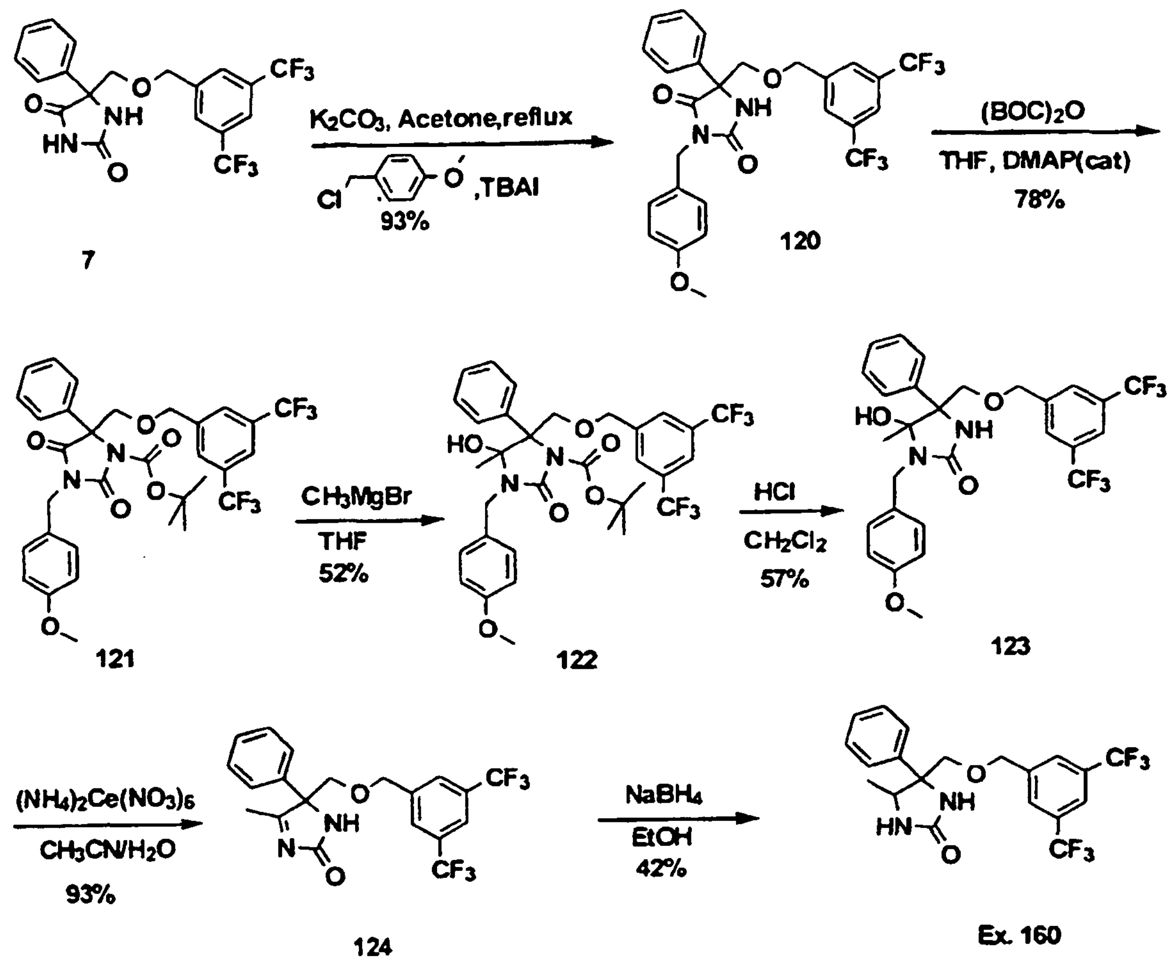


7

120

121

122

123

124

Ex. 160

Step 1 A mixture of hydantoin 7 (9.2 g, 21.3 mmol, 1 equiv.), $K_2CO_3$ (8.8g, 63.9 mmol, 3.0 equiv.) and tetrabutylammonium iodide (0.79 g, 2.13 mmol, 0.1 equiv.) in 180 mL acetone was stirred at room temperature for 30 minutes, treated with p-methoxybenzyl chloride (3.25 mL, 23.9 mmol, 1.12 equiv.) at reflux for 2 hours, then cooled to room temperature and filtered through sintered glass funnel. The filtrate was concentrated to give pale yellow solid, which was washed with small amount of ice-cold $Et_2O$ (2 × 5 mL) to give 11 g of the crude racemic p-methoxybenzyl (pmb) -hydantoin **120**. It was used in the next step without further purification.

Step 2 A mixture of pmb-hydantoin **120** (6.0 g, 10.9 mmol, 1 equiv.), 4-(dimethylamino)pyridine (DMAP) (0.02 g, 0.16 mmol, 1.5% equiv.) in 60 mL dry THF was stirred at room temperature for 1.5 hours. The solvent was evaporated under vacuum. The residue was taken up in $CH_2Cl_2$ (150 mL) and washed with saturated sodium bicarbonate (50 mL). The organic layer was dried over anhydrous $Na_2SO_4$, then concentrated. The crude was purified with Biotage (EtOAc/ Hexane=10%) to give 5.5 g (77.7% yield) of BOC-pmb-hydantoin 121 as white solid.

Step 3 A solution of BOC-pmb-hydantoin **121** (540 mg, 0.83 mmol, 1 equiv.) in 10 mL dry THF was treated with 3.0 M methylmagnesium bromide $Et_2O$ solution (0.42 mL, 1.25 mmol, 1.5 equiv.) at 0 C. After addition, the reaction was warmed up to room temperature gradually and stirred for another hour. THF was evaporated under vacuum. The residue was taken up with 100 mL $CH_2Cl_2$, then washed with 10 mL of saturated aq. $NaHCO_3$ solution. The resulting white solid was filtered off. The filtrate was concentrated and purified by Biotage (EtOAc/Hexane=20%) to give 290 mg (52.3% yield) of Methyl-hydroxy-BOC-pmb-urea **122** as white solid.

Step 4 A solution of methyl-hydroxy-BOC-pmb-urea **122**(310 mg, 0.46 mmol, 1 equiv.) in 5 mL of dry $CH_2Cl_2$ was treated with 1.16 mL of 4.0 M HCl in 1,4-dioxane at 0 C. The reaction was allowed to warm up to room temperature and stirred overnight. The crude product was taken up with $Et_2O$, then washed with 3 mL of saturated $NaHCO_3$. The aqueous layer was further extracted with ether. The combined organic layer was dried over $Na_2SO_4$, then concentrated, and purified with Biotage (EtOAc/Hexane=15%) to give 150 mg (56.5% yield) of Methyl-hydroxy-pmb-urea **123** as white solid.

Step 5 A white suspension of Methyl-hydroxy-pmb-urea **123** (1g, 1.76 mmol, 1 equiv.) in 22.5 mL of $CH_3CN$ and 6.75 mL of water was treated with ceric ammonium nitrate (7.72 g, 14.0 mmol, 8 equiv.). Stirred at room temperature

for 8 hours, then partitioned between 300 mL of EtOAc and 100 mL of saturated aq. $NaHCO_3$. The yellow solid was filtered off and aqueous layer was further extracted with 2 x 100 mL EtOAc. Combined organic layers were dried over anhy. $Na_2SO_4$, filtered and concentrated. Flashed over Biotage (EtOAc/Hexane/$Et_3N$=1:1:2%) to give 703 mg (93% yield) of **124** as white solid.

Step 6 A solution of **124** (312 mg, .93 mmol, 1 equiv.) in 8 mL EtOH was reacted with $NaBH_4$ (600 mg, 15.9 mmol, 17 equiv.) at room temperature for 2 days. The crude was partitioned between 2 X 100 mL of $CH_2Cl_2$ and 100 mL saturated aq. $NaHCO_3$, then washed with 80 mL of Brine. The combined organic layer was dried over anhy. $Na_2SO_4$, filtered, concentrated to give 300 mg of liquid as crude. It was purified with Biotage (EtOAc/Hexane=30%) to give two diastereomers (100mg and 70 mg each), which were separated on HPLC with a ChiralCel OD column to give the 4 stereoisomers of example 160. MS: (M+1)= 433.

Example 161

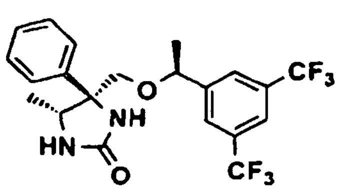

**Example 161a**

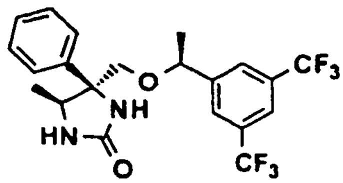

**Example 161b**

Examples **161a** and **161b** were synthesized using procedure similar to example **160** starting from optical pure hydantoin **105.** MS: (M+1)=447.

Examples 162-164

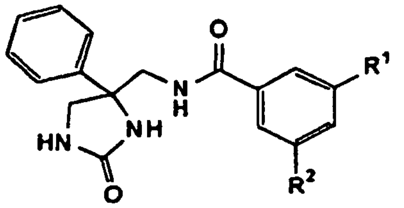

| Example | $R^1$ | $R^2$ | MS $[M+1]^+$ |
|---|---|---|---|
| 162 | F | $CF_3$ | 382.1. |
| 163 | Cl | Cl | 364.1. |
| 164 | $CF_3$ | $CF_3$ | 432.1 |

The title compounds were prepared using the acylation procedure similar to those used in example 21 using amnie **39** and the appropriate substituted benzoyl chloride to provide the title benzamides.

Example 165

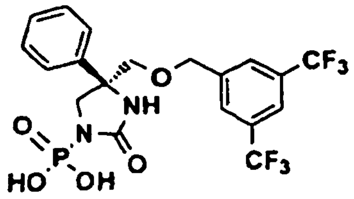

• 2 N-methylglucamine

**Example 165a**

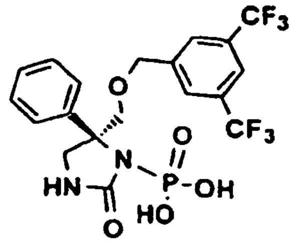

• 2 N-methylglucamine

**Example 165b**

Step 1

A solution of **Example 92** (224 mg, 0.536 mmol, 1 equiv.) in THF (12 mL) at 0°C was treated with 2.5 M n-BuLi (215 μL, 0.536 mmol, 1 equiv.). The resulting mixture was stirred cold for 5 min. Tetrabutyl pyrophosphate (405 mg. 0.751 mmol, 1.4 equiv.) was added to the reaction mixture as a solid in one portion. The cooling bath was removed and the reaction was stirred at room temperature for 45 minutes at which point a white suspension formed. The reaction was quenched with sat. aq. $NaHCO_3$ (20 mL) and extracted with EtOAc (2 x 30 mL). The solution was dried ($Na_2SO_4$), filtered, and concentrated to give crude product (461 mg). This was not purified and used crude in the next step.

Step 2

A solution of the product of step 1 (364 mg, 0.536 mmol, 1 equiv.) in MeOH (10 mL), a solution of N-Me-D-glucamine (206 mg, 1.072 mmol, 2 equiv.) in $H_2O$ (2 mL), and 10% Pd/C (29 mg) were combined and the mixture was hydrogenated at 40 psi for 2 h. The reaction mixture was filtered through a pad of Celite and rinsed with MeOH (80 mL). The solution was concentrated under reduced pressure and the crude product redissolved in MeOH (5 mL)_. $^{i}$PrOH (25 mL) was added to the solution and the resulting mixture was aged at room temperature for 30 min to form a white precipitate. The precipitate was filtered, washed with $^{i}$PrOH (15 mL) and EtOAc (15 mL), and dried. The solid was partitioned between EtOAc (30 mL) and $H_2O$ (30 mL) and an emulsion formed. The emulsion was transferred in 10-12 mL portions to centrifuge tubes and centrifuged at 3000 rpm for 15 min. Decanted away the organic layer. The aqueous layers were combined, filtered, and lyophilized to provide example 165 (306 mg, 64% yield, 1:1 mixture of regioisomers 165a and 165b).

Example 166

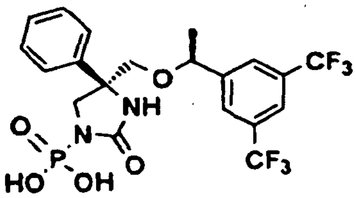

• 2 N-methylglucamine

**Example 166a**

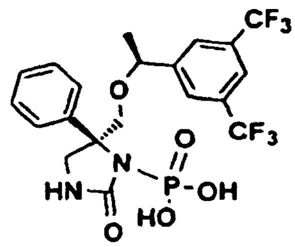

• 2 N-methylglucamine

**Example 166b**

Using procedures similar to those in example 165 and substituting Example 93 for Example 92, the title compounds were prepared.

Compounds of formula I have been found to be antagonists of the $NK_1$ receptor and of the effect of Substance P at its receptor site, and are therefore useful in treating conditions caused or aggravated by the activity of said receptor.

The *in vitro* and *in vivo* activity of the compounds of formula I can be determined by various procedures known in the art, such as a test for their ability to inhibit the activity of the $NK_1$ agonist Substance P. % Inhibition of neurokinin

agonist activity is the difference between the percent of maximum specific binding (MSB) and 100%. The percent of MSB is defined by the following equation, wherein "dpm" is disintegrations per minute:

$$\% \text{ MSB} = \frac{(\text{dpm of unknown}) - (\text{dpm of nonspecific binding})}{(\text{dpm of total binding}) - (\text{dpm of nonspecific binding})} \times 100$$

The concentration at which the compound produces 50% inhibition of binding is then used to determine the inhibition constant (ki) using the Chang-Prusoff equation.

In addition, functional antagonism of calcium channel activity is measured using FLIPR technology known to those skilled in the art.

*In vivo* activity is measured by inhibition of agonist-induced foot tapping in the gerbil, as descibed in Science, (1998), 281, p. 1640-1695.

It will be recognized that compounds of formula I exhibit $NK_1$ antagonist activity to varying degrees, e.g., certain compounds have strong $NK_1$ antagonist activity, while others are weaker $NK_1$ antagonists.

Compounds of the present invention exhibit a range of activity: Ki values range from about 0.1 to 1000 nM, with Ki values of about 0.1 to 100 being preferred and Ki values of 0.1 to 25 nM being more preferred. Most preferred are compounds having a Ki ≤10nM for the $NK_1$ receptor.

For preparing pharmaceutical compositions from the compounds described by this invention, inert, pharmaceutically acceptable carriers can be either solid or liquid. Solid form preparations include powders, tablets, dispersible granules, capsules, cachets and suppositories. The powders and tablets may be comprised of from about 5 to about 95 percent active ingredient. Suitable solid carriers are known in the art, e.g. magnesium carbonate, magnesium stearate, talc, sugar or lactose. Tablets, powders, cachets and capsules can be used as solid dosage forms suitable for oral administration. Examples of pharmaceutically acceptable carriers and methods of manufacture for various compositions may be found in A. Gennaro (ed.), Remington's Pharmaceutical Sciences, 18th Edition, (1990), Mack Publishing Co., Easton, PA.

Liquid form preparations include solutions, suspensions and emulsions. As an example may be mentioned water or water-propylene glycol solutions for parenteral injection or addition of sweeteners and opacifiers for oral solutions, suspensions and emulsions. Liquid form preparations may also include solutions for intranasal administration.

Aerosol preparations suitable for inhalation may include solutions and solids in powder form, which may be in combination with a pharmaceutically acceptable carrier, such as an inert compressed gas, e.g. nitrogen.

Also included are solid form preparations which are intended to be converted, shortly before use, to liquid form preparations for either oral or parenteral administration. Such liquid forms include solutions, suspensions and emulsions.

The compounds of the invention may also be deliverable transdermally. The transdermal compositions can take the form of creams, lotions, aerosols and/or emulsions and can be included in a transdermal patch of the matrix or reservoir type as are conventional in the art for this purpose.

Preferably the compound is administered orally.

Preferably, the pharmaceutical preparation is in a unit dosage form. In such form, the preparation is subdivided into suitably sized unit doses containing appropriate quantities of the active component, e.g., an effective amount to achieve the desired purpose.

The quantity of $NK_1$ receptor antagonists in combination with selective serotonin reuptake inhibitors in a unit dose of preparation may be varied or adjusted from about 10 mg to about 300 mg of $NK_1$ receptor antagonists with about 10 mg to about 100 mg of SSRI. A further quantity of $NK_1$ receptor antagonists in combination with selective serotonin reuptake inhibitors in a unit dose of preparation may be varied or adjusted from about 50 mg to about 300 mg of $NK_1$ receptor antagonists with about 10 mg to about 100 mg of SSRI. An even further quantity of $NK_1$ receptor antagonists in combination with selective serotonin reuptake inhibitors in a unit dose of preparation may be varied or adjusted from about 50 mg to about 300 mg of $NK_1$ receptor antagonists with about 20 mg of SSRI, according to the particular application.

The actual dosage employed may be varied depending upon the requirements of the patient and the severity of the condition being treated. Determination of the proper dosage regimen for a particular situation is within the skill of the art. For convenience, the total daily dosage may be divided and administered in portions during the day as required.

The amount and frequency of administration of the compounds of the invention and/or the pharmaceutically acceptable salts thereof will be regulated according to the judgment of the attending clinician considering such factors as age, condition and size of the patient as well as severity of the symptoms being treated. A typical recommended daily dosage regimen for oral administration can range from about 1 mg/day to about 300 mg/day, preferably 1 mg/day to 75 mg/day, in two to four divided doses.

While the present has been described in conjunction with the specific embodiments set forth above, many alternatives, modifications and variations thereof will be apparent to those of ordinary skill in the art.

## Claims

1. A compound represented by the structural formula

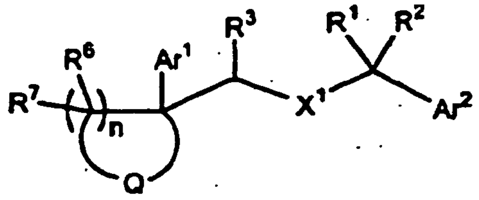

I

or a pharmaceutically acceptable salt thereof, wherein
$Ar^1$ and $Ar^2$ are independently selected from the group consisting of $R^{17-}$ heteroaryl and

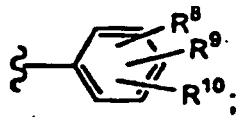

$X^1$ is -O-, -S-, -SO-, $-SO_2-$, $-NR^{12}-$, $-N(COR^{12})-$ or $-N(SO_2R^{15})-$;
$R^1$, $R^2$, $R^3$ and $R^7$ are each independently selected from the group consisting of H, $C_1$-$C_8$ alkyl, hydroxy($C_1$-$C_3$) alkyl, $C_3$-$C_8$ cycloalkyl, $-CH_2F$, $-CHF_2$ and $-CF_3$; or $R^1$ and $R^2$, together with the carbon to which they are attached, form a $C_3$-$C_8$ alkylene ring; or, when $X^1$ is -O-, -S- or $-NR^{12}-$, $R^1$ and $R^2$ together are =O;
each $R^6$ is independently selected from H, $C_1$-$C_6$ alkyl, $-OR^{13}$ or $-SR^{12}$;
n is 1-4, if n is greater than 1, then $R^6$ and $R^7$ can be the same or different on each carbon;

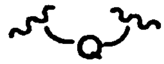

is selected from the group consisting of

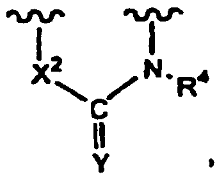 , 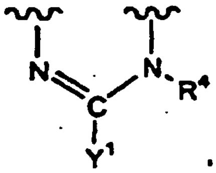 , 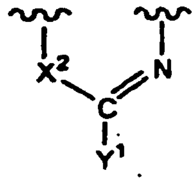 and 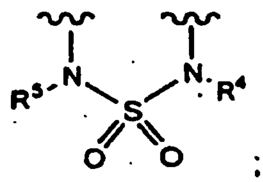 ;

$X^2$ is -O-, -S- or $-NR^5-$;
Y is =O, =S or $=NR^{11}$;
Y' is H, $C_1$-$C_6$ alkyl, $-NR^{17}R^{13}$, $-SCH_3$, $R^{19}$-aryl$(CH_2)_{n6}$-, $R^{19}$-heteroaryl-$(CH_2)_{n6}$-, $-(CH_2)_{n6}$-heterocycloalkyl, -($C_1$-$C_3$) alkyl-NH-C(O)O($C_1$-$C_6$)alkyl or $-NHC(O)R^{15}$;
$R^5$ is H or $-(CH_2)_{n1}$-G, wherein $n_1$ is 0-5, G is H, $-CF_3$, $-CHF_2$. $-CH_2F$ -OH, -O-($C_1$-$C_6$ alkyl), $-SO_2R^{13}$, -O-($C_3$-$C_8$ cycloalkyl), $-NR^{13}R^{14}$, $-SO_2NR^{13}R^{14}$, $-NR^{13}SO_2R^{15}$, $-NR^{13}COR^{12}$, $-NR^{12}(CONR^{13}R^{14})$, $-CONR^{13}R^{14}$, $-COOR^{12}$, $C_3$-$C_8$ cycloalkyl, $R^{18}$-aryl, $R^{19}$-heteroaryl,

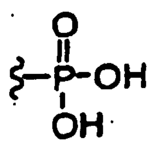 , 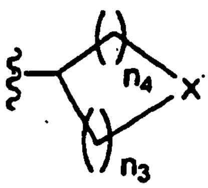 , 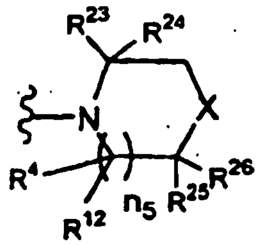 , 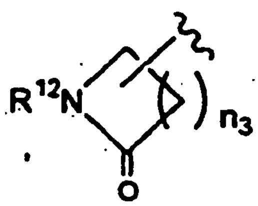 ,

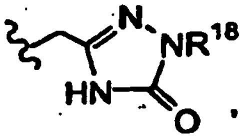, 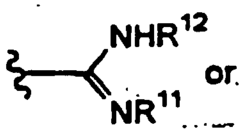 or 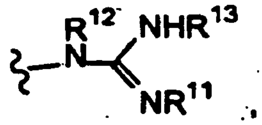;

or when $n_1$ is 0, $R^5$ can also be $-C(O)R^{13}$ or $-C(S)R^{13}$; provided that G is not H when $n_1$=0;

X is $-NR^{20}-$, $-N(CONR^{13}R^{14})-$, $-N(CO_2R^{13})-$, $-N(SO_2R^{15})-$, $-N(COR^{12})-$, $N(SO_2NHR^{13})-$, -O-, -S-, -SO-, $-SO_2-$, $-CF_2-$, $-CH_2-$ or $-CR^{12}F-$;

$R^8$, $R^9$ and $R^{10}$ are independently selected from the group consisting of H, $C_1$-$C_6$ alkyl, $C_3$-$C_8$ cycloalkyl, $-OR^{12}$, halogen, -CN, $-NO_2$, $-CF_3$, $-CHF_2$, $-CH_2F$, $-OCF_3$, $-OCHF_2$, $-OCH_2F$, $-COOR^{12}$, $-CONR^{21}R^{22}$, $-NR^{21}COR^{12}$, $-NR^{21}CO_2R^{15}$, $-NR^{21}CONR^{21}R^{22}$, $-NR^{21}SO_2R^{15}$, $-NR^{21}R^{22}$, $-SO_2NR^{21}R^{22}$, $-S(O)_{n5}R^{15}$, $R^{16}$-aryl and $R^{19}$-heteroaryl;

$R^{11}$ is H, $C_1$-$C_6$ alkyl, $C_3$-$C_8$ cycloalkyl, $-NO_2$, -CN, OH, $-OR^{12}$, $-O(CH_2)_{n8}R^{12}$, $-(C_1$-$C_3)$alkyl-C(O)NHR$^{12}$, $R^{19}$-aryl $(CH_2)_{n6}$- or $R^{19}$-heteroaryl-$(CH_2)_{n6}$-;

$R^4$ and $R^{12}$ are each independently selected from the group consisting of H, $C_1$-$C_6$ alkyl and $C_3$-$C_8$ cycloalkyl;

$R^{13}$ and $R^{14}$ are independently selected from the group consisting of H, $C_1$-$C_6$ alkyl, $C_3$-$C_8$ cycloalkyl, $R^{19}$-aryl $(CH_2)_{n6}$- or $R^{19}$-heteroaryl-$(CH_2)_{n6}$-, ; or $R^{13}$ and $R^{14}$ together are $C_3$-$C_6$ alkylene and with the nitrogen to which they are attached form a 4-7 membered ring, or one of the carbon atoms in the alklyene chain formed by $R^{13}$ and $R^{14}$ is replaced by a heteroatom selected from the group consisting of -O-, -S- and - $NR^{12}$-;

$R^{15}$ is $C_1$-$C_6$ alkyl, $C_3$-$C_8$ cycloalkyl or-$CF_3$;

$R^{16}$ is 1 to 3 substituents independently selected from the group consisting of H, $C_1$-$C_6$ alkyl, $C_3$-$C_8$ cycloalkyl, $C_1$-$C_6$ alkoxy, halogen and $-CF_3$;

$R^{17}$ is H, $C_1$-$C_6$ alkyl, $C_3$-$C_8$ cycloalkyl, $-COOR^{12}$, $-CONR^{21}R^{22}$, $-NR^{21}R^{22}$, - $NR^{21}COR^{12}$, $-NR^{21}CO_2R^{12}$, $-NR^{21}CONR^{21}R^{22}$, $-NR^{21}SO_2R^{15}$ or $-S(O)_{n5}R^{15}$;

$R^{18}$ is H, $C_1$-$C_6$ alkyl or $-P(O)(OH)_2$;

$R^{19}$ is 1 to 3 substituents independently selected from the group consisting of H, $C_1$-$C_6$ alkyl, $C_3$-$C_8$ cycloalkyl, -OH, halogen, -CN, $-NO_2$, $-CF_3$, $-CHF_2$, $-CH_2F$, $-OCF_3$, $-OCHF_2$, $-OCH_2F$, -O-($C_1$-$C_6$ alkyl), -O-($C_3$-$C_8$ cycloalkyl), $-COOR^{12}$, $-CONR^{21}R^{22}$, $-NR^{21}R^{22}$, $-NR^{21}COR^{12}$, $-NR^{21}CO_2R^{12}$, $-NR^{21}CONR^{21}R^{22}$, $-NR^{21}SO_2R^{15}$ and $-S(O)_{n5}R^{15}$;

$R^{20}$ is H, $C_1$-$C_6$ alkyl, $C_3$-$C_8$ cycloalkyl or $-(CH_2)_{n6}$-heterocycloalkyl ;

$R^{21}$ and $R^{22}$ are independently selected from the group consisting of H, $C_1$-$C_6$ alkyl, $C_3$-$C_8$ cycloalkyl and benzyl; or $R^{21}$ and $R^{22}$ together are $C_3$-$C_6$ alkylene and with the nitrogen to which they are attached form a 4-7 membered ring, or one of the carbon atoms in the alkylene chain formed by $R^{21}$ and $R^{22}$ is replaced by a heteroatom selected from the group consisting of -O-, -S- and $-NR^{12}$-;

$R^{23}$, $R^{24}$, $R^{25}$ and $R^{26}$ are H, $C_1$-$C_6$ alkyl and can be together as = O; when $n_6$ = O, and $R^{25}$ and $R^{26}$ = H, X is not O, N, S;

$n_3$ and $n_4$ are independently 1-5, provided that the sum of $n_3$ and $n_4$ is 2-6;

$n_5$ is independently 0-2; and

$n_6$ is independently 0-3,

wherein

"aryl" means phenyl, naphthyl, indenyl, tetrahydronaphthyl, indanyl, anthracenyl or fluorenyl,

"heteroaryl" means 5- to 10-membered single or benzofused aromatic rings comprising 1 to 4 heteroatoms independently selected from the group consisting of -O-, -S-, -N = and -NH-, provided that the rings do not include adjacent oxygen and/or sulfur atoms, and

"heterocycloalkyl" means a 4- to 7-membered saturated ring comprising 1 to 3 heteroatoms independently selected from the group consisting of -O-, -S- and $-NR^{21}$-, wherein $R^{21}$ is H or $C_1$-$C_8$ alkyl, and wherein the remaining ring members are carbon.

2. The compound of claim 1 wherein $R^1$, $R^3$, $R^4$ and $R^7$ are each hydrogen.

3. The compound of claim 1 wherein $R^6$ is H or OH.

4. The compound of claim 1 wherein $X^1$ is -O- or $-NR^{12}$-.

5. The compound of claim 1 wherein $Ar^1$ and $Ar^2$ are each $R^8$,$R^9$,$R^{10}$-phenyl,

6. The compound of claim 1 wherein

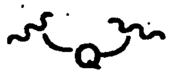

is

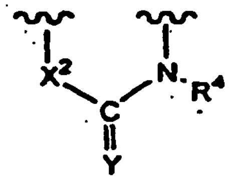

7. The compound of claim 6 wherein n is 1 or 2.

8. The compound of claim 7 wherein $X^2$ is $-NR^5-$ and Y is =O.

9. The compound of claim 1 wherein $R^1$, $R^3$, $R^4$ and $R^7$ are each hydrogen; $X^1$ is -O- or $-NR^{12}-$; $Ar^1$ and $Ar^2$ are each $R^8$, $R^9$,$R^{10}$-phenyl; n is 1 or 2; $X^2$ is $-NR^5-$; and $R^6$ is H or-OH, Y is =O.

10. The compound of claim 1 selected from the group of compounds represented by the formula

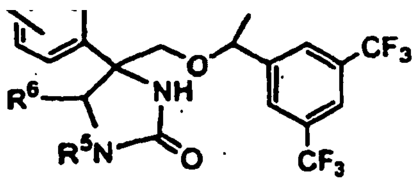

and

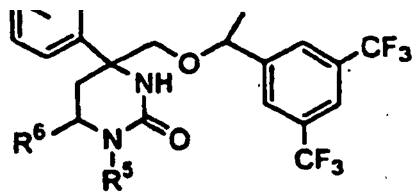

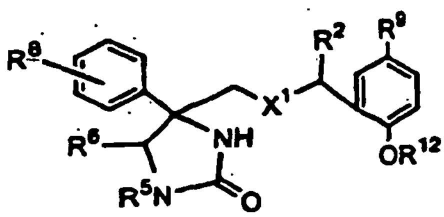

and

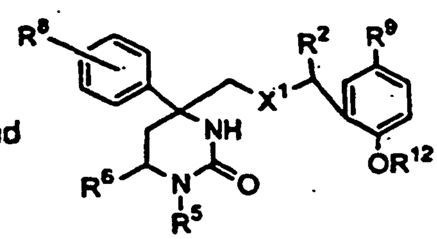

wherein $R^8$ is H or halogen; $R^2$ is H, -CH, or $-CH_2OH$ ; $R^6$ is H or -OH; and $R^5$ is selected from the group consisting of hydrogen and groups of the formula $-(CH_2)_{n1}-G$.

11. The compound of claim 10 wherein $X^1$ is -O-, -NH-, $-N(CH_3)-$ or $-N(COCH_3)$; $R^8$ is H or halogen; $R^2$ is H, $-CH_3$ or $-CH_2OH$; $R^6$ is H or -OH; $R^9$ is $-OCF_3$ or 5-(trifluoromethyl)-1H-tetrazol-1-yl; $R^{12}$ is $-CH_3$ or cyclopropyl; and $R^5$ is selected from the group consisting of hydrogen and groups of the formula $-(CH_2)_{n1}-G$, wherein $-(CH_2)_{n1}-G$ is selected from the group consisting of

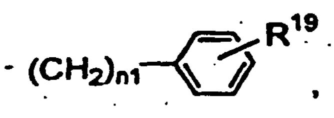, $-(CH_2)_{n1}-R^{19}$-heteroaryl , 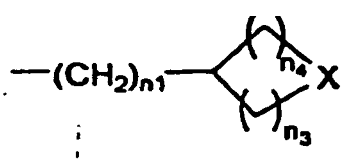 ,

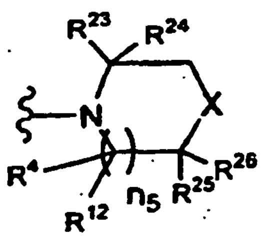 and 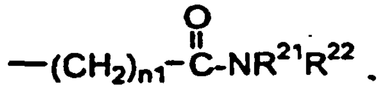

or $-(CH_2)_{n1}$-G', wherein n1 is 2-4 and G' is H, -OH, $-OCH_3$-, ethoxy, isopropyloxy, -O-cyclopropyl, or $-CONR^{13}R^{14}$, wherein $R^{13}$ and $R^{14}$ are independently selected from the group consisting of H, $-CH_3$, ethyl, isopropyl, or cyclopropyl.

**12.** A compound according to Claim 1 selected from the following structures:

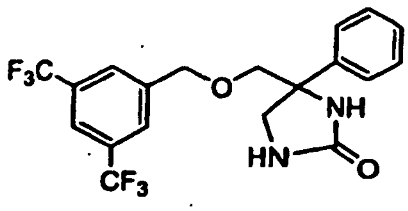

1

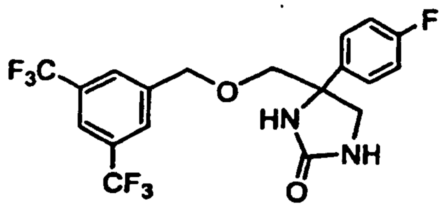

2

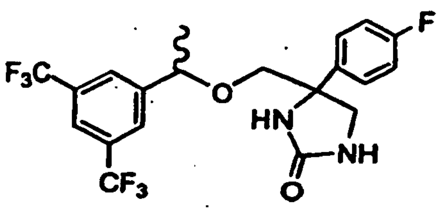

3

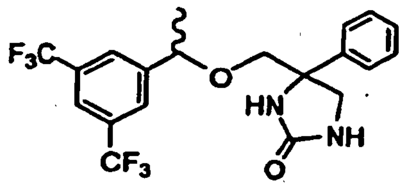

4

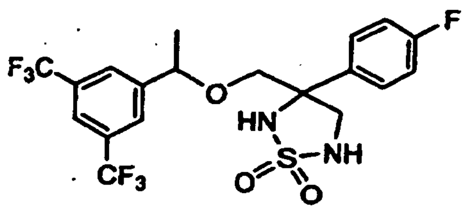

5

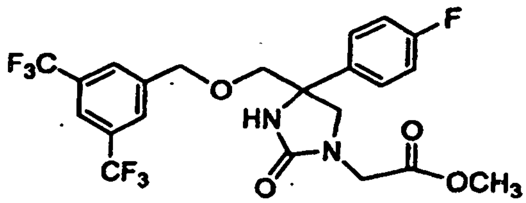

6

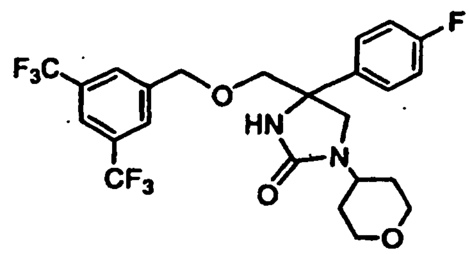

7

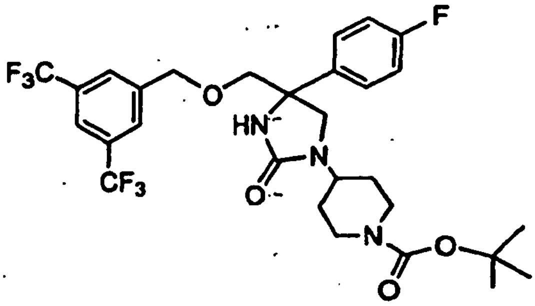

9

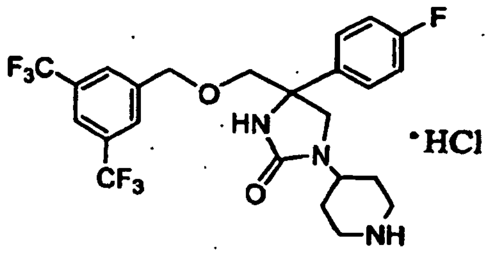

•HCl

10

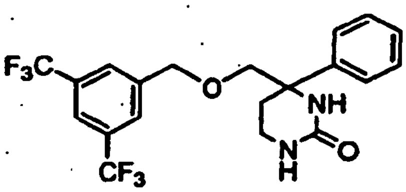

11

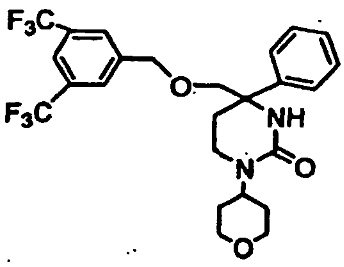

12

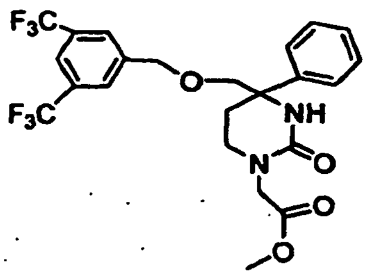

13

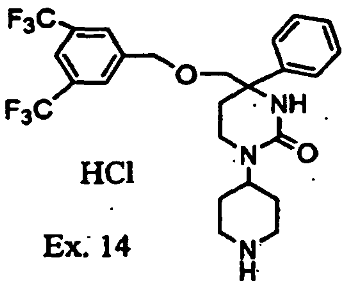

HCl

Ex. 14

14

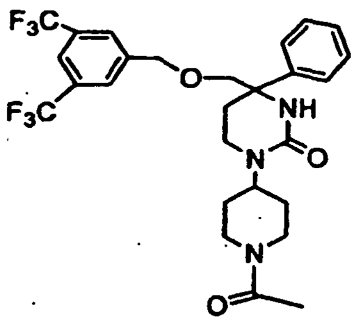

15

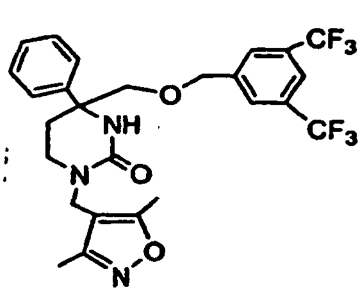

16

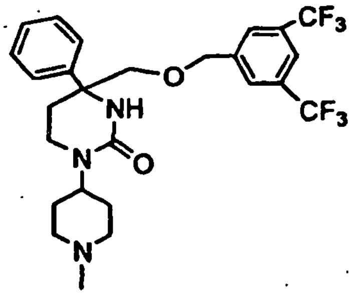

17

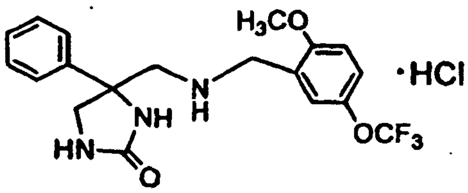

18

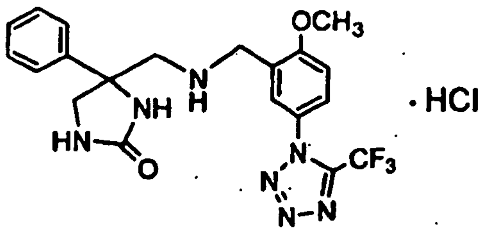

19

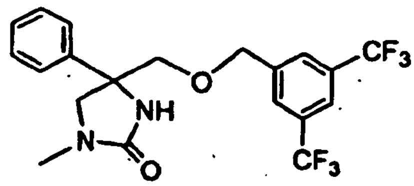

20

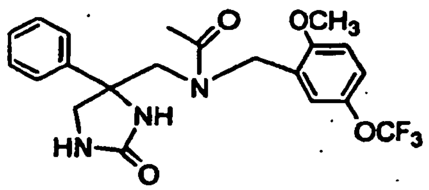

21

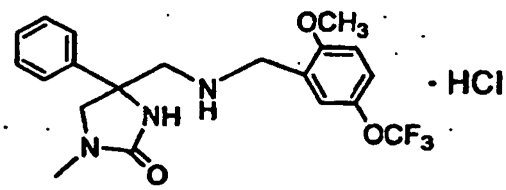

22

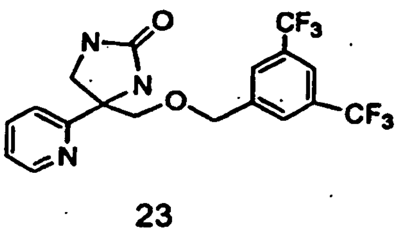

23

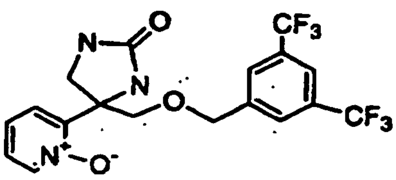

24

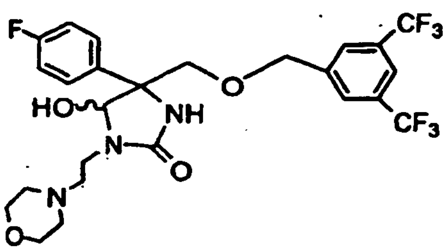

25

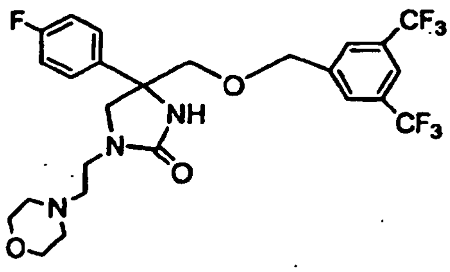

26

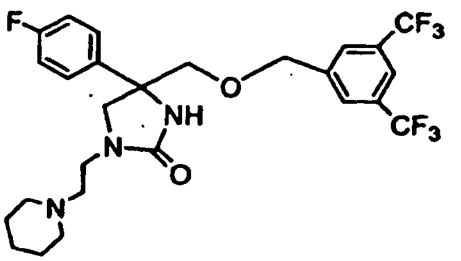

27

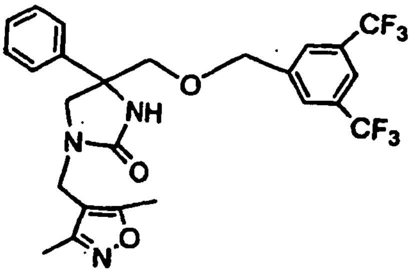

28

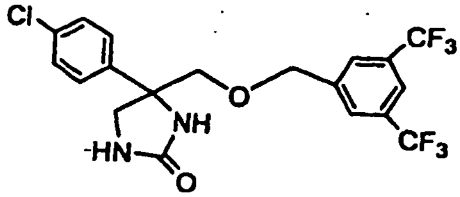

29

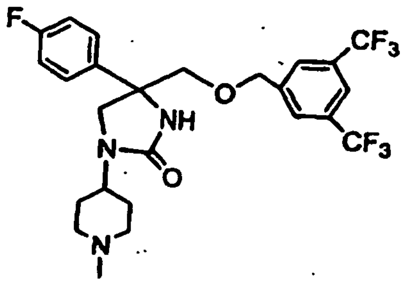

30

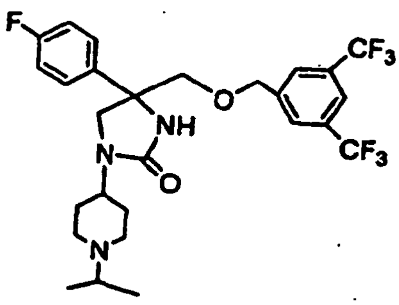

31

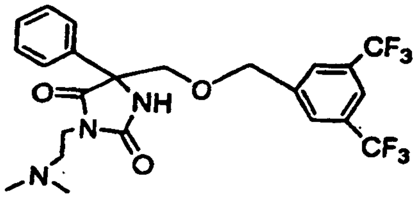

32

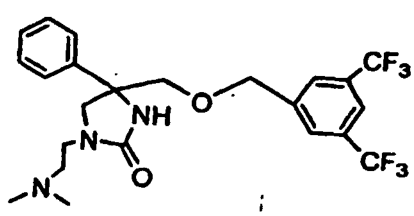

33

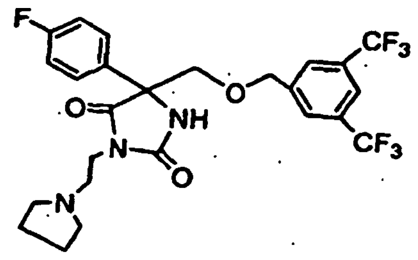

34

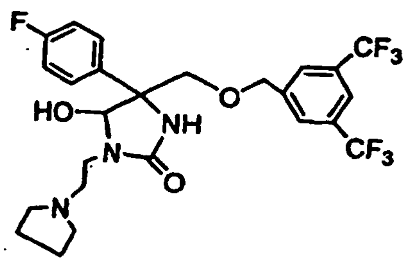

35

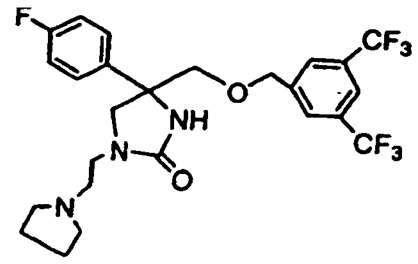

36

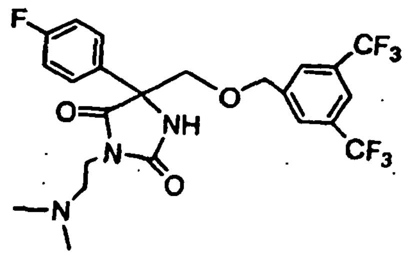

37

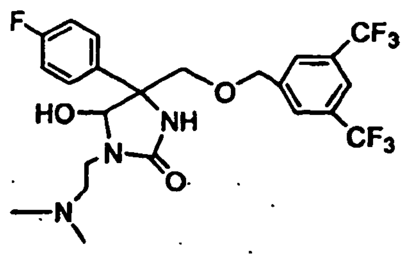

38

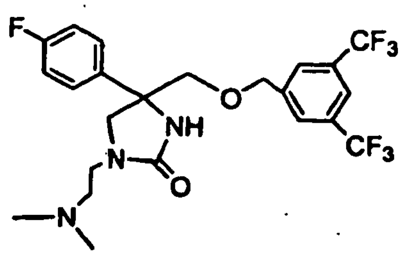

39

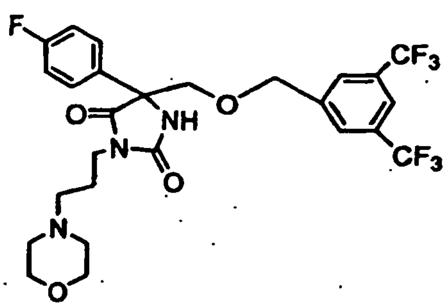

40

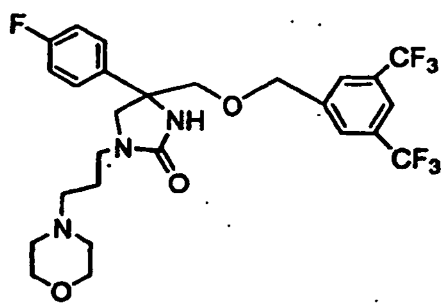

41

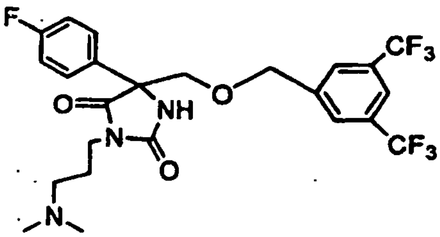

42

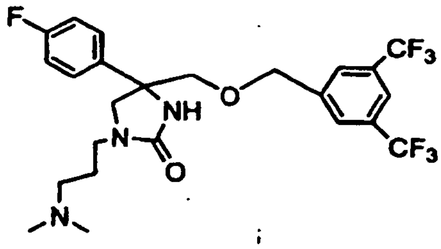

43

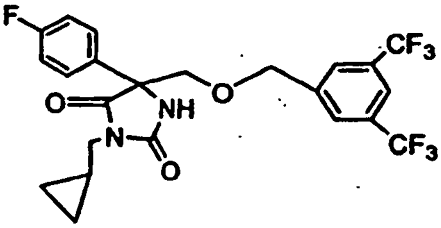

44

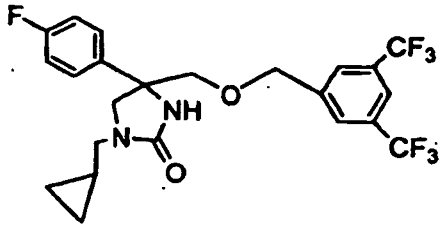

45

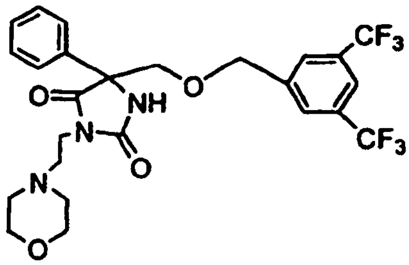

46

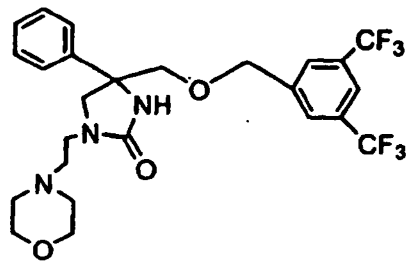

47

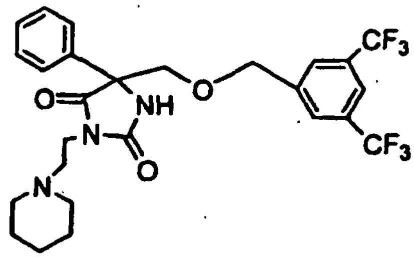

48

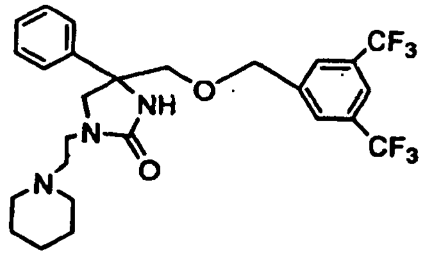

49

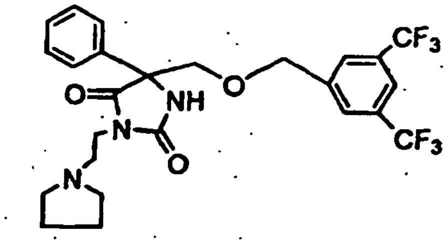

50

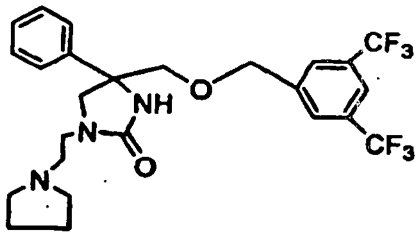

51

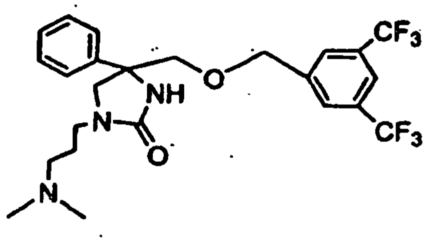

52

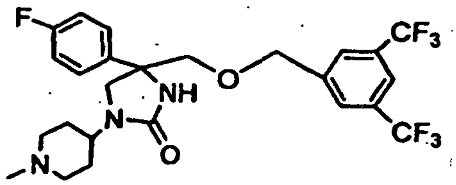

53

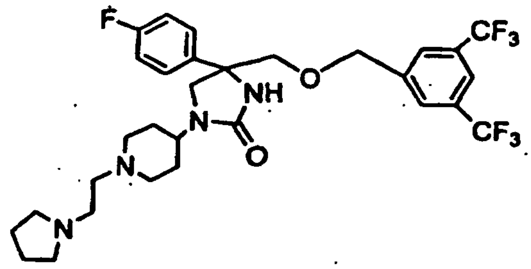

54

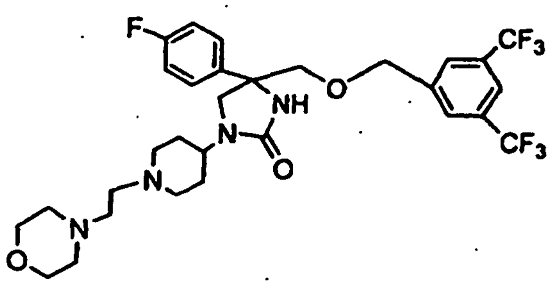

55

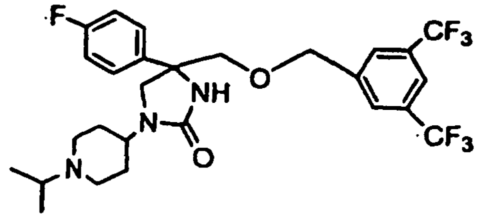

56

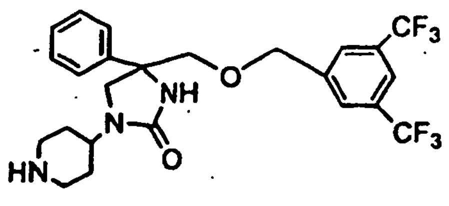

57

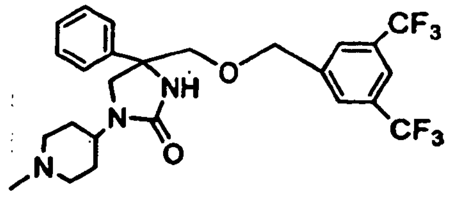

58

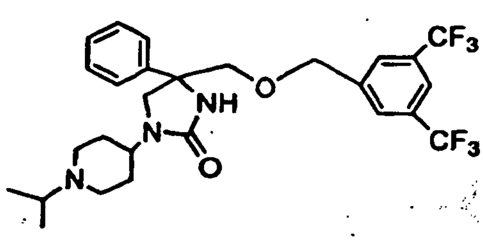

59

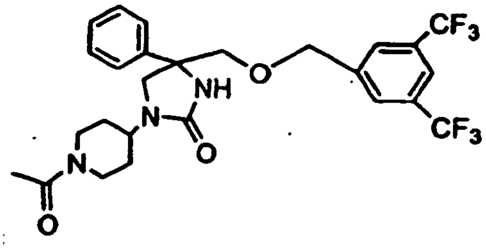

60

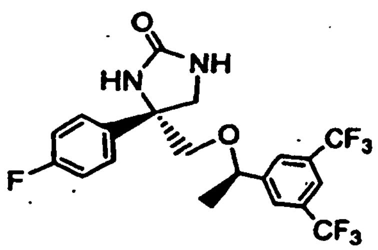

61

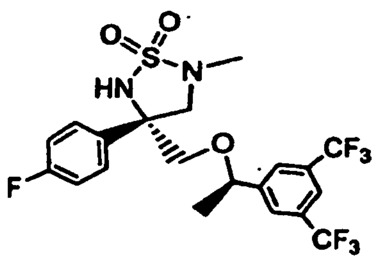

63

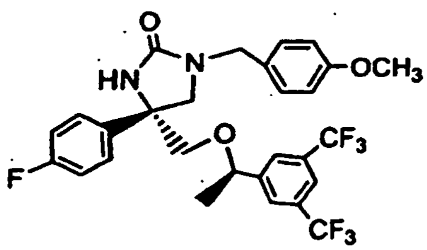

64

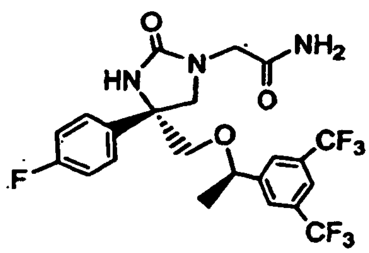

65

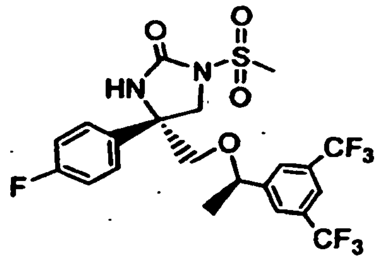

66

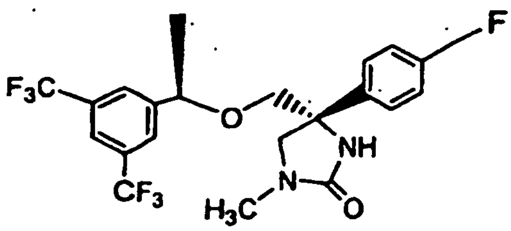

67

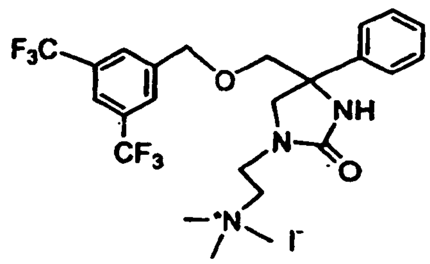

68

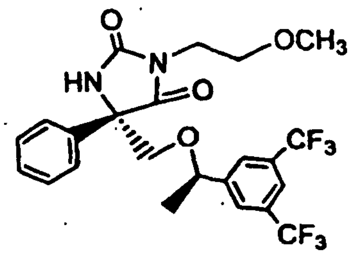

69

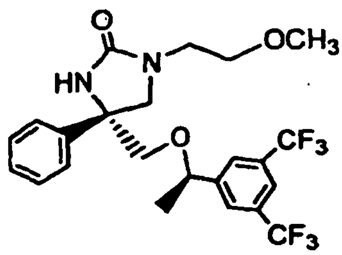

70a

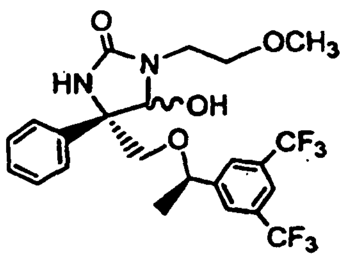

70b

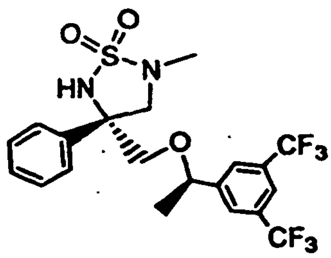

72

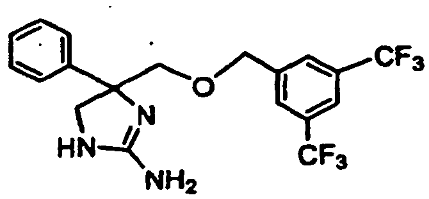

73

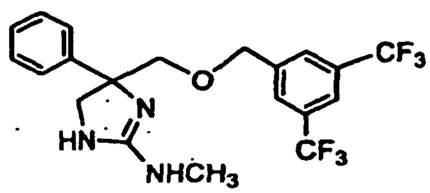

74

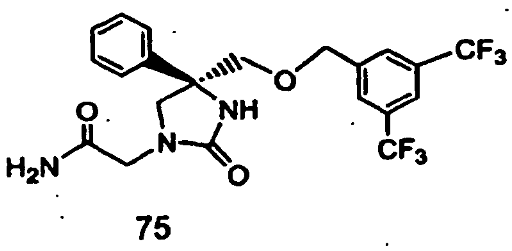

75

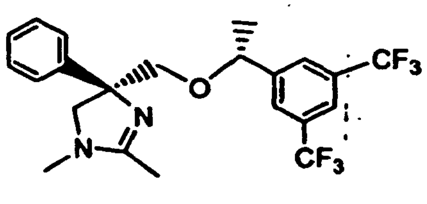

76

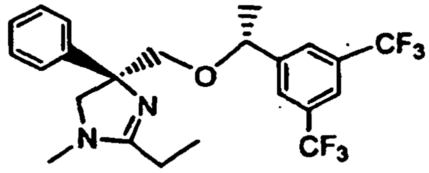

77

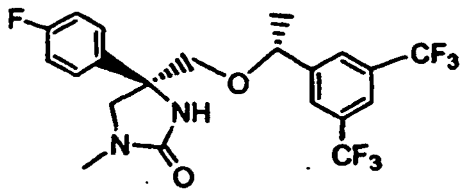

78

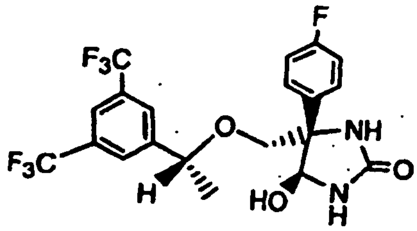

79a

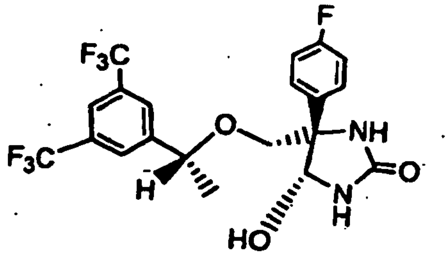

79b

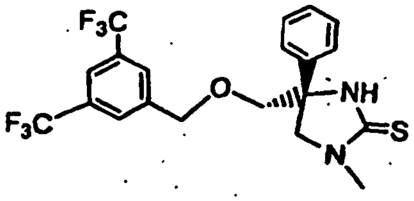

80

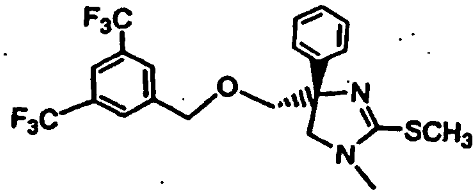

81

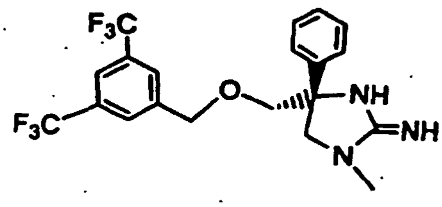

82

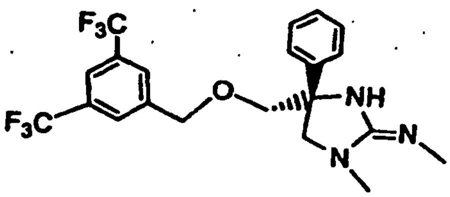

83

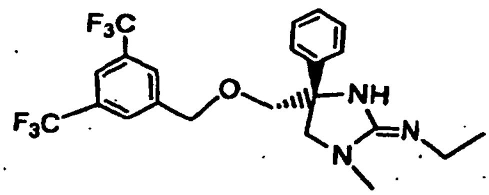

84

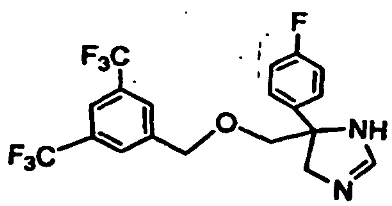

85

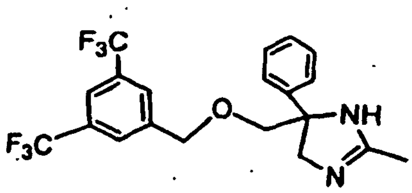

86

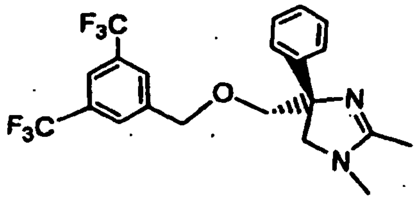

87

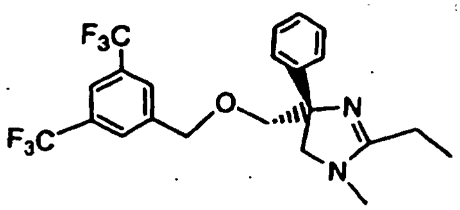

88

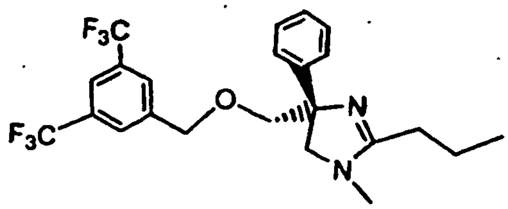

89

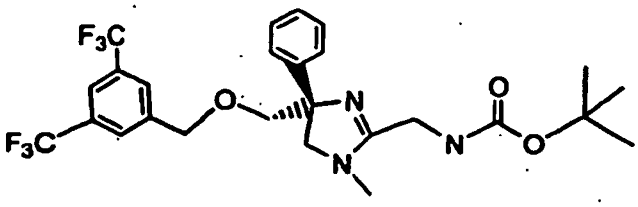

90

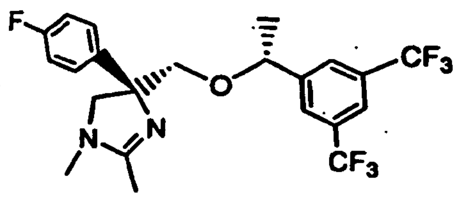

91

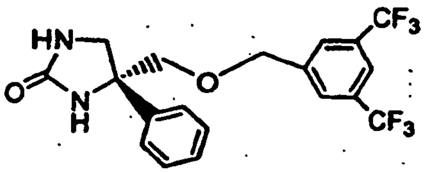

92

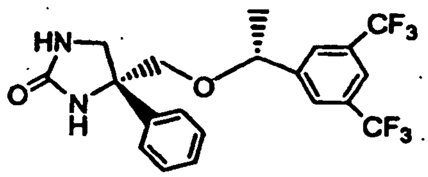

93

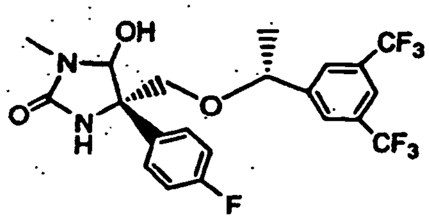

94

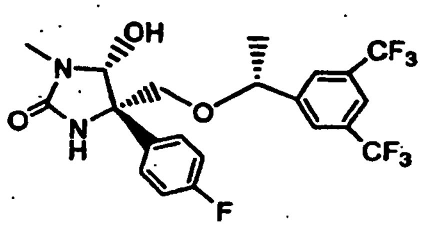

95

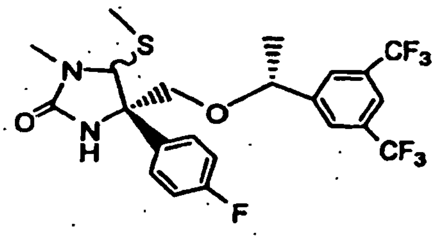

96

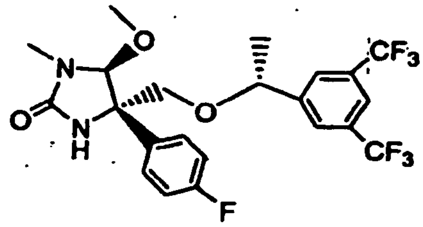

97

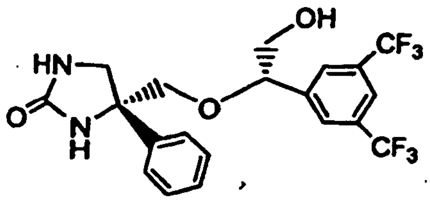

98

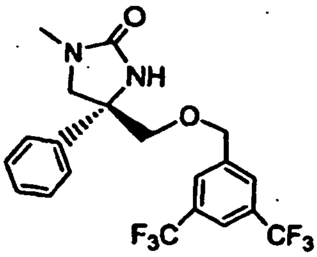

99

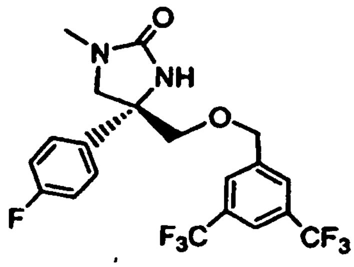

100

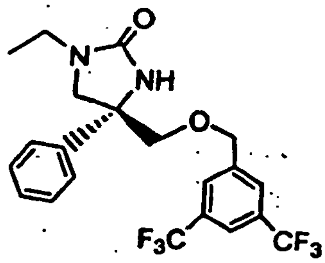

101

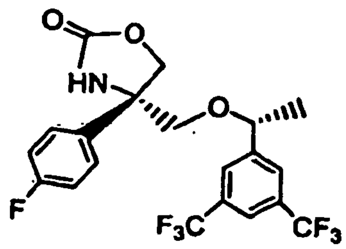

102

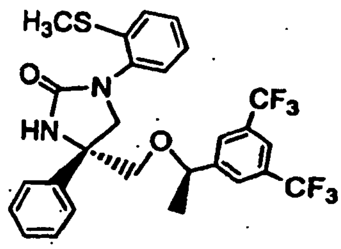

103

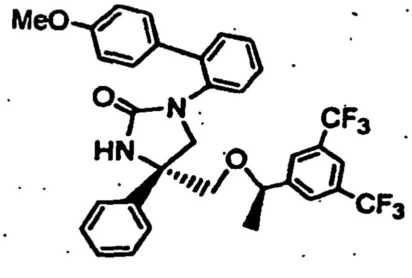

104

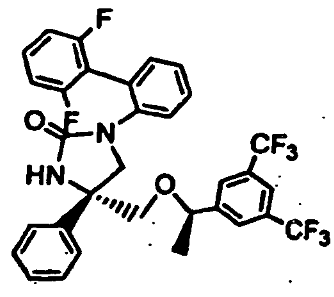

105

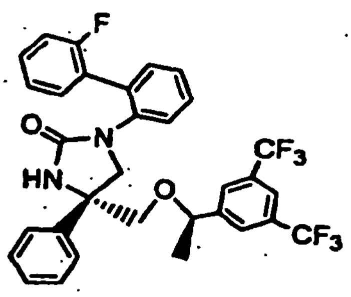

106

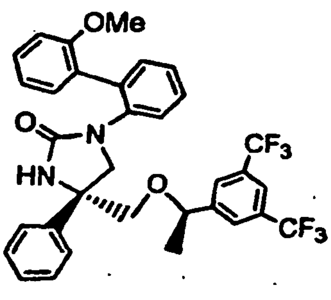

107

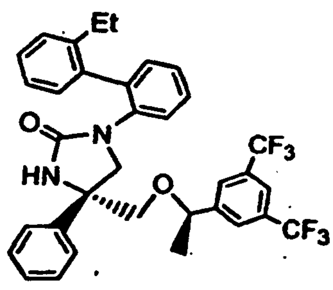

108

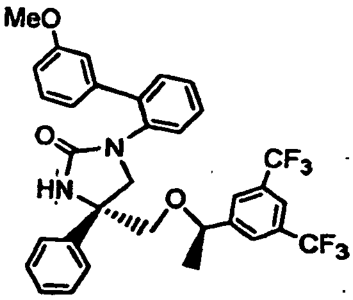

109

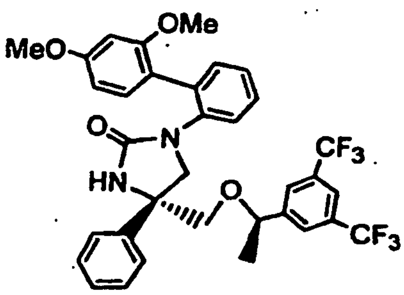

110

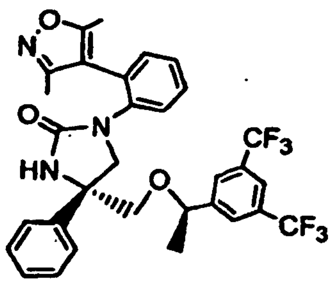

111

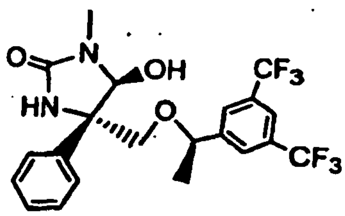

112

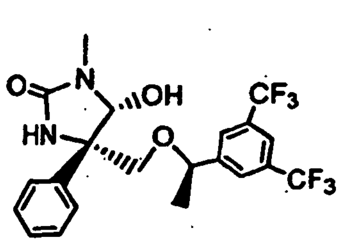

113

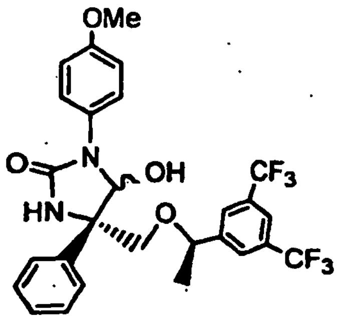

114

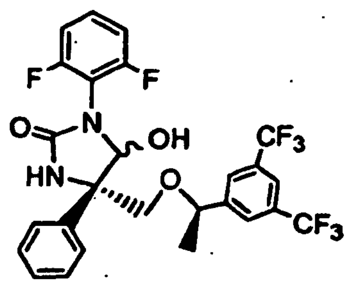

115

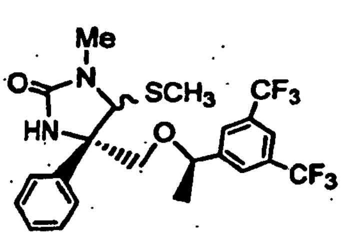

116

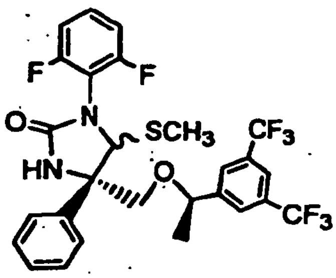

117

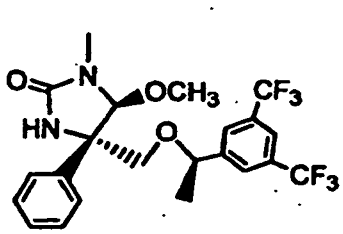

118

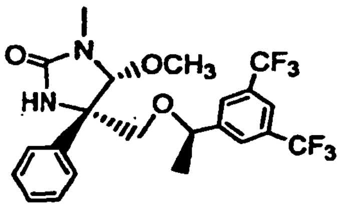

119

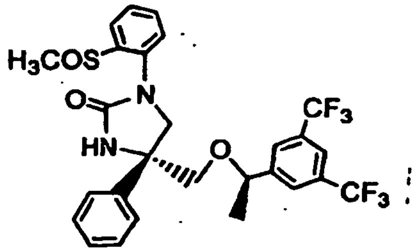

120

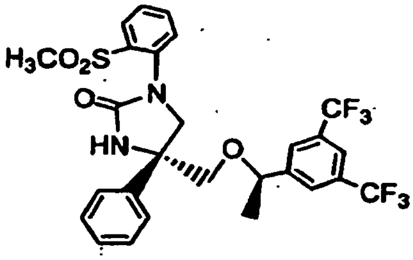

121

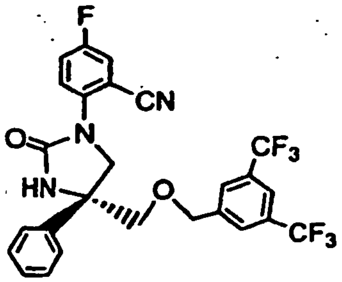

122

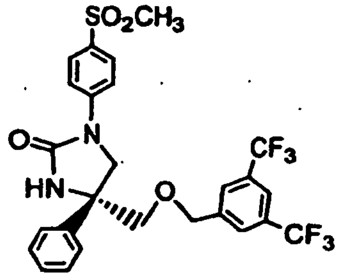

123

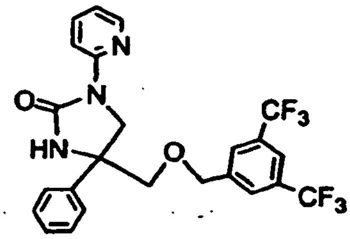

124

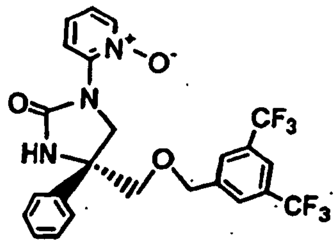

125

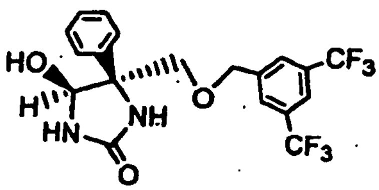

126

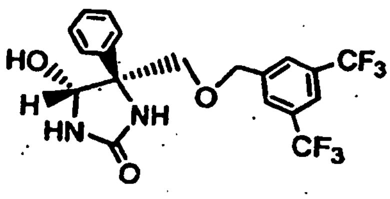

127

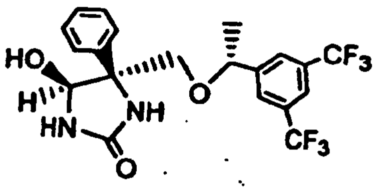

128

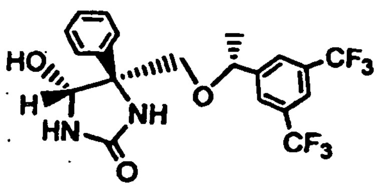

129

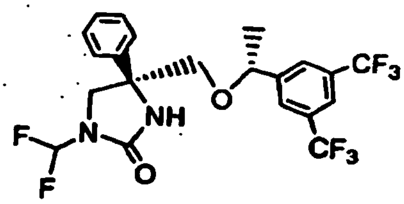

130

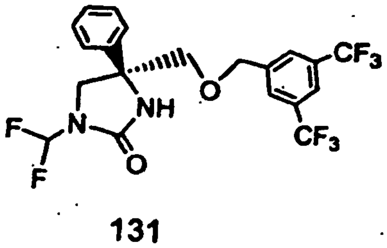

131

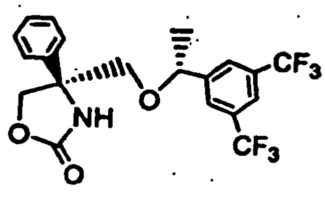

132

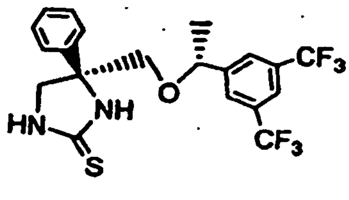

133

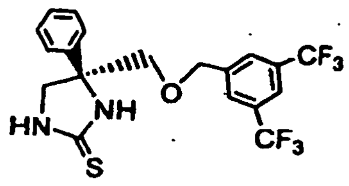
134

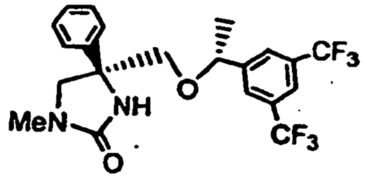
135

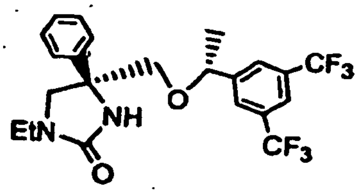
136

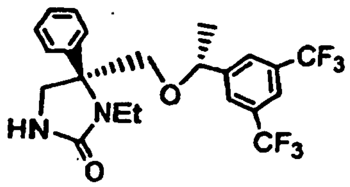
137

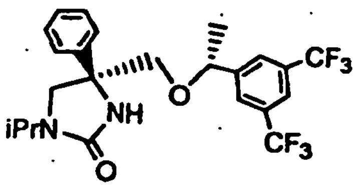
138

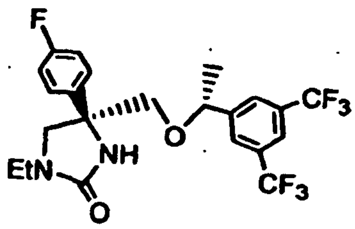
139

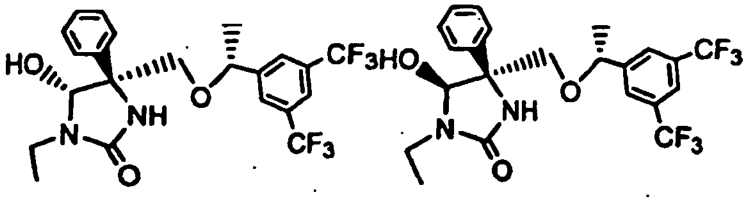
140

141

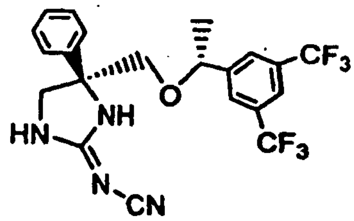
142

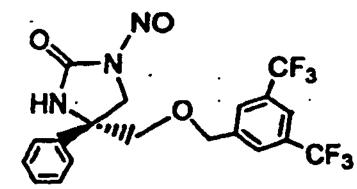
143

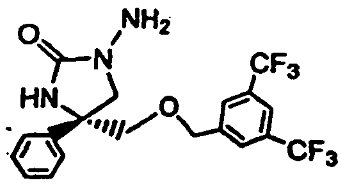
144

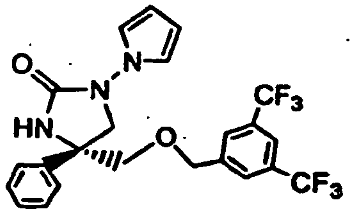
145

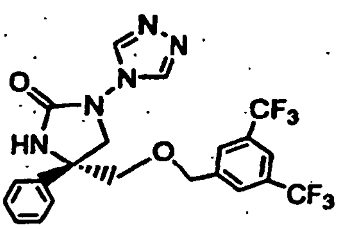
146

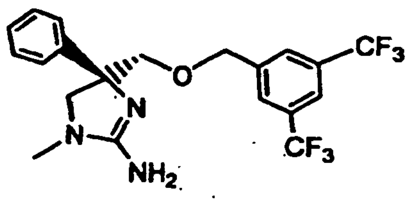
147

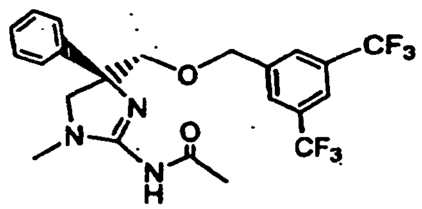

148

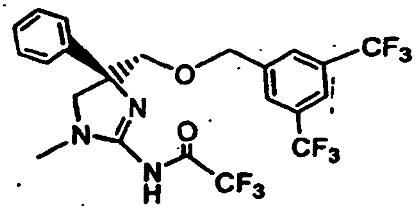

149

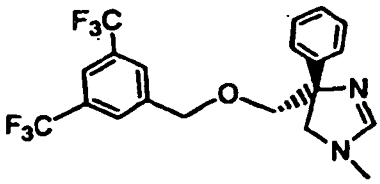

150

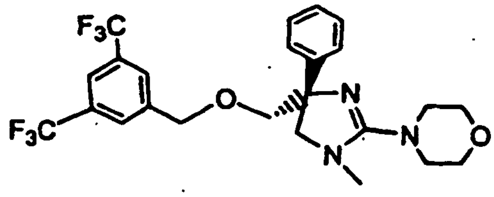

151

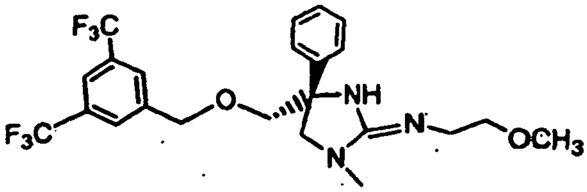

152

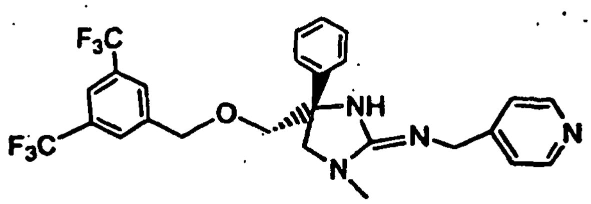

153

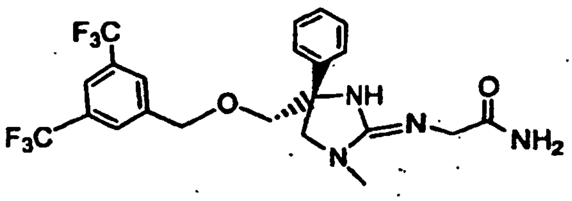

154

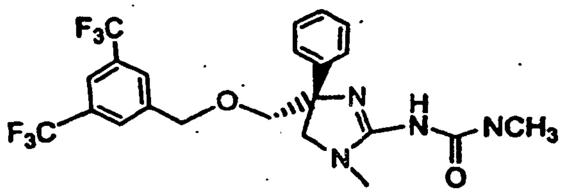

155

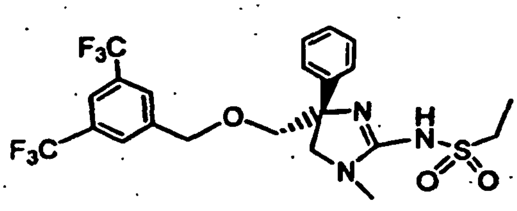

156

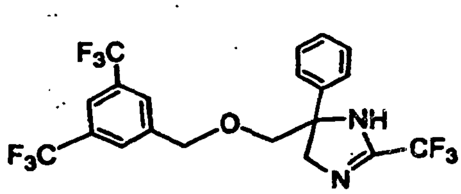

157

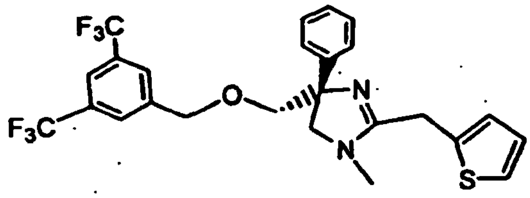

158

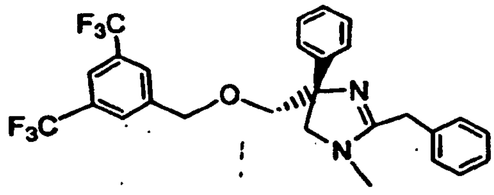

159

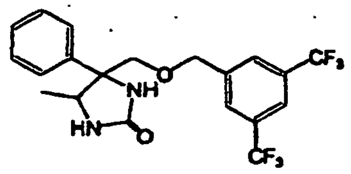

160

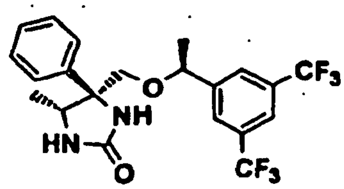

Example 161a

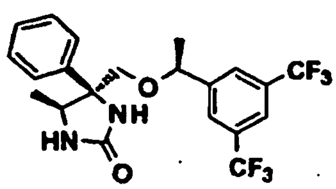

Example 161b

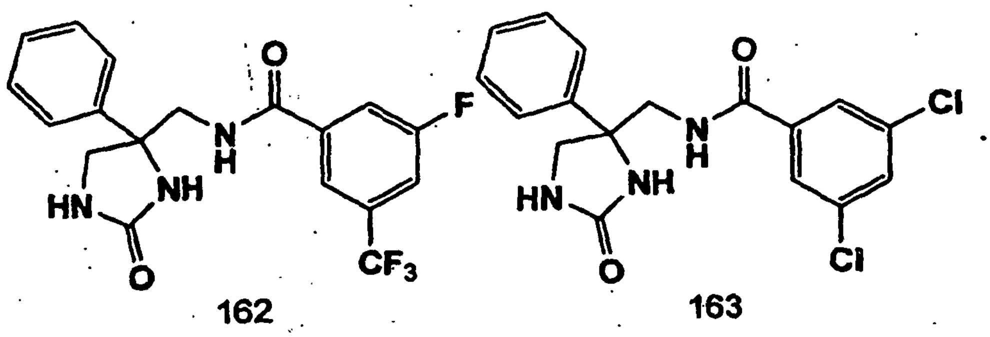

162

163

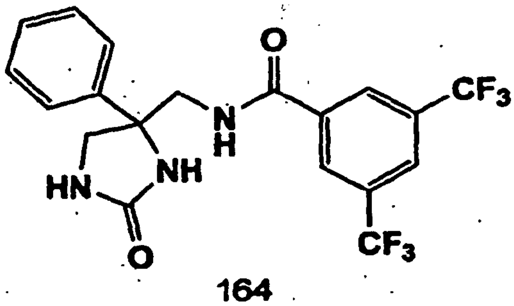

164

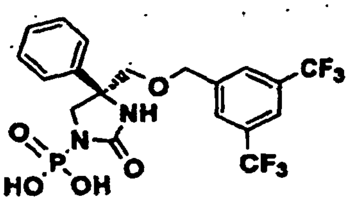

• 2 N-methylglucamine

Example 165a

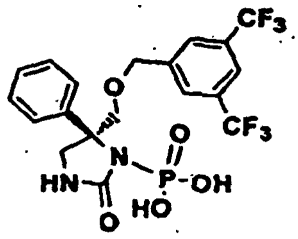

• 2 N-methylglucamine

Example 165b

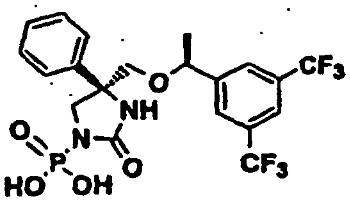

• 2 N-methylglucamine

Example 166a

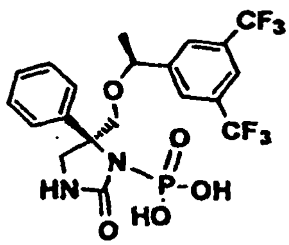

• 2 N-methylglucamine

Example 166b

**13.** A compound according to Claim 12 selected from compound Nos. 2, 17, 19, 31, 59, 61, 65, 67, 76, 77, 78, 79a, 79b, 92, 93, 99, 100, 103, 104, 110, 111, 113, 120, 121, 126, 127, 128, 129, 130, 135, 142, 165, 165a, 165b, 166a, 166b or compound 118.

**14.** A pharmaceutical composition comprising an effective amount of a compound of claims 1-13 and a pharmaceutically acceptable carrier.

**15.** A pharmaceutical composition comprising an effective amount of a compound of claims 1-13, in combination with an effective amount of a selective serotonin reuptake inhibitor and a pharmaceutically acceptable carrier.

**16.** The use of the compounds of claims 1-13 for the manufacture of a medicament for treating respiratory diseases; inflammatory diseases; skin disorders; ophthamalogical disorders; addictions; stress related disorders; obsessive/compulsive disorders; eating disorders; mania; premenstrual syndrome; central nervous system conditions; gastrointestinal disorders; bladder disorders; atherosclerosis; fibrosing disorders; obesity; Type II diabetes; pain related disorders; and genitourinary disorders.

**17.** The use of the compounds of claims 1-13 in combination with a selective serotonin reuptake inhibitor for the manufacture of a medicament for treating respiratory diseases; inflammatory diseases; skin disorders; ophthalmalogical disorders; addictions; stress related disorders; obsessive/compulsive disorders; eating disorders; mania; premenstrual syndrome; central nervous system conditions; gastrointestinal disorders; bladder disorders; atherosclerosis; fibrosing disorders; obesity; Type II diabetes; pain related disorders; and genitourinary disorders.

**18.** The use of the compounds of claims 1-13 for the manufacture of a medicament for treating emesis, depression, anxiety and cough.

**19.** The use of the compounds of claims 1-13 in combination with a selective serotonin reuptake inhibitor for the manufacture of a medicament for treating treating emesis, depression, anxiety and cough.

**Patentansprüche**

**1.** Verbindung mit der Strukturformel

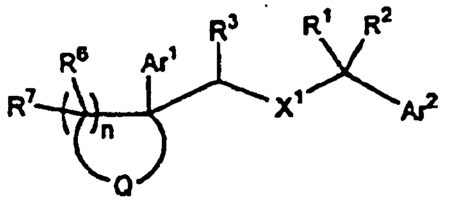

I

oder ein pharmazeutisch annehmbares Salz davon, worin $Ar^1$ und $Ar^2$ unabhängig ausgewählt sind aus der Gruppe bestehend aus $R^{17}$-Heteroaryl und

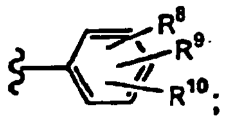

;

$x^1$ -O-, -S-, -SO-, $-SO_2-$, $-NR^{12}-$, -N ($COR^{12}$) - oder -N ($SO_2R^{15}$) - ist;
$R^1$, $R^2$, $R^3$ und $R^7$ jeweils unabhängig ausgewählt sind aus der Gruppe bestehend aus H, $C_1$-$C_6$-Alkyl, Hydroxy ($C_1$-$C_3$) - alkyl, $C_3$-$C_8$-Cycloalkyl, $-CH_2F$, $-CHF_2$ und $-CF_3$; oder $R^1$ und $R^2$ zusammen mit dem Kohlenstoff, an den sie gebunden sind, einen $C_3$-$C_6$-Alkylenring bilden; oder, wenn $X^1$ -O- , -S- oder $-NR^{12}-$ ist, $R^1$ und $R^2$ zusammen =O sind;
jedes $R^6$ unabhängig ausgewählt ist aus H, $C_1$-$C_6$-Alkyl, $-OR^{13}$ oder $-SR^{12}$;
n 1 bis 4 ist, wenn n größer als 1 ist, können $R^6$ und $R^7$ an jedem Kohlenstoff gleich oder verschieden sein;

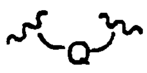

ausgewählt ist aus der Gruppe bestehend aus

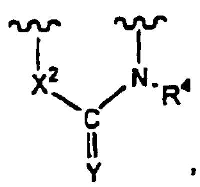 , 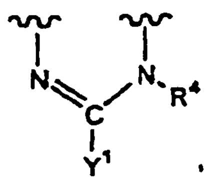 , 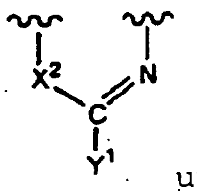 und 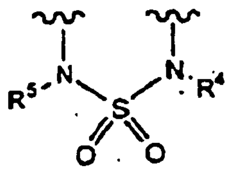 ;

$X^2$ -O-, -S- oder $-NR^5-$ ist;
Y =O, =S oder $=NR^{11}$ ist;
$Y^1$ H, $C_1$-$C_6$-Alkyl , $-NR^{17}R^{13}$, $-SCH_3$, $R^{19}$-Aryl $(CH_2)_{n6}$-, $R^{19}$-Heteroaryl- $(CH_2)_{n6}$-, - $(CH_2)_{n6}$-Heterocycloalkyl, - ($C_1$-$C_3$) - Alkyl-NH-C (O) O ($C_1$-$C_6$) alkyl oder $-NHC(O)R^{15}$ ist;
$R^5$ H oder - $(CH_2)_{n1}$-G ist, wobei $n_1$ 0 bis 5 ist, G H, $-CF_3$, $-CHF_2$, $-CH_2F$, -OH, -O- ($C_1$-$C_6$-Alkyl), $-SO_2R^{13}$, -O- ($C_3$-$C_8$-Cycloalkyl) , $-NR^{13}R^{14}$, $-SO_2N^{13}R^{14}$-, $-NR^{13}SO_2R^{15}$, $-NR^{13}COR^{12}$, $-NR^{12}$ ($CONR^{13}R^{14}$), $-CONR^{13}R^{14}$, $-COOR^{12}$, $C_3$-$C_8$-Cycloalkyl, $R^{19}$-Aryl, $R^{19}$-Heteroaryl,

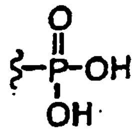 , 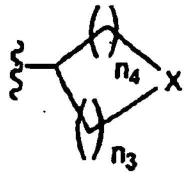 , 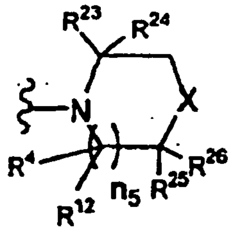 , 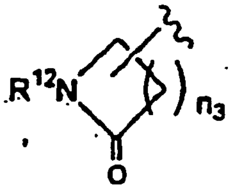 ,

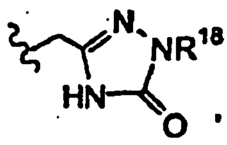 , 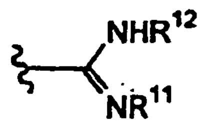 , oder 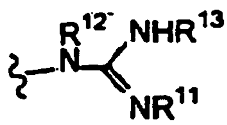

ist;

oder wenn $n_1$ 0 ist, $R^5$ auch -C (O) $R^{13}$ oder -C (S) $R^{13}$ sein kann, mit der Maßgabe, dass G nicht H ist, wenn $n_1 = 0$ ist;

X -$NR^{20}$-, -N ($CONR^{13}R^{14}$) -, -N ($CO_2R^{13}$) -, -N ($SO_2R^{15}$) -, -N ($COR^{12}$) -, N ($SO_2NHR^{13}$)-, -O-, -S-, -SO-, -$SO_2$-, -$CF_2$-, -$CH_2$- oder -$CR^{12}F$- ist;

$R^8$, $R^9$ und $R^{10}$ unabhängig ausgewählt sind aus der Gruppe bestehend aus H, $C_1$-$C_6$-Alkyl, $C_3$-$C_8$-Cycloalkyl, -$OR^{12}$, Halogen, -CN, -$NO_2$, -$CF_3$, -$CHF_2$, -$CH_2F$, -$OCF_3$, -$OCHF_2$, -$OCH_2F$, -$COOR^{12}$, -$CONR^{21}R^{22}$, -$NR^{21}COR^{12}$, -$NR^{21}CO_2R^{15}$, -$NR^{21}CONR^{21}R^{22}$, -$NR^{21}SO_2R^{15}$, -$NR^{21}R^{22}$, -$SO_2NR^{21}R^{22}$, -S (O) $_{n5}R^{15}$, $R^{16}$-aryl und $R^{19}$-Heteroaryl;

$R^{11}$ H, $C_1$-$C_6$-Alkyl, $C_3$-$C_8$-Cycloalkyl, -$NO_2$, -CN, OH, -$OR^{12}$, - O ($CH_2$) $_{n6}R^{12}$, -($C_1$-$C_3$) -Alkyl-C (O) $NHR^{12}$, $R^{19}$-Aryl ($CH_2$) $_{n6}$- oder $R^{19}$-Heteroaryl- ($CH_2$) $_{n6}$- ist;

$R^4$ und $R^{12}$ jeweils unabhängig ausgewählt sind aus der Gruppe bestehend aus H, $C_1$-$C_6$-Alkyl und $C_3$-$C_8$-Cycloalkyl;

$R^{13}$ und $R^{14}$ unabhängig ausgewählt sind aus der Gruppe bestehend aus H, $C_1$-$C_6$-Alkyl, $C_3$-$C_8$-Cycloalkyl, $R^{19}$-Aryl-$(CH_2)_{n6}$- oder $R^{19}$-Heteroaryl-$(CH_2)_{n6}$-; oder $R^{13}$ und $R^{14}$ zusammen $C_3$-$C_8$-Alkylen sind und mit dem Stickstoff, an den sie gebunden sind, einen 4-7-gliedrigen Ring bilden, oder eines der Kohlenstoffatome in der durch $R^{13}$ und $R^{14}$ gebildeten Alkylengruppe durch ein Heteroatom ausgewählt aus der Gruppe bestehend aus -O-, -S- und -$NR^{12}$- ersetzt wird;

$R^{15}$ $C_1$-$C_6$-Alkyl, $C_3$-$C_9$-Cycloalkyl oder -$CF_3$ ist;

$R^{16}$ 1 bis 3 Substituenten ist, die unabhängig ausgewählt sind aus der Gruppe bestehend aus H, $C_1$-$C_6$-Alkyl, $C_3$-$C_8$-Cycloalkyl, $C_1$-$C_6$-Alkoxy, Halogen und -$CF_3$;

$R^{17}$ H, $C_1$-$C_6$-Alkyl, $C_3$-$C_9$-Cycloalkyl, -$COOR^{12}$, $CONR^{21}R^{22}$, -$NR^{21}R^{22}$, -$NR^{21}COR^{12}$, -$NR^{21}CO_2R^{12}$, -$NR^{21}CONR^{21}R^{22}$, -$NR^{21}SO_2R^{15}$ oder -$S(O)_{n5}R^{15}$ ist;

$R^{18}$ H, $C_1$-$C_6$-Alkyl oder -P(O) $(OH)_2$ ist;

$R^{19}$ 1 bis 3 Substituenten ist, die unabhängig ausgewählt sind aus der Gruppe bestehend aus H, $C_1$-$C_6$-Alkyl, $C_3$-$C_8$-Cycloalkyl, -OH, Halogen, -CN, -$NO_2$, -$CF_3$, -$CHF_2$, -$CH_2F$, -$OCF_3$, -$OCHF_2$, -$OCH_2F$, -O-($C_1$-$C_6$-Alkyl), -O-($C_3$-$C_8$-Cycloalkyl), -$COOR^{12}$, -$CONR^{21}R^{22}$, -$NR^{21}R^{22}$, -$NR^{21}COR^{12}$, -$NR^{21}CO_2R^{12}$, -$NR^{21}CONR^{21}R^{22}$, -$NR^{21}SO_2R^{15}$ und -S (O) $_{n5}R^{15}$;

$R^{20}$ H, $C_1$-$C_6$-Alkyl, $C_3$-$C_8$-Cycloalkyl oder - ($CH_2$) $_{n6}$-Heterocycloalkyl ist;

$R^{21}$ und $R^{22}$ unabhängig ausgewählt sind aus der Gruppe bestehend aus H, $C_1$-$C_6$-Alkyl, $C_3$-$C_8$-Cycloalkyl und Benzyl; oder $R^{21}$ und $R^{22}$ zusammen $C_3$-$C_6$-Alkylen sind und mit dem Stickstoff, an den sie gebunden sind, einen 4-7-gliedrigen Ring bilden, oder eines der Kohlenstoffatome in der durch $R^{21}$ und $R^{22}$ gebildeten Alkylengruppe durch ein Heteroatom ausgewählt aus der Gruppe bestehend aus -O-, -S- und -$NR^{12}$- ersetzt ist;

$R^{23}$, $R^{24}$, $R^{25}$ und $R^{26}$ H, $C_1$-$C_6$-Alkyl sind und zusammen als =O vorliegen können; wenn $n_5 = 0$, und $R^{25}$ und $R^{26}$ = H, ist X nicht O, N, S;

$n_3$ und $n_4$ unabhängig 1 bis 5 sind, mit der Maßgabe, dass die Summe von $n_3$ und $n_4$ 2 bis 6 ist;

$n_5$ unabhängig 0 bis 2 ist; und

$n_6$ unabhängig 0 bis 3 ist,

worin:

"Aryl" Phenyl, Naphthyl, Indenyl, Tetrahydronaphthyl, Indanyl, Anthracenyl oder Fluorenyl bedeutet,

"Heteroaryl" 5- bis 10-gliedrige einzelne oder benzokondensierte aromatische Ringe bedeutet, die 1 bis 4 Heteroatome aufweisen, die unabhängig aus der Gruppe bestehend aus -O-, -S-, -N= und -NH- ausgewählt sind, mit der Maßgabe, dass die Ringe keine benachbarten Sauerstoff-und/oder Schwefelatome einschließen, und

"Heterocycloalkyl" 4- bis 7-gliedrige gesättigte Ringe bedeutet, die 1 bis 3 Heteroatome aufweisen, die unabhängig ausgewählt sind aus der Gruppe bestehend aus -O-, -S-und -$NR^{21}$-, worin $R^{21}$ H oder $C_1$-$C_6$-Alkyl ist, und wobei die restlichen Ringglieder Kohlenstoff sind.

**2.** Verbindung nach Anspruch 1, bei der $R^1$, $R^3$, $R^4$ und $R^7$ jeweils Wasserstoff sind.

**3.** Verbindung nach Anspruch 1, bei der $R^6$ H oder OH ist.

**4.** Verbindung nach Anspruch 1, bei der $X^1$ -O- oder $-NR^{12}-$ ist.

**5.** Verbindung nach Anspruch 1, bei der $Ar^1$ und $Ar^2$ jeweils $R^8$, $R^9$, $R^{10}$-Phenyl sind.

**6.** Verbindung nach Anspruch 1, bei der

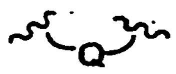

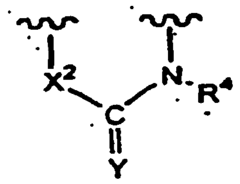

ist.

**7.** Verbindung nach Anspruch 6, bei der n 1 oder 2 ist.

**8.** Verbindung nach Anspruch 7, bei der $X^2$ $-NR^5-$ ist und Y =O ist.

**9.** Verbindung nach Anspruch 1, bei der $R^1$, $R^3$, $R^4$ und $R^7$ jeweils Wasserstoff sind; $X^1$ -O- oder $-NR^{12}-$ ist; $Ar^1$ und $Ar^2$ jeweils $R^8$,$R^9$,$R^{10}$-Phenyl sind; n 1 oder 2 ist; $X^2$ $-NR^5-$ ist; und $R^6$ H oder -OH ist, Y =O ist.

**10.** Verbindung nach Anspruch 1 ausgewählt aus der Gruppe von Verbindungen, die durch die Formel

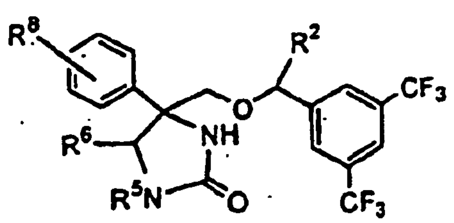

und

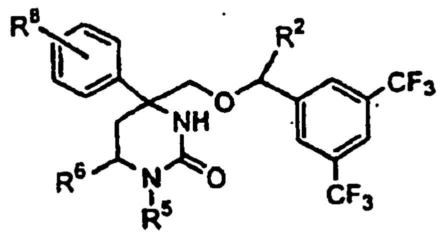

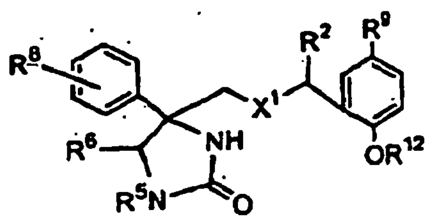

und

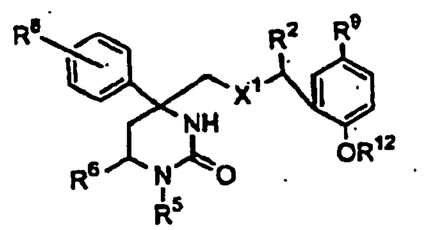

wiedergegeben werden, worin $R^8$ H oder Halogen ist; $R^2$ H, $-CH_3$ oder $-CH_2OH$ ist; $R^6$ H oder -OH ist; und $R^5$ ausgewählt ist aus der Gruppe bestehend aus Wasserstoff und Gruppen mit der Formel $(CH_2)_{n1}$-G.

**11.** Verbindung nach Anspruch 10, bei der $X^1$ -O-, -NH-, $-N(CH_3)$ - oder $-N(COCH_3)$ ist; $R^8$ H oder Halogen ist; $R^2$ H, $-CH_3$ oder $-CH_2OH$ ist; $R^6$ H oder -OH ist; $R^9$ $-OCF_3$ oder 5-(Trifluoromethyl)-1H-tetrazol-1-yl ist; $R^{12}$ $-CH_3$ oder Cyclopropyl ist, und $R^5$ ausgewählt ist aus der Gruppe bestehend aus Wasserstoff und Gruppen mit der Formel $-(CH_2)_{n1}$-G, wobei - $(CH_2)$ $n_1$-G ausgewählt ist aus der Gruppe bestehend aus

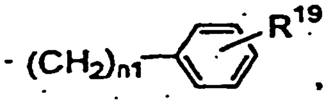, —$(CH_2)_{n1}$–$R^{19}$-heteroaryl, 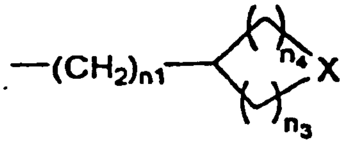

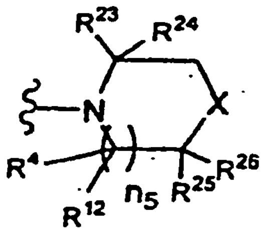 und 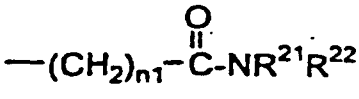.

oder -$(CH_2)_{n1}$-G', wobei n1 2 bis 4 ist und G' H, -OH, $-OCH_3$, Ethoxy, Isopropyloxy, -O-Cyclopropyl oder- $CONR^{13}R^{14}$ ist, wobei $R^{13}$ und $R^{14}$ unabhängig ausgewählt sind aus der Gruppe bestehend aus H, $-CH_3$, Ethyl, Isopropyl oder Cyclopropyl.

**12.** Verbindung nach Anspruch 1 ausgewählt aus den folgenden Strukturen:

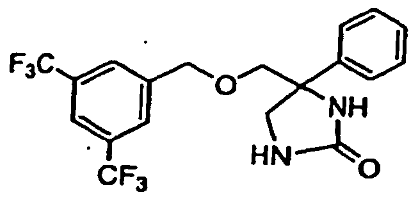

1

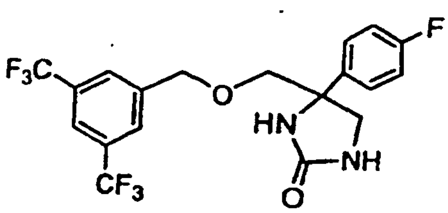

2

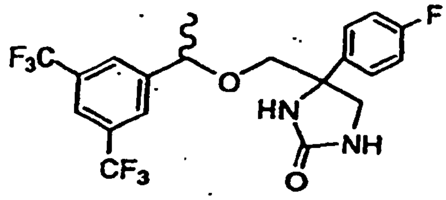

3

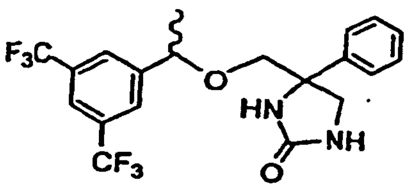

4

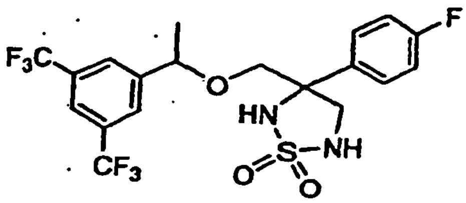

5

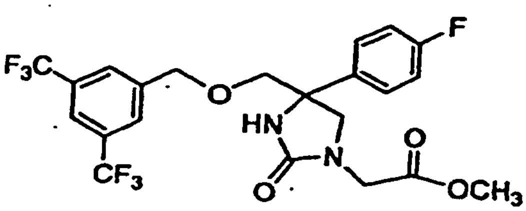

6

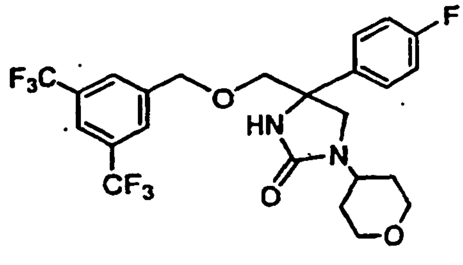

7

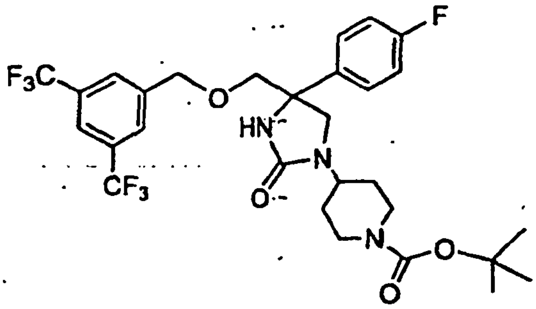

9

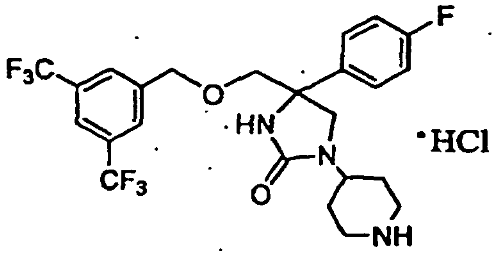

10

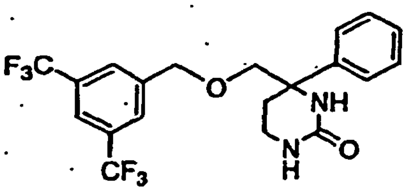

11

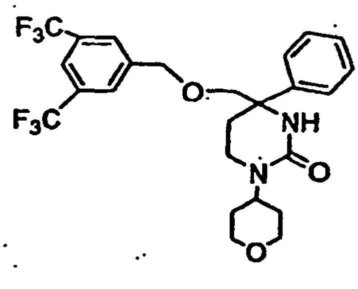

12

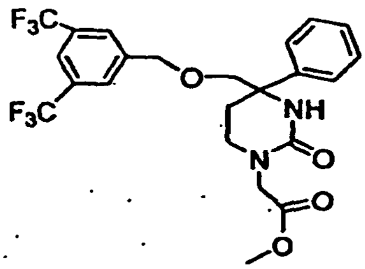

13

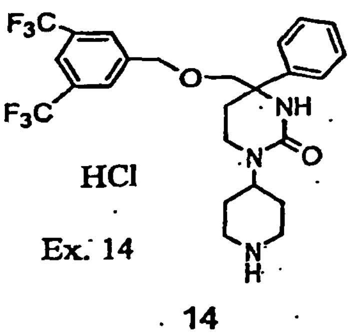

Ex. 14

14

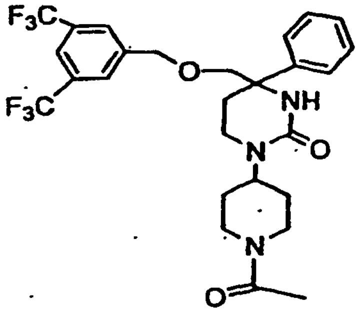

15

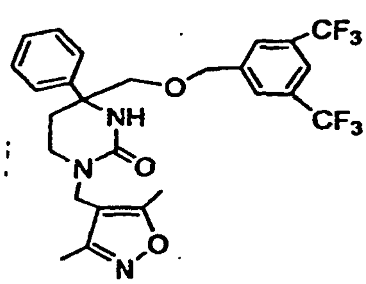

16

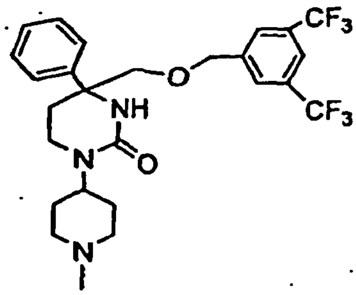

17

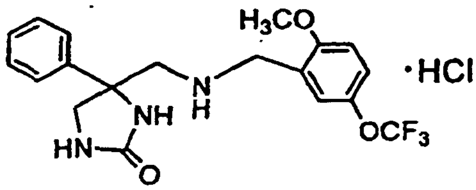

18

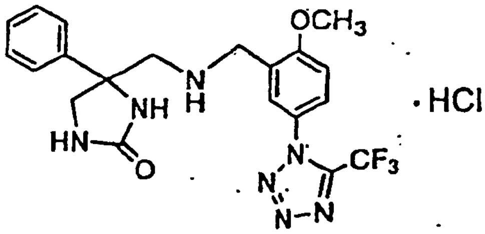

19-

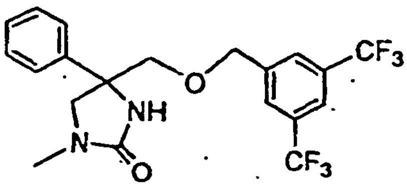

20

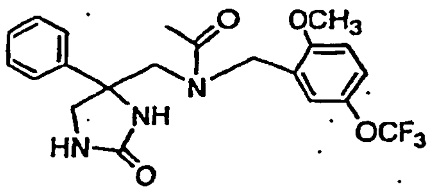

21

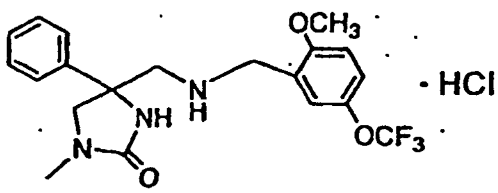

22

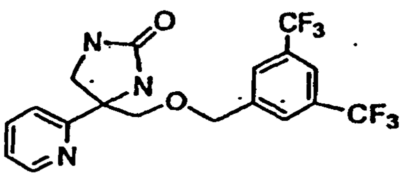

23

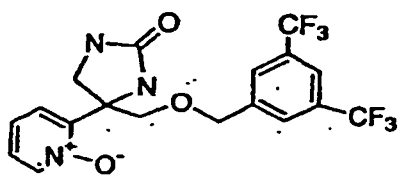

24

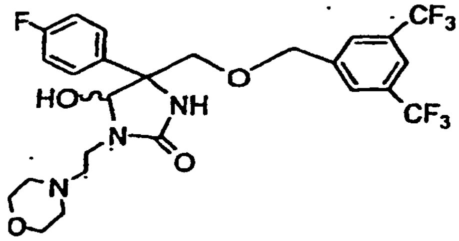

25

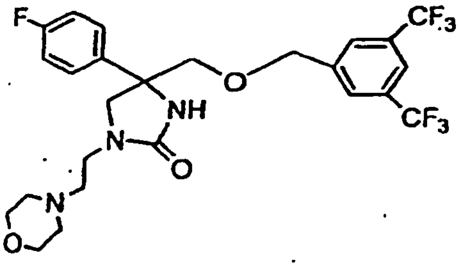

26

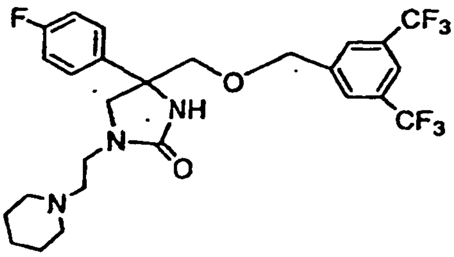

27

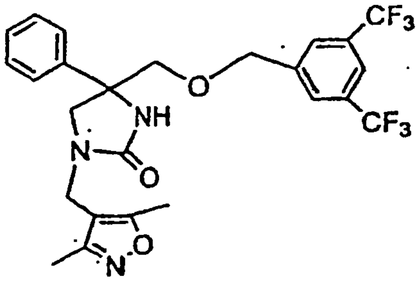

28

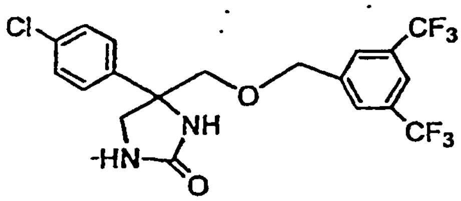

29

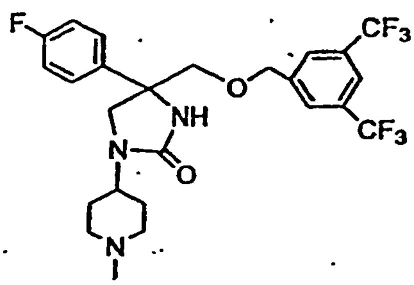

30

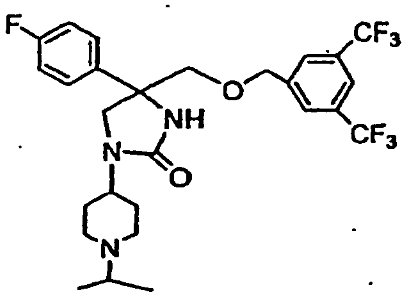

31

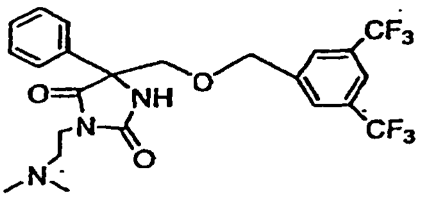

32

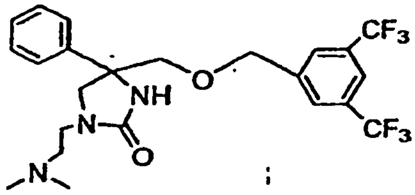

33

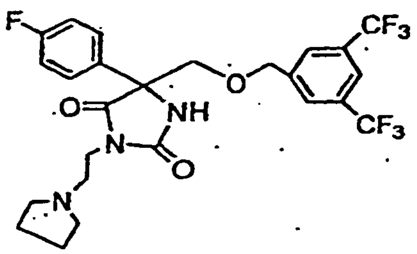

34

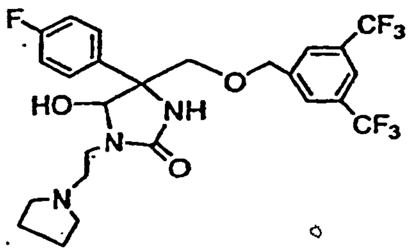

35

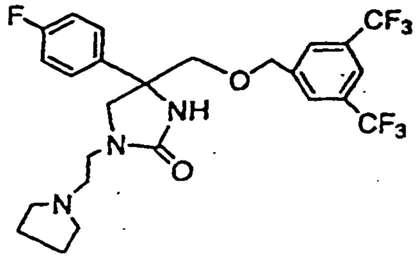

36

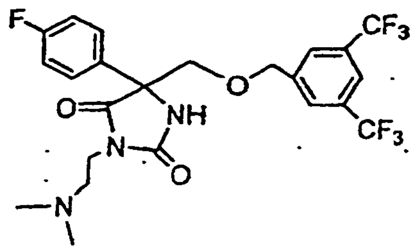

37

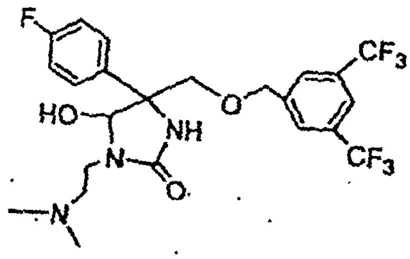

38

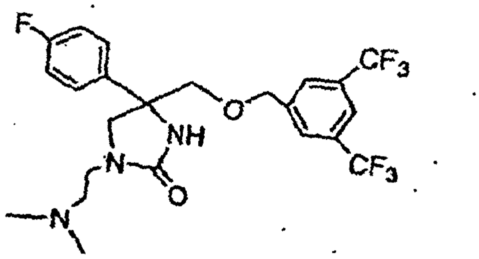

39

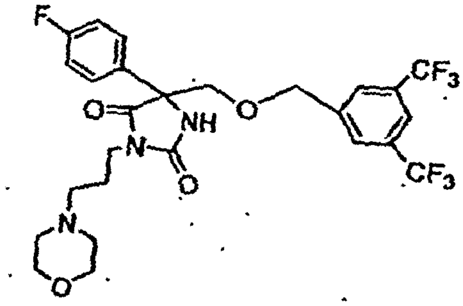

40

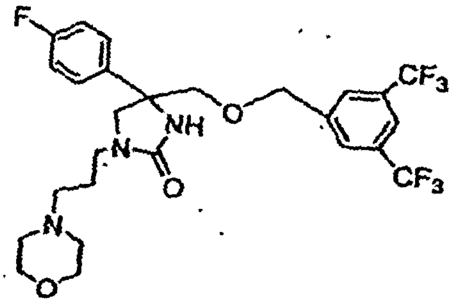

41

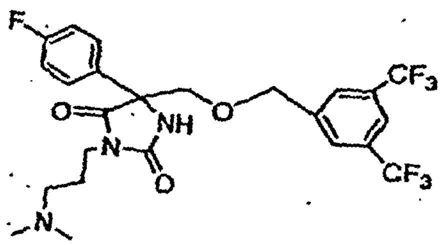

42

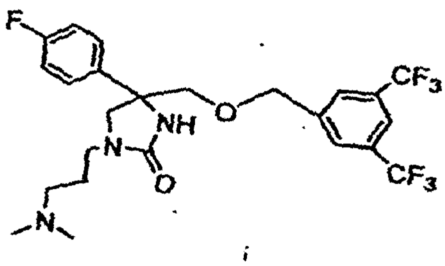

43

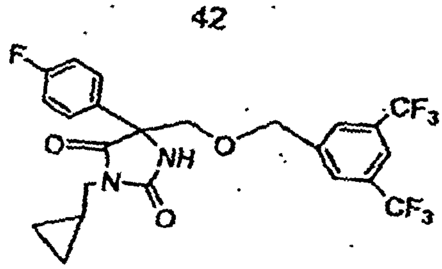

44

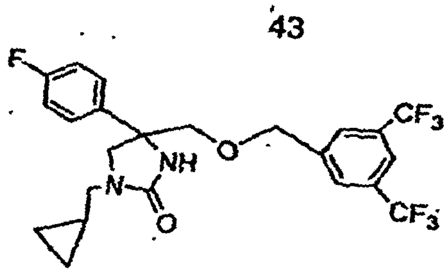

45

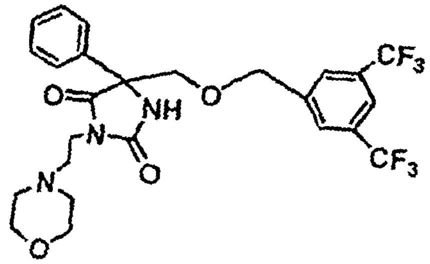

46

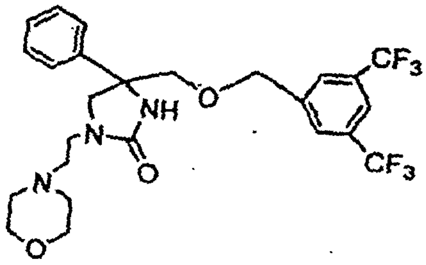

47

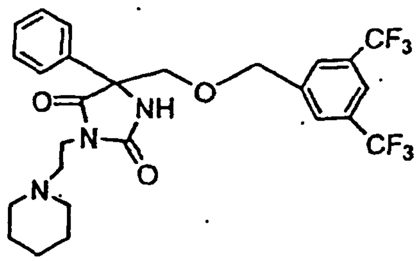

48

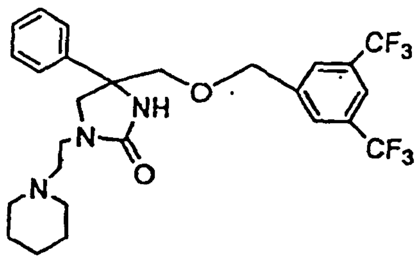

49

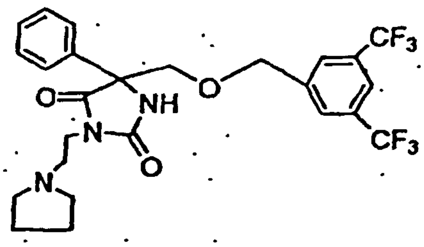

50

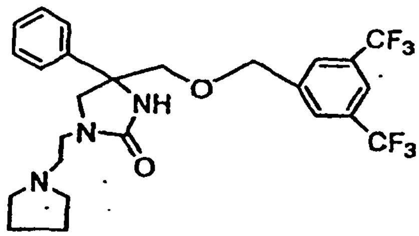

51

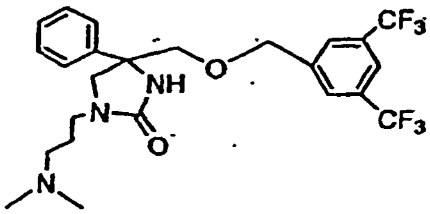

52

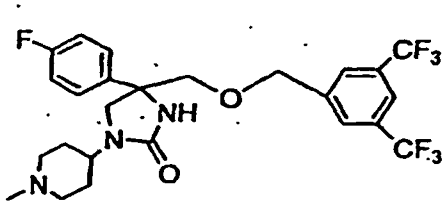

53

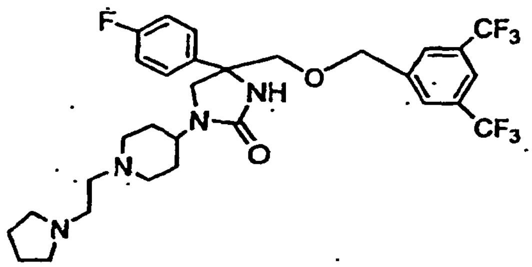

54

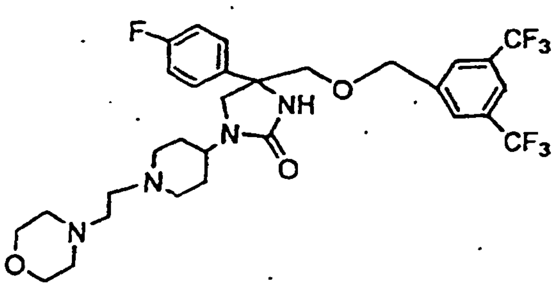

55

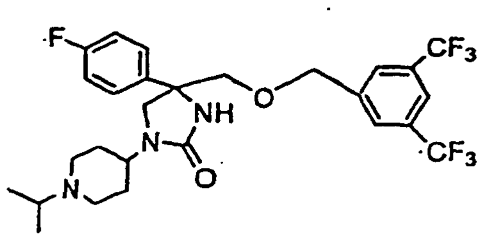

56

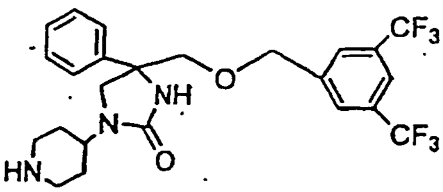

57

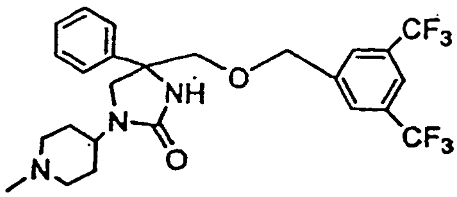

58

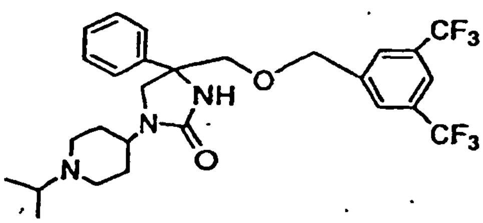

59

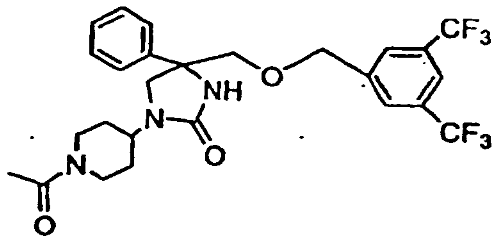

60

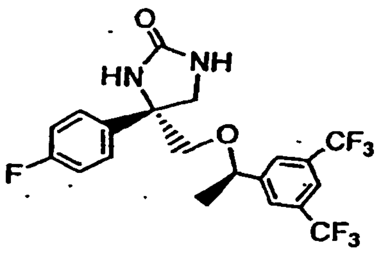

61

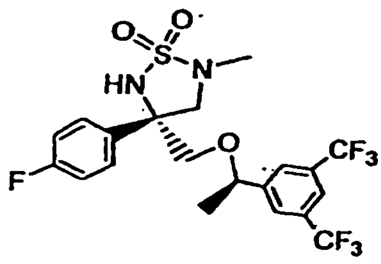

63

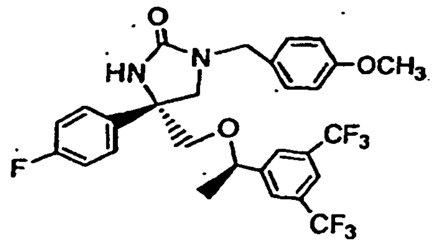

64

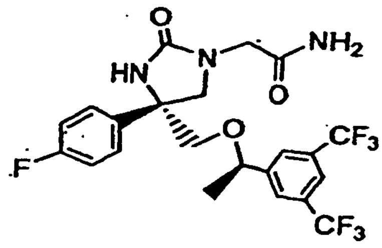

65

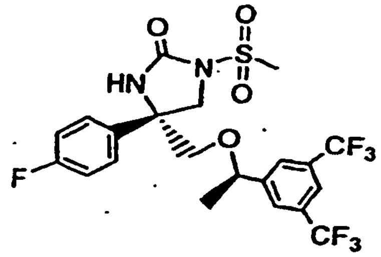

66

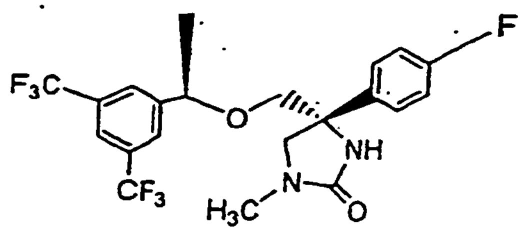

67

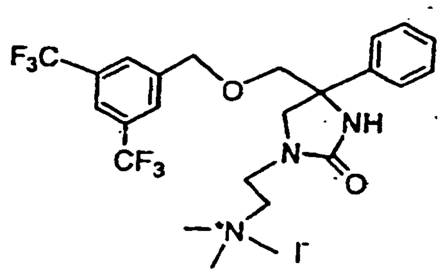

68

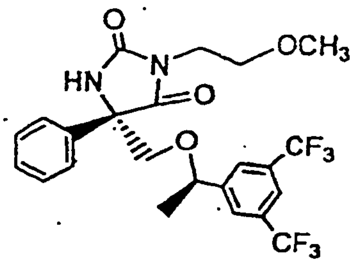

69

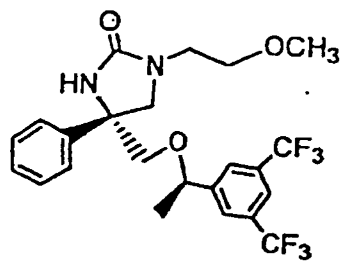

70a

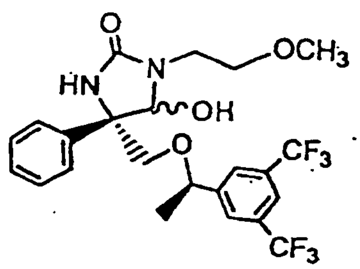

70b

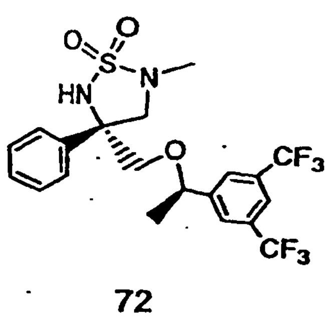

72

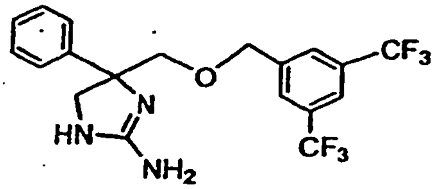
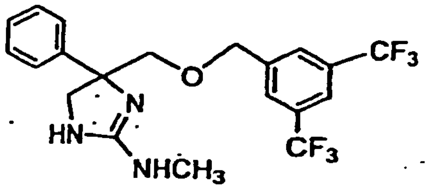

73 74

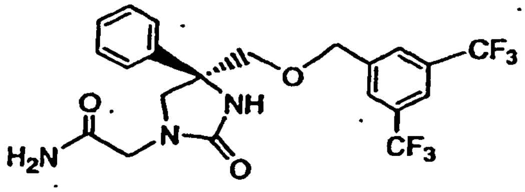
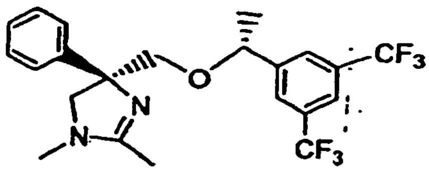

75 76

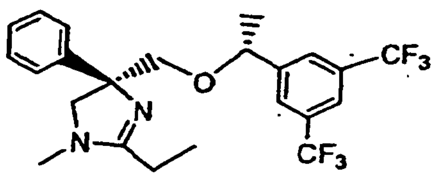
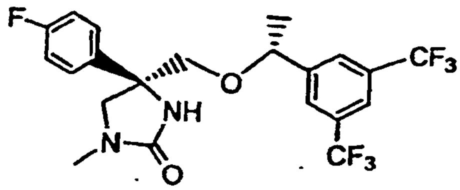

77 78

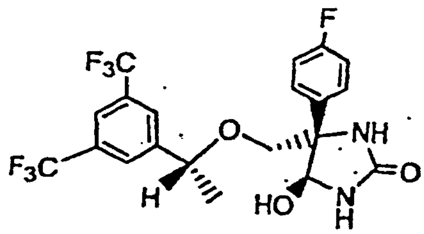
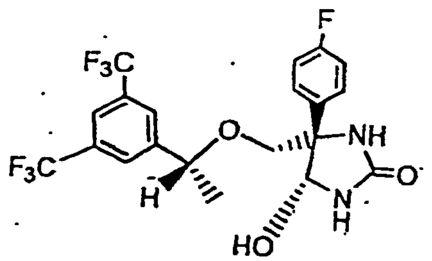

79a 79b

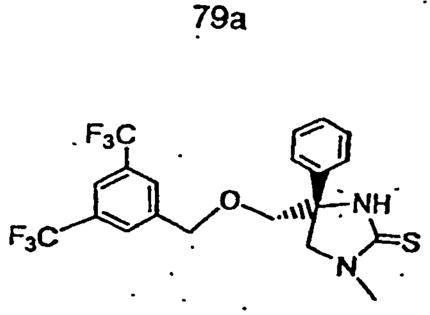
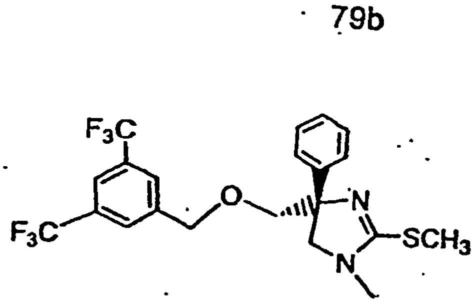

80 81

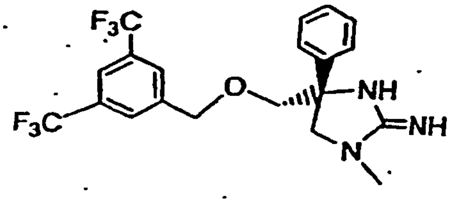

82

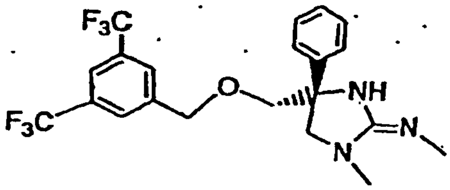

83

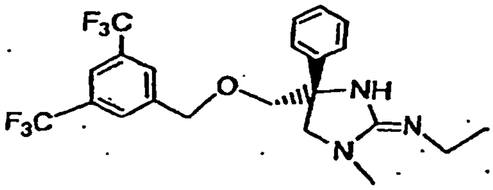

84

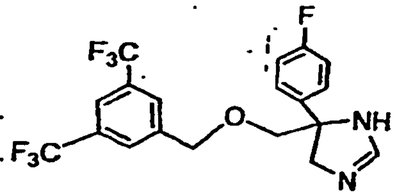

85

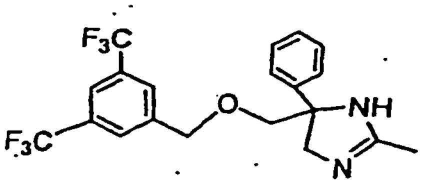

86

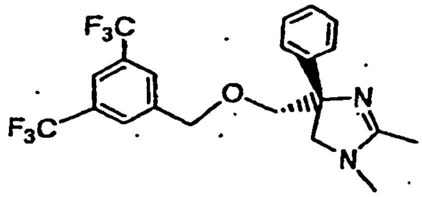

87

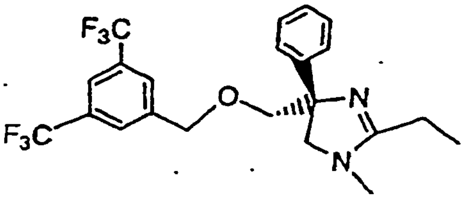

88

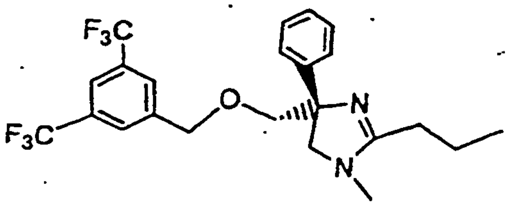

89

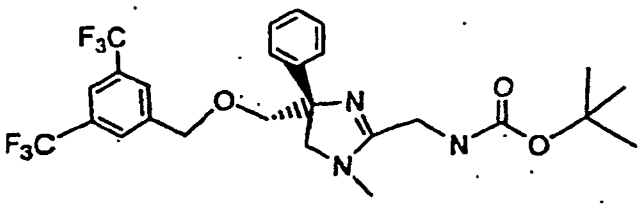

90

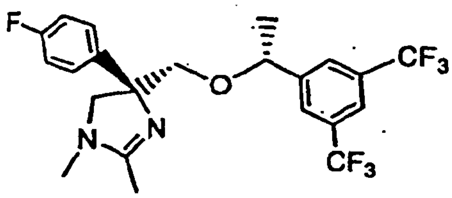

91

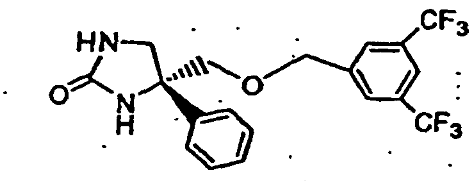

92

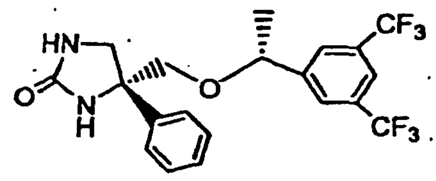

93

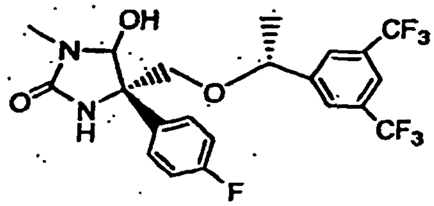

94

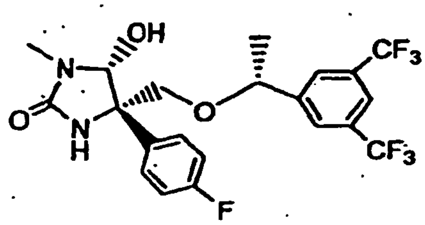

95

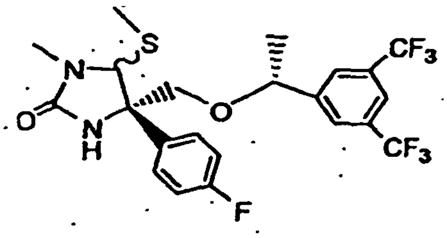

96

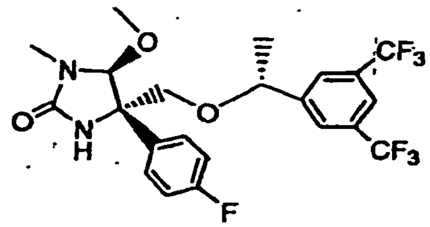

97

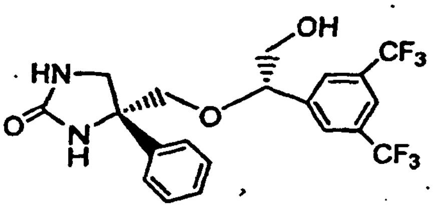

98

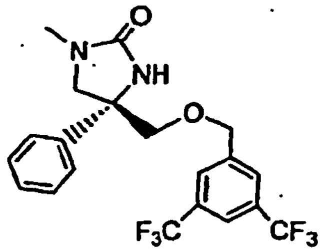

99

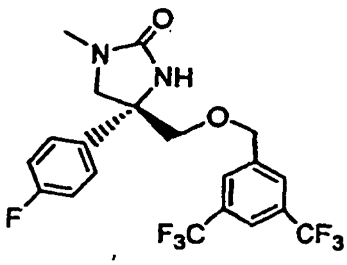

100

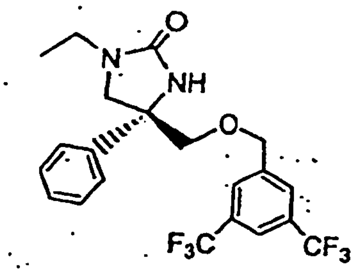

101

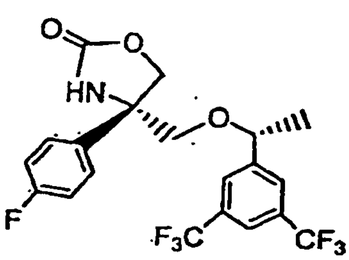

102

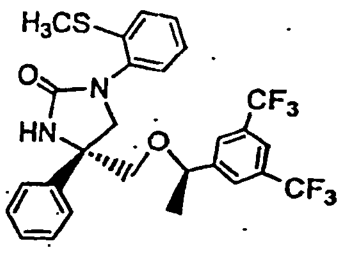

103

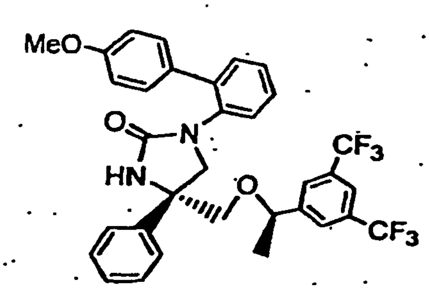

104

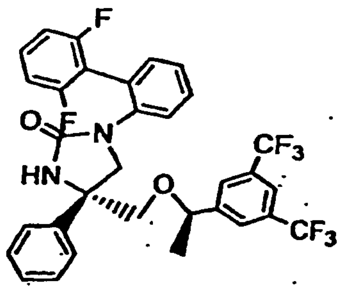

105

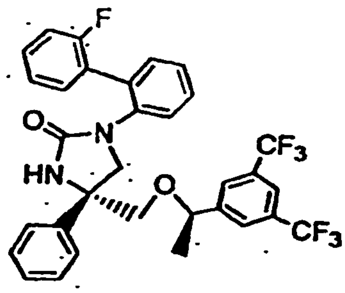

106

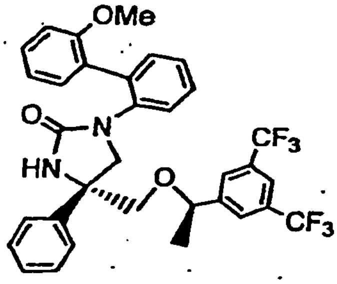

107

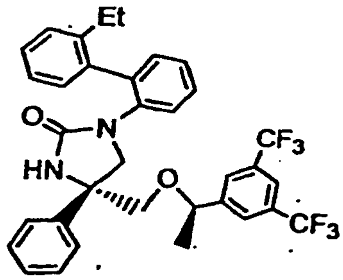

108

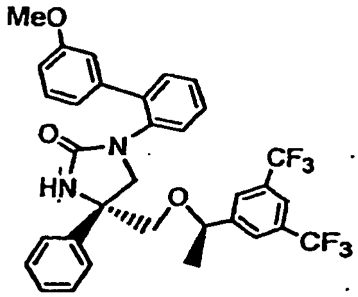

109

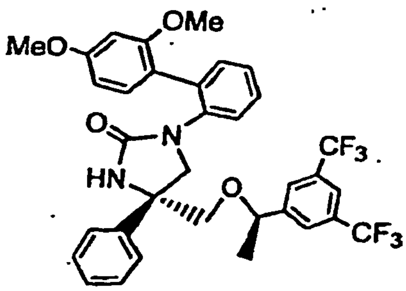

110

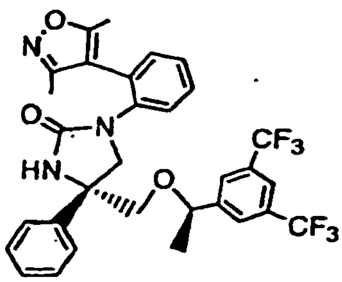

111

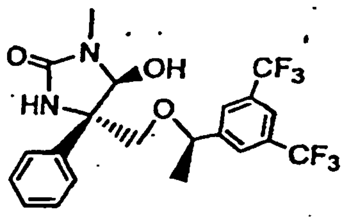

112

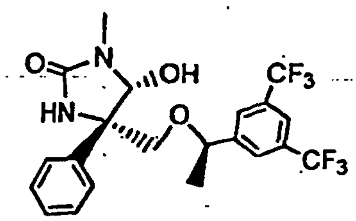

113

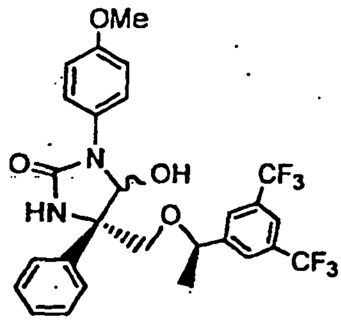

114

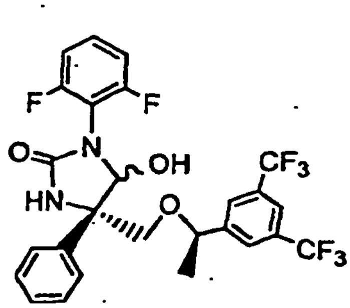

115

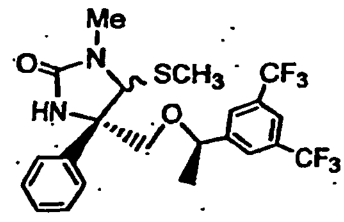

116

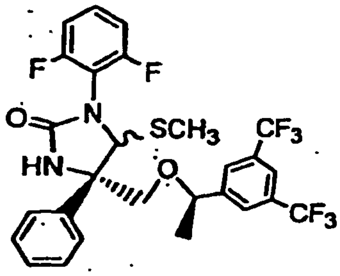

117

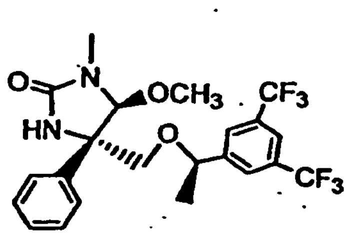

118

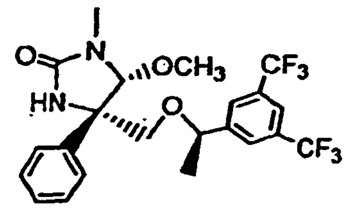

119

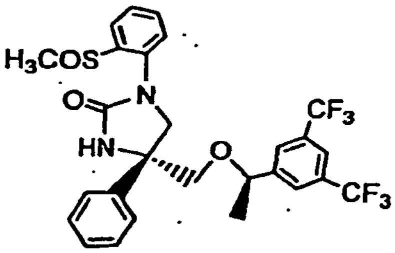

120

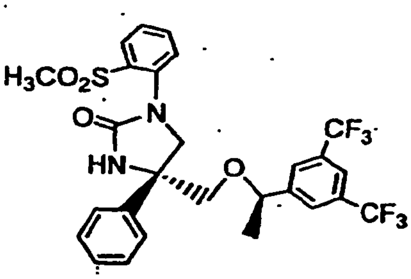

121

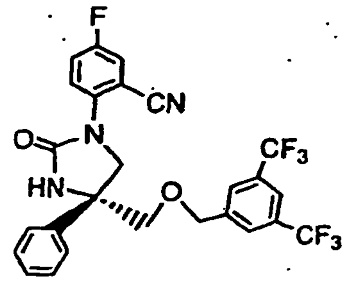

122

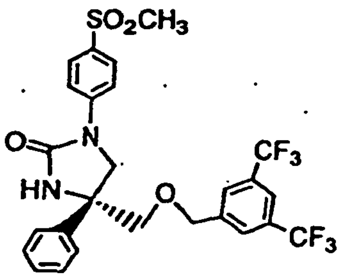

123

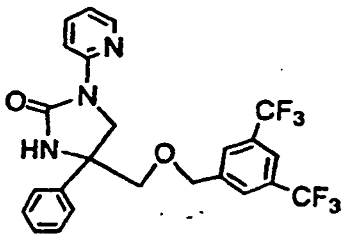

124

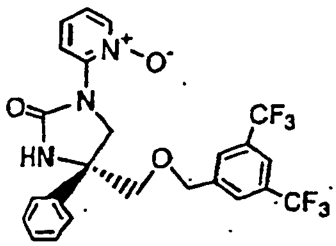

125

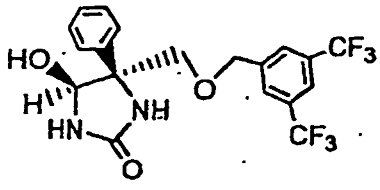

126

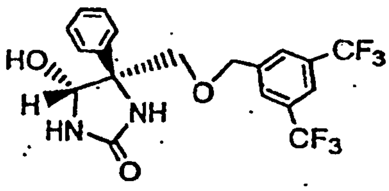

127

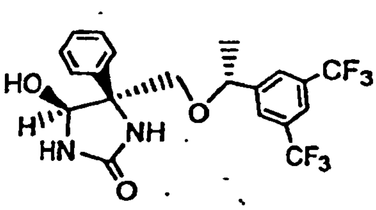

128

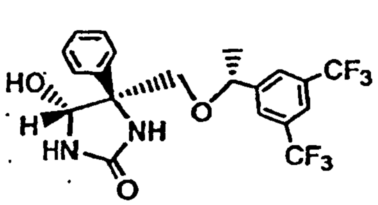

129

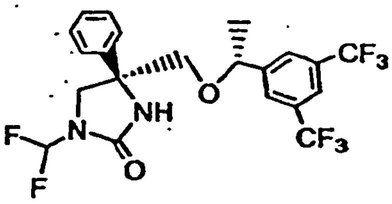

130

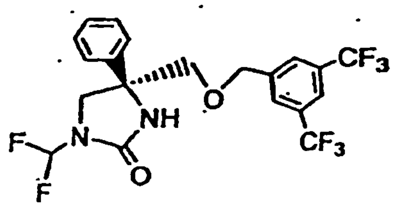

131

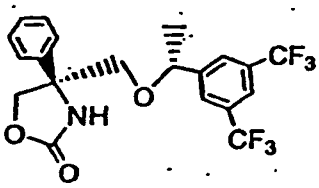

132

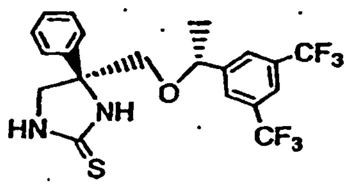

133

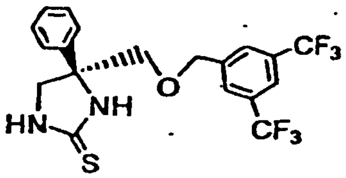

134

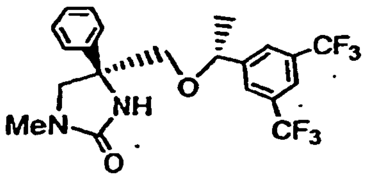

135

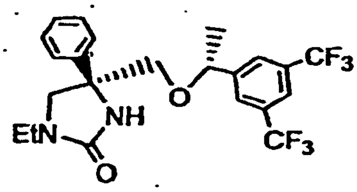

136

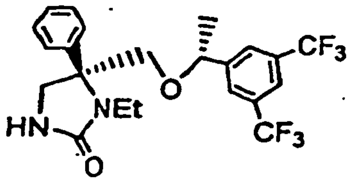

137

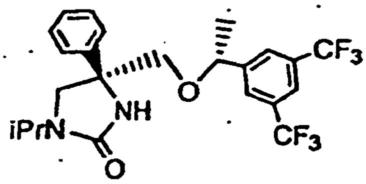

138

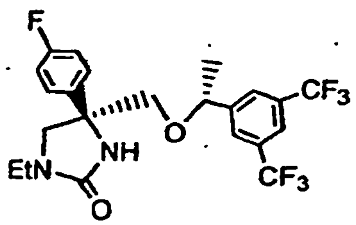

139

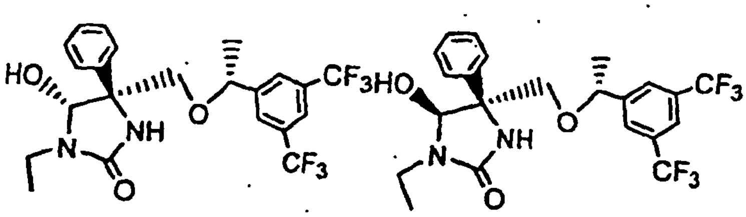

140

141

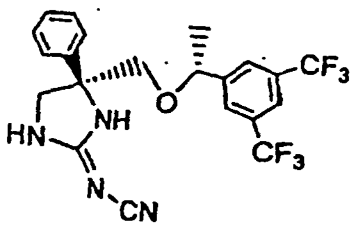

142

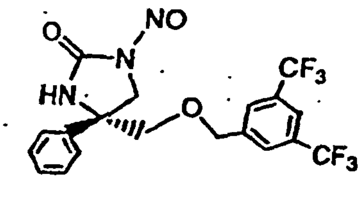

143

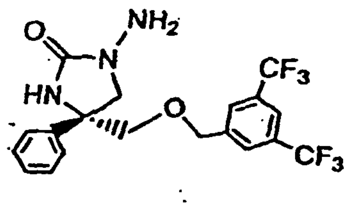

144

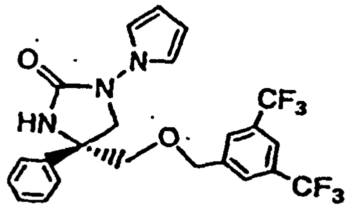

145

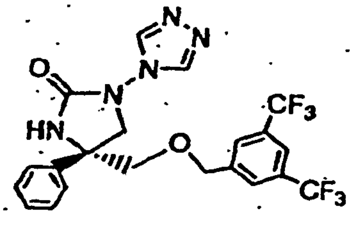

146

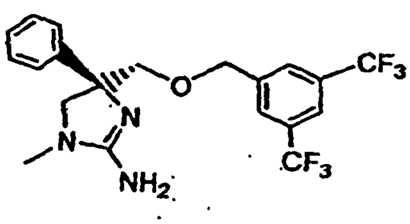

147

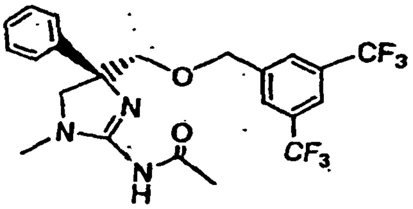

148

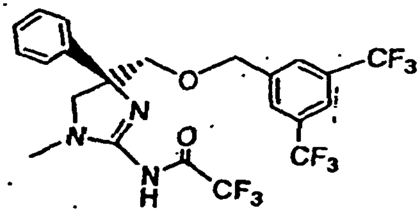

149

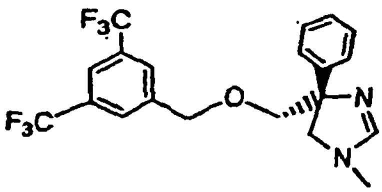

150

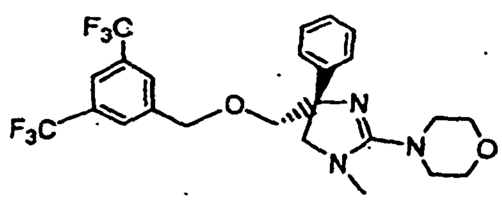

151

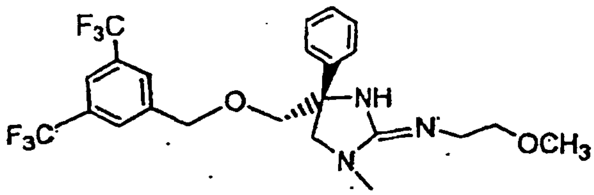

152

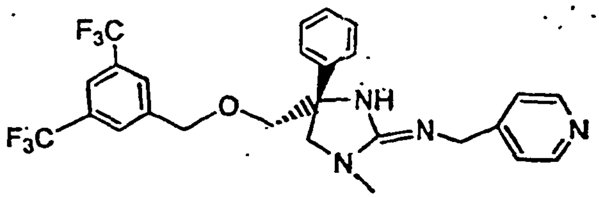

153

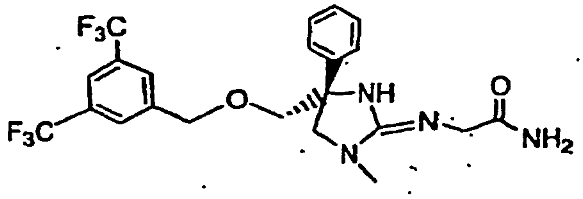

154

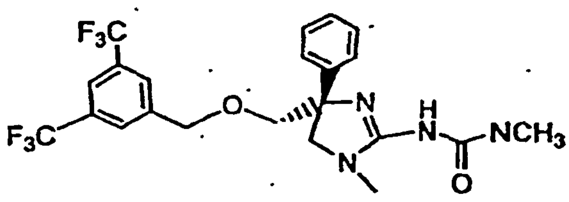

155

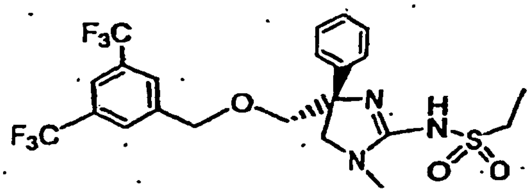

156

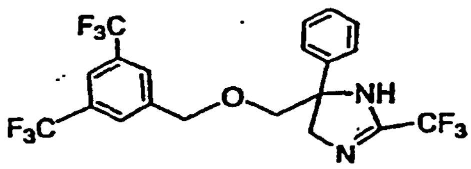

157

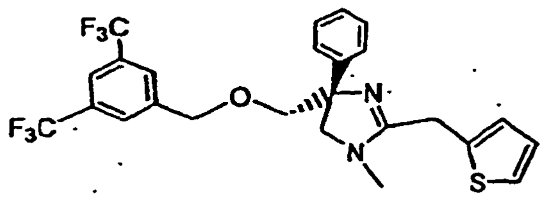

158

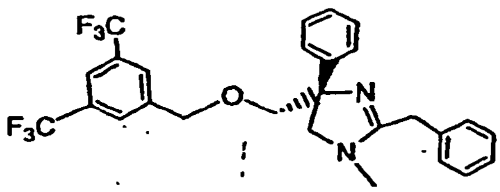

159

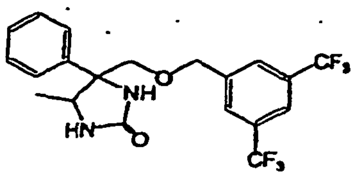

160

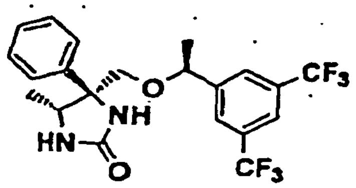

Beispiel 161a

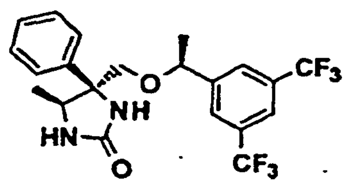

Beispiel 161b

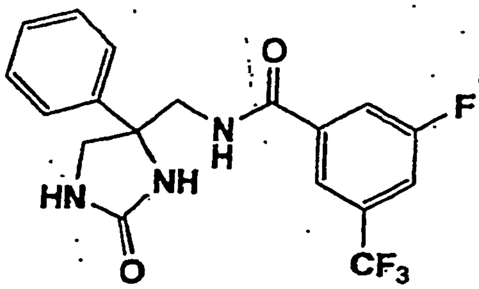

162

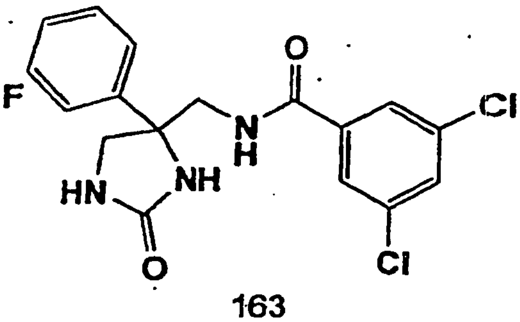

163

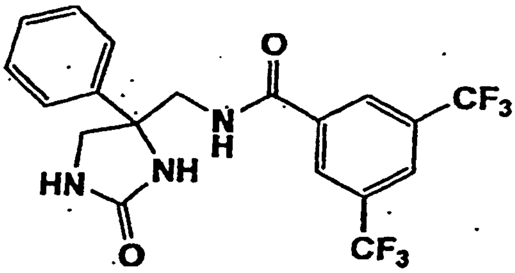

164

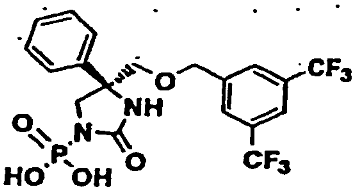

• 2 N-Methylglucamin

Beispiel 165a

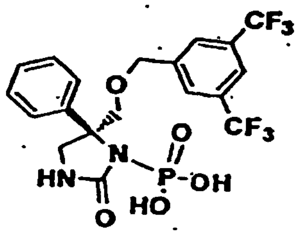

• 2 N-Methylglucamin

Beispiel 165b

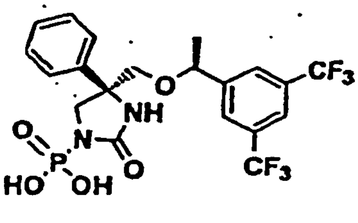

• 2 N-Methylglucamin

Beispiel 166a

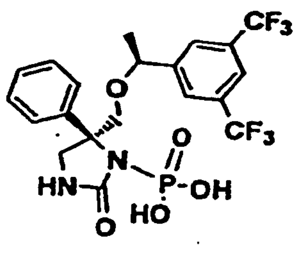

• 2 N-Methylglucamin

Beispiel 166b

**13.** Verbindung nach Anspruch 12 ausgewählt aus den Verbindungen Nr. 2, 17, 19, 31, 59, 61, 65, 67, 76, 77, 78, 79a, 79b, 92, 93, 99, 100, 103, 104, 110, 111, 113, 120, 121, 126, 127, 128, 129, 130, 135, 142, 165, 165a, 165b, 166a, 166b oder Verbindung 118.

**14.** Pharmazeutische Zusammensetzung, die eine wirksame Menge einer Verbindung gemäß Anspruch 1 bis 13 und einen pharmazeutisch annehmbaren Träger enthält.

**15.** Pharmazeutische Zusammensetzung, die eine wirksame Menge einer Verbindung gemäß Anspruch 1 bis 13 in Kombination einer wirksamen Menge eines selektiven Serotonin-Wiederaufnahmeinhibitors und einem pharmazeutisch annehmbaren Träger enthält.

**16.** Verwendung der Verbindungen gemäß Ansprüchen 1 bis 13 zur Herstellung eines Medikaments zur Behandlung von Atemwegserkrankungen, entzündlichen Erkrankungen, Hautproblemen, ophthalmologischen Störungen, Suchterkrankungen, mit Stress zusammenhängenden Störungen, Zwangsstörungen, Essstörungen, Manie, prämenstruellem Syndrom, Zuständen des zentralen Nervensystems, gastrointestinalen Störungen, Blasenstörungen, Arteriosklerose, fibrosierenden Störungen, Fettleibigkeit, Diabetes Typ II, mit Schmerz zusammenhängenden Störungen und Urogenitalstörungen.

**17.** Verwendung der Verbindungen gemäß Ansprüchen 1 bis 13 in Kombination mit einem selektiven Serotonin-Wiederaufnahmeinhibitors zur Herstellung eines Medikaments zur Behandlung von Atemwegserkrankungen, entzündlichen Erkrankungen, Hautproblemen, ophthalmologischen Störungen, Suchterkrankungen, mit Stress zusammenhängenden Störungen, Zwangsstörungen, Essstörungen, Manie, prämenstruellem Syndrom, Zuständen des zentralen Nervensystems, gastrointestinalen Störungen, Blasenstörungen, Arteriosklerose, fibrosierenden Störungen, Fettleibigkeit, Diabetes Typ II, mit Schmerz zusammenhängenden Störungen und Urogenitalstörungen.

**18.** Verwendung der Verbindungen gemäß Ansprüchen 1 bis 13 zur Herstellung eines Medikaments zur Behandlung von Erbrechen, Depression, Ängstlichkeit und Husten.

**19.** Verwendung der Verbindungen gemäß Ansprüchen 1 bis 13 in Kombination mit einem selektiven Serotonin-Wiederaufnahmeinhibitor zur Herstellung eines Medikaments zur Behandlung von Erbrechen, Depression, Ängstlichkeit und Husten.

## Revendications

**1.** Composé représenté par la formule structurale

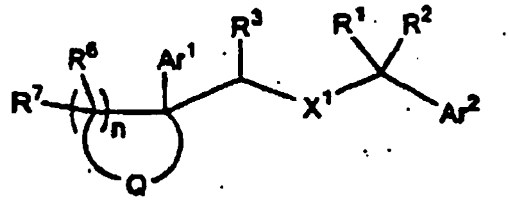

I

ou sel pharmacologiquement admissible d'un tel composé,
dans laquelle formule :

$Ar^1$ et $Ar^2$ représentent chacun, indépendamment, un groupe choisi dans l'ensemble constitué par $R^{17}$-hétéroaryle et

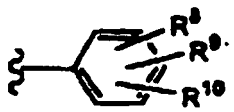

;

$X^1$ représente -O-, -S-, -SO-, $-SO_2-$, $-NR^{12}-$, $-N(COR^{12})-$ ou $-N(SO_2R^{15})-$ ;
$R^1$, $R^2$, $R^3$ et chaque symbole $R^7$ représentent chacun, indépendamment, un atome d'hydrogène ou un groupe alkyle en $C_{1-6}$, hydroxyalkyle en $C_{1-3}$, cycloalkyle en $C_{3-8}$, $-CH_2F$, $-CHF_2$ ou $-CF_3$, ou $R^1$ et $R^2$ représentent ensemble un groupe alkylène qui, avec l'atome de carbone auquel il est attaché, forme un cycle en $C_{3-6}$, ou encore, si $X^1$ représente -O-, -S- ou $-NR^{12}-$, $R^1$ et $R^2$ représentent ensemble =O ;

chaque symbole $R^6$ représente indépendamment un atome d'hydrogène ou un groupe alkyle en $C_{1-6}$, $-OR^{13}$ ou $-SR^{12}$ ;

n vaut de 1 à 4, et si n est supérieur à 1, $R^6$ et $R^7$ peuvent représenter des entités identiques ou différentes sur chaque atome de carbone ;

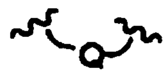

représente un fragment choisi dans l'ensemble formé par

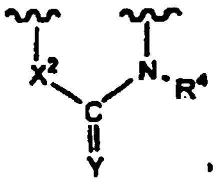, 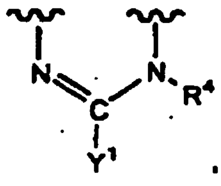, 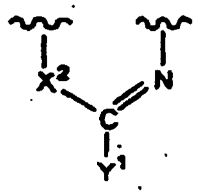 et 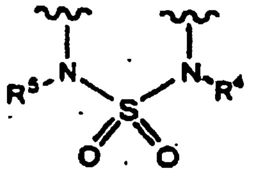 ;

$X^2$ représente -O-, -S- ou $-NR^5-$ ;

Y représente =O, =S ou $=NR^{11}$ ;

Y' représente un atome d'hydrogène ou un groupe alkyle en $C_{1-6}$, $-NR^{17}R^{13}$, $-SCH_3$, $R^{19}$ -aryl-$(CH_2)n_6$-, $R^{19}$ -hétéroaryl-$(CH_2)_{n6}$-, $-(CH_2)_{n6}$-hétérocycloalkyle, -(alkyle en $C_{1-3}$)-NH-C(O)O(alkyle en $C_{1-6}$) ou $-NHC(O)R^{15}$ ;

$R^5$ représente un atome d'hydrogène ou un groupe $-(CH_2)_{n1}$-G où $n_1$ vaut de 0 à 5 et G représente un atome d'hydrogène ou un groupe $-CF_3$, $-CHF_2$, $-CH_2F$, -OH, -O-(alkyle en $C_{1-6}$), $-SO_2R^{13}$, -O-(cycloalkyle en $C_{3-8}$), $-NR^{13}R^{14}$, $-SO_2NR^{13}R^{14}$, $-NR^{13}SO_2R^{15}$, $-NR^{13}COR^{12}$, $-NR^{12}(CONR^{13}R^{14})$, —$CONR\text{-}^{13}R^{14}$, $-COOR^{12}$, cycloalkyle en $C_{3-8}$, $R^{16}$-aryle, $R^{19}$-hétéroaryle,

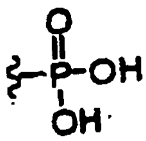, 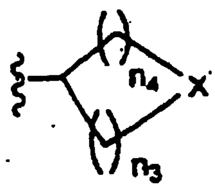, 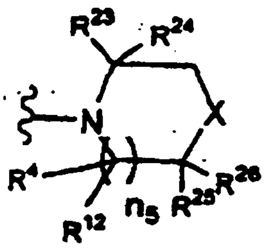, 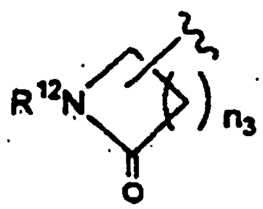,

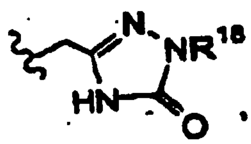, 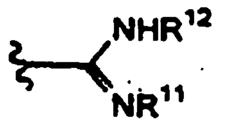 ou 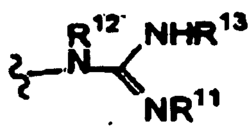,

étant entendu que, si $n_1$ vaut 0, $R^5$ peut aussi représenter $-C(O)R^{13}$ ou $-C(S)R^{13}$, et que G ne représente pas un atome d'hydrogène si $n_1$ vaut 0 ;

X représente un chaînon $-NR^{20}$-, $-N(CONR^{13}R^{14})$-, $-N(CO_2R^{13})$-, $-N(SO_2R^{15})$-, $-N(COR^{12})$-, $-N(SO_2NHR^{13})$-, -O-, -S-, -SO-, $-SO_2$-, $-CF_2$-, $-CH_2$- ou $-CFR^{12}$- ;

$R^8$, $R^9$ et $R^{10}$ représentent chacun, indépendamment, un atome d'hydrogène ou d'halogène ou un groupe alkyle en $C_{1-6}$, cycloalkyle en $C_{3-8}$, $-OR^{12}$, -CN, $-NO_2$, $-CF_3$, $-CHF_2$, $-CH_2F$, $-OCF_3$, $-OCHF_2$, $-OCH_2F$, $-COOR^{12}$, $-CONR^{21}R^{22}$, $-NR^{21}COR^{12}$, $-NR^{21}CO_2R^{15}$, $-NR^{21}CONR^{21}R^{22}$, $-NR^{21}SO_2R^{15}$, $-NR^{21}R^{22}$, $-SO_2NR^{21}R^{22}$, $-S(O)_{n5}R^{15}$, $R^{16}$-aryle ou $R^{19}$-hétéroaryle ;

$R^{11}$ représente un atome d'hydrogène ou un groupe alkyle en $C_{1-6}$, cycloalkyle en $C_{3-8}$,$-NO_2$, -CN, -OH, $-OR^{12}$, $-O(CH_2)_{n6}R^{12}$, -(alkyle en $C_{1-3}$)-$C(O)NHR^{12}$, $R^{19}$-aryl-$(CH_2)_{n6}$- ou $R^{19}$-hétéroaryl-$(CH_2)_{n6}$- ;

$R^4$ et $R^{12}$ représentent chacun, indépendamment, un atome d'hydrogène ou un groupe alkyle en $C_{1-6}$ ou cycloalkyle en $C_{3-8}$ ;

$R^{13}$ et $R^{14}$ représentent chacun, indépendamment, un atome d'hydrogène ou un groupe alkyle en $C_{1-6}$, cycloalkyle en $C_{3-8}$, $R^{19}$-aryl-$(CH_2)_{n6}$- ou $R^{19}$- hétéroaryl-$(CH_2)_{n6}$-, ou bien $R^{13}$ et $R^{14}$ représentent ensemble un groupe alkylène en $C_{3-6}$ qui, avec l'atome d'azote auquel il est attaché, forme un cycle de 4 à 7 chaînons, l'un des atomes de carbone de la chaîne de ce groupe alkylène représenté par $R^{13}$ et $R^{14}$ pouvant être remplacé par un chaînon hétéroatomique choisi parmi -O-, -S- et -$NR^{12}$- ;
$R^{15}$ représente un groupe alkyle en $C_{1-6}$, cycloalkyle en $C_{3-8}$ ou -$CF_3$ ;
$R^{16}$ représente 1 à 3 substituants choisis indépendamment dans l'ensemble formé par les atomes d'hydrogène et d'halogène et les groupes alkyle en $C_{1-6}$, cycloalkyle en $C_{3-8}$, alcoxy en $C_{1-6}$ et -$CF_3$ ;
$R^{17}$ représente un atome d'hydrogène ou un groupe alkyle en $C_{1-6,}$ cycloalkyle en $C_{3-8}$, -$COOR^{12}$, -$CONR^{21}R^{22}$, -$NR^{21}R^{22}$, -$NR^{21}$ $COR^{12}$, -$NR^{21}CO_2R^{12}$, -$NR^{21}CONR^{21}R^{22}$, -$NR^{21}SO_2R^{15}$ ou -$S(O)_{n5}R^{15}$ ;
$R^{18}$ représente un atome d'hydrogène ou un groupe alkyle en $C_{1-6}$ ou -$P(O)(OH)_2$ ;
$R^{19}$ représente 1 à 3 substituants choisis indépendamment dans l'ensemble formé par les atomes d'hydrogène et d'halogène et les groupes alkyle en $C_{1-6}$, cycloalkyle en $C_{3-8}$, -OH, -CN, -$NO_2$, -$CF_3$, -$CHF_2$, -$CH_2F$, -$OCF_3$, -$OCHF_2$, -$OCH_2F$, -O-(alkyle en $C_{1-6}$), -O-(cycloalkyle en $C_{3-8}$), -$COOR^{12}$, -$CONR^{21}R^{22}$, -$NR^{21}R^{22}$, -$NR^{21}COR^{12}$, -$NR^{21}CO_2R^{12}$, -$NR^{21}CONR^{21}R^{22}$, -$NR^{21}SO_2R^{15}$ et -$S(O)_{n5}R^{15}$ ;
$R^{20}$ représente un atome d'hydrogène ou un groupe alkyle en $C_{1-6}$, cycloalkyle en $C_{3-8}$ ou -$(CH_2)_{n6}$-hétérocycloalkyle ;
$R^{21}$ et $R^{22}$ représentent chacun, indépendamment, un atome d'hydrogène ou un groupe alkyle en $C_{1-6}$, cycloalkyle en $C_{3-8}$ ou benzyle, ou bien $R^{21}$ et $R^{22}$ représentent ensemble un groupe alkylène en $C_{3-6}$ qui, avec l'atome d'azote auquel il est attaché, forme un cycle de 4 à 7 chaînons, l'un des atomes de carbone de la chaîne de ce groupe alkylène représenté par $R^{21}$ et $R^{22}$ pouvant être remplacé par un chaînon hétéroatomique choisi parmi -O-, -S- et -$NR^{12}$- ;
$R^{23}$ , $R^{24}$ , $R^{25}$ et $R^{26}$ représentent chacun un atome d'hydrogène ou un groupe alkyle en $C_{1-6}$, et peuvent aussi représenter conjointement =O, étant entendu que, si $n_5$ vaut 0 et si $R^{25}$ et $R^{26}$ représentent chacun un atome d'hydrogène, X ne représente pas un chaînon O, N ou S ;
$n_3$ et $n_4$ valent chacun, indépendamment, de 1 à 5, sous réserve que la somme de $n_3$ et $n_4$ vaille de 2 à 6 ;
$n_5$ vaut indépendamment de 0 à 2 ; et
$n_6$ vaut indépendamment de 0 à 3, étant entendu que :

le terme "aryle" désigne un groupe phényle, naphtyle, indényle, tétrahydronaphtyle, indanyle, anthracényle ou fluorényle ;
le terme "hétéroaryle" désigne un cycle aromatique unique ou des cycles aromatiques benzocondensés, comportant 5 à 10 chaînons au nombre desquels 1 à 4 chaînons hétéroatomiques choisis indépendamment dans l'ensemble constitué par -O-, -S-, -N= et -NH-, sous réserve qu'il n'y ait pas d'atomes d'oxygène et/ou de soufre adjacents dans ces cycles ;
et le terme "hétérocycloalkyle" désigne un cycle saturé comportant 4 à 7 chaînons au nombre desquels 1 à 3 chaînons hétéroatomiques choisis indépendamment dans l'ensemble constitué par -O-, -S- et -$NR^{21}$- où $R^{21}$ représente un atome d'hydrogène ou un groupe alkyle en $C_{1-6}$, les autres chaînons du cycle étant des atomes de carbone.

**2.** Composé conforme à la revendication 1, dans lequel $R^1$, $R^3$, $R^4$ et $R^7$ représentent chacun un atome d'hydrogène.

**3.** Composé conforme à la revendication 1, dans lequel $R^6$ représente un atome d'hydrogène ou un groupe -OH.

**4.** Composé conforme à la revendication 1, dans lequel $X^1$ représente -O- ou -$NR^{12}$-.

**5.** Composé conforme à la revendication 1, dans lequel $Ar^1$ et $Ar^2$ représentent chacun un groupe $R^8$,$R^9$,$R^{10}$-phényle.

**6.** Composé conforme à la revendication 1, dans lequel

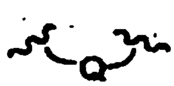

représente

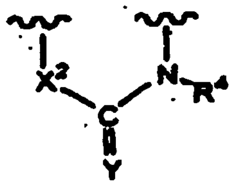

7. Composé conforme à la revendication 6, dans lequel n vaut 1 ou 2.

8. Composé conforme à la revendication 7, dans lequel $X^2$ représente $-NR^5-$ et Y représente =O.

9. Composé conforme à la revendication 1, dans lequel $R^1$, $R^3$, $R^4$ et $R^7$ représentent chacun un atome d'hydrogène, $X^1$ représente -O- ou $-NR^{12}-$, $Ar^1$ et $Ar^2$ représentent chacun un groupe $R^8,R^9,R^{10}$-phényle, n vaut 1 ou 2, $X^2$ représente $-NR^5-$, $R^6$ représente un atome d'hydrogène ou un groupe -OH, et Y représente =O.

10. Composé conforme à la revendication 1, choisi dans l'ensemble des composés représentés par les formules suivantes :

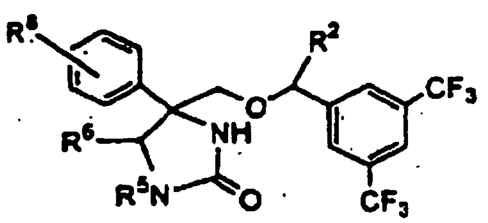 , 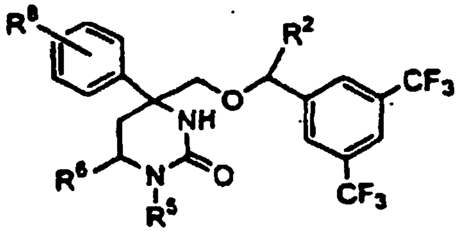 ,

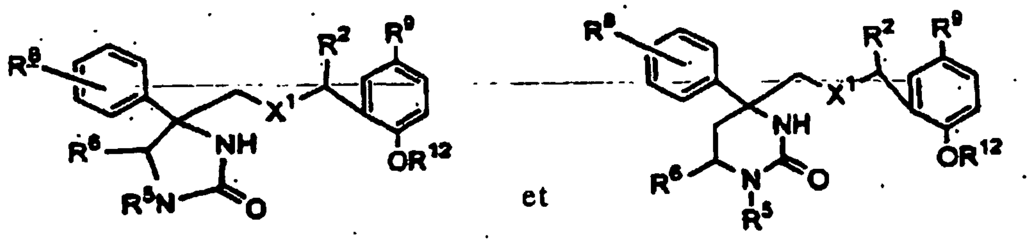 ,

dans lesquelles $R^8$ représente un atome d'hydrogène ou d'halogène, $R^2$ représente un atome d'hydrogène ou un groupe $-CH_3$ ou $-CH_2OH$, $R^6$ représente un atome d'hydrogène ou un groupe -OH, et $R^5$ représente un atome d'hydrogène ou l'un des groupes de formule $-(CH_2)_{n1}-G$.

11. Composé conforme à la revendication 10, dans lequel $X^1$ représente -O-, -NH-, $-N(CH_3)-$ ou $-N(COCH_3)-$, $R^8$ représente un atome d'hydrogène ou d'halogène, $R^2$ représente un atome d'hydrogène ou un groupe $-CH_3$ ou $-CH_2OH$, $R^6$ représente un atome d'hydrogène ou un groupe -OH, $R^9$ représente un groupe $-OCF_3$ ou 5-(trifluorométhyl)-1H-tétrazol-5-yle, $R^{12}$ représente un goupe méthyle ou cyclopropyle, et $R^5$ représente un atome d'hydrogène ou un groupe de formule $-(CH_2)_{n1}-G$ choisi parmi les suivants : $-(CH_2)_{n1}-(R^{19}$-hétéroaryle), $-(CH_2)_{n1}-C(=O)-NR^{21}R^{22}$, $—(CH_2)_{n1}$

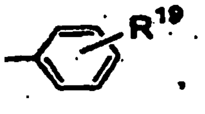

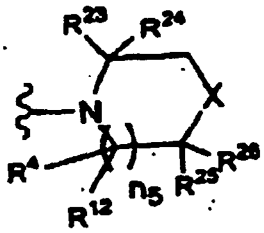

et

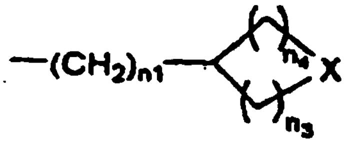

,

ou de formule $-(CH_2)_{n1}$-G', où $n_1$ vaut de 2 à 4 et G' représente un atome d'hydrogène ou un groupe hydroxy, méthoxy, éthoxy, isopropyloxy, cyclopropyloxy ou de formule $-CONR^{13}R^{14}$ où $R^{13}$ et $R^{14}$ représentent chacun, indépendamment, un atome d'hydrogène ou un groupe méthyle, éthyle, isopropyle ou cyclopropyle.

**12.** Composé conforme à la revendication 1, choisi parmi ceux dont les structures sont les suivantes :

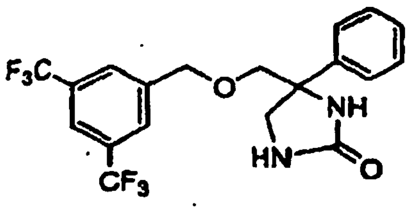

1

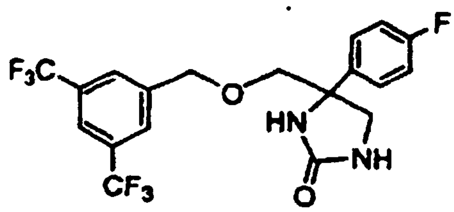

2

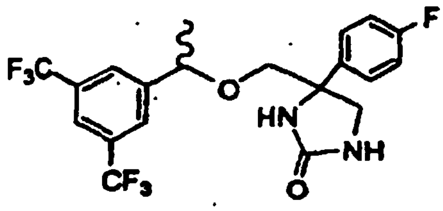

3

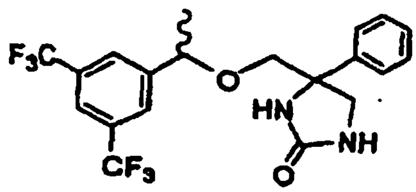

4

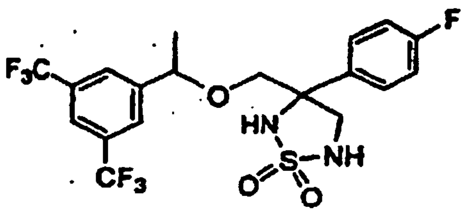

5

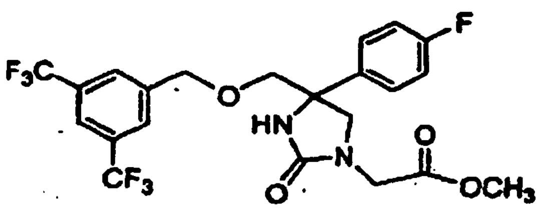

6

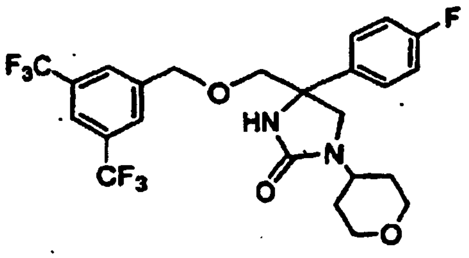

7

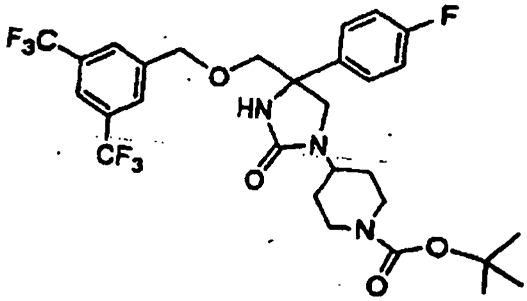

9

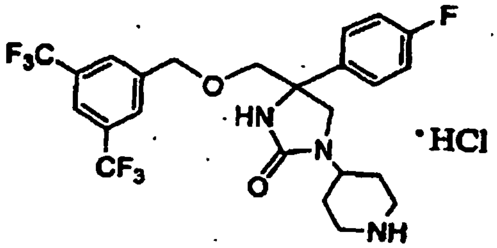

·HCl

10

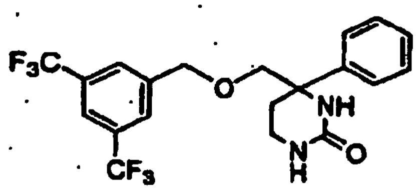

11

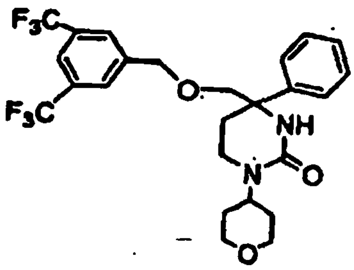

12

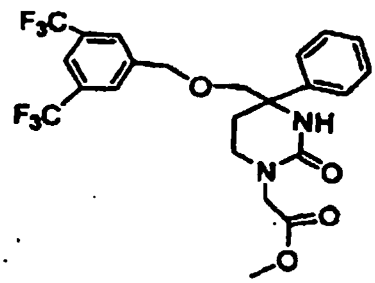

13

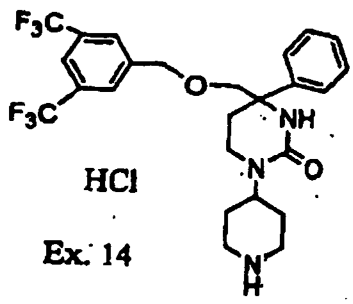

HCl

Ex. 14

14

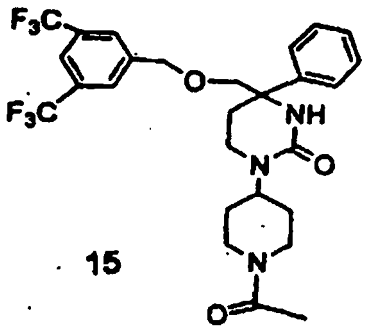

15

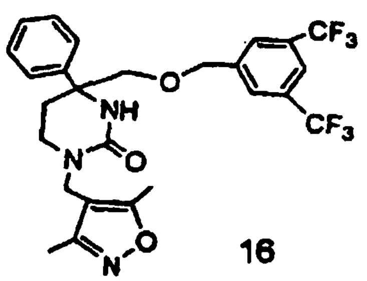

16

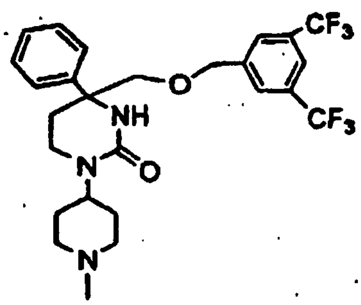

17

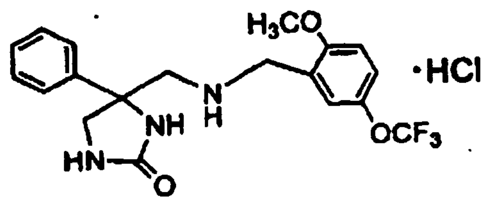

18

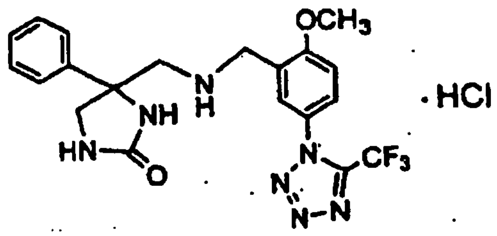

19

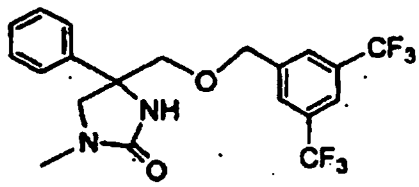

20

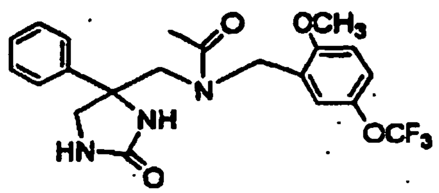

21

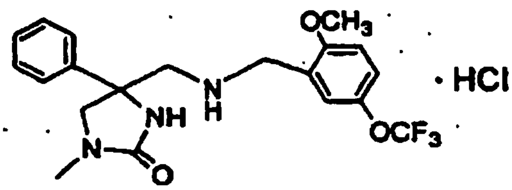

22

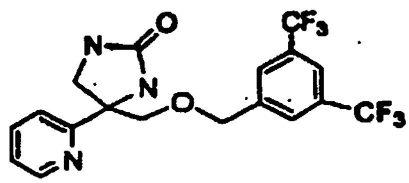

23

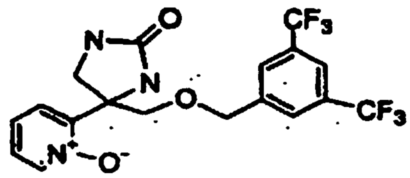

24

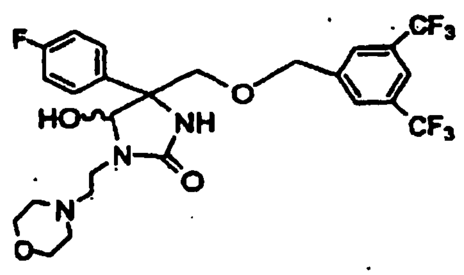

25

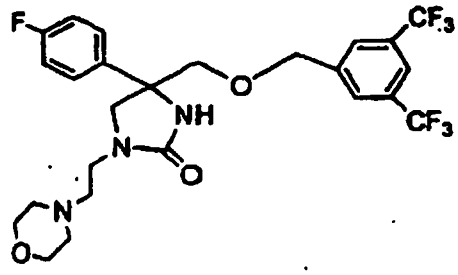

26

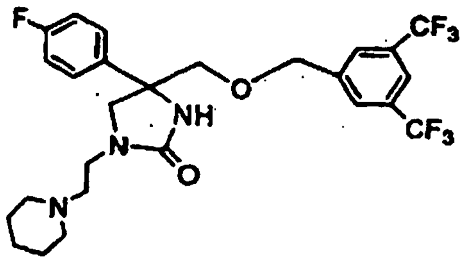

27

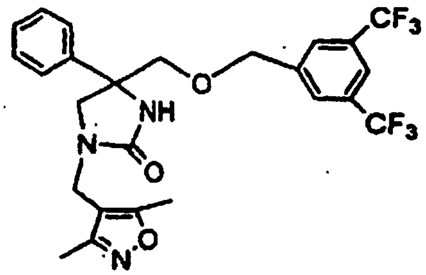

28

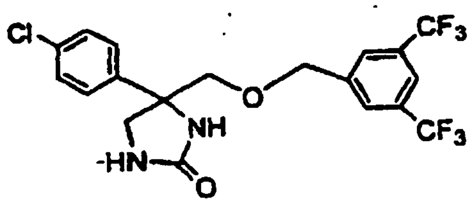

29

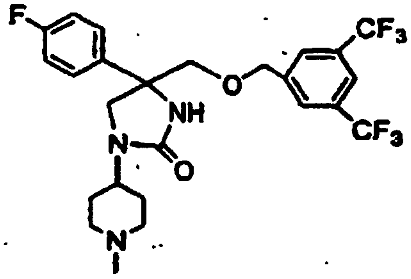

30

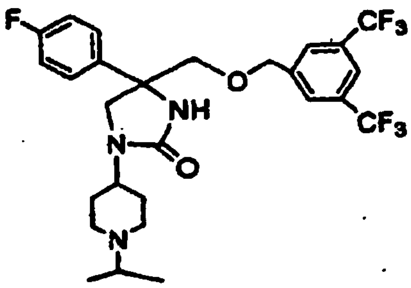

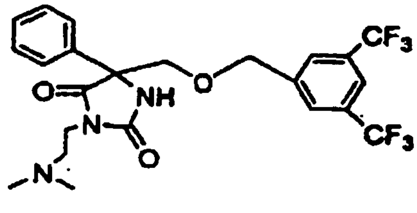

32

31

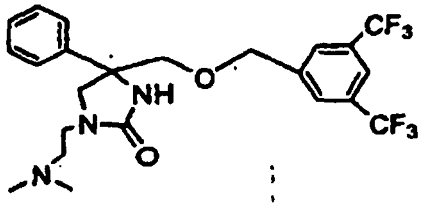

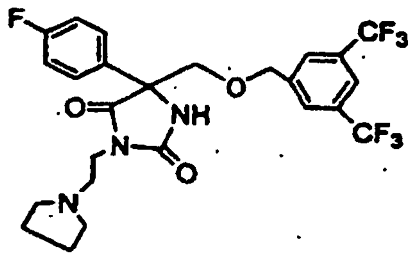

33

34

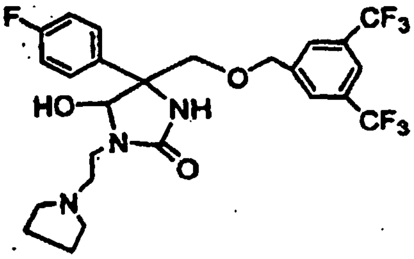

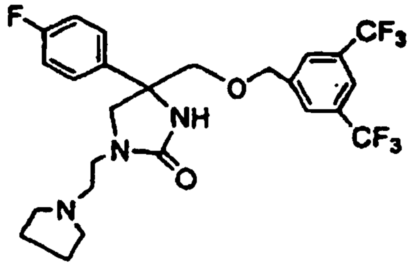

35

36

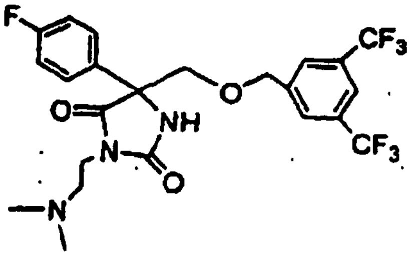

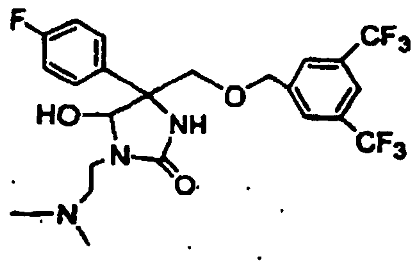

37

38

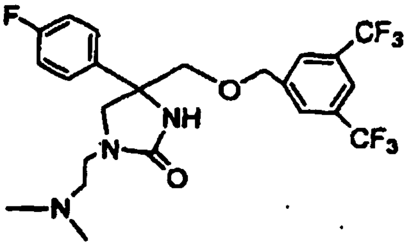

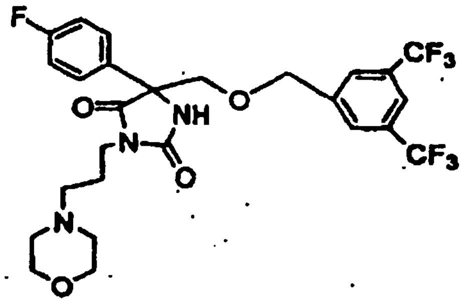

39

40

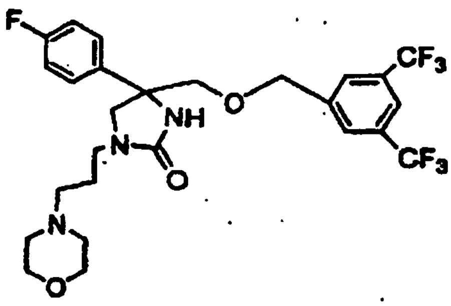

**41**

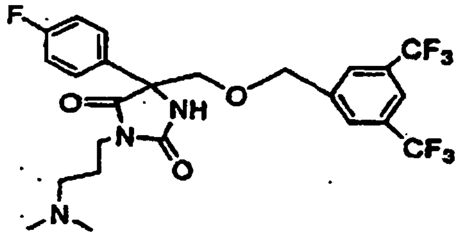

**42**

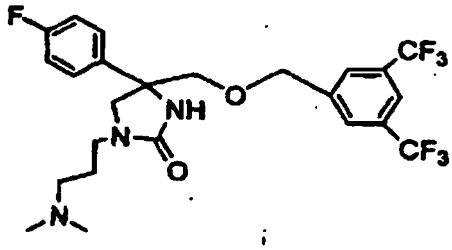

**43**

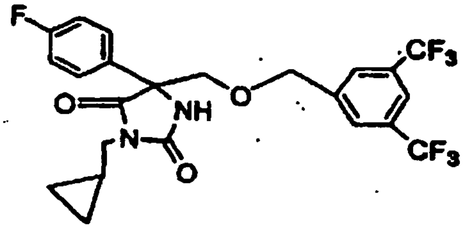

**44**

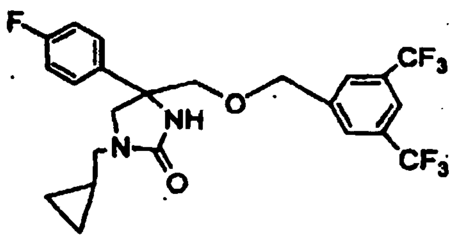

**45**

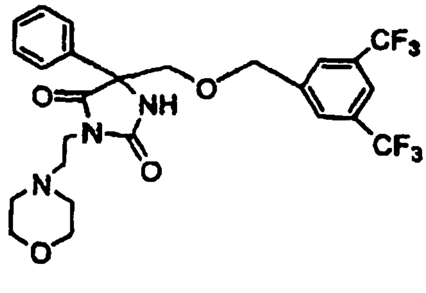

**46**

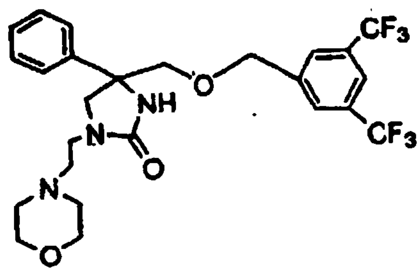

**47**

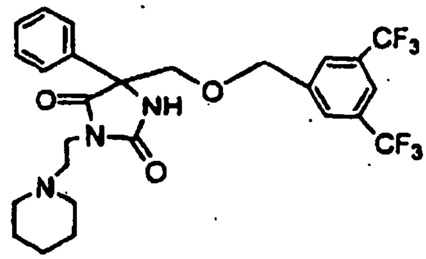

**48**

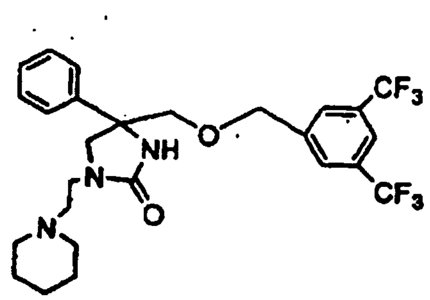

49

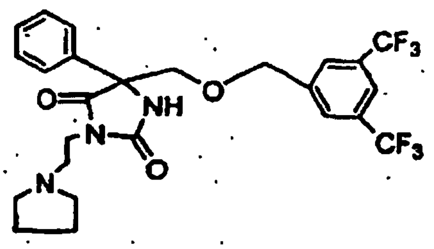

50

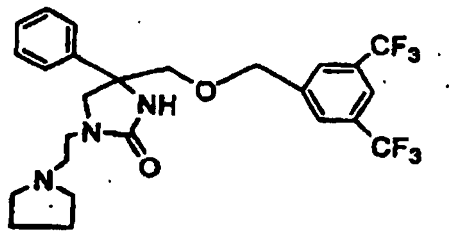

51

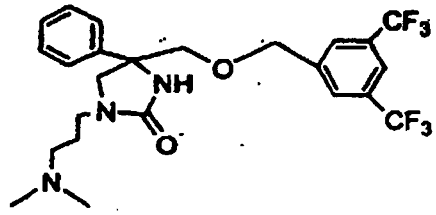

52

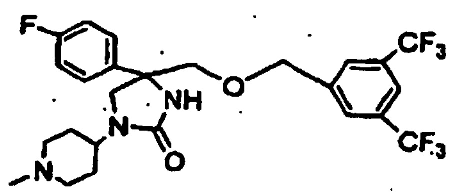

53

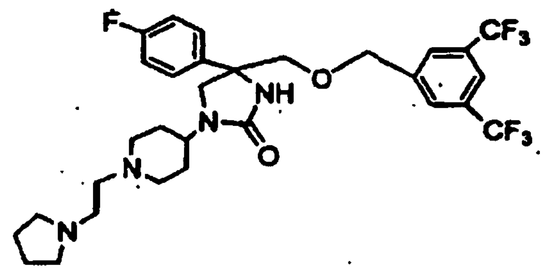

54

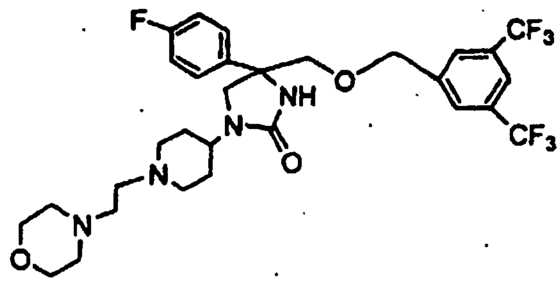

55

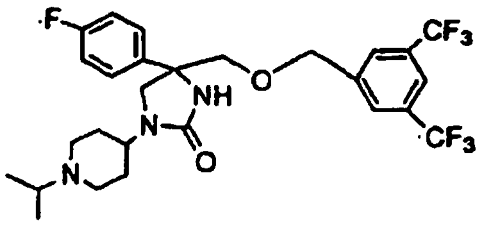

56

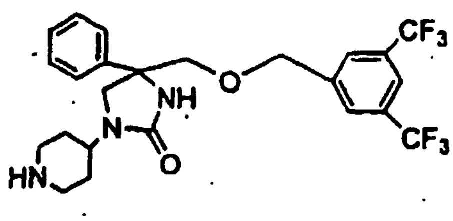

57

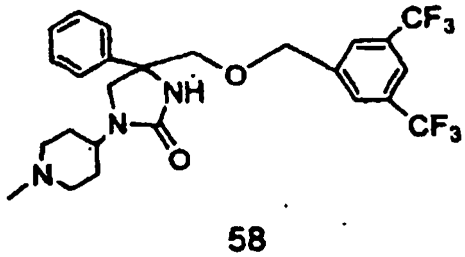

58

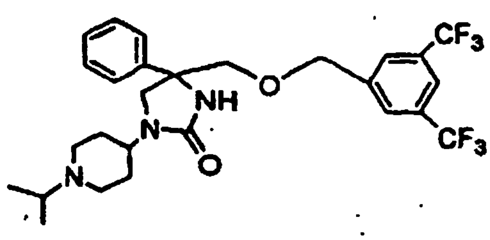

59

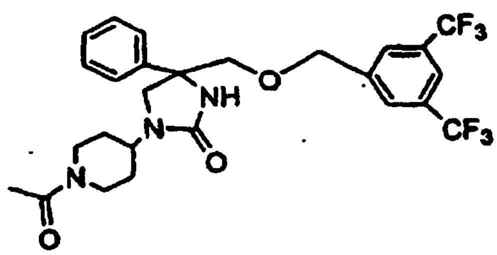

60

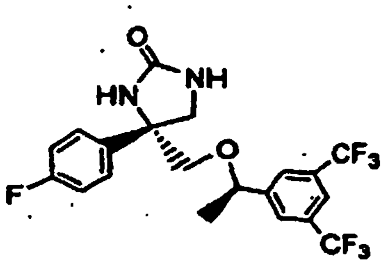

61

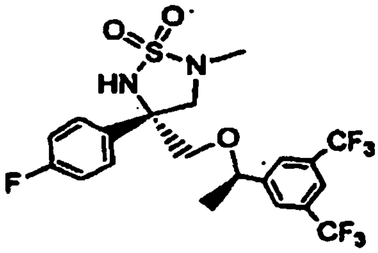

63

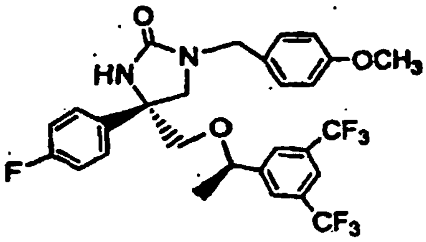

64

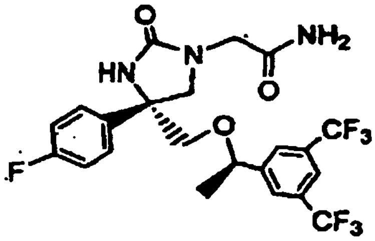

65

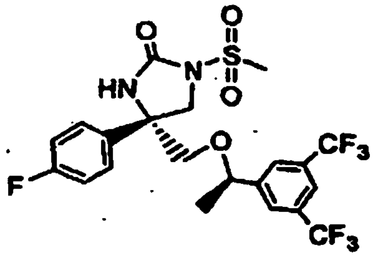

66

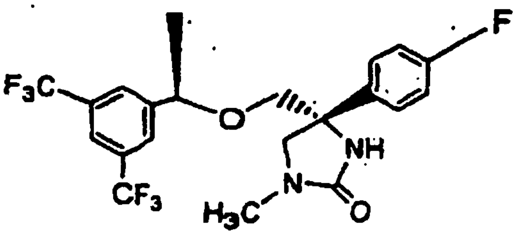

67

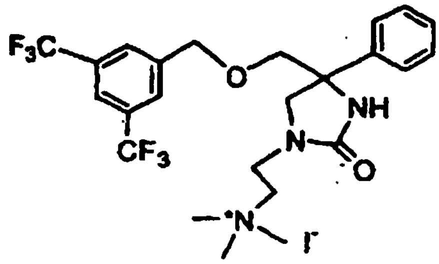

68

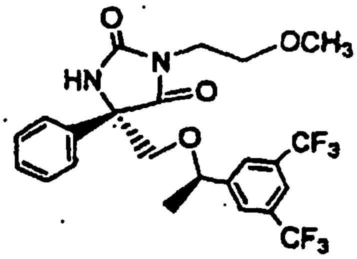

69

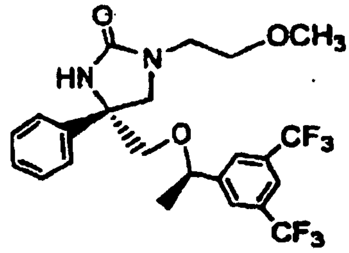

70a

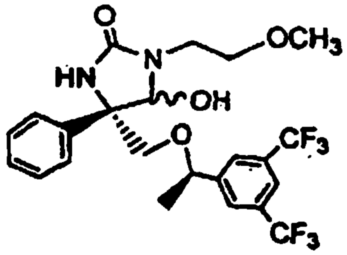

70b

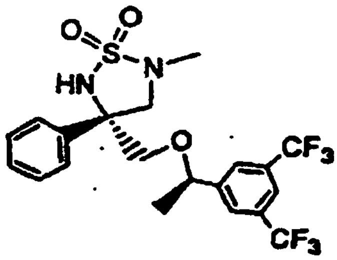

72

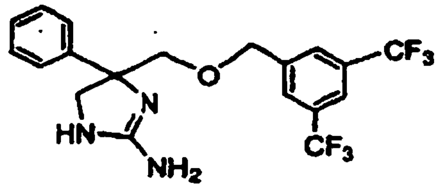

73

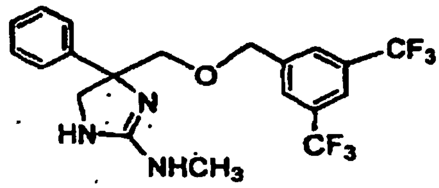

74

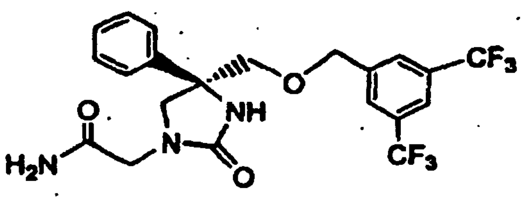

75

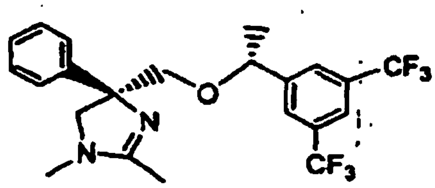

76

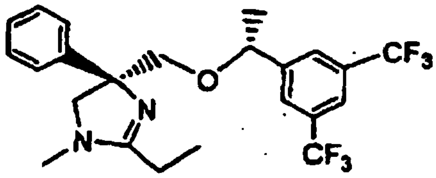

77

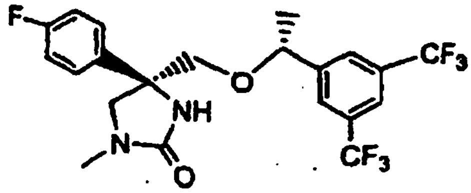

78

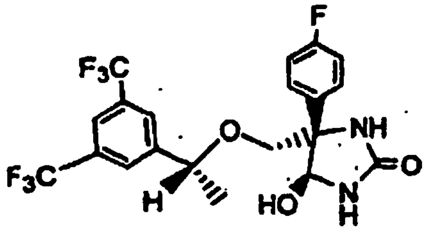

79a

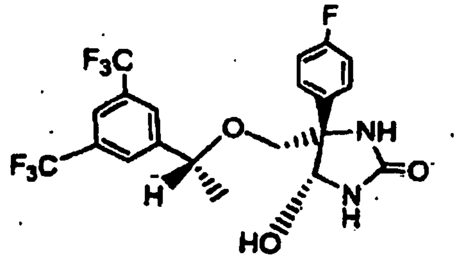

79b

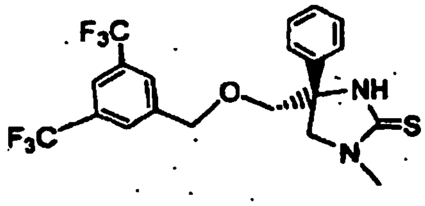

80

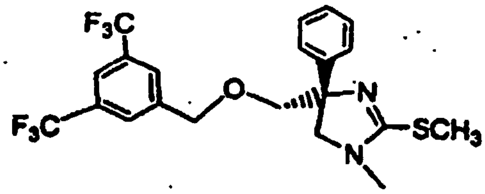

81

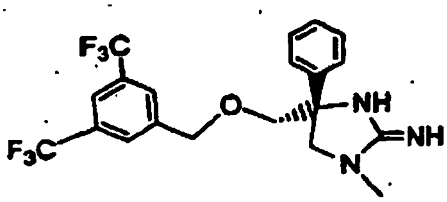

82

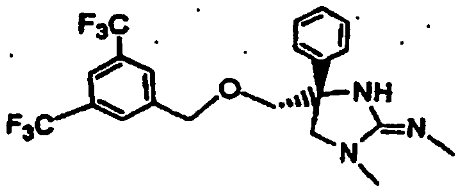

83

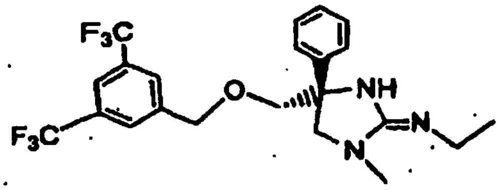

84

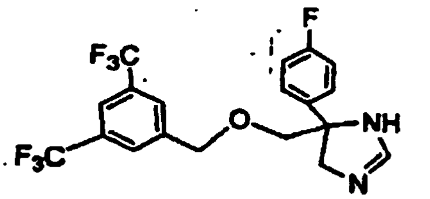

85

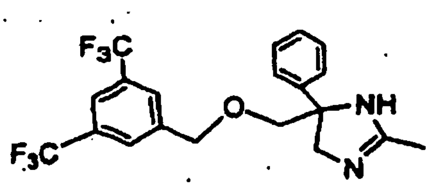

86

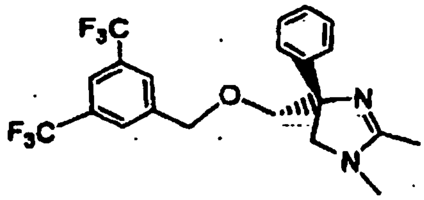

87

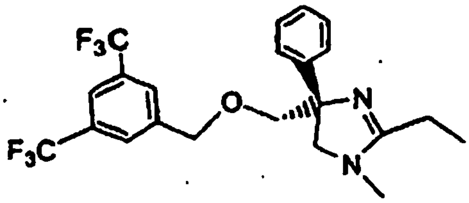

88

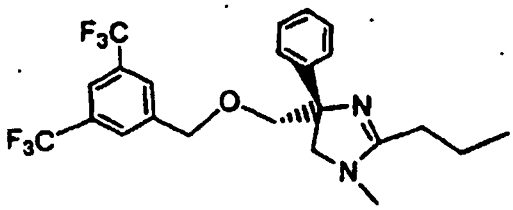

89

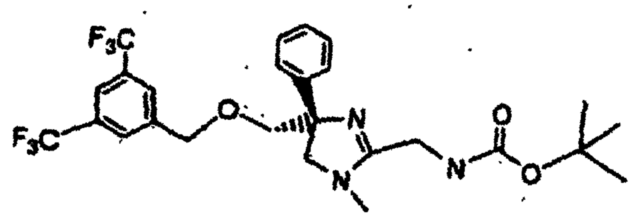

90

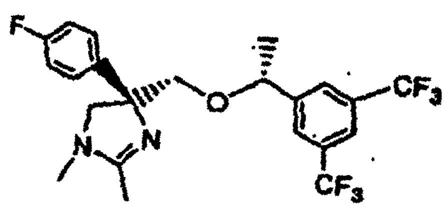

91

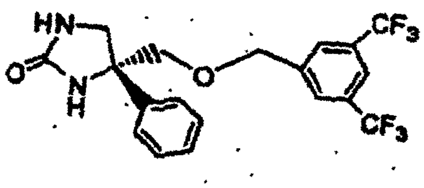

92

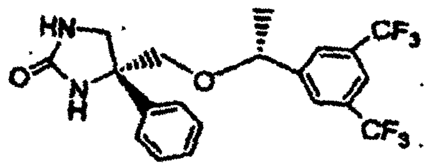

93

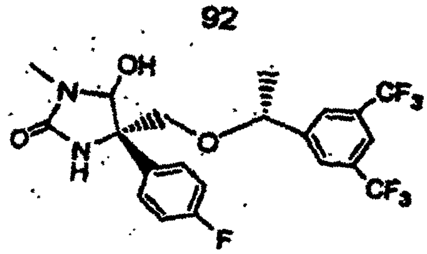

94

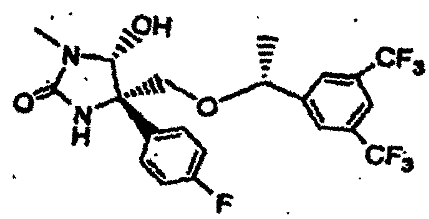

95

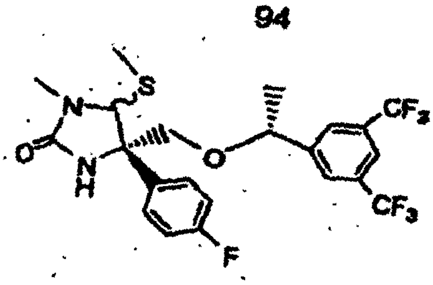

96

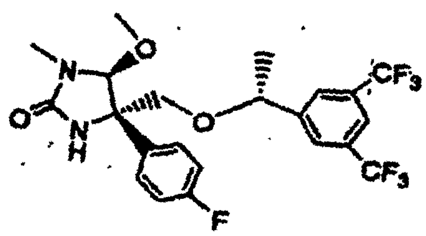

97

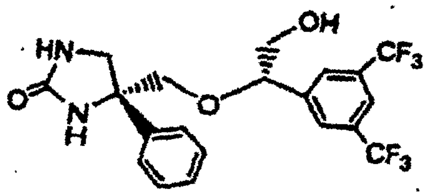

98

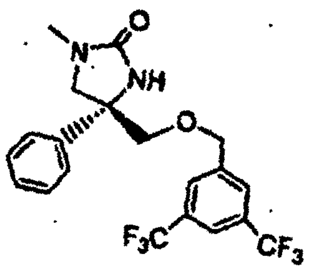

99

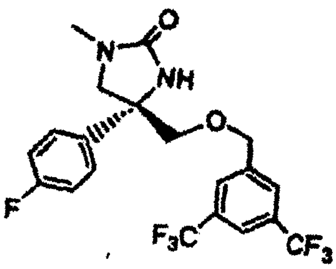

100

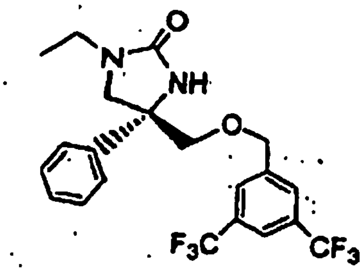

101

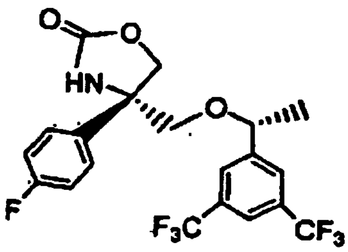

102

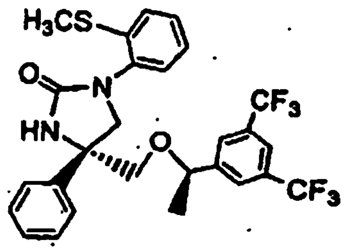

103

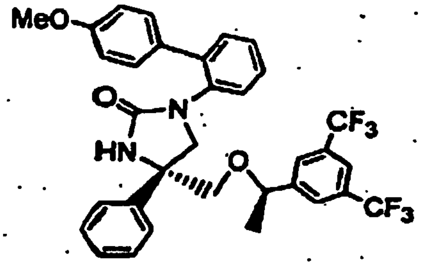

104

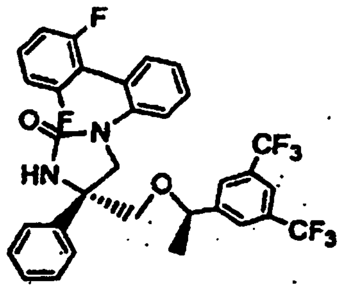

105

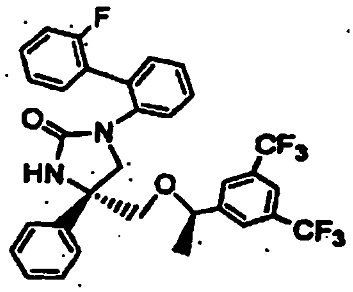

106

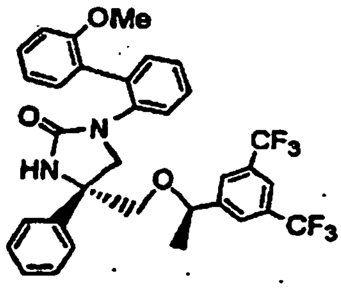

107

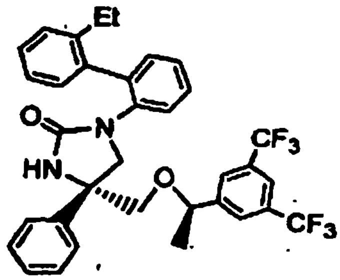

108

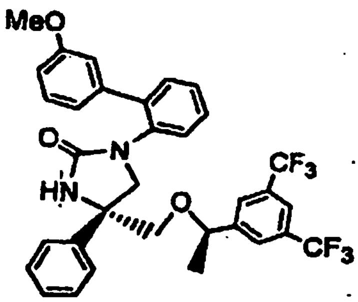

109

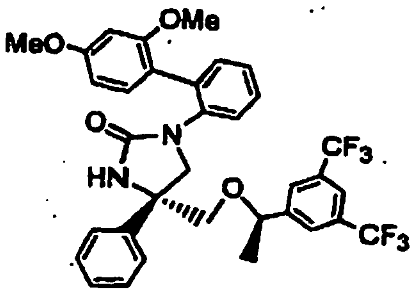

110

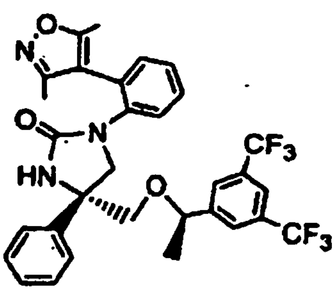

111

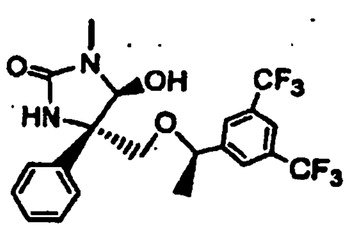

112

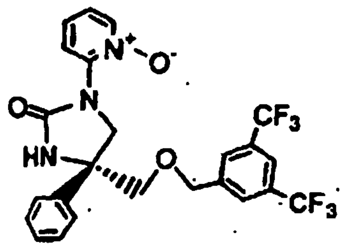

125

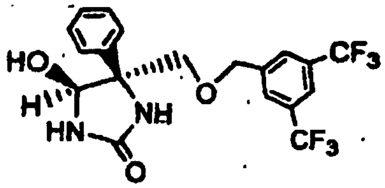

126

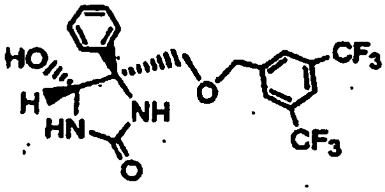

127

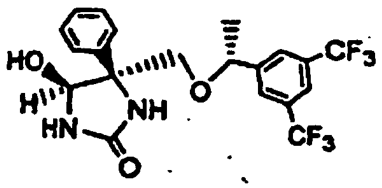

128

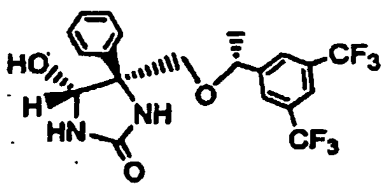

129

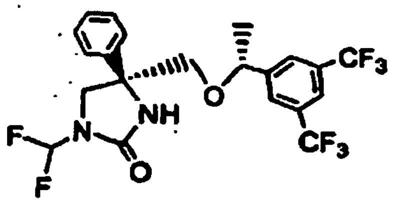

130

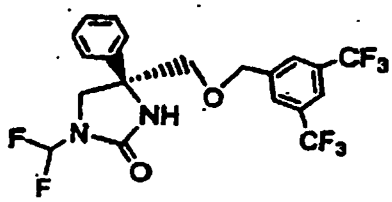

131

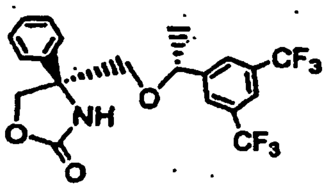

132

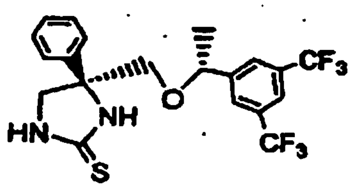

133

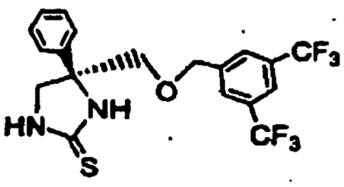

134

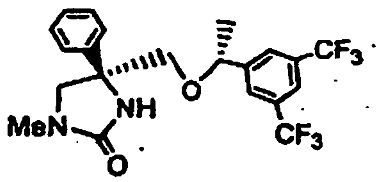

135

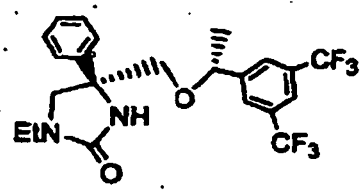

136

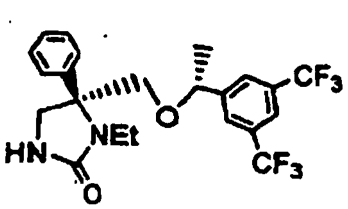

137

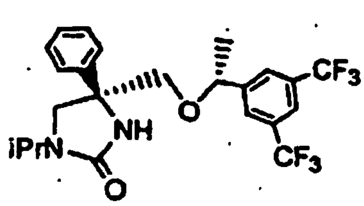

138

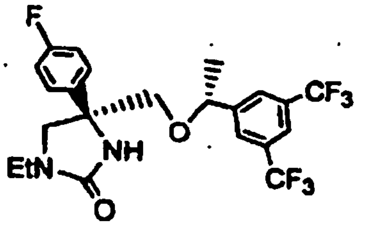

139

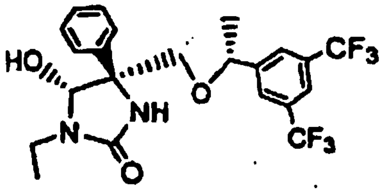

140

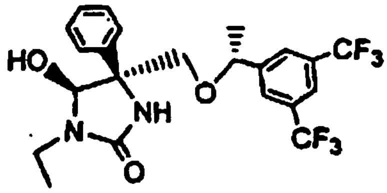

141

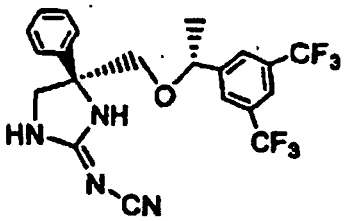

142

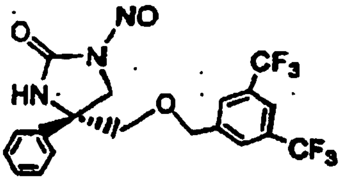

143

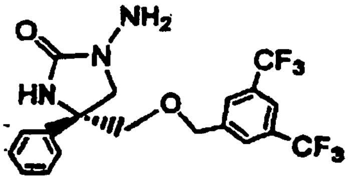

144

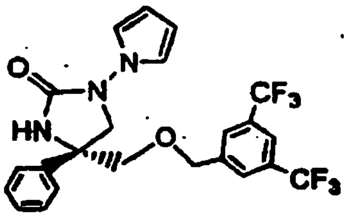

145

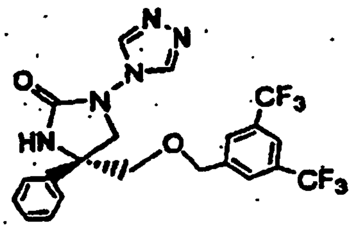

146

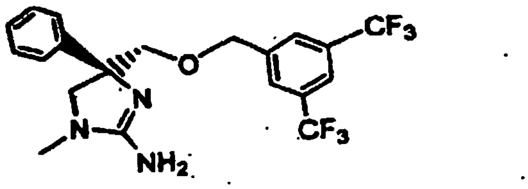

147

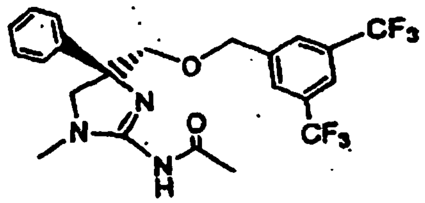

148

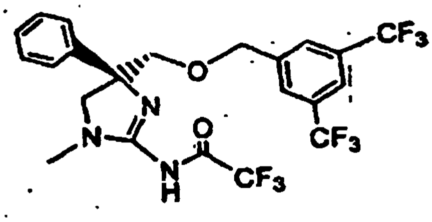

149

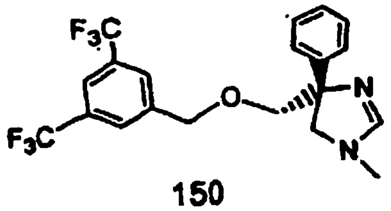

150

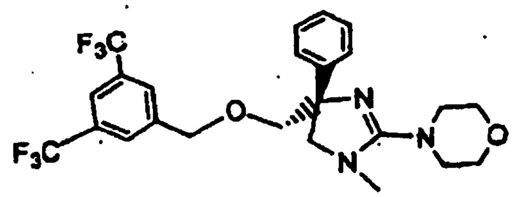

151

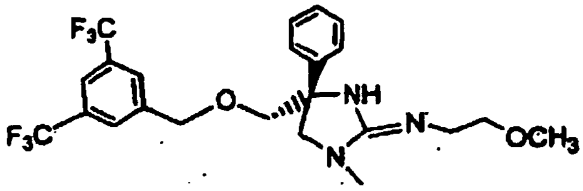

152

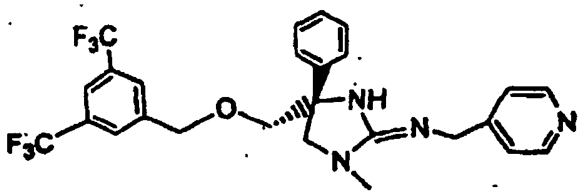

153

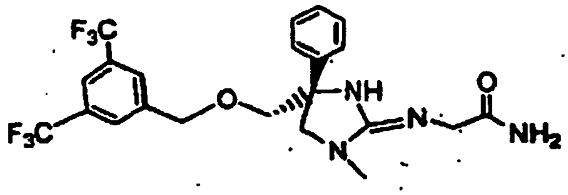

154

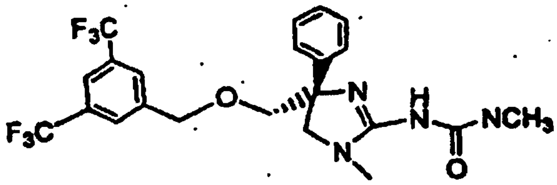

155

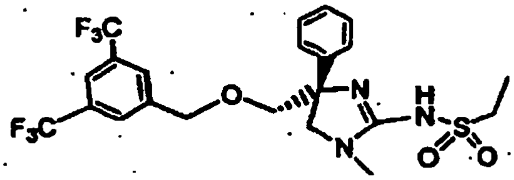

156

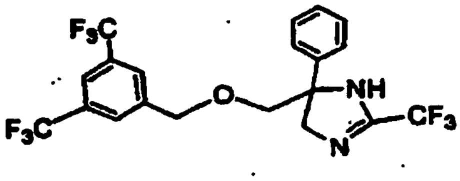

157

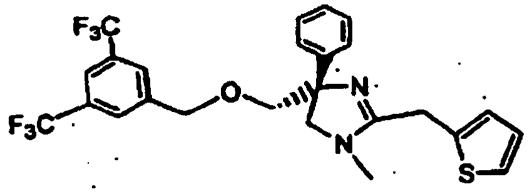

158

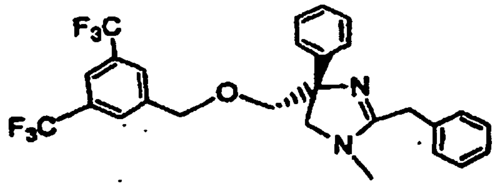

159

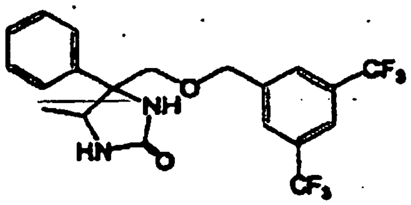

160

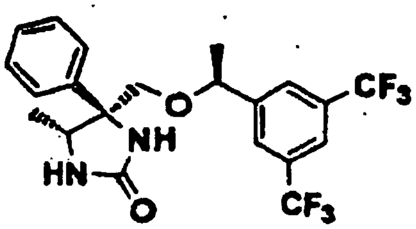

Exemple 161a

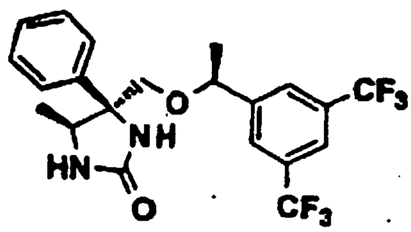

Exemple 161b

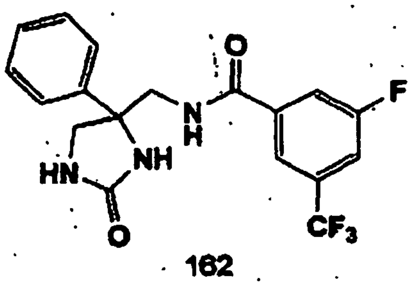

162

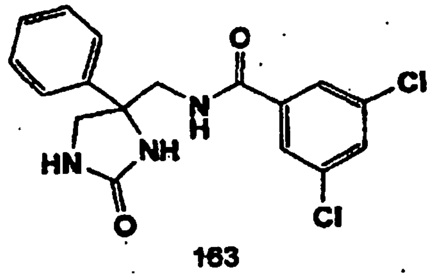

163

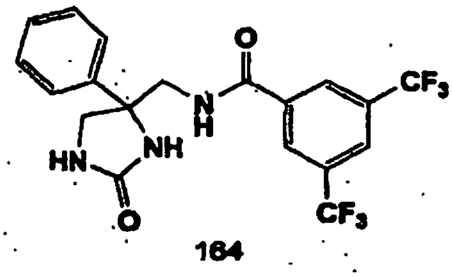

164

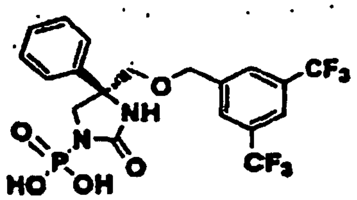

2 N-méthyl-glucamine

Exemple 165a

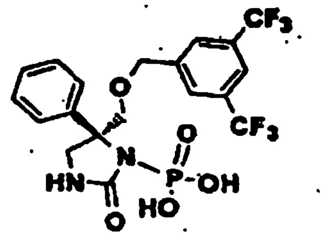

2 N-méthyl-glucamine

Exemple 165b

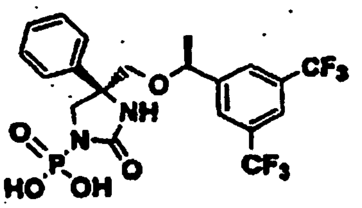

2 N-méthyl-glucamine

Exemple 166a

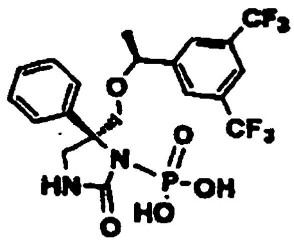

2 N-méthyl-glucamine

Exemple 166b

**13.** Composé conforme à la revendication 12, choisi parmi les composés numérotés 2, 17, 19, 31, 59, 61, 65, 67, 76, 77, 78, 79a, 79b, 92, 93, 99, 100, 103, 104, 110, 111, 113, 120, 121, 126, 127, 128, 129, 130, 135, 142, 165, 165a, 165b, 166a, 166b et 118.

**14.** Composition pharmaceutique comprenant une quantité efficace d'un composé conforme à l'une des revendications 1 à 13, et un véhicule pharmacologiquement admissible.

**15.** Composition pharmaceutique comprenant une quantité efficace d'un composé conforme à l'une des revendications 1 à 13, associée avec une quantité efficace d'un inhibiteur spécifique de la recapture de sérotonine, et un véhicule pharmacologiquement admissible.

**16.** Emploi d'un composé conforme à l'une des revendications 1 à 13 en vue de la fabrication d'un médicament destiné au traitement de maladies respiratoires, de maladies inflammatoires, de maladies de la peau, de troubles ophtalmologiques, d'accoutumances, de troubles liés au stress, de troubles obsessionnels compulsifs, de troubles de l'alimentation, de manies, du syndrome prémenstruel, de troubles du système nerveux central, de troubles gastro-intestinaux, de troubles de la vessie, de l'athérosclérose, de maladies fibrosantes, de l'obésité, du diabète de type II, de troubles liés à la souffrance, ou de troubles génito-urinaires.

**17.** Emploi d'un composé conforme à l'une des revendications 1 à 13, en association avec un inhibiteur spécifique de la recapture de sérotonine, en vue de la fabrication d'un médicament destiné au traitement de maladies respiratoires, de maladies inflammatoires, de maladies de la peau, de troubles ophtalmologiques, d'accoutumances, de troubles liés au stress, de troubles obsessionnels compulsifs, de troubles de l'alimentation, de manies, du syndrome prémenstruel, de troubles du système nerveux central, de troubles gastro-intestinaux, de troubles de la vessie, de l'athérosclérose, de maladies fibrosantes, de l'obésité, du diabète de type II, de troubles liés à la souffrance, ou de troubles génito-urinaires.

**18.** Emploi d'un composé conforme à l'une des revendications 1 à 13 en vue de la fabrication d'un médicament destiné au traitement de vomissements, de la dépression, de l'anxiété ou de la toux.

**19.** Emploi d'un composé conforme à l'une des revendications 1 à 13, en association avec un inhibiteur spécifique de la recapture de sérotonine, en vue de la fabrication d'un médicament destiné au traitement de vomissements, de la dépression, de l'anxiété ou de la toux.